# FASCICULE DE BREVET EUROPEEN

(11) **EP 3 397 768 B1**
(45) Date de publication et mention de la délivrance du brevet: **09.12.2020**
(21) Numéro de dépôt: 16831496.1
(22) Date de dépôt: 21.12.2016
(51) Int. Cl.: C12N 15/67, C12N 1/14, C12N 9/14, C12N 15/81

(54) **SOUCHES MUTANTES DETRICHODERMA REESEI**
MUTANTENSTÄMME VON TRICHODERMA REESEI
MUTANT STRAINS OFTRICHODERMA REESEI

(30) Priorité: 28.12.2015 FR 1563382
(43) Date de publication de la demande: 07.11.2018
(73) Titulaire: IFP Energies nouvelles, 92500 Rueil-Malmaison (FR)
(72) Inventeur: BIDARD-MICHELOT, Frédérique, 78620 L'Etang la Ville (FR); MARGEOT, Antoine, 75018 Paris (FR)
(74) Mandataire: Plasseraud IP
(86) Numéro de dépôt international: PCT/FR2016/053601
(87) Numéro de publication internationale: WO 2017/115033

(56) Documents cités:
- WO-A1-92/03541
- WO-A1-2008/156605
- WO-A2-2011/151515
- CN-A- 104 862 237
- FR-A1- 2 979 111
- FR-A1- 3 018 522
- FR-A1- 3 022 558
- LINDSEY N. ANDERSON ET AL: "Activity-based protein profiling of secreted cellulolytic enzyme activity dynamics in Trichoderma reesei QM6a, NG14, and RUT-C30", MOLECULAR BIOSYSTEMS, vol. 9, no. 12, 1 décembre 2013 (2013-12-01), pages 2992-3000, XP055272434, GB ISSN: 1742-206X, DOI: 10.1039/c3mb70333a
- M. G. SLATTERY ET AL: "The Function and Properties of the Azf1 Transcriptional Regulator Change with Growth Conditions in Saccharomyces cerevisiae", EUKARYOTIC CELL, vol. 5, no. 2, 1 février 2006 (2006-02-01) , pages 313-320, XP055272597, US ISSN: 1535-9778, DOI: 10.1128/EC.5.2.313-320.2006
- SHAOWEN WANG ET AL: "Enhancing cellulase production in Trichoderma reesei RUT C30 through combined manipulation of activating and repressing genes", JOURNAL OF INDUSTRIAL MICROBIOLOGY & BIOTECHNOLOGY, vol. 40, no. 6, 7 mars 2013 (2013-03-07), pages 633-641, XP055137169, ISSN: 1367-5435, DOI: 10.1007/s10295-013-1253-y
- JUNXIN LI ET AL: "Achieving efficient protein expression in Trichoderma reesei by using strong constitutive promoters", MICROBIAL CELL FACTORIES, BIOMED CENTRAL, vol. 11, no. 1, 18 juin 2012 (2012-06-18), page 84, XP021115901, ISSN: 1475-2859, DOI: 10.1186/1475-2859-11-84 cité dans la demande
- L. L. NEWCOMB ET AL: "AZF1 Is a Glucose-Dependent Positive Regulator of CLN3 Transcription in Saccharomyces cerevisiae", MOLECULAR AND CELLULAR BIOLOGY., vol. 22, no. 5, 1 mars 2002 (2002-03-01), pages 1607-1614, XP055272588, US ISSN: 0270-7306, DOI: 10.1128/MCB.22.5.1607-1614.2002

### SEQUENCE LISTING

<110> IFPEN
<120> Souches mutantes de Trichoderma reesei
<130> BFF150464
<160> 160
<170> PatentIn version 3.5
<210> 1
   <211> 1521
   <212> DNA
   <213> Artificial Sequence
<220>
   <223> Séquence nucléotidique du gène TrAZF1 de T.reesei
<400> 1
   atggccctcg cagctcaaca gcatactcag gccgattggg gccgctggtc tcaccagatt      60
   ccacagagtt ttcccatgat gggctctcca ggattcatgt catacgatcc cagagctcag     120
   gacggcagtc agatgcagcg tcaggtgtct gctcagtacc tggtgaactc gaactacaac     180
   cagcccccga tgcccactgc ttcgtctccc cagtatcaac acgcagggcc attttcctat     240
   gtgccttacc acagcccgcc gccgtccact cctcttggtt ccccattcaa gagcgaattt     300
   cccgagcacc ctcttacgcg catgacacac tctacggtcg atcgacacca ttctcaggcc     360
   atgagggact accaacctta ttctcctgta tcgaggaggg gatcgatttc ttcagtcgcc     420
   accaagccct cagcagctcc cgtcacacca ggtccaacta ctcctggctc tttcacttca     480
   agttccgacg cccagagccc cagcactcca aacccccaga ctgcgtctca gcctgtcagc     540
   tccaagactc tcacttacaa tgagaccgtt catccgggcg ataggatcag cttcagaacc     600
   gatgttgatg aactcatgaa ggccatccag aagacacaga cgaccgacga gtgtcagcaa     660
   acactcacac ctgcgcgaac accaaagaac tgtaccacaa gtactcccgt acttcgtaca     720
   caaagcggga agccgagaaa acagtgggtt tgcgatggcc ccaactgcgg caaggccttt     780
   gtccagaaga cgcatcgcga cattcaccga cgcactcaca ccggccatcg accatacgtc     840
   tgcaccatgg aaaattgcgg tcttacgttc tcgcagcgag gaaacctcaa gactcacata     900
   cgacgccaca caggtgaaaa gccgttctct tgcgctgctt gtggcaagtg cttcgctcag     960
   cgtgggaatc ttcgatccca cgaggagaca cacaaaggcc tgaagccctt cgtctgccgg    1020
   ctcgatgatt gcaacaagtc gttttctcag ctgggcaata tgaagactca tcagaacaac    1080
   tttcacaaag aaacgctcca gaaactcaca cacatgtttg tgcaattctc ggagaacggc    1140
   gaggtgccca gagactatca ggatcttttc gaatacttcc agaagcacta caagaatagc    1200
   aacaagggag tcaagggccg aggaaagact cgcgctgtgg cagctcgtgg gcctcaagat    1260
   tccgcgtttc ggcaggctgc ctccccagtg cccgcgttac tgaagacgcc ggctacgact    1320
   catttgcccc agatgacaat gccagcccat gatccccatg gcagaatctc accatacgcc    1380
   atgacccagg gagctgcgaa cactctgagc aatgtcctgc gcaaccccaa cccctcttac    1440
   ggcctttatg gacccacgtt tgccccgggc cctgtacgag atggcgtctt tcacatgggc    1500
   attgcgagcc acctatcctg a                                              1521
<210> 2
   <211> 506
   <212> PRT
   <213> Artificial Sequence
<220>
   <223> Protéine TrAZF1 de T.reesei
<400> 2
   Met Ala Leu Ala Ala Gln Gln His Thr Gln Ala Asp Trp Gly Arg Trp
   1               5                   10                  15
   Ser His Gln Ile Pro Gln Ser Phe Pro Met Met Gly Ser Pro Gly Phe
               20                  25                  30
   Met Ser Tyr Asp Pro Arg Ala Gln Asp Gly Ser Gln Met Gln Arg Gln
           35                  40                  45
   Val Ser Ala Gln Tyr Leu Val Asn Ser Asn Tyr Asn Gln Pro Pro Met
       50                  55                  60
   Pro Thr Ala Ser Ser Pro Gln Tyr Gln His Ala Gly Pro Phe Ser Tyr
   65                  70                  75                  80
   Val Pro Tyr His Ser Pro Pro Pro Ser Thr Pro Leu Gly Ser Pro Phe
                   85                  90                  95
   Lys Ser Glu Phe Pro Glu His Pro Leu Thr Arg Met Thr His Ser Thr
               100                 105                 110
   Val Asp Arg His His Ser Gln Ala Met Arg Asp Tyr Gln Pro Tyr Ser
           115                 120                 125
   Pro Val Ser Arg Arg Gly Ser Ile Ser Ser Val Ala Thr Lys Pro Ser
       130                 135                 140
   Ala Ala Pro Val Thr Pro Gly Pro Thr Thr Pro Gly Ser Phe Thr Ser
   145                 150                 155                 160
   Ser Ser Asp Ala Gln Ser Pro Ser Thr Pro Asn Pro Gln Thr Ala Ser
                   165                 170                 175
   Gln Pro Val Ser Ser Lys Thr Leu Thr Tyr Asn Glu Thr Val His Pro
   180 185 190
   Gly Asp Arg Ile Ser Phe Arg Thr Asp Val Asp Glu Leu Met Lys Ala
   195 200 205
   Ile Gln Lys Thr Gln Thr Thr Asp Glu Cys Gln Gln Thr Leu Thr Pro
   210 215 220
   Ala Arg Thr Pro Lys Asn Cys Thr Thr Ser Thr Pro Val Leu Arg Thr
   225 230 235 240
   Gln Ser Gly Lys Pro Arg Lys Gln Trp Val Cys Asp Gly Pro Asn Cys
   245 250 255
   Gly Lys Ala Phe Val Gln Lys Thr His Arg Asp Ile His Arg Arg Thr
   260 265 270
   His Thr Gly His Arg Pro Tyr Val Cys Thr Met Glu Asn Cys Gly Leu
   275 280 285
   Thr Phe Ser Gln Arg Gly Asn Leu Lys Thr His Ile Arg Arg His Thr
   290 295 300
   Gly Glu Lys Pro Phe Ser Cys Ala Ala Cys Gly Lys Cys Phe Ala Gln
   305 310 315 320
   Arg Gly Asn Leu Arg Ser His Glu Glu Thr His Lys Gly Leu Lys Pro
   325 330 335
   Phe Val Cys Arg Leu Asp Asp Cys Asn Lys Ser Phe Ser Gln Leu Gly
   340 345 350
   Asn Met Lys Thr His Gln Asn Asn Phe His Lys Glu Thr Leu Gln Lys
   355 360 365
   Leu Thr His Met Phe Val Gln Phe Ser Glu Asn Gly Glu Val Pro Arg
   370 375 380
   Asp Tyr Gln Asp Leu Phe Glu Tyr Phe Gln Lys His Tyr Lys Asn Ser
   385 390 395 400
   Asn Lys Gly Val Lys Gly Arg Gly Lys Thr Arg Ala Val Ala Ala Arg
   405 410 415
   Gly Pro Gln Asp Ser Ala Phe Arg Gln Ala Ala Ser Pro Val Pro Ala
                   420                 425                 430
   Leu Leu Lys Thr Pro Ala Thr Thr His Leu Pro Gln Met Thr Met Pro
           435                 440                 445
   Ala His Asp Pro His Gly Arg Ile Ser Pro Tyr Ala Met Thr Gln Gly
       450                 455                 460
   Ala Ala Asn Thr Leu Ser Asn Val Leu Arg Asn Pro Asn Pro Ser Tyr
   465                 470                 475                 480
   Gly Leu Tyr Gly Pro Thr Phe Ala Pro Gly Pro Val Arg Asp Gly Val
                   485                 490                 495
   Phe His Met Gly Ile Ala Ser His Leu Ser
               500                 505
<210> 3
   <211> 1699
   <212> DNA
   <213> Artificial Sequence
<220>
   <223> séquence nucléique génomique TrAZF1
<400> 3
   atggccctcg cagctcaaca gcatactcag gccgattggg gccgctggtc tcaccagatt 60
   ccacagagtt ttcccatgat gggctctcca ggattcatgt catacgatcc cagagctcag 120
   gacggcagtc agatgcagcg tcaggtgtct gctcagtacc tggtgaactc gaactacaac 180
   cagcccccga tgcccactgc ttcgtctccc cagtatcaac acgcagggcc attttcctat 240
   gtgccttacc acagcccgcc gccgtccact cctcttggtt ccccattcaa gagcgaattt 300
   cccgagcacc ctcttacgcg catgacacac tctacggtcg atcgacacca ttctcaggcc 360
   atgagggact accaacctta ttctcctgta tcgaggaggg gatcgatttc ttcagtcgcc 420
   accaagccct cagcagctcc cgtcacacca ggtccaacta ctcctggctc tttcacttca 480
   agttccgacg cccagagccc cagcactcca aacccccaga ctgcgtctca gcctgtcagc 540
   tccaagactc tcacttacaa tgagaccgtt catccgggcg ataggatcag cttcagaacc 600
   gatgttgatg aactcatgaa ggccatccag aagacacaga cgaccgacga gtgtcagcaa 660
   acactcacac ctgcgcgaac accaaagaac tgtaccacaa gtactcccgt acttcgtaca 720
   caaagcggga agccgagaaa acagtgggtt tgcgatggcc ccaactgcgg caaggccttt 780
   gtccagaaga cgcatcgcga cattcaccga cgcactcaca ccggccatcg accatacgta 840
   cgcgcccagc tcctcttcac tgcaacgccg gctaattaaa tgttgatagg tctgcaccat 900
   ggaaaattgc ggtcttacgt tctcgcagcg aggaaacctc aaggtaagct tcagctgcta 960
   agaatctcct ttgagaatgc gtatactgac cagatggtgt gtggacagac tcacatacga 1020
   cgccacacag gtgaaaagcc gttctcttgc gctgcttgtg gcaagtgctt cgctcagcgt 1080
   gggaatcttc gatcccacga ggagacacac aaaggcctga agcccttcgt ctgccggctc 1140
   gatgattgca acaagtcgtt ttctcagctg ggcaatatga aggtatgcaa catctagcac 1200
   atgaaagcag tatgagaacg ctctaacgct gagggaactg cagactcatc agaacaactt 1260
   tcacaaagaa acgctccaga aactcacaca catgtttgtg caattctcgg agaacggcga 1320
   ggtgcccaga gactatcagg atcttttcga atacttccag aagcactaca agaatagcaa 1380
   caagggagtc aagggccgag gaaagactcg cgctgtggca gctcgtgggc ctcaagattc 1440
   cgcgtttcgg caggctgcct ccccagtgcc cgcgttactg aagacgccgg ctacgactca 1500
   tttgccccag atgacaatgc cagcccatga tccccatggc agaatctcac catacgccat 1560
   gacccaggga gctgcgaaca ctctgagcaa tgtcctgcgc aaccccaacc cctcttacgg 1620
   cctttatgga cccacgtttg ccccgggccc tgtacgagat ggcgtctttc acatgggcat 1680
   tgcgagccac ctatcctga 1699
<210> 4
   <211> 1437
   <212> DNA
   <213> Artificial Sequence
<220>
   <223> promoteur gpd
<400> 4
   gacgcagaag aaggaaatcg ccccgccggt tccgtaaaaa aaatatgagc gcagggacaa      60
   gcacagcctt ggccctgggc ctagccttgc gccttgtttg atgcaatcgg cgacatgtcg     120
   aatgctgtaa ttttttttgt ttaggttccc cttttccttt tgtgttaata ataattctcg     180
   aagggcgctg attttgaaat ttgtcggtga gagccaaacg gatatacagg cgcggctgat     240
   gaataatgat gaatcgagct gacttgatgc tgtatgtaca atattgactg cgaggaccat     300
   caggtgttgt atggatggaa tcattctgta accaccaagg tgcatgcatc ataaggtatt     360
   ctcctcagct caccaacaac gaacgatggc catgttagta aaggcaccgt gatggcaaga     420
   tagaaccact attgcatctg cgcttcccac gcacagtacg tcaatgtaac gtcaaagccg     480
   ccctcccgta acctcgcccg ttgttgctcc ccccgattgc ctcaatcaca tagtacctac     540
   ctatgcatta tggcgcctca acccaccccc ccagattgag agctacctta catcaatatg     600
   gccagcacct cttcggcgat acatactcgc caccccagcc ggggcgattg tgtgtactag     660
   gtaggctcgt actataccag caggagaggt gctgcttggc aatcgtgctc agctgttagg     720
   ttgtacttgt atggtacttg taaggtggtc atgcagttgc taaggtacct agggagggat     780
   tcaacgagcc ctgcttccaa tgtccatctg gataggatgg cggctggcgg ggccgaagct     840
   gggaactcgc caacagtcat atgtaatagc tcaagttgat gataccgttt tgccaggatt     900
   aggatgcgag aagcagcatg aatgtcgctc atccgatgcc gcatcaccgt tgtgtcagaa     960
   acgaccaagc taagcaacta aggtacctta ccgtccacta tctcaggtaa ccaggtacta    1020
   ccagctaccc tacctgccgt gcctacctgc tttagtatta atctttccac ctccctcctc    1080
   aatcttcttt tccctcctct cctctttttt ttttcttcct cctcttcttc tccataacca    1140
   ttcctaacaa catcgacatt ctctcctaat caccagcctc gcaaatcctc aggttagtat    1200
   tactactact acaatcatca ccacgatgct ccgcccgacg atgcggcttc tgttcgcctg    1260
   cccctcctct cactcgtgcc cttgacgagc taccccgcca gactctcctg cgtcaccaat    1320
   ttttttccct atttacccct cctccctctc tccctctcgt ttcttcctaa caaacaacca    1380
   ccaccaaaat ctctttggaa gctcacgact cacgcaagct caattcgcag atacaaa       1437
<210> 5
   <211> 645
   <212> DNA
   <213> Artificial Sequence
<220>
   <223> terminateur gpd
<400> 5
   tctagaaaga aggattacct ctaaacaagt gtacctgtgc attctgggta aacgactcat      60
   aggagagttg taaaaaagtt tcggccggcg tattgggtgt tacggagcat tcactaggca     120
   accatgcatc cttactattg tataccatct tagtaggaat gatttcgagg tttataccta     180
   cgatgaatgt gtgtcctgta ggcttgagag ttcaaggaag aaacatgcaa ttatctttgc     240
   gaacccaggg ctggtgacgg aattttcata gtcaagctat cagagtaaag aagaggagca     300
   tgtcaaagta caattagaga caaatatata gtcgcgtgga gccaagagcg gattcctcag     360
   tctcgtaggt ctcttgacga ccgttgatct gcttgatctc gtctcccgaa aatgaaaata     420
   gctctgctaa gctattcttc tcttcgccgg agcctgaagg cgttactagg ttgcagtcaa     480
   tgcattaatg cattgcagat gagctgtatc tggaagaggt aaacccgaaa acgcgtttta     540
   ttcttgttga catggagcta ttaaatcact agaaggcact ctttgctgct tggacaaatg     600
   aacgtatctt atcgagatcc tgaacaccat ttgtctcaac tccgg                     645
<210> 6
   <211> 475
   <212> PRT
   <213> Artificial Sequence
<220>
   <223> blaslBnnG_09418T0
<400> 6
   Met Glu Thr Ala Val Pro Val Ala Tyr Asn Val Thr Asn His Asp Met
   1               5                   10                  15
   Asn Ala Val Ala His Arg Arg Met Met Ala Pro His Gln Gln His His
               20                  25                  30
   His His His His His His Asp Gln Asn Ile Ala Tyr Phe Ser Asn Pro
           35                  40                  45
   Pro Ile Pro Tyr Ser Thr Pro Leu Gln Pro Pro Ser Phe Gly Gly Phe
       50                  55                  60
   Gly His Ile Leu Asn Asn His Pro His His Thr Ser Tyr Gln Gly Tyr
   65                  70                  75                  80
   Phe Asn Ser Asn Pro Asn Pro Asn Pro Gln Val Asn Ser His His His
                   85                  90                  95
   Arg Leu Ala Thr Glu Thr Asn Pro Val His Gln Val Ser Asp Ile Arg
               100                 105                 110
   His Val Lys Asn Pro Asn Gln Arg Leu Ile Arg Pro Ser Pro Pro Lys
           115                 120                 125
   Glu Asp Thr Leu Pro Pro Ala Pro Ala Pro Ala Pro Ala Val Ala Pro
       130                 135                 140
   Pro Pro Pro Pro Arg Leu Pro Pro Gln Arg Val Ser Val Ala Pro Val
   145                 150                 155                 160
   Arg Asn Thr Thr Gln His Glu Asp Ser Lys Pro Met Pro Lys Pro Gly
                   165                 170                 175
   Val Glu Phe Gly Thr Glu Val Asp Thr Leu Met Lys Ala Ile Gln Ser
               180                 185                 190
   Lys Pro Gln Pro Ala Ser Pro Gln Val Glu His Gln Leu Pro Pro Leu
           195                 200                 205
   His Gln Lys Phe Asn Asn Gly Val Ala Asn Trp Ile His Pro Ala Tyr
       210                 215                 220
   Ala Asn Gln Met Ala Gly Asn Gln Ala Ile Phe Pro Asn Ala Pro Gln
   225                 230                 235                 240
   Asp Arg Val Pro Pro Pro Asn Gln Lys Ala Lys Arg Lys Tyr Glu Cys
                   245                 250                 255
   Thr Leu Pro His Cys Arg Lys Ser Phe Phe Gln Lys Thr His Leu Asp
               260                 265                 270
   Ile His Met Arg Ala His Thr Gly Asp Lys Pro Phe Thr Cys Lys Glu
           275                 280                 285
   Pro Ser Cys Gly Gln Arg Phe Ser Gln Leu Gly Asn Leu Lys Thr His
       290                 295                 300
   Glu Arg Arg His Thr Gly Glu Lys Pro Tyr Ser Cys Glu Ile Cys His
   305                 310                 315                 320
   Lys Lys Phe Ala Gln Arg Gly Asn Val Arg Ala His Lys Ile Thr His
                   325                 330                 335
   Glu Gln Ala Lys Pro Phe Lys Cys Gln Leu Asp Asp Cys Gly Lys Gln
               340                 345                 350
   Phe Thr Gln Leu Gly Asn Leu Lys Ser His Gln Asn Lys Phe His Ala
           355                 360                 365
   Gln Thr Leu Arg Asn Leu Thr Leu Arg Phe Ala Ser Ile Gly Asp Ile
       370                 375                 380
   Asp Arg Met Ser Pro Gln Asp Lys Glu Leu Trp Ser Tyr Phe Ser Thr
   385                 390                 395                 400
   Leu Tyr Arg Asn Ser Asn Lys Gly Ile Lys Gly Arg Gly Lys Asp Arg
                   405                 410                 415
   Arg Val Ser Thr Ala Lys Thr Ser Thr Ser Ala Tyr Asp Gly Ser His
               420                 425                 430
   Ser Glu Ser Glu Gly Glu Ala Lys Gly Arg Ser Arg Ser Tyr Asp Arg
           435                 440                 445
   Ala Ser Ala Val Met Thr Ser Ser Ser Asp Glu Pro Asp Tyr Arg Glu
       450                 455                 460
   Gln Leu Tyr His His Arg Asn Gly Ala His His
   465                 470                 475
<210> 7
   <211> 389
   <212> PRT
   <213> Artificial Sequence
<220>
   <223> artolgi345568033gbEGX50934.1
<400> 7
   Met Asp Ile Val Asn Ile Leu Asn Lys Lys Glu Ala Ser Ala Ser Ser
   1               5                   10                  15
   Asp Asn Ala Val Ser Pro Thr Ser Arg Gln Pro Glu Thr Gly Gln Ala
               20                  25                  30
   Arg Phe Glu Lys Gln Asp Ala Val Arg Leu Pro Ser Met Ala Pro Asn
           35                  40                  45
   Ser Phe Ser Thr Tyr Pro His Ala Thr Phe Ala Pro Arg Tyr Ser Tyr
       50                  55                  60
   Glu Thr Gln His Ser Pro Pro Pro Ala Ala Ser Pro Ser Asn Pro Phe
   65                  70                  75                  80
   Ala Thr Ser Pro Gly Pro Ser Ser Glu Gly Pro His Ser Asn Asn Gly
                   85                  90                  95
   Asn Asp Asp Pro Thr Lys Pro Tyr His Cys Lys Thr Cys Ser Lys Gly
               100                 105                 110
   Phe Ala Arg Arg Ser Asp Leu Ser Arg His Glu Arg Ile His Ser Gly
           115                 120                 125
   Ile Arg Pro His Ile Cys Asp Phe Glu Gly Cys Gly Lys Gln Phe Ile
       130                 135                 140
   Gln Arg Ser Ala Leu Thr Val His Ala Arg Val His Thr Gly Glu Lys
   145                 150                 155                 160
   Pro His Met Cys Glu Ala Cys Gly Lys Pro Phe Ser Asp Ser Ser Ser
                   165                 170                 175
   Leu Ala Arg His Arg Arg Ile His Ser Gly Lys Arg Pro Tyr Lys Cys
               180                 185                 190
   Pro Tyr Ala Asp Cys Gln Lys Thr Phe Thr Arg Arg Thr Thr Leu Thr
           195                 200                 205
   Arg His Gln Asn His His Thr Gly Thr Ile Ser Glu Ala Ala Ala Ala
       210                 215                 220
   Thr Ala Ala Ala Leu Ala Ser Arg Arg Ser Ser Ser Lys Ser Ser Val
   225                 230                 235                 240
   Gln Ser Leu Ser Leu Thr Gly Asn Ser Pro Arg Glu Asp Asn Met Glu
                   245                 250                 255
   Ser Val Ser Gln Ser Arg Thr Gly Ser Asn Ser Ser Leu Arg Leu Ser
               260                 265                 270
   Gln Gly Ser Ser Pro Ala Ala Asp Leu Asp Lys Gln Val Ile Leu Ala
           275                 280                 285
   Trp Arg Glu Ser Pro Pro Tyr Gln Ala Val Thr Ser Leu Pro Thr Pro
       290                 295                 300
   Val Ser Thr Pro Thr Ile Pro Ala Asn Leu Val Thr Gly Thr Ala Ala
   305                 310                 315                 320
   Leu Gln Ile Ser Asp Ser Arg Gln Asn Leu Arg Gly Leu Pro Pro Leu
                   325                 330                 335
   Gly Ser Gly Ser Gln Pro Arg Ser Leu Ser Pro Leu His Arg Ser Pro
               340                 345                 350
   Ser Ser Gly Ile Ser His His Ile Trp Gln His His Thr His Asn Gly
           355                 360                 365
   Ser Pro Thr Ser Leu Leu Pro Ile His Thr Ala Ile Ser Leu Ser Pro
       370                 375                 380
   Gln Pro Ala Tyr Arg
   385
<210> 8
   <211> 482
   <212> PRT
   <213> Artificial Sequence
<220>
   <223> clagrfgenesh1_kg.4_#_138_#_isotig07432
<400> 8
   Met Ala Ala Arg Pro Phe Gln Cys Glu Trp Pro Glu Gly Cys Ser Lys
   1               5                   10                  15
   Ser Phe Asn Arg Lys Ser Asp Leu Gln Arg His Tyr Arg Ile His Thr
               20                  25                  30
   Asn Glu Arg Pro Tyr Ala Cys Asp Tyr Lys Pro Cys Thr Lys Thr Phe
           35                  40                  45
   Ile Gln Arg Ser Ala Leu Thr Val His Arg Arg Thr His Thr Gly Asp
       50                  55                  60
   Lys Pro His Lys Cys Pro Phe Leu Gly Cys Gly Lys Cys Phe Ser Asp
   65                  70                  75                  80
   Ser Ser Ser Leu Ala Arg His Arg Arg Ile His Thr Asn Asp Lys Pro
                   85                  90                  95
   Tyr Ile Cys Arg Asn Gln Arg Cys Lys Lys Ala Phe Cys Arg Lys Thr
               100                 105                 110
   Thr Met Thr Lys His Ile Arg Lys Glu His Pro Ala Glu Pro Ile Gln
           115                 120                 125
   Glu Asp Gln Asp Ala Glu Tyr Ser Asp Val Asp Pro Ser Asp Asp Glu
       130                 135                 140
   Val Leu Glu Asp Asp Ile Asp Glu Ile Lys Glu Glu Ala Gly Ser Pro
   145                 150                 155                 160
   Tyr Gln Glu Asp Pro Lys Asp Ser Arg Leu Thr Arg Pro Leu Ser Asn
                   165                 170                 175
   Tyr His Ala Asn Leu Trp Pro Leu Pro Ala Gln Thr Ala Gln Arg Pro
               180                 185                 190
   Thr Leu His Arg Ser Glu Ser Ser Ser His Asp Ile Lys Leu Glu Arg
           195                 200                 205
   Thr Ser Ser Gly Thr Pro Gln Arg Ser Leu Thr Asp Pro Tyr Pro Asp
       210                 215                 220
   Ser Ser Ile His Ser Ala Gly Leu Asn Gly Val Thr Ile His Ser Val
   225                 230                 235                 240
   Met Pro His Gly Leu Asn Thr Ser Asn Met Pro Gln Gln Phe Gln Tyr
                   245                 250                 255
   Arg Ser His Asp Asn Asn Val Gly Leu Trp Ser Pro Gly His Asp Ser
               260                 265                 270
   Pro Thr Ser Ile Thr Asn Ser Ser Pro Ser Ser Val Ser Ala Gln Thr
           275                 280                 285
   His Pro Ile Tyr Thr Ser Gln Pro Tyr Gln Leu Gln Pro Asn Ser Leu
   290 295 300
   Pro Thr His Ala Gln Ile Gln Tyr Ser His Asp Gly Met Val Ser Asn
   305 310 315 320
   Ile Gln Gln Pro Met Asn Asp Leu Ala Val His Asp Ile His Leu Asp
   325 330 335
   Gln Pro Gln Gln His Gln Tyr His Asp Met Ala Ser Thr Pro Ile Gln
   340 345 350
   Gln Gln Gln Gln Tyr Glu Gly Val Pro Gln Gln Val Ser Pro Gln Asp
   355 360 365
   His Tyr Ile Glu Met Ser Arg Asp Pro Pro Gln His Pro Ala Tyr Ala
   370 375 380
   Glu Gly Pro Pro Gln Ala Ala Val His His Phe Gln Ser Glu Val Pro
   385 390 395 400
   Pro Thr Pro Ala Thr Asn Arg Asp Leu Ser Arg Tyr Ala Thr Ser Ile
   405 410 415
   Pro Gln Glu Gln Thr Tyr Gln Gln Pro Gln Leu Leu Pro Ile Glu Glu
   420 425 430
   Thr Phe Ser Ile Gly His Gln Tyr Phe Leu Pro Asn His Gly Ser Tyr
   435 440 445
   Gln Ile Asn Gln Pro Ser Tyr Phe Phe Asp Glu Leu Lys Asp Lys Pro
   450 455 460
   Asp Thr Met Trp Ala Gly Pro Leu Pro Asp Gln Arg Leu Lys Asp Glu
   465 470 475 480
   Trp Pro
<210> 9
   <211> 397
   <212> PRT
   <213> Artificial Sequence
<220>
   <223> dotseestExt_fgenesh1_kg.C_6_t10037
<400> 9
   Met Ala Gly Val Met Ser Ser Phe Asn Met Ala Phe Gln Pro Asp Thr
   1 5 10 15
   Ser Ala Ile Leu Pro Tyr Met Gln Met Gln Glu Gln Glu Arg His Thr
   20 25 30
   Met His Ala Pro Ser Ile Ile Pro Ser Asp Leu Met Asn Thr Gln Ile
   35 40 45
   Asn Ala Asp Leu Ser Val Gly Leu Gly Met Asp Ala Ser Phe Ala Asn
   50 55 60
   Ala His Ala Met Asn Val Asp Tyr Asn Asn Ala Thr Phe Gly Asn Leu
   65 70 75 80
   Ala Ala Trp Pro Ala Pro Ile Asn Thr Asn Phe Asn Ala Met Pro Arg
   85 90 95
   Pro Val Met Met Gln Ala Glu Ser Tyr Ser Ser Thr Asp Ser Ser Glu
   100 105 110
   Pro Tyr Ile Lys Thr Glu Asp Asp Ser Pro Val His Ala Ser Gln Val
   115 120 125
   Phe Tyr Asn Ala Asn Ala Tyr Ala Ser Ser Pro Glu Ser Gln Thr Gly
   130 135 140
   Thr Asp Glu Pro Glu Glu Thr Lys Pro Thr Val Phe Ala Thr Asp Ile
   145 150 155 160
   Asp Thr Leu Met Arg Ala Ile Gln Thr Lys Thr Gly Ser Ala Ala Glu
   165 170 175
   Gln Arg Gln Lys Asp Gln Thr Glu Val Thr Ile Thr Glu Ile Ser Pro
   180 185 190
   Val Lys Pro Lys Lys Arg Tyr Glu Cys Asn Val Pro Asp Cys Gly Lys
   195 200 205
   Ala Phe Tyr Gln Lys Thr His Leu Glu Ile His Thr Arg Ala His Thr
   210 215 220
   Gly Ile Lys Pro Phe Leu Cys Arg Glu Pro Ser Cys Gly Gln Arg Phe
   225 230 235 240
   Ser Gln Leu Gly Asn Leu Lys Thr His Glu Arg Arg His Thr Gly Glu
   245 250 255
   Arg Pro Tyr His Cys Asp Ile Cys Gly Lys Thr Phe Ala Gln His Gly
                   260                 265                 270
   Asn Val Arg Ala His Lys Ile Val His Thr Ala Ala Lys Pro Phe Thr
           275                 280                 285
   Cys Lys Leu Asp Ser Cys Asn Lys Gln Phe Thr Gln Leu Gly Asn Leu
       290                 295                 300
   Lys Ser His Gln Asn Lys Phe His Ile Glu Thr Ile Arg Arg Leu Lys
   305                 310                 315                 320
   Ser Arg Phe Glu Ser Phe Arg Glu Gly Asp Val Val Asp Thr Trp Glu
                   325                 330                 335
   Lys Glu Met Trp Glu Tyr Phe Ala Ser Leu Tyr Lys Asn Cys Asn Lys
               340                 345                 350
   Gly Ile Lys Gly Arg Gly Lys Asp Arg Arg Ile Ser Asn Thr Ala Ala
           355                 360                 365
   Ala Gln Arg Arg Asn Ser Ile Val Ser Ser Val Gly Ser Ser Ser Gly
       370                 375                 380
   Ser Met Ser Ala Gly His Ser Val Pro Gln Arg Thr Tyr
   385                 390                 395
<210> 10
   <211> 434
   <212> PRT
   <213> Artificial Sequence
<220>
   <223> geodeGMDG_07394T0
<400> 10
   Met Glu Leu Met Glu Leu Val Glu Asn Glu Pro Thr Val Arg Pro Phe
   1               5                   10                  15
   Gln Cys Glu Trp Pro Ser Cys Gln Lys Asn Phe Asn Arg Lys Ser Asp
               20                  25                  30
   Leu Gln Arg His Phe Arg Ile His Thr Asn Glu Arg Pro Tyr Ser Cys
           35                  40                  45
   Leu Thr Pro Gly Cys Asn Lys Ser Phe Ile Gln Arg Ser Ala Leu Thr
       50                  55                  60
   Val His Ile Arg Thr His Thr Gly Glu Lys Pro His Gln Cys Gln Gln
   65                  70                  75                  80
   Leu Glu Cys Gly Lys Arg Phe Ser Asp Ser Ser Ser Leu Ala Arg His
   85 90 95
   Arg Arg Ile His Thr Gly Lys Arg Pro Tyr Lys Cys Ala His Glu Gly
   100 105 110
   Cys Leu Lys Ser Phe Cys Arg Lys Thr Thr Met Val Lys His Gln Arg
   115 120 125
   Arg Ser His Gln Arg Gly Gly Gly Val Asn Ser Ser Glu Leu Glu Asp
   130 135 140
   Gly Asp Thr Ser Asp Ser Asp Met Gly Glu Ser Pro Ser Thr Pro Gln
   145 150 155 160
   Leu Val Met Gln Ala His Trp Pro Gln Asp Leu Asn Thr Val Ile Thr
   165 170 175
   His Ser Val Met Gln Ser His His Gln Ala Thr His Arg Ser Gln Ser
   180 185 190
   Phe Ala Asp Phe Gly His Tyr His Asn Ala Asn Ser Gln Pro Tyr Val
   195 200 205
   Thr Thr Asp Val Tyr Pro Pro Arg His Ser Leu Ser Glu Ser Pro Gln
   210 215 220
   His Tyr Gln His Arg Ser Leu Ser Leu Gln Asp Gln Arg His Pro Gln
   225 230 235 240
   Asp Leu Ile Gln Gln Gln Gln Arg Gln His Gln His Pro Tyr Phe Ile
   245 250 255
   Pro Glu Gln Asn Asn Pro Gly Val Ala Thr Met Asn Thr Asn Pro Asn
   260 265 270
   Ser His Ile Gln Thr Tyr Ile Ala Arg Gln Ile Pro Asp Leu Gln Ile
   275 280 285
   Pro Asp Leu Gln Ile Pro Tyr Thr Thr Gln Pro Leu Pro Ala Ser Val
   290 295 300
   Tyr Ala Gly Ser Asp Thr Tyr Ser Ala Leu Ser Ala Arg Thr Pro Pro
   305 310 315 320
   Pro Pro Glu Leu Tyr Tyr Ala His Gln Gln Pro Ile Gln Lys Phe Glu
   325 330 335
   Tyr Pro Ala His Gln Pro Ser Pro Ile Asp Gln Gln Gln Gln His Gln
   340 345 350
   Gln Val Pro Val Val Gln Tyr Gln Arg Leu Gln Val Gln Gln Val Val
   355 360 365
   Pro Thr Pro Gln Gln Gln Gln Gln Tyr Gln Thr Pro Gln Glu Gln Trp
   370 375 380
   Tyr Ala Gln Ala Pro Phe Gln Glu Pro Val Glu Leu Ala Ser Ala Ile
   385 390 395 400
   Asn Ser Tyr Thr Ser Ala Gly Leu Asn Asp Pro Trp Gln Leu Lys Met
   405 410 415
   Glu Ala Phe Glu Asp Pro Ser Met Gln Leu Pro Ser Ala Arg Cys Glu
   420 425 430
   Asn Leu
<210> 11
   <211> 471
   <212> PRT
   <213> Artificial Sequence
<220>
   <223> fusoxFOXG_01564T0
<400> 11
   Met Met Ala Ser Pro His Met Leu Pro Phe Asp Ser Arg Ala Thr Thr
   1               5                   10                  15
   Thr Gly Pro Leu Gln Arg Pro Val Met Ala Pro Tyr Met Val His Pro
               20                  25                  30
   Pro Tyr Ser Ser Gly Pro Val Asn Ser Leu Thr Ala Pro His Tyr Gln
           35                  40                  45
   Val Pro Asn Pro Tyr Gln Phe Gly Gly Tyr Gln Gly Pro Pro Thr Pro
       50                  55                  60
   Pro His His Ser Thr Pro Phe Lys Met Glu Tyr Asn Asp Arg Arg Pro
   65                  70                  75                  80
   Met Gly His Asp Asn Asp His Gly Arg Met Pro Ser Tyr Ser Arg Glu
                   85                  90                  95
   Met Lys Tyr Thr Tyr Ala Glu Gln Ala Pro Ser Pro Ala Arg Ser Asp
               100                 105                 110
   Ser Gln Ala Ser Thr Val Arg Ser Ser Gly Thr Asn Pro Ser Met Gly
           115                 120                 125
   Ser Lys Thr Ile Thr Ser Asn Glu Thr Leu Asn Pro Gly Asp Gln Ile
       130                 135                 140
   Asn Phe Glu Thr Glu Val Asp Glu Leu Met Lys Ala Ile Gln Arg Lys
   145                 150                 155                 160
   Val Asp Leu Gln Val Asp Thr Val Gln Gln Pro Leu Thr Pro Gly Met
                   165                 170                 175
   Ser Pro Val Ser Glu Ala Ser Phe Glu Ser Gln Gly Thr Pro Gly Pro
               180                 185                 190
   Met Asp Ser Lys Thr Ala Arg Lys Arg Tyr Arg Cys Asp Gly Pro Asn
           195                 200                 205
   Cys Gln Lys Ser Phe Thr Gln Lys Thr His Leu Asp Ile His Arg Arg
       210                 215                 220
   Thr His Thr Gly Ile Lys Pro Tyr Asn Cys Asp Phe Pro Gly Cys Asp
   225                 230                 235                 240
   Leu Thr Phe Ser Gln Leu Gly Asn Leu Lys Thr His Arg Arg Arg His
                   245                 250                 255
   Thr Gly Glu Arg Pro Phe Ala Cys Asp Lys Cys Asp Arg His Phe Ala
               260                 265                 270
   Gln Arg Gly Asn Leu Arg Ala His Leu Gln Thr His Gln Gly Leu Lys
           275                 280                 285
   Pro Phe Ile Cys Ile Leu Asp Asp Cys Asn Lys Thr Phe Ser Gln Leu
       290                 295                 300
   Gly Asn Met Lys Thr His Gln Asn Asn Phe His Lys Lys Thr Leu Lys
   305                 310                 315                 320
   Lys Leu Thr Met Lys Phe Ala Asn Ile Ile Ala Ser Gly Glu Glu Val
                   325                 330                 335
   Ser Glu Ala Asp Arg Glu Leu Phe Glu Tyr Phe Ala Ala His Tyr Lys
   340 345 350
   Asn Ser Asn Lys Gly Ile Lys Gly Arg Gly Lys Ala Arg Thr Val Ala
   355 360 365
   Asp Arg Lys Ala Lys Thr Ser Gln Ser Pro Pro Ala Asn Thr Met Thr
   370 375 380
   Ala Val Pro Gln Tyr Pro Leu Pro Gln Ile Ala Pro Thr Pro Val Thr
   385 390 395 400
   Pro His Gly Leu Pro Val Ser Gly Ser Leu Ala Ser Tyr Ser Val Thr
   405 410 415
   Arg Gly Gln Pro Gly Pro Ile Asn His Met Ser Arg Gln Thr His Thr
   420 425 430
   Gly Gly Tyr Glu Val Tyr Asp Ile Gln Gly His His His Ile Gln Pro
   435 440 445
   Pro Asn Asn Asn Gly Met Leu Tyr Glu Thr Gly Ser Thr Arg Glu Met
   450 455 460
   Gly Tyr His Gly Arg Met Tyr
   465 470
<210> 12
   <211> 413
   <212> PRT
   <213> Artificial Sequence
<220>
   <223> picanestExt_fgeneshl_pg.C_70236
<400> 12
   Met Asp Val Ser Ala Ile Thr Asn His Asp Thr Ala Asn Thr Glu Pro
   1               5                   10                  15
   Gly Ala Ala Lys Glu Thr Glu Leu Lys Gln Ser Gln Asp His Ala Phe
               20                  25                  30
   Ser Ile Ser Ser Met Met Asn Asn Asp Glu Glu Arg Thr Val Ser Pro
           35                  40                  45
   Asp Pro Ile Gln Thr Glu Glu Arg Glu Gln Glu Asn Gly Lys Glu Glu
       50                  55                  60
   Asn Gly Ile His His Ser Asp Ser Gln Asn Asp Leu Val Pro Lys Asp
   65                  70                  75                  80
   Val Asp Ala Gly Ser Glu Ser Thr Leu Ser Asp Val Glu Ser Thr Lys
                   85                  90                  95
   Ala Gln Ser Glu Asp Glu Phe Thr Asp Leu Glu Leu Ser Asp Asp Asn
               100                 105                 110
   Ala Glu Glu Ile Glu Ser Lys Arg Ile Glu Ala Asp Cys His Trp Ser
           115                 120                 125
   Gly Cys Gln Asp Lys Phe Leu Asn Ile Asp Asp Leu Val Gln His Leu
       130                 135                 140
   Asn Ser Lys His Ile Thr Asn Val Asp Ser Val Tyr Arg Cys Glu Trp
   145                 150                 155                 160
   Asp Asn Cys Phe Arg Lys Gly Val Thr Gln Pro Ser Arg Phe Ala Leu
                   165                 170                 175
   Ile Ser His Leu Arg Thr His Thr Gly Glu Lys Pro Phe Phe Cys Ile
               180                 185                 190
   Ile Pro Glu Cys Ser Lys His Phe Thr Arg Ala Asp Ala Leu Ser Gln
           195                 200                 205
   His Ile Arg Thr Val His Gln Thr Ser Asn Leu Ser Val Glu Tyr Pro
       210                 215                 220
   Phe Trp Tyr Ser Tyr Leu Asn Gly Leu Lys Asn Asp Glu Thr Phe Ala
   225                 230                 235                 240
   Lys Arg Phe Arg Leu Met Pro Glu Met His Ser Leu Glu Arg Tyr Ser
                   245                 250                 255
   Lys Leu Asp Ser Gly Asp Gln Phe Gln Lys Glu Leu Ala Asn Lys Lys
               260                 265                 270
   Ile Gln Phe Asp Asn Ile His Asn Ile Glu Leu Thr Asp Leu Thr Glu
           275                 280                 285
   Pro Thr Ser Lys Arg Gln Lys Thr Ser Leu Lys Asp Ile Asn Ala Gly
       290                 295                 300
   Leu Lys Ala Ser Phe Glu Lys Glu Asp Ala Asp Phe Lys His His Ile
   305                 310                 315                 320
   Ala Ala Ala Ala Lys Asp Thr Phe Lys Ser Leu Glu Gly Val Glu Ser
   325 330 335
   Leu Pro Thr Asp Ala Ser Ile Glu Ser Val Gln Ser Leu Glu Glu Leu
   340 345 350
   Gln Ala Leu His Glu Ser Leu Arg Arg Lys Tyr Val Trp Gly Leu Glu
   355 360 365
   Val Glu Lys Leu Ile Thr Lys Glu Leu Lys Gln Leu Arg Thr Glu Lys
   370 375 380
   Thr Gln Leu Trp Leu Lys Lys Glu Thr Leu Leu Asp Ala His Ile Glu
   385 390 395 400
   Leu Glu Ile Ser Glu Asn Arg Asp Leu Tyr Ser Leu Lys
   405 410
<210> 13
   <211> 373
   <212> PRT
   <213> Artificial Sequence
<220>
   <223> Afu4g10380
<400> 13
   Met Arg Tyr Pro Pro His Gly Gln Pro Ser Ser Gly Met Arg Ile Leu
   1               5                   10                  15
   Gln Asn Pro Tyr Val Pro Gly Ala Tyr Thr Gly Ser Ala Gln Ile Pro
               20                  25                  30
   Gly Gly Ala Thr Pro Gln Arg Ala Asp Pro Pro Pro Lys Thr Phe His
           35                  40                  45
   Cys Ser Thr Cys Gly Lys Gly Phe Ala Arg Arg Ser Asp Leu Ala Arg
       50                  55                  60
   His Glu Arg Ile His Thr Gly Ile Arg Pro His Ala Cys Asp Trp Pro
   65                  70                  75                  80
   Gly Cys Gly Lys Gln Phe Ile Gln Arg Ser Ala Leu Thr Val His Ser
                   85                  90                  95
   Arg Val His Thr Gly Glu Lys Pro His Met Cys Glu Arg Cys Gly Lys
               100                 105                 110
   Pro Phe Ser Asp Ser Ser Ser Leu Ala Arg His Arg Arg Ile His Ser
           115                 120                 125
   Gly Lys Arg Pro Tyr Lys Cys Pro Tyr Ala Asn Cys Gln Lys Thr Phe
       130                 135                 140
   Thr Arg Arg Thr Thr Leu Thr Arg His Gln Asn His His Thr Gly Thr
   145                 150                 155                 160
   Ile Glu Glu Ala Ala Ala Glu Thr Glu Ala Asn Leu Arg Gln Asn Lys
                   165                 170                 175
   Glu Arg Val Arg Ala Pro Gly Asp Gly Gly Phe Ser Glu His Ala Ser
               180                 185                 190
   Val His Ser Thr Pro Ser Pro Ala His His Pro Val Ser Ile Pro Pro
           195                 200                 205
   Pro Gly Asp Leu Pro Pro Leu Asn Met Pro Arg Ser Ala Gly Asp Tyr
       210                 215                 220
   Tyr Met Gly Asn Gly Ser Ile Pro Pro His Val Arg Gly Asp Phe Ser
   225                 230                 235                 240
   Gln Ala Ser Pro Arg Ser Ser Pro Thr Ala Thr Ser Pro Ser Leu Ser
                   245                 250                 255
   Ser Phe Ser Gly Ala Pro His Gln Arg Pro Ser Met Thr Ser His Pro
               260                 265                 270
   Ser Gly Tyr Ala Pro Pro Gln Pro Leu Glu Pro Pro Ala Asn Ser Asp
           275                 280                 285
   His Arg Pro Asn Ser Val Ser Gly Ser Pro His Met Thr Ser Leu Gly
       290                 295                 300
   Trp Ala Ser Pro Ser His Gly Ser Ile Pro Ser Pro Gly Ser Val Asn
   305                 310                 315                 320
   Asp Phe Asn Tyr Pro Glu Pro Ser Gly Pro Ala Tyr Pro Ser Ser Met
                   325                 330                 335
   Pro Pro His Met Tyr Phe Pro Asn Ser Thr Ile Arg Arg Pro Thr Ser
               340                 345                 350
   Thr Glu Pro Glu Asn Tyr Glu Leu Lys Pro Arg Leu Gly Asp Asn Gly
           355                 360                 365
   Trp Ser Thr Pro Val
   370
<210> 14
   <211> 435
   <212> PRT
   <213> Artificial Sequence
<220>
   <223> miccaMCYG_06457T0
<400> 14
   Met Asp Ile Thr Thr Ile Leu Asn Lys Lys Ala Ser Val Ser Val Val
   1               5                   10                  15
   Ala Ala Glu Val Gln Gln Leu Gln Gln His Leu Ala Gln Ala Gln Ala
               20                  25                  30
   Ala Asp Asp Ala Lys Ser Arg Ser Pro Ser Glu Met Gly Ser Glu His
           35                  40                  45
   Gly Ser Ala Ser Ala Pro Pro Ser Glu His Gln Gln Thr Tyr Pro Ser
       50                  55                  60
   Ser Thr Gln Ser Leu Pro Gln Met Ala His Leu Ala Gln Tyr His Val
   65                  70                  75                  80
   Gln Ala Gln Ala Thr His Asn Asn Tyr Ala His Ser Ala His Gly Ser
                   85                  90                  95
   Asp Tyr Gly Arg Asn Gly Ser Gly Asn Met Arg Pro Ser Gly Leu Pro
               100                 105                 110
   Ala Leu Lys Thr Phe His Cys Gln Thr Cys Ser Lys Gly Phe Ala Arg
           115                 120                 125
   Arg Ser Asp Leu Ala Arg His Glu Arg Ile His Ser Gly Ile Arg Pro
       130                 135                 140
   His Ala Cys Asp Trp Pro Gly Cys Gly Lys Gln Phe Ile Gln Arg Ser
   145                 150                 155                 160
   Ala Leu Thr Val His Thr Arg Val His Thr Gly Glu Lys Pro His Met
                   165                 170                 175
   Cys Asp Arg Cys Gly Lys Pro Phe Ser Asp Ser Ser Ser Leu Ala Arg
               180                 185                 190
   His Arg Arg Ile His Ser Gly Lys Arg Pro Tyr Lys Cys Pro Tyr Ala
           195                 200                 205
   Asn Cys Gln Lys Thr Phe Thr Arg Arg Thr Thr Leu Thr Arg His Gln
       210                 215                 220
   Asn His His Thr Gly Thr Ile Glu Glu Ala Ala Ala Glu Thr Glu Ala
   225                 230                 235                 240
   Asn Leu Arg His Ser Lys Asp Arg Val Ser Arg Pro Ser Asp Tyr Ser
                   245                 250                 255
   Glu Ala Gly Ser Ala His Ser Thr Pro Ser Pro Ala Gln Arg Pro Ser
               260                 265                 270
   Leu Ser Pro Gly Asn Glu Leu Pro Pro Leu Asn Leu Ser Arg Pro Ser
           275                 280                 285
   Gly Asp Tyr Tyr Thr Ser Leu Pro Pro His Leu Arg Thr Asp Phe Gln
       290                 295                 300
   Gln Ser Ser Pro Arg Ala Ser Pro Thr Ser Pro Ser Pro Thr Leu Thr
   305                 310                 315                 320
   Ser Leu Ser Gly Pro Ala Ser Tyr His Asn Arg Pro Ser Leu Thr Ser
                   325                 330                 335
   His Pro Ser Gly Tyr Gly Pro Pro Gln Pro Leu Glu Pro Pro Ala Asn
               340                 345                 350
   Ser Asp Pro Arg Pro Asn Ser Val Ala Gly Ser Pro His Leu Ser Ser
           355                 360                 365
   Val Gly Trp Ala Ser Pro Thr Leu Ser Ser Met Pro Ser Pro Ser Ser
       370                 375                 380
   Ala Ser Ala Pro Glu Tyr Ser Tyr Pro Glu Pro Ser Ser His Gln Tyr
   385                 390                 395                 400
   Pro Gly Gly Ile Thr Pro His Met Phe Phe Ser Gly Gln Gly Leu Arg
                   405                 410                 415
   Arg Pro Gln Ser Ser Glu Pro Glu Asn Tyr Glu Met Lys Pro Lys Leu
               420                 425                 430
   Glu Met Ser
           435
<210> 15
   <211> 436
   <212> PRT
   <213> Artificial Sequence
<220>
   <223> fusoxFOXG_03183T0
<400> 15
   Met Ser Gly Met Arg Ser Pro Ala Pro Thr Glu Val Met Asp Ile Thr
   1               5                   10                  15
   Asn Met Leu Asn Lys Lys Gly Gln Met Gln Gln Leu Thr Ser Gly Leu
               20                  25                  30
   Leu Asp His Gln Gln Tyr Gln His Ala Phe Val Lys His Glu Pro Gly
           35                  40                  45
   Met Glu Arg Ser Ala Ser Pro His Gly Ser Glu His Ser Gln Tyr Ser
       50                  55                  60
   Asn Pro His Ser Ile Ala Arg Ala Tyr Pro Ser Pro Ser Thr Met Gln
   65                  70                  75                  80
   Ala Pro Met His Met Pro Asn Pro Met Ser Ala Ala Met Thr Ile Gln
                   85                  90                  95
   Gly Tyr Pro Glu Met Pro Asn Met Gly Gly Met Pro His Met His Met
               100                 105                 110
   Gln Gln Met Pro Gln Gln Pro Pro Pro Pro Pro Gln Gln Pro Ile Lys
           115                 120                 125
   Ala Tyr Pro Cys Ser Thr Cys Gly Lys Gly Phe Ala Arg Arg Ser Asp
       130                 135                 140
   Leu Ala Arg His Glu Arg Ile His Ser Gly Val Arg Pro His Val Cys
   145                 150                 155                 160
   Asp Trp Pro Gln Cys Gly Lys Gln Phe Ile Gln Arg Ser Ala Leu Thr
                   165                 170                 175
   Val His Gln Arg Val His Thr Gly Glu Lys Pro His His Cys Glu Thr
               180                 185                 190
   Cys Ala Lys Pro Phe Ser Asp Ser Ser Ser Leu Ala Arg His Arg Arg
           195                 200                 205
   Thr His Ser Gly Lys Arg Pro Tyr Lys Cys Pro Tyr Ala Asp Cys Gln
       210                 215                 220
   Lys Thr Phe Thr Arg Arg Thr Thr Leu Thr Arg His Gln Asn His His
   225                 230                 235                 240
   Ser Gly Thr Ile Glu Glu Ala Ala Ala Ala Thr Ala Ala Ala Leu Ala
                   245                 250                 255
   Ala Ser Lys Ser Lys Gly Leu Ser Gln Ala Arg Ser Glu Ser Asp His
               260                 265                 270
   Met Ser Asn His Gly Ser Pro Leu Thr Thr Pro Ser Pro Ser Gln Arg
           275                 280                 285
   Thr Met Ser Met Ser Pro Ser Val Asp Leu Ser Gly Asn Asn Ser Ile
       290                 295                 300
   Pro Arg His Pro Gly Asp Phe Gln Tyr Leu Ser Gln Asn Gly Ser Leu
   305                 310                 315                 320
   Pro Met His Met Arg Val Gly Ser Pro Thr Ser Thr Ser Ser Gly Gly
                   325                 330                 335
   Tyr Asn Met Met Arg Pro Thr Ser His Pro Thr Ser Tyr Gly Pro Pro
               340                 345                 350
   Pro Thr Leu Glu Pro Asn Leu Asp Asn Ser Gln Gly Thr Pro Ser Ser
           355                 360                 365
   Asn Gly Gly Ser Pro His Met Ala Asn Val Gly Trp Gln Ser Pro Ser
       370                 375                 380
   His Met Ala Ser Pro Ser Gln Asn Asn Ala Ser Tyr Val Tyr Pro Asp
   385                 390                 395                 400
   Pro Ala Asp Ala Tyr Pro Ala Asn Gln Ala Met Asn Gln Met Tyr Tyr
                   405                 410                 415
   Ser Ala Ala Thr His Met Arg Arg Pro Gln Ser Thr Glu Pro Gly Leu
               420                 425                 430
   Val His Met Ala
           435
<210> 16
   <211> 445
   <212> PRT
   <213> Artificial Sequence
<220>
   <223> triruTERG_07147T0
<400> 16
   Met Asp Ile Thr Thr Ile Leu Asn Lys Lys Ala Ser Val Ser Ile Val
   1               5                   10                  15
   Ala Ala Asp Val His Gln Leu Gln Gln His Leu Ala Gln Ala Pro Val
               20                  25                  30
   Thr Val Thr Val Ala Val Thr Ser Arg Ser Pro Ser Glu Leu Gly Ala
           35                  40                  45
   Ser Glu His His Arg Ser Ala Ser Ala Pro Pro Ser Glu His His His
       50                  55                  60
   His His His His His Gln Pro Thr Ser Ser Phe Pro Ser Ser Thr Gln
   65                  70                  75                  80
   Ser Leu Pro Gln Met Ala His Leu Ala Gln Tyr His Val Gln Ala Gln
                   85                  90                  95
   Ala Asn His Ala Gly Thr Ser Gln Gly Tyr Ala His Ser Ala His Gly
               100                 105                 110
   Ser Asp Tyr Gly Arg Ser Thr Thr Ser Met Arg Pro Thr Gly Leu Pro
           115                 120                 125
   Ala Leu Lys Thr Phe His Cys Gln Thr Cys Ser Lys Gly Phe Ala Arg
       130                 135                 140
   Arg Ser Asp Leu Ala Arg His Glu Arg Ile His Ser Gly Ile Arg Pro
   145                 150                 155                 160
   His Ala Cys Asp Trp Pro Gly Cys Gly Lys Gln Phe Ile Gln Arg Ser
                   165                 170                 175
   Ala Leu Thr Val His Thr Arg Val His Thr Gly Glu Lys Pro His Met
               180                 185                 190
   Cys Asp Arg Cys Gly Lys Pro Phe Ser Glu Ser Ser Ser Leu Ala Arg
           195                 200                 205
   His Cys Arg Ile His Ser Gly Lys Arg Pro Tyr Lys Cys Pro Tyr Ala
       210                 215                 220
   Asn Cys Gln Lys Thr Phe Thr Arg Arg Thr Thr Leu Thr Arg His Gln
   225                 230                 235                 240
   Asn His His Thr Gly Thr Ile Glu Glu Ala Ala Ala Glu Thr Glu Ala
   245 250 255
   Asn Leu Arg His Ser Lys Asp Arg Ser Thr Thr Ser Arg Pro Ser Asp
   260 265 270
   Ala Leu Leu Ser Pro Leu Asn Leu Ser Ser Arg Pro Thr Ser Gly Trp
   275 280 285
   Arg Pro Leu Leu Ser Ala Gly Gly Ala Ala Asp Tyr Tyr Thr Ser Leu
   290 295 300
   Arg Pro Ile Ser Glu Arg Thr Phe Ala Lys Leu Gln Ser Leu Ala Pro
   305 310 315 320
   Arg Leu Leu Pro Leu Arg Gln Pro Ser Pro Ala Ser Pro Gly Pro Ala
   325 330 335
   Ser Phe His His His His Asn His Asn Arg Pro Ser Leu Thr Ser His
   340 345 350
   Pro Ser Gly Tyr Ala Pro Pro Gln Pro Leu Glu Pro Pro Ala Asn Ser
   355 360 365
   Asp Pro Arg Pro Asn Ser Val Ala Gly Ser Pro His Leu Ser Ser Val
   370 375 380
   Gly Trp Ala Ser Pro Thr Leu Ser Ser Met Pro Ser Pro Ser Ser Ala
   385 390 395 400
   Ser Ala Pro Glu Tyr Pro Tyr Pro Glu Pro Ser Asn His Gln Tyr Pro
   405 410 415
   Gly Ser Ile Thr Pro His Met Phe Phe Pro Arg Arg Pro Gln Ser Ser
   420 425 430
   Glu Pro Glu Asn Tyr Glu Leu Lys Pro Lys Leu Glu Leu
   435 440 445
<210> 17
   <211> 230
   <212> PRT
   <213> Artificial Sequence
<220>
   <223> canluCLUT_00076
<400> 17
   Met Gln Pro Leu Ser Lys Thr Glu Lys Pro Pro Lys Asp Pro Ala Asn
   1               5                   10                  15
   Thr Asn Thr Ala Ser Pro Leu Ala Ala Ala Ser Val Ser Gln Thr Ser
               20                  25                  30
   Gln Thr Thr Gln Pro Leu Gln Thr Ser Gln Val Ser Ala Pro Ser Gln
           35                  40                  45
   Ala Pro Gln Thr Ser Gln Ser Thr Ser Pro Asn Ser Ser Thr Thr Gln
       50                  55                  60
   Ser Ser Pro Gln Ser Thr Ser Gln Thr Ser Ser Ser Phe Ser Arg Glu
   65                  70                  75                  80
   Pro Lys Asp Ala Pro Gln Lys Glu Ser Ser Asn Glu Ala Ser Asn Glu
                   85                  90                  95
   Ala Ser Asp Ser Asp Lys Ser Leu Ser Thr Ser Lys Ser Ala Pro Glu
               100                 105                 110
   Ser Ala Gly Ser Ala Glu Asn Gln Ala Asp His Asp Asp Ala Lys Glu
           115                 120                 125
   Lys Lys Leu Glu Lys His Phe Phe Cys Arg Ile Cys Lys Gln Gly Phe
       130                 135                 140
   Thr Arg Lys His Asn Met Val Ser His Glu Leu Ile His Ser Ser Asn
   145                 150                 155                 160
   Lys Pro His Val Cys Ser Val Cys Asp Ala Thr Phe Arg Arg Ile His
                   165                 170                 175
   Asp Leu Arg Arg His Glu Lys Leu His Ser Gly Glu Lys Pro Phe His
               180                 185                 190
   Cys Gln His Cys Asn Arg Gly Phe Ala Arg Thr Asp Ala Leu Thr Arg
           195                 200                 205
   His Ile Asn Ser Ser Asn Ala Cys Leu Val Lys Gln Arg Gln Thr Arg
       210                 215                 220
   Lys Arg Thr Asn Leu Ile
   225                 230
<210> 18
   <211> 259
   <212> PRT
   <213> Artificial Sequence
<220>
   <223> R03G_16212
<400> 18
   Met Asp Ile Arg Asn Leu Leu Asn Glu Thr Glu Asn Ile Glu Lys Pro
   1 5 10 15
   Phe Arg Cys Thr Trp Asn Asp Cys Asp Lys Lys Phe Ser Arg Lys Ser
   20 25 30
   Asp Leu Ser Arg His Ile Arg Ile His Thr Gly Glu Arg Pro Tyr His
   35 40 45
   Cys Gly Trp Pro Ser Cys Gly Lys Gln Phe Ile Gln Arg Ser Ala Leu
   50 55 60
   Thr Val His Tyr Arg Thr His Thr Gly Glu Arg Pro His Val Cys Glu
   65 70 75 80
   Tyr Asp Ser Cys Gly Lys Ala Phe Ser Asp Ser Ser Ser Leu Ala Arg
   85 90 95
   His Arg Arg Thr His Thr Gly Lys Arg Pro Tyr Ser Cys Leu His Pro
   100 105 110
   Gly Cys Gly Lys Ser Phe Thr Arg Lys Thr Thr Leu Ser Arg His Gln
   115 120 125
   Arg Cys His Asp Pro Lys Trp Glu Ser Tyr Asn Leu Lys Asn His Lys
   130 135 140
   Ile Thr Phe Asn Lys His Glu Glu Ser Asn Glu Cys Leu Ile Val Asp
   145 150 155 160
   Gln Gln Pro Phe His Gln Thr Ser Ala Ala Ser Pro Ile Ser Ser Ser
   165 170 175
   Ser Asp Ser Glu Leu Asp Ser Pro Leu Asn Ser Pro Val Glu Tyr Phe
   180 185 190
   Asn Leu Tyr His Gln Arg Gln Gln Leu Pro Phe Ile Lys His His His
   195 200 205
   Pro Met Val Glu Met Pro Gln Arg Ile Lys His Val His Thr Tyr Arg
   210 215 220
   Ile Leu Asp Tyr Asn Ser Phe Gly Asn Asn Pro Ser Ile Ala Pro Leu
   225 230 235 240
   Gln Gln Gln Pro Ser Phe Ile His Gln Lys Thr Arg Met Ser Tyr Tyr
   245 250 255
   Asn Phe Pro
<210> 19
   <211> 436
   <212> PRT
   <213> Artificial Sequence
<220>
   <223> colgrGLRG_05374T0
<400> 19
   Met Gln Gln His Ala Ser Leu His Asn Gly Ser Thr Pro Glu Arg Gln
   1 5 10 15
   Met His Gln Leu Pro Gln Leu Thr Ala Arg Asn Met Pro Glu Pro Pro
   20 25 30
   Met Asp Arg Ala Gly Ser Pro His Gly Ser Glu His Ser Gln Tyr Ser
   35 40 45
   Arg His Thr Met Glu Gly Val Pro Arg Ser Tyr Pro Ser Pro Ser Ala
   50 55 60
   Met Val Gly Ala Pro Met Gln Met Gln Val Pro Val Ser His Met Gly
   65 70 75 80
   Pro Gly Pro Val Ile Leu Pro Gly Ile Pro Gln His Asp Met Ser Gln
   85 90 95
   Gly Met Pro Gln Tyr Lys Val Pro Asp Gln Gln Ala Pro Gln Gln Pro
   100 105 110
   Thr Lys Ala Tyr Pro Cys Ser Thr Cys Gly Lys Gly Phe Ala Arg Arg
   115 120 125
   Ser Asp Leu Ala Arg His Glu Arg Ile His Ser Gly Ile Arg Pro His
   130 135 140
   Val Cys Asp Phe Pro Asn Cys Gly Lys Gln Phe Ile Gln Arg Ser Ala
   145 150 155 160
   Leu Thr Val His Gln Arg Val His Thr Gly Glu Lys Pro His Met Cys
   165 170 175
   Glu Arg Cys Gly Lys Pro Phe Ser Asp Ser Ser Ser Leu Ala Arg His
   180 185 190
   Arg Arg Ile His Ser Gly Lys Arg Pro Tyr Lys Cys Pro Tyr Ala Asp
   195 200 205
   Cys Gln Lys Thr Phe Thr Arg Arg Thr Thr Leu Thr Arg His Gln Asn
   210 215 220
   His His Thr Gly Thr Val Glu Glu Ala Ala Ala Ala Thr Ala Ala Ala
   225 230 235 240
   Leu Ala Ala Arg Gly Ala Thr Ala Lys Pro Asn Ala Thr Arg Ser Asp
   245 250 255
   Gly Asp Ala Val Ser Asn Arg Gly Ser Pro Leu Thr Thr Pro Ser Pro
   260 265 270
   Gly Gln Arg Thr Leu Ser Met Ser Pro Ser Ala Asp Gly Met His Arg
   275 280 285
   Thr Asn Ser Glu Phe Gln Tyr Met Gly Asn Ser Ser Leu Pro Val His
   290 295 300
   Leu Gln Arg Asp Met His Val Gly Ser Pro Ala Ser Thr Thr Ser Gly
   305 310 315 320
   Gly Tyr Asn Thr Gly Met Arg Pro Thr Ser His Pro Thr Ser Tyr Gly
   325 330 335
   Pro Pro Pro Thr Leu Glu Pro Ser Val Glu Gln His Ser Gly Pro Gly
   340 345 350
   Ser Ala Gly Gly Ser Pro His Met Ser Asn Val Gly Trp Gln Ser Pro
   355 360 365
   Ser His Val Ala Ser Pro Ser His Ser Ser Asn Gly Gly Gly Tyr Thr
   370 375 380
   Tyr Pro Asp Pro Asp Ala Tyr Pro Ala Ala Pro Ser Leu Ser Gly Gln
   385 390 395 400
   Met Phe Tyr Gly Asn Pro Gly Gln Val Arg Arg Pro Gln Ser Thr Glu
   405 410 415
   Pro Pro Ala Gly Ala Tyr Asp Ile Lys Gly Arg Gln Ser Glu Leu Trp
   420 425 430
   Ala Gly Ala His
   435
<210> 20
   <211> 470
   <212> PRT
   <213> Artificial Sequence
<220>
   <223> fusveFVEG_07926T0
<400> 20
   Met Met Glu Ser Pro His Met Ile Pro Phe Asp Ser Arg Ala Thr Thr
   1 5 10 15
   Thr Gly Pro Leu Gln Arg Pro Val Met Ala Pro Tyr Ile Val Gln Pro
   20 25 30
   Pro Tyr Ser Ser Gly Pro Val Asn Ser Leu Thr Ala Pro His Tyr Gln
   35 40 45
   Val Pro Asn Pro Tyr Gln Phe Gly Gly Tyr Gln Gly Pro Pro Thr Pro
   50 55 60
   Pro His His Ser Thr Pro Phe Lys Ile Glu Tyr Asn Asp Arg Arg Pro
   65 70 75 80
   Met Gly His Asp Asn Asp His Gly Arg Val Pro Ser Tyr Ser Arg Glu
   85 90 95
   Met Lys Cys Thr Tyr Ala Glu Gln Ala Pro Ser Pro Ala Arg Ser Asp
   100 105 110
   Ser Gln Ala Ser Thr Val Arg Ser Ser Gly Thr Asn Pro Ser Met Cys
   115 120 125
   Ser Lys Thr Ile Thr Ser Asn Glu Thr Leu Asn Pro Gly Asp Gln Ile
   130 135 140
   Asn Phe Glu Thr Glu Val Asp Glu Leu Met Lys Ala Ile Gln Arg Lys
   145 150 155 160
   Ala Asp Leu Gln Val Asp Thr Val Gln Gln Pro Leu Thr Pro Gly Met
   165 170 175
   Ser Pro Val Ser Glu Ala Ser Phe Glu Ser Gln Gly Thr Pro Gly Pro
               180                 185                 190
   Met Asp Asn Lys Thr Ala Arg Lys Arg Tyr Arg Cys Asp Gly Pro Asn
           195                 200                 205
   Cys Gln Lys Ser Phe Thr Gln Lys Thr His Leu Asp Ile His Arg Arg
       210                 215                 220
   Thr His Thr Gly Ile Lys Pro Tyr Asn Cys Asp Phe Pro Gly Cys Asp
   225                 230                 235                 240
   Leu Thr Phe Ser Gln Leu Gly Asn Leu Lys Thr His Arg Arg Arg His
                   245                 250                 255
   Thr Gly Glu Arg Pro Phe Ala Cys Asp Lys Cys Asp Arg His Phe Ala
               260                 265                 270
   Gln Arg Gly Asn Leu Arg Ala His Leu Gln Thr His Gln Gly Leu Lys
           275                 280                 285
   Pro Phe Val Cys Ile Leu Asp Asp Cys Asn Lys Thr Phe Ser Gln Leu
       290                 295                 300
   Gly Asn Met Lys Thr His Gln Asn Asn Phe His Lys Lys Thr Leu Lys
   305                 310                 315                 320
   Lys Leu Thr Met Lys Phe Ala Lys Ile Ile Ala Ser Gly Glu Glu Val
                   325                 330                 335
   Thr Glu Ala Asp Arg Glu Leu Phe Glu Tyr Phe Ala Thr His Tyr Lys
               340                 345                 350
   Asn Ser Asn Lys Gly Ile Lys Gly Arg Gly Lys Ala Arg Thr Val Ala
           355                 360                 365
   Glu Arg Lys Thr Lys Ala Ser Gln Ser Pro Ala Asn Thr Met Thr Ala
       370                 375                 380
   Val Pro Gln Tyr Pro Leu Pro Gln Ile Ala Ser Thr Pro Val Thr Pro
   385                 390                 395                 400
   His Gly Leu Pro Val Ser Gly Ser Leu Ala Ser Tyr Ser Val Thr Arg
                   405                 410                 415
   Gly Gln Pro Ala Pro Ile Asn His Met Ser Arg Gln Thr His Asn Gly
               420                 425                 430
   Gly Tyr Glu Val Tyr Asp Met His Gly His His His Val Gln Pro Pro
   435 440 445
   Asn Asn Asn Gly Met Leu Tyr Glu Thr Gly Ser Thr Arg Glu Met Gly
   450 455 460
   Tyr His Gly Arg Met Tyr
   465 470
<210> 21
   <211> 445
   <212> PRT
   <213> Artificial Sequence
<220>
   <223> neofi4499
<400> 21
   Met Glu Thr Ala Glu Pro Val Ser Tyr Glu Phe Thr Gly His Ala Met
   1 5 10 15
   Gly Ser Met Ala Pro Arg Arg Leu Leu His Ala Gly Leu Gly Ser Asn
   20 25 30
   Phe Ser Tyr Tyr Asn Asn Pro Ala Thr Ser Phe Pro Leu Pro Tyr His
   35 40 45
   Gln Ser Ser Ser Thr Pro Tyr Gly Phe Ser His Thr Ile Asn Gln His
   50 55 60
   Pro His Gln Leu Pro Gly Tyr Gln His Phe Tyr Leu Thr Gly His Pro
   65 70 75 80
   Ser Leu Asn Leu Gln Pro Val Arg Leu Ser Ser Glu Pro Pro Thr Val
   85 90 95
   Gln Gln Ile Pro Asp Ile Arg Pro Ala Lys Asn Ala Ile Ser Arg Ile
   100 105 110
   Val Gly Lys Pro Leu Ala Lys Ser Glu Gln Ser Val Ser Ser Gln Pro
   115 120 125
   Ile Ala Ala Ala Gln Pro Pro Ala Thr Gly Ala Thr Gln Glu Lys Gly
   130 135 140
   Pro Gly Ser Thr Glu Ile Glu Phe Ser Thr Glu Val Asp Ile Leu Met
   145 150 155 160
   Lys Ala Ile Gln Ala Lys Ala Val Pro Gln Gln Ser Ala Leu Gln Ser
   165 170 175
   Leu Pro Pro Leu Gln Gln Leu Thr His Ser Gly Glu Asn Gly Phe Ser
   180 185 190
   Gln Thr Ser Tyr Ala Met Ala Ser Ser Thr Pro Arg Cys Asn Met Ala
   195 200 205
   Val Glu Glu Leu Ala Ser Arg Pro Gly Lys Lys Arg Lys Tyr Ile Cys
   210 215 220
   Arg Leu Ser Asn Cys Gly Lys Ser Phe Ala Gln Lys Thr His Leu Asp
   225 230 235 240
   Ile His Met Arg Ala His Thr Gly Asp Lys Pro Phe Val Cys Lys Glu
   245 250 255
   Pro Ser Cys Gly Gln Arg Phe Ser Gln Leu Gly Asn Leu Lys Thr His
   260 265 270
   Gln Arg Arg His Thr Gly Glu Lys Pro Phe Ser Cys Asp Ile Cys Gln
   275 280 285
   Lys Arg Phe Ala Gln Arg Gly Asn Val Arg Ala His Lys Ile Thr His
   290 295 300
   Gln His Ala Lys Pro Phe Thr Cys Leu Leu Asp Asp Cys Gly Lys Gln
   305 310 315 320
   Phe Thr Gln Leu Gly Asn Leu Lys Ser His Gln Asn Lys Phe His Ala
   325 330 335
   Ala Thr Leu Arg Thr Leu Thr Leu Lys Phe Ser Gln Met Ser Glu Ala
   340 345 350
   Gln Leu Met Asn Pro Gln Asp Arg Gln Leu Trp Glu Tyr Phe Ala Thr
   355 360 365
   Leu Tyr Lys Asn Ser Asn Lys Gly Ile Lys Gly Arg Gly Lys Asp Arg
   370 375 380
   Arg Ile Ser Pro Thr Ser Lys Ser Ser Asp Ser Ser Ala Ser Cys Thr
   385 390 395 400
   Arg Met Gln Ser Phe Glu Ser Asp Glu Asp Ile Lys Ala Ser Arg Asp
   405 410 415
   Ser Arg Glu Ala Ser Ser Ala Tyr Thr Gly Ser Ser Ser Gly Glu Glu
   420 425 430
   Glu Asp Pro Glu Ile Tyr Tyr Thr Asp Arg Arg Gly His
   435 440 445
<210> 22
   <211> 441
   <212> PRT
   <213> Artificial Sequence
<220>
   <223> cocrsCIMG_00576T0
<400> 22
   Met Asp Ile Thr Thr Ile Leu Asn Lys Asn Arg Ser Ala Val Ala Ala
   1 5 10 15
   Ala Ala Glu Ala Gln Leu Gln His Gln Leu Ala Gln Thr Ala Gln Ile
   20 25 30
   Lys Ser Arg Ser Pro Ser Glu Leu Gly Ser Glu His Asp Ala Gln Gln
   35 40 45
   Pro Gly Arg His Ser Glu Pro Tyr His Pro Ala His Gln Pro Ile Gln
   50 55 60
   Leu Pro Thr Ile Ser Gln Tyr His Ser Pro Ile Gln Thr Asn His His
   65 70 75 80
   Ala Ser Met Leu Arg Gly Asp Tyr Ala Gln Asn Gly His Glu Asn Met
   85 90 95
   Phe Arg Asn Met Pro Thr Ser Asn Ser Ser Pro Gly Arg Gly Thr Gly
   100 105 110
   Glu Pro Ala Pro Lys Ser Phe His Cys Gln Thr Cys Ser Lys Gly Phe
   115 120 125
   Ala Arg Arg Ser Asp Leu Ala Arg His Glu Arg Ile His Ser Gly Ile
   130 135 140
   Arg Pro His Val Cys Asp Trp Pro Gly Cys Gly Lys Gln Phe Ile Gln
   145 150 155 160
   Arg Ser Ala Leu Thr Val His Thr Arg Val His Thr Gly Glu Lys Pro
   165 170 175
   His Met Cys Asp Arg Cys Gly Lys Pro Phe Ser Asp Ser Ser Ser Leu
               180                 185                 190
   Ala Arg His Arg Arg Ile His Ser Gly Lys Arg Pro Tyr Lys Cys Pro
           195                 200                 205
   Tyr Ala Asn Cys Gln Lys Thr Phe Thr Arg Arg Thr Thr Leu Thr Arg
       210                 215                 220
   His Gln Asn His His Thr Gly Thr Ile Glu Glu Ala Ala Ala Glu Thr
   225                 230                 235                 240
   Glu Ala Asn Leu Arg Gln Gly Lys Val Val Arg Pro Asp Gly Val Tyr
                   245                 250                 255
   Ser Glu Thr Ala Ser Pro His Gly Thr Ala Ser Pro Ala Gln Arg Ser
               260                 265                 270
   Ser Val Ser Pro Arg His Glu Leu Pro Pro Leu His Ser His Arg Gln
           275                 280                 285
   Met Gly Asp Tyr Phe Met Ala Ser Ser Ser Leu Pro Pro His Leu Arg
       290                 295                 300
   Asp Phe Gln Gln Ala Ser Pro Arg Ala Ser Pro Ser Thr Pro Ser Pro
   305                 310                 315                 320
   Thr Leu Ser Thr Phe Ser Ala Ala His His Pro Arg Pro Ser Leu Thr
                   325                 330                 335
   Ser His Pro Thr Ser Tyr Gly Pro Pro Gln Pro Leu Glu Pro Pro Pro
               340                 345                 350
   Asn Ser Gly Ser Arg Pro Gly Ser Val Thr Gly Ser Pro His Met Thr
           355                 360                 365
   Thr Val Gly Trp Ala Ser Pro Ser Leu Asn Ser Val Pro Ser Pro Gly
       370                 375                 380
   Ser Val Ser Ala Pro Glu Tyr Thr Tyr Pro Glu Pro Ser Gly Pro Pro
   385                 390                 395                 400
   Phe Ala Gly Gly Met Pro Ala His Met Tyr Tyr Pro Asn Ser Thr Ile
                   405                 410                 415
   Arg Arg Pro Gln Ser Thr Glu Pro Glu Asn Tyr Glu Leu Lys Pro Lys
               420                 425                 430
   Val Glu Glu Pro Trp Ala Thr His Ala
   435 440
<210> 23
   <211> 395
   <212> PRT
   <213> Artificial Sequence
<220>
   <223> Ef02.087100.1
<400> 23
   Met Glu Leu Leu Glu Leu Val Glu Tyr Glu Ser Thr Thr Arg Pro Phe
   1 5 10 15
   Gln Cys Asp Trp Glu Ser Cys Asn Lys Ser Phe Asn Arg Lys Ser Asp
   20 25 30
   Leu Gln Arg His Tyr Arg Ile His Thr Asn Glu Arg Pro Tyr Thr Cys
   35 40 45
   Thr Thr Pro Gly Cys Gly Lys Ser Phe Ile Gln Arg Ser Ala Leu Thr
   50 55 60
   Val His Ile Arg Thr His Thr Gly Glu Lys Pro His Gln Cys Gln His
   65 70 75 80
   Val Gly Cys Gly Lys Arg Phe Ser Asp Ser Ser Ser Leu Ala Arg His
   85 90 95
   Arg Arg Ile His Thr Gly Lys Arg Pro Tyr Lys Cys Ala His Asp Gly
   100 105 110
   Cys Leu Lys Ser Phe Cys Arg Lys Thr Thr Met Val Lys His Gln Arg
   115 120 125
   Arg Ser His Gln Arg Gly Met Asn Pro Asn Asp Ile Leu Asp Asp Cys
   130 135 140
   Thr Ser Glu Ser Asp Met Gly Glu Ser Pro Ser Thr Pro Gly Gln Thr
   145 150 155 160
   Ala Met Ser Trp Ser Met Pro Gly Thr Ile Ile His Pro Ala Met Ala
   165 170 175
   His Ser His Pro Met His Arg Ser Ala Thr Phe Gly Asp Phe Gly Gln
   180 185 190
   Gln Met Thr Pro Tyr Gly Met Asp His Gln Ile Ala Gln Arg Gln Ser
   195 200 205
   Val Pro Ala Glu Val His Glu Phe His Gly Gln Asp Pro Asn Met His
   210 215 220
   Met Ile Gln Arg Thr Ala Asn Ile Pro His Gln Pro Tyr Tyr Val Ile
   225 230 235 240
   Asp Gln Asn Asn Pro Gly Ile Ala Thr Met Asn Thr Asn Val Pro Ala
   245 250 255
   Ala Tyr His Ile Pro Arg Gln His Val Glu Arg Ser Leu Glu Met Ala
   260 265 270
   Tyr Asn Ser Gly Ser Met Pro Ser Leu Ser Ser Ser Pro Ala Ser Phe
   275 280 285
   Ser Pro Ala Ser Gly His Ser Ser Ser Met His Asp Gly Leu Tyr Thr
   290 295 300
   His Gln Pro Pro Ala Ser Ala Thr Tyr Gly Leu Gln Glu Ala His Ala
   305 310 315 320
   Val Glu Pro Ser Asn Asn Met Val Ser Tyr Thr Gln Gln Met His Gln
   325 330 335
   Thr Arg Ser Ser Gln Pro Glu Ala Glu Trp Val Tyr Gln Tyr Gln Ser
   340 345 350
   Pro Val Glu Val Ala Thr Ile Gly Gln Ile Pro Ala Phe Gly Thr Gly
   355 360 365
   Val Tyr Asp Met Tyr Ser Gly Pro Lys Ile Asp Phe Asp Asp Pro Thr
   370 375 380
   Met Gln Leu Pro Ser Ser Arg Val Glu Thr Leu
   385 390 395
<210> 24
   <211> 447
   <212> PRT
   <213> Artificial Sequence
<220>
   <223> parraPABG_03071T0
<400> 24
   Met Asp Ile Thr Thr Ile Leu Asn Lys Lys Gly Thr Ala Ala Val Ala
   1 5 10 15
   Ala Ala Glu Ala His Leu Gln Gln His Leu Val His Ala Thr His Met
   20 25 30
   Lys Ser Lys Thr Pro Ser Glu Ile Ser Ser Glu His Gly Thr Pro Gln
   35 40 45
   Asn Gly Asp Gln Ser Asn Ile Tyr His Pro Thr Ser Gln Pro Asn Pro
   50 55 60
   Gln Leu Val Gly Ser Ser Lys Tyr His His Ser Gln Ser Gln Gly His
   65 70 75 80
   Ala Gly Ile Pro Pro Val Arg Ser Asn Tyr Val Gln Asp Gly Gln Gly
   85 90 95
   Ile Val Lys Tyr Leu Gln Asn Gly Asn Gly Gly Asn Gly Arg Ser Ser
   100 105 110
   Gly Glu Pro Val Pro Lys Thr Phe His Cys Ser Thr Cys Ser Lys Gly
   115 120 125
   Phe Ala Arg Arg Ser Asp Leu Ala Arg His Glu Arg Ile His Ser Gly
   130 135 140
   Ile Arg Pro His Val Cys Asp Trp Pro Gly Cys Gly Lys Gln Phe Ile
   145 150 155 160
   Gln Arg Ser Ala Leu Thr Val His Thr Arg Val His Thr Gly Glu Lys
   165 170 175
   Pro His Met Cys Asp Arg Cys Gly Lys Pro Phe Ser Asp Ser Ser Ser
   180 185 190
   Leu Ala Arg His Arg Arg Ile His Ser Gly Lys Arg Pro Tyr Lys Cys
   195 200 205
   Pro Tyr Ala Asn Cys Gln Lys Thr Phe Thr Arg Arg Thr Thr Leu Thr
   210 215 220
   Arg His Gln Asn His His Thr Gly Thr Ile Glu Glu Ala Ala Ala Glu
   225 230 235 240
   Thr Glu Ala Asn Leu Arg Gln Asn Lys Glu Arg Ala Val Arg Pro Pro
   245 250 255
   Asp Gly Met Tyr Ser Glu Ala Gly Ser Thr Gln Ser Thr Pro Ser Pro
   260 265 270
   Gly Gln Arg His Ser Leu Ser Pro Gly Asn Glu Leu Pro Pro Met Tyr
   275 280 285
   Leu Pro Arg Gln Met Ser Asp Tyr Tyr Met Gly Asn Thr Pro Ile Pro
   290 295 300
   Ala His Leu Arg Gly Asp Phe Gln Gln Ala Ser Pro Arg Ala Ser Pro
   305 310 315 320
   Ser Thr Ser Ser Pro Pro Leu Ser Thr Tyr Ser Ser Val Pro His Ala
   325 330 335
   Arg Pro Ser Met Thr Ser His Pro Ser Gly Tyr Gly Pro Pro Gln Pro
   340 345 350
   Val Glu Pro Pro Ala Asn Ser Asp Pro Arg Pro Asn Ser Val Ala Gly
   355 360 365
   Ser Pro His Ile Thr Ser Met Gly Trp Ala Ser Pro Thr Leu His Ser
   370 375 380
   Ile Gly Ser Pro Asp Ser Ala Ser Ala Thr Glu Tyr Ser Tyr Pro Glu
   385 390 395 400
   Pro Ser Ala Pro Ser Tyr Pro Ser Gln Ile Pro Pro His Met Tyr Phe
   405 410 415
   Pro Asn Ser Thr Ile Arg Arg Pro Gln Ser Thr Glu Pro Glu Asn Tyr
   420 425 430
   Glu Pro Lys Pro Lys Leu Asn Gly Asp Ser Trp Thr Thr Pro Val
   435 440 445
<210> 25
   <211> 368
   <212> PRT
   <213> Artificial Sequence
<220>
   <223> altbrAB09078.1
<400> 25
   Met Gln Gln Thr Ser Tyr Tyr Ser Pro Asn Met Pro His Pro Val Ser
   1               5                   10                  15
   Tyr Ala Gln Ser His Asp Ile Gln Gln Leu Pro Thr Leu Pro Asp Val
   20 25 30
   Arg His Val Phe Asn Pro Asn Val Lys Ser Glu Gly Thr Ser Pro Ala
   35 40 45
   Gln Ser Asn Gln Met Tyr Ala Asn Gln Ser Tyr Gly Thr Glu Leu Lys
   50 55 60
   Arg Ser Thr Ser Glu Pro Thr Glu Gly Ser Ser Thr Asn Phe Ala Thr
   65 70 75 80
   Asp Val Asp Thr Leu Met Arg Ala Ile Gln Ala Lys Gln Thr Asn Leu
   85 90 95
   Pro Gln Glu Asn Asp Gln Lys Asp Asp Ala Ala Lys Thr Ala Gln Lys
   100 105 110
   Pro Arg Lys Arg Tyr Gln Cys Thr Val Pro Asn Cys Asn Lys Ser Phe
   115 120 125
   Tyr Gln Lys Thr His Leu Glu Ile His Ile Arg Ala His Thr Gly Asp
   130 135 140
   Lys Pro Phe Pro Cys Lys Ala Pro Gly Cys Gly Gln Ser Phe Ser Gln
   145 150 155 160
   Leu Gly Asn Leu Lys Thr His Glu Arg Arg His Thr Gly Glu Arg Pro
   165 170 175
   Tyr Ser Cys Asp Ile Cys Gly Lys Thr Phe Ala Gln Arg Gly Asn Val
   180 185 190
   Arg Ala His Lys Ile Val His Gln Gln Ile Lys Pro Phe Ser Cys Lys
   195 200 205
   Leu Asp Asp Cys Gly Lys Gln Phe Thr Gln Leu Gly Asn Leu Lys Ser
   210 215 220
   His Gln Asn Lys Phe His Ala Ala Thr Leu Arg Tyr Leu Thr Thr Lys
   225 230 235 240
   Phe Ala Thr Ile Asn Gln Gly Asp Tyr Val Ser Gln Glu Asp Lys Glu
   245 250 255
   Leu Trp Glu Tyr Phe Ala Ser Leu Tyr Lys Asn Ser Asn Lys Gly Ile
   260 265 270
   Lys Gly Arg Gly Lys Asp Arg Arg Ile Ser Ala Met Ser Ser Ser Ala
           275                 280                 285
   Ser Ser Tyr Pro Ser Ser Tyr Ala Ala Met Pro Met Ala Ser Met Ser
       290                 295                 300
   Arg Gly Tyr Ala Ser Ser Phe His Gln His Ser Ser Asp Arg Ser Ser
   305                 310                 315                 320
   Arg Cys Ser Ser Met Ser Ser Asp Thr Ile Pro Ile Gln Arg Ala Asp
                   325                 330                 335
   Ser Ala Tyr Asp Phe Ser Ala Pro Met His Asn Gly Tyr His Gln Pro
               340                 345                 350
   Gln Gly Asn Gly Tyr Asp Asp Met Val Phe Pro Glu Arg Lys Met Tyr
           355                 360                 365
<210> 26
   <211> 467
   <212> PRT
   <213> Artificial Sequence
<220>
   <223> tritoTESG_05283T0
<400> 26
   Met Asp Ile Thr Thr Ile Leu Asn Lys Lys Ala Ser Val Ser Ile Val
   1               5                   10                  15
   Ala Ala Asp Val His Gln Leu Gln Gln His Leu Ala Gln Ala Gln Ser
               20                  25                  30
   Gln Ser Gln Thr Asp Asp Ala Val Lys Ser Arg Ser Pro Ser Glu Leu
           35                  40                  45
   Gly Ala Ser Glu His His Arg Ser Ala Ser Ala Pro Pro Ser Glu His
       50                  55                  60
   His His His His His His His His Gln Pro Thr Ser Ser Phe Pro Ser
   65                  70                  75                  80
   Ser Thr Gln Ser Leu Pro Gln Met Ala His Leu Ala Gln Tyr His Val
                   85                  90                  95
   Gln Ala Gln Ala Asn His Ala Gly Thr Ser Pro Gly Tyr Ala His Ser
               100                 105                 110
   Ala His Gly Ser Asp Tyr Gly Arg Ser Ser Thr Ser Met Arg Pro Thr
           115                 120                 125
   Gly Leu Pro Ala Leu Lys Thr Phe His Cys Gln Thr Cys Ser Lys Gly
       130                 135                 140
   Phe Ala Arg Arg Ser Asp Leu Ala Arg His Glu Arg Ile His Ser Gly
   145                 150                 155                 160
   Ile Arg Pro His Ala Cys Asp Trp Pro Gly Cys Gly Lys Gln Phe Ile
                   165                 170                 175
   Gln Arg Ser Ala Leu Thr Val His Thr Arg Val His Thr Gly Glu Lys
               180                 185                 190
   Pro His Met Cys Asp Arg Cys Gly Lys Pro Phe Ser Asp Ser Ser Ser
           195                 200                 205
   Leu Ala Arg His Arg Arg Ile His Ser Gly Lys Arg Pro Tyr Lys Cys
       210                 215                 220
   Pro Tyr Ala Asn Cys Gln Lys Thr Phe Thr Arg Arg Thr Thr Leu Thr
   225                 230                 235                 240
   Arg His Gln Asn His His Thr Gly Thr Ile Glu Glu Ala Ala Ala Glu
                   245                 250                 255
   Thr Glu Ala Asn Leu Arg His Ser Lys Asp Arg Ser Thr Ala Ser Arg
               260                 265                 270
   Pro Ser Glu Tyr Ser Glu Pro Gly Ser Ala His Ser Thr Pro Ser Pro
           275                 280                 285
   Ala Gln Arg Pro Ser Leu Ser Pro Gly Asn Glu Leu Pro Pro Leu Asn
       290                 295                 300
   Leu Ser Ser Arg Pro Ser Ala Ala Gly Gly Pro Thr Ser Ala Gly Gly
   305                 310                 315                 320
   Ser Ala Asp Tyr Tyr Thr Ser Leu Pro Pro His Leu Arg Thr Asp Phe
                   325                 330                 335
   Ala Asn Ser Ser Pro Arg Ala Ser Pro Thr Ser Pro Ser Pro Thr Leu
               340                 345                 350
   Thr Ser Leu Ser Gly Pro Ala Ser Phe His His His Asn His Asn Arg
           355                 360                 365
   Pro Ser Leu Thr Ser His Pro Ser Gly Tyr Ala Pro Pro Gln Pro Leu
       370                 375                 380
   Glu Pro Pro Ala Asn Ser Asp Pro Arg Pro Asn Ser Val Ala Gly Ser
   385                 390                 395                 400
   Pro His Leu Ser Ser Val Gly Trp Ala Ser Pro Thr Leu Ser Ser Met
                   405                 410                 415
   Pro Ser Pro Ser Ser Ala Ser Ala Pro Glu Tyr Pro Tyr Pro Glu Pro
               420                 425                 430
   Ser Ser His Gln Tyr Pro Gly Ser Ile Thr Pro His Met Phe Phe Pro
           435                 440                 445
   Arg Arg Pro Gln Ser Ser Glu Pro Glu Asn Tyr Glu Leu Lys Pro Lys
       450                 455                 460
   Leu Glu Leu
   465
<210> 27
   <211> 475
   <212> PRT
   <213> Artificial Sequence
<220>
   <223> mycpoestExt_fgenesh1_kg.C_4_t10200
<400> 27
   Met Gln Tyr Glu Val Pro Met Pro Leu Ser Met Asn His Asp Met His
   1               5                   10                  15
   Thr Pro Asn Gly Asn Tyr Leu Gly Gln Asn Leu Tyr Val Pro Thr Ala
               20                  25                  30
   Asn Arg Ile Lys Ser Glu Asn Gly Ser Asp Arg Gly Val Ser Pro His
           35                  40                  45
   Ser Ser Glu His Ser Ser Arg Tyr Ser Ser Thr Ala Pro Gln Asn Ser
       50                  55                  60
   Val Ala Tyr Gln Gln Ile Ala Ala Gln Leu Thr Asn Gly Met Arg Tyr
   65                  70                  75                  80
   Pro Ser Pro Ser Met Pro Gln Ser Asn Ser Met Pro Met Leu Gln Gln
                   85                  90                  95
   His Tyr Gln Pro Thr Gly Thr Pro Asp Ser Asn Tyr Pro Gln His Thr
               100                 105                 110
   Pro Thr Leu Gly Ala Thr Gln Gln Ser Thr Pro Gln Glu Pro Ser Pro
           115                 120                 125
   Met Glu Gly Ala Arg Thr Ser Thr Gly Ser Ser Gly Leu Pro Lys Ala
       130                 135                 140
   Phe Ala Cys Ser Thr Cys Asn Lys Gly Phe Ala Arg Arg Ser Asp Leu
   145                 150                 155                 160
   Ala Arg His Glu Arg Ile His Ser Gly Val Arg Pro His Val Cys Asp
                   165                 170                 175
   Tyr Ala Gly Cys Gly Lys Gln Phe Ile Gln Arg Ser Ala Leu Thr Val
               180                 185                 190
   His Ser Arg Val His Thr Gly Glu Lys Pro His Met Cys Glu Arg Cys
           195                 200                 205
   Gly Lys Pro Phe Ser Asp Ser Ser Ser Leu Ala Arg His Arg Arg Ile
       210                 215                 220
   His Ser Gly Lys Arg Pro Tyr Lys Cys Pro Tyr Ala Asp Cys Gln Lys
   225                 230                 235                 240
   Thr Phe Thr Arg Arg Thr Thr Leu Thr Arg His Gln Asn His His Thr
                   245                 250                 255
   Gly Thr Ile Glu Glu Ser Glu Ala Ala Thr Ala Ala Ala Leu Ala Ser
               260                 265                 270
   Arg Val Ser Met Gln Gly Gln Arg Ser Arg Gly Ser Asp Asp Glu Asn
           275                 280                 285
   Asp Phe Ser Asp Asn Gly Lys Ser Pro Leu Pro Gln Pro Asp Arg Gln
       290                 295                 300
   Ser Thr Asn Ser Pro Ala Ala Gly Met Asn Asn Met Pro Thr Leu Gln
   305                 310                 315                 320
   Arg Gln Thr Ser Glu Phe Tyr Met Asn Ala Ile Ser Gly Gly Met Ala
                   325                 330                 335
   Ala Val Pro Pro His Met Arg Ser Gln Val Gln Ala Ser Pro Arg Ala
               340                 345                 350
   Gln Ser Pro Gln Gln Tyr Ser Met Pro Gly Asn Asn Gly Thr Pro Gln
           355                 360                 365
   Pro Arg Ile Thr Ser Ile Ser Ala Ala Gly Tyr Asn Gly Pro Pro Gln
       370                 375                 380
   Ile Met Glu Pro Pro Thr Thr Asn Gly His Gln Gln Thr Gly Ser Gly
   385                 390                 395                 400
   His Asn Ser Pro His Leu Asn Gly Trp Gln Ser Pro His Asn Gly Met
                   405                 410                 415
   Gly Pro Gly Gln His Gln Thr Thr Asp Tyr Ser Tyr Gln Asp Ala Ser
               420                 425                 430
   Gly Tyr Pro Ser His Ser Val Asn Ala Met His Ala Tyr Tyr Gln Ala
           435                 440                 445
   Pro Asn Val Ser Arg Pro His Ser Thr Gly Pro Val Asp Tyr Ser Ser
       450                 455                 460
   Gly Leu Arg Gln Asp Val Trp Thr Gln Gln Pro
   465                 470                 475
<210> 28
   <211> 482
   <212> PRT
   <213> Artificial Sequence
<220>
   <223> thitefgenesh_kz1_kg.chromosome_1_#_3815_#_28091
<400> 28
   Met Ser Gln Ser Pro Ser Ser Gly Gly Ala Met Gly Ile Gly Ser Val
   1               5                   10                  15
   Leu Asn Ile Lys Ala Glu Ser Gly Met Leu Gln Pro Gln Gln Gly Leu
               20                  25                  30
   Ala Pro Glu Gln Gln Gln Gln Gln Gln Gln Gln Glu Gln Gln Gln Gln
           35                  40                  45
   Gln Gln Gln Gln Pro Gln Pro Gln Pro Gln Gln Gln Gln Gln Gln Gln
       50                  55                  60
   Gln Gln Gln Pro Ser Arg Pro Ala Gln Thr His Gln Ile Gln Gly Asp
   65                  70                  75                  80
   Gln Pro Asp Ala Ser His Gly Ser Glu Gln Ser Arg Tyr Ser Gly Pro
                   85                  90                  95
   Met Asn Ala Ser Tyr Pro Ser Pro Thr Ala Met Ala Ala Ala Pro Leu
               100                 105                 110
   Pro Pro Val Pro Ser Ala Asn Met Ala Pro Ala Pro Met Asn Ser Ala
           115                 120                 125
   Met Leu His Pro Asp Leu Pro Pro Glu Leu Val Ala Gly Ile Pro Pro
       130                 135                 140
   Gly Gly Tyr Ala Pro Ala Glu Ala Arg Ala Gln Pro Ala Lys Ser Phe
   145                 150                 155                 160
   Pro Cys Gly Thr Cys Gly Lys Pro Phe Thr Arg Arg Ser Asp Leu Ala
                   165                 170                 175
   Arg His Glu Arg Ile His Ser Gly Ile Arg Pro His Ala Cys Asp Tyr
               180                 185                 190
   Pro Gly Cys Asp Lys Arg Phe Ile Gln Arg Ser Ala Leu Thr Val His
           195                 200                 205
   Ser Arg Val His Thr Gly Glu Lys Pro His Gln Cys Glu Arg Cys Gly
       210                 215                 220
   Lys Pro Phe Ser Asp Ser Ser Ser Leu Ala Arg His Arg Arg Ile His
   225                 230                 235                 240
   Ser Gly Lys Arg Pro Tyr Lys Cys Pro Tyr Glu Asp Cys Gln Lys Thr
                   245                 250                 255
   Phe Thr Arg Arg Thr Thr Leu Thr Arg His Gln Asn His His Val Gly
               260                 265                 270
   Thr Val Glu Asp Ser Ala Arg Ala Arg Ala Glu Ala Leu Ala Gln Gly
           275                 280                 285
   Ala Ser Ala Ala Ala Ala Ala Ala Ala Arg Asn Lys Ser Arg Ser Ser
       290                 295                 300
   Ser Glu Gln Ala Ser Asn His Glu Ser Pro Leu Thr Thr Pro Ser Pro
   305                 310                 315                 320
   Ala Gln Gly Ala Met Ser Met Ser Pro Ser Ala Asp Leu Ala Asn Met
                   325                 330                 335
   Asn Ser Met Gln Tyr Leu Ala Asn Asn Ser Leu Pro Pro His Leu Arg
   340 345 350
   Gly Asp Val His Val Gly Ser Pro Ser Pro Thr Ala Ser Thr Gly Tyr
   355 360 365
   Asn Asn Gly Met Arg Pro Thr Ser His Pro Thr Gly Tyr Ala Pro Pro
   370 375 380
   Gln Thr Met Glu Pro Ser Ile Glu His Gln Gln Gly Pro Gly Ser Val
   385 390 395 400
   Ala Gly Ser Pro His Val Ala Ser Val Gly Trp Pro Ser Pro Gly Pro
   405 410 415
   Val Gly Ser Pro Thr Gln Ser Pro Asn Gly Asn Gly Tyr Thr Tyr Pro
   420 425 430
   Asp Pro Ser Asp Val Tyr Gln Ser Gly Thr Pro Ile Ala Gln Met Phe
   435 440 445
   Tyr Asn Ser Ala Pro Ala Thr Arg Arg Pro Gly Ser Ala Glu Pro Gly
   450 455 460
   Asn Pro Ala Tyr Asp Ala Lys Gly Arg Gln Ala Glu Leu Trp Thr Asn
   465 470 475 480
   Ala Gln
<210> 29
   <211> 388
   <212> PRT
   <213> Artificial Sequence
<220>
   <223> VDAG_08452
<400> 29
   Met Ser Pro Ser Pro Thr Pro Gln Pro Trp Asp Gln Trp Ser Gln Gln
   1               5                   10                  15
   Gln Gln Gln Gln Gln Gln Gln Gln Gln Gln Pro Gln His His Ser Gly
               20                  25                  30
   Tyr Pro Leu Met Asp Gln Ser Val Phe Pro Gln Tyr Ala Ser Gln Pro
           35                  40                  45
   Thr Gln Tyr Asp Pro Arg Thr Ser Gln Tyr Asp Pro Arg Thr Val Val
           50                  55                  60
   Thr Ser Gly Pro Leu Val Ala Pro His Met Ala Thr His Tyr Val Thr
   65                  70                  75                  80
   Ala Pro Ala Tyr Gly Ala Ser Pro Ile Ser Asn Met Lys Pro His Phe
                   85                  90                  95
   Pro Ala Gln Thr His Tyr Ser Tyr Gly Gln Tyr Asp Gln Pro Ala Gly
               100                 105                 110
   His Leu Ala Met Ala Tyr Ser Ser His Pro Ala Ser Pro Met Thr Pro
           115                 120                 125
   Gly Pro Met Asn Thr Lys Asp Ile Thr Tyr Asn Lys Pro Ala Leu Gln
       130                 135                 140
   Lys Arg Lys Asp Lys Glu Glu Leu Ala Ser Ser Asn Ser Ser Glu Ala
   145                 150                 155                 160
   Gly Ala Gly Ser Val Lys Ser Glu Pro Ser Ser Pro His Pro Ser Ser
                   165                 170                 175
   Thr Asp Gly Ser Gln Glu Thr Thr Asp Lys Val Lys Lys Tyr Arg Cys
               180                 185                 190
   Pro Tyr Asp Gly Cys Pro Lys Ser Phe Gln Gln Ser Thr His Leu Glu
           195                 200                 205
   Thr His Lys Arg Ala His Thr Gly Asp Lys Pro Tyr Lys Cys Glu Trp
       210                 215                 220
   His Gly Cys Gly Arg Arg Phe Ser Gln Pro Gly Asn Leu Lys Thr His
   225                 230                 235                 240
   Thr Arg Leu His Thr Gly Glu Arg Pro Phe Glu Cys Glu Met Cys Gly
                   245                 250                 255
   Thr Cys Phe Ala Gln Arg Gly Asn Leu Thr Ala His Lys Ala Thr His
               260                 265                 270
   Ser Lys Thr Lys Pro Phe Val Cys Lys Leu Asp Thr Cys Asn Lys Cys
           275                 280                 285
   Phe Thr Thr Arg Gly Asn Leu Lys Asn His Gln Asn Lys Tyr His Lys
       290                 295                 300
   Glu Thr Ile Ala Gln Leu Val Asp Trp Ile Ile Ser Leu Thr Asp Val
   305 310 315 320
   Asp Ala Leu Ser Pro Lys Asp Arg Asp Leu Leu Trp Tyr Phe Ser Asn
   325 330 335
   Ile Tyr Lys Asn Ser Asn Lys Gly Ile Lys Gly Arg Gly Arg Asp Arg
   340 345 350
   Arg Val Ser Glu Val Arg Met Gly Lys Thr Lys Leu Asn Ser Pro Gly
   355 360 365
   Met Gly Leu Pro Arg Ser Lys Met Leu Asp Ser Ser Phe Ser Arg Leu
   370 375 380
   Gly Leu Gly Phe
   385
<210> 30
   <211> 335
   <212> PRT
   <213> Artificial Sequence
<220>
   <223> phlgiestExt_Genemarkl.C_10108
<400> 30
   Met Arg Pro Ser Lys Leu Lys Arg Pro Ile Leu Leu Lys Cys Ala Phe
   1               5                   10                  15
   Cys Ile Leu Glu Asn Pro Cys Glu Pro Val Lys Thr Trp Gly Arg Arg
               20                  25                  30
   Gly Asp Leu Asn Arg His Met Arg Lys His Gly Asp Val Lys Pro Tyr
           35                  40                  45
   Tyr Cys Thr Arg Cys Val Lys Gly Phe Val Gln Lys Ser Ala Met Glu
       50                  55                  60
   Thr His Met Asn Thr His Thr Gly Ala Lys Pro His Ala Cys Pro Val
   65                  70                  75                  80
   Ser Gly Cys Gly Lys Lys Phe Gly Asp Pro Ser Ser Arg Thr Arg His
                   85                  90                  95
   Lys Asn Glu Ala His Ser Ser Ile Ala Ala Phe Arg Cys Pro Glu Val
               100                 105                 110
   Gly Cys Pro Thr Arg Ile Lys Arg Lys Gly Thr Phe Ile Lys His Leu
           115                 120                 125
   Thr Val His His Asn Trp Leu Thr Arg Pro Thr Glu Gln Gln Leu Met
       130                 135                 140
   Asp Met Ile Val Tyr His Ala Lys Gly Glu Ser Ala Lys Gln Glu Ser
   145                 150                 155                 160
   Glu Gly Ala Lys Gln Glu Gly Gln Gly Ala Lys Gln Glu Val Gln Ser
                   165                 170                 175
   Ala Lys Gln Glu Ser Gln Val Val Lys Gln Glu Thr Gln Val Val Lys
               180                 185                 190
   Gln Glu Thr Gln Val Val Lys Gln Glu Ser Pro Ser Ala Lys Gln Glu
           195                 200                 205
   Ser Val Glu Pro Ser Val Leu Asp Ala Leu Val Ser Arg Ser Ser Pro
       210                 215                 220
   Ser Pro Thr Thr Arg Arg Leu Ser Ser Ser Leu Leu Pro Leu Asn Ala
   225                 230                 235                 240
   Pro Tyr Ser Phe Asp Lys Tyr Thr Lys Leu Tyr Pro Glu Pro Pro Ser
                   245                 250                 255
   Tyr Asp Ser Tyr His Thr Gly Thr Thr Val Tyr Ser Gln Pro Val Asp
               260                 265                 270
   Pro Trp Gln Glu Leu Ser Ser Tyr Ala Asp Gly Leu Leu Leu Ser Ser
           275                 280                 285
   Thr Ser Ser Ser Ser Leu Pro Val Ser Thr Ser Met Ile Ser Trp Asp
       290                 295                 300
   Ser Asp Pro Asp Met Met Val Tyr Pro Thr Tyr Arg Ser Gly Tyr Thr
   305                 310                 315                 320
   Ser His Pro Pro Leu Ser Tyr Asp Trp Ser Leu Gly Leu Leu Gly
                   325                 330                 335
<210> 31
   <211> 456
   <212> PRT
   <213> Artificial Sequence
<220>
   <223> AO090701000019
<400> 31
   Met Glu Thr Ala Glu Pro Val Ser Tyr Glu Phe Pro Gly His Thr Ile
   1               5                   10                  15
   Gly Ala Val Ala Pro Arg Arg Met Met Thr Ser Asn Leu Gly His Asn
               20                  25                  30
   Phe Pro Phe Tyr Ala Thr Pro Ala Ala Ser Phe Pro Leu Pro Phe His
           35                  40                  45
   Gln Ser Ser Ser Thr Ala Tyr Gly Phe Gly His Ala Leu Asn His His
       50                  55                  60
   His His Asn His His Gln Pro Ser Tyr Pro Gln Phe Phe Val Ala Ser
   65                  70                  75                  80
   His Glu Ser Ile Asn Ser Gln Pro Met Arg Leu Ser Ser Glu Pro Pro
                   85                  90                  95
   Pro Val Gln Ser Ile Pro Asp Ile Arg Pro Ala Lys Asn Ala Val Asn
               100                 105                 110
   Arg Val Ser Arg Asp Pro Leu Val Lys Asn Asp Pro Ser Ser Asn Thr
           115                 120                 125
   Gln Gln Thr Pro Met Ala Arg Ser Ser Thr His Gly Ala Ala Ala Gln
       130                 135                 140
   Ser Lys Ser Pro Ser Val Ser Glu Ile Glu Phe Thr Thr Glu Val Asp
   145                 150                 155                 160
   Ile Leu Met Lys Ala Ile Gln Ser Arg Asn Ser Val Gln Pro Pro Asn
                   165                 170                 175
   Thr Gln Ser Leu Pro Pro Leu Gln Gln Leu Thr His Arg Gly Cys His
               180                 185                 190
   Gly Tyr Pro Gln Thr Phe Ser Leu His Pro Ser Gly Asn Thr Arg Cys
           195                 200                 205
   Asn Met Met Ala Glu Glu Val Gln Ser Arg Ser Gly Lys Lys Arg Lys
       210                 215                 220
   Tyr Val Cys Thr Leu Pro His Cys Gly Lys Ser Phe Ala Gln Lys Thr
   225                 230                 235                 240
   His Leu Asp Ile His Thr Arg Ala His Thr Gly Asp Lys Pro Phe Ile
                   245                 250                 255
   Cys Lys Glu Pro Ser Cys Gly Gln Arg Phe Ser Gln Leu Gly Asn Leu
               260                 265                 270
   Lys Arg Glu Lys Thr Asp Ser Asn Pro Gly Ala His Lys Gln Thr His
           275                 280                 285
   Gln Arg Arg His Thr Gly Glu Lys Pro Phe Ser Cys Asp Ile Cys Gln
       290                 295                 300
   Lys Arg Phe Ala Gln Arg Gly Asn Val Arg Ala His Lys Ile Thr His
   305                 310                 315                 320
   Gln His Ala Lys Pro Phe Thr Cys Leu Leu Asp Asp Cys Gly Lys Gln
                   325                 330                 335
   Phe Thr Gln Leu Gly Asn Leu Lys Ser His Gln Asn Lys Phe His Ala
               340                 345                 350
   Thr Thr Leu Arg Asp Leu Thr Leu Lys Phe Ser Gln Val Thr Ile Gly
           355                 360                 365
   Asp Pro Met Ser Pro Gln Asp Arg Lys Leu Trp Glu Tyr Phe Ala Thr
       370                 375                 380
   Leu Tyr Lys Asn Ser Asn Lys Gly Ile Lys Gly Arg Gly Lys Asp Arg
   385                 390                 395                 400
   Arg Ile Ser Pro Thr Ser Arg Ser Gly Pro Gly Lys Arg His Gln Thr
                   405                 410                 415
   Leu Gly Asn Asn Asp Asp Lys Leu Gln Arg Pro Ile Tyr Glu Glu Ser
               420                 425                 430
   Ser Val Tyr Thr Gly Gly Ser Ser Ser Asp Glu Glu Asp Ala Glu Ala
           435                 440                 445
   Tyr Tyr Ile Asp Arg Gln Asn His
       450                 455
<210> 32
   <211> 457
   <212> PRT
   <213> Artificial Sequence
<220>
   <223> BC_P076400.1
<400> 32
   Met Asp Ile Lys Glu Ile Val Asn Ser Lys Gly Ala Lys Gly Ala Ala
   1               5                   10                  15
   Ala Ala Ala Ala Ala Ser Ala Ser Gly Gln Ala Gln Ala Gln Ala Gln
               20                  25                  30
   Asp Leu His Leu Leu Gln Thr Ile Ser Gln Ala Asn Gly Met Arg Met
           35                  40                  45
   Ser Asp Thr Gly Ser Glu Arg Gly Thr Ser Pro His Gly Ser Glu His
       50                  55                  60
   Ser Gln Tyr Ser Thr Pro Arg Met Gly Met Asn Val Met Asn Gly Ala
   65                  70                  75                  80
   Pro Asn Gly Met Arg Tyr Pro Ser Pro Pro Gly Met Gln Asn Ala Met
                   85                  90                  95
   Pro Met Met Gln Gln Pro Tyr Arg Pro Asp Ile Pro Phe Asp Asn Ser
               100                 105                 110
   Met Met Pro Thr Gln Gln Gln Asp Asn Thr Arg Gln Arg Gln Pro Gly
           115                 120                 125
   Asp Val Thr Val Thr Lys Ala Phe Pro Cys Ser Thr Cys Gly Lys Gly
       130                 135                 140
   Phe Ala Arg Arg Ser Asp Leu Ala Arg His Glu Arg Ile His Ser Gly
   145                 150                 155                 160
   Val Arg Pro His Val Cys Asp Tyr Pro Gly Cys Gly Lys Gln Phe Ile
                   165                 170                 175
   Gln Arg Ser Ala Leu Thr Val His Leu Arg Val His Thr Gly Glu Lys
               180                 185                 190
   Pro His Thr Cys Glu Arg Cys Ala Lys Pro Phe Ser Asp Ser Ser Ser
           195                 200                 205
   Leu Ala Arg His Arg Arg Ile His Ser Gly Lys Arg Pro Tyr Lys Cys
       210                 215                 220
   Pro Tyr Ala Asp Cys Gln Lys Thr Phe Thr Arg Arg Thr Thr Leu Thr
   225                 230                 235                 240
   Arg His Gln Asn His His Thr Gly Thr Val Glu Glu Ala Ala Ala Ala
   245 250 255
   Thr Ala Ala Ala Leu Ala Thr Arg Pro Gly Asn Asn Arg Pro Ser Arg
   260 265 270
   Gln Arg Ser Asp Gly Glu Ser Tyr Ser Asn Asn Gly Ser Pro Met Ser
   275 280 285
   Thr Pro Ser Pro Gly Arg His Leu Ser Ala Ser Pro Ser Ser Asp Met
   290 295 300
   Ala Pro Met Asn Ser Met Gln Arg His Pro Gly Asp Tyr Gly Tyr Met
   305 310 315 320
   Asn Asn Ser Ser Leu Pro Gly His Leu Arg Gly Asp Tyr Gly Val Pro
   325 330 335
   Ser Gln Pro Pro Pro Val Ser Thr Ala Phe Ala Asn Gly Met Gln Gln
   340 345 350
   Gln Arg Pro Thr Ser His Pro Thr Gly Tyr Gly Pro Pro Asn Ile Leu
   355 360 365
   Glu Pro Pro Ala Asn Met Glu Gln Arg Gln Pro Gly Ser Ala Asn Gly
   370 375 380
   Ser Pro His Met Thr Asn Met Gly Trp Gln Ser Pro Ser His Met Gly
   385 390 395 400
   Ser Pro Ser His Asn Asn Asn Tyr Val Tyr Pro Asp Pro Asp Pro Tyr
   405 410 415
   Gly Ser Gly Ala Ala Ile Gly Gln Met Tyr Tyr Pro Asn Ser Asn Met
   420 425 430
   Arg Arg Pro Gln Ser Thr Glu Pro Glu Gln Tyr Asp Met Lys Pro Arg
   435 440 445
   Met Gly Asp Leu Trp Ala Thr Ala Gln
   450 455
<210> 33
   <211> 472
   <212> PRT
   <213> Artificial Sequence
<220>
   <223> NCU00038
<400> 33
   Met Lys Arg Lys Gln Asp Asp Glu Arg Leu Asp Gly His Asp Gly Pro
   1 5 10 15
   Asp His Ala Gln Val Gln Ser Lys Lys Arg Ala Thr Asp Trp Val Gln
   20 25 30
   Gln Gln Gln Glu Glu Glu Leu Ser Gln Val Glu Gln Leu Glu Ala Pro
   35 40 45
   Ala Leu Pro Ser Gln Ser Arg Ser Pro Pro Ala Gly Ala Glu Ala Ala
   50 55 60
   Ala Ser Asp Gly Asn Gly Asn Asp Ser Leu Glu Leu Asp Pro Asp Ser
   65 70 75 80
   Ala Ser Val Ala Ser Val Ala Asn Thr Ser Leu Thr His Ala Asp Pro
   85 90 95
   Thr Thr Thr Val Ala Ala Thr Ser Ile Asn Thr Thr Thr Thr Arg Ala
   100 105 110
   Ser Arg Arg Phe Pro Ser Asp Leu Lys Thr Ile Arg Cys Thr Phe Ala
   115 120 125
   Gly Cys Thr Lys Thr Phe Asn Arg Pro Ala Arg Leu Ala Ala His Leu
   130 135 140
   Arg Ser His Thr Asn Asp Arg Pro Phe Lys Cys Pro Tyr Asp Asp Cys
   145 150 155 160
   Asp Lys Asp Tyr Leu Glu Glu Lys His Leu Ser Gln His Ile Lys Gly
   165 170 175
   Ser His Thr Asn Asp Arg Lys Tyr Thr Cys Pro Glu Pro Gly Cys Gly
   180 185 190
   Lys Ser Phe Val Thr Asn Thr Arg Leu Arg Arg His Ala Leu Val His
   195 200 205
   Glu Gly Ala Asp Arg Tyr Arg Cys Arg Gly Tyr Gly Asp Cys Val Gln
   210 215 220
   Ser Phe Arg Lys His Gln Thr Leu Gln Arg His Ile Arg Thr Val His
   225 230 235 240
   Leu Gly Lys Ser Ala Tyr Pro Cys Gly Asn Asp Gly Cys Asp Ala Gly
   245 250 255
   Phe Asp Thr Ala Ser Ala Leu Arg Arg His Val Glu Arg Glu His Gly
   260 265 270
   Asp Leu Lys Phe Trp Cys Asp Glu Cys Asn Ala Glu Gly Glu Gly Asp
   275 280 285
   Asp Ser Gly Gly Arg Arg Val Gly Phe Thr Thr Met Leu Leu Leu Gln
   290 295 300
   Ala His Met Lys Lys Glu His Asn Asn Cys Ala Phe Cys Gly Val Arg
   305 310 315 320
   Cys Gly Thr Gln Ser Asn Met Met Arg His Val Glu Leu Tyr His Ser
   325 330 335
   Ala Lys Thr Val Glu Asp Arg Lys Thr Ile Ala Cys Thr Trp Glu Gly
   340 345 350
   Cys Asp Lys Lys Phe Thr Arg Val Ser Asn Leu Asn Thr His Ile Lys
   355 360 365
   Ser Ala His Glu Gly His Arg Phe Val Cys Gly Gln Thr Asp Thr Tyr
   370 375 380
   Asp Ala Lys Val Ser Glu Ile Ala Asp Trp Asn Phe Ala Glu Glu Gly
   385 390 395 400
   Cys Gly Gln Gly Phe Thr Thr Arg Val Lys Leu Glu Glu His Val Leu
   405 410 415
   His Val His Leu Gly Lys Lys Arg Pro Pro Lys Leu Tyr Pro Val Pro
   420 425 430
   Ser Met Val Ala Gln Ala Gln Gln Ala Gln Gln Ala Leu Leu Asp Thr
   435 440 445
   Arg Asp Leu Ala Cys Pro Ala Arg Gly Thr Gly Ser Tyr Gly Thr Ile
   450 455 460
   Ser Glu Cys Cys Gln Leu Glu Arg
   465 470
<210> 34
   <211> 441
   <212> PRT
   <213> Artificial Sequence
<220>
   <223> aspcaestExt_Genewise1Plus.C_41182
<400> 34
   Met Asp Ile Thr Thr Ile Leu Asn Arg Lys Gly Ser Ala Ala Met Val
   1               5                   10                  15
   Ala Ala Asp Gly Gln Phe Asp His Gln His Leu Val Pro Asn His Ser
               20                  25                  30
   Ile Asp His Thr Ser Ser Pro Lys Met Lys Ser Glu Pro Gly Val Ser
           35                  40                  45
   Asp Ala Gly Asp Gln Ala Val Leu Ala Tyr Pro Pro His Gly Pro Leu
       50                  55                  60
   Gly Gln Val Pro Thr Met Pro His Asp Met Arg Tyr Ala Pro Gln Ala
   65                  70                  75                  80
   His Pro Ala Pro Gly Leu Pro Leu Leu Gln Asn Pro Phe Val Ser Gly
                   85                  90                  95
   Gly Tyr Ala Gly Ser Ala Gln Ile Pro Asn Ala Gly Ala Pro Gln Gly
               100                 105                 110
   Arg Pro Asp Pro Pro Pro Lys Thr Phe His Cys Gly Thr Cys Gly Lys
           115                 120                 125
   Gly Phe Ala Arg Arg Ser Asp Leu Ala Arg His Glu Arg Ile His Ser
       130                 135                 140
   Gly Val Arg Pro His Ala Cys Asp Trp Pro Gly Cys Gly Lys Gln Phe
   145                 150                 155                 160
   Ile Gln Arg Ser Ala Leu Thr Val His Ser Arg Val His Thr Gly Glu
                   165                 170                 175
   Lys Pro His Met Cys Glu Arg Cys Gly Lys Pro Phe Ser Asp Ser Ser
               180                 185                 190
   Ser Leu Ala Arg His Arg Arg Ile His Ser Gly Lys Arg Pro Tyr Lys
           195                 200                 205
   Cys Pro Tyr Ala Asn Cys Gln Lys Thr Phe Thr Arg Arg Thr Thr Leu
       210                 215                 220
   Thr Arg His Gln Asn His His Thr Gly Thr Ile Glu Glu Ala Ala Ala
   225 230 235 240
   Glu Thr Glu Ala Asn Leu Arg Gln Asn Lys Glu Arg Ala Arg Leu Pro
   245 250 255
   Gly Asp Met Phe Ser Glu His Gly Ser Val His Ser Thr Pro Ser Pro
   260 265 270
   Ala His His Pro Ile Ser Pro Val Gly Asp Leu Pro Pro Leu Asn Met
   275 280 285
   Pro Arg Ser Asp Tyr Tyr Met Pro Asn Gly Ser Ile Pro Ala His Val
   290 295 300
   Arg Gly Gly Phe Thr Gln Ala Ser Pro Arg Ala Ser Pro Thr Ala Thr
   305 310 315 320
   Ser Pro Ser Leu Ser Ser Tyr Gly Ser Ala Pro His Pro Arg Pro Ser
   325 330 335
   Met Thr Ser His Pro Ser Gly Tyr Gly Pro Pro Gln Pro Leu Glu Pro
   340 345 350
   Pro Ala Asn Asn Asp His Arg Pro Asn Ser Val Ser Gly Ser Pro His
   355 360 365
   Met Thr Ser Leu Gly Trp Ala Ser Pro Ser His Gly Ser Ile Pro Ser
   370 375 380
   Pro Gly Ser Ala Thr Asp Phe Tyr Pro Glu Thr Ser Gly Pro Ala Tyr
   385 390 395 400
   Pro Thr Ser Met Pro Pro His Met Tyr Phe Pro Asn Ser Thr Ile Arg
   405 410 415
   Arg Pro Thr Ser Thr Glu Pro Asp Ser Tyr Glu Leu Lys Pro Lys Met
   420 425 430
   Gly Glu Ser Ala Trp Ser Thr Pro Val
   435 440
<210> 35
   <211> 406
   <212> PRT
   <213> Artificial Sequence
<220>
   <223> baucoe_gw1.4.1097.1
<400> 35
   Met Ala Tyr Leu Ala Asn Gln Leu Asn Gly Gly Val Pro Arg Tyr Pro
   1               5                   10                  15
   Ser Pro Ser Met Gln Gln Gln Gly Ala Met Pro Leu Ile Gln His Thr
               20                  25                  30
   Tyr His Pro Asn Ala Gly Ser Asp Gln Ser Tyr Gln Pro Gln Ala Ser
           35                  40                  45
   Met Gly Pro Val Gln Gly Ala Leu Pro Pro Asp Pro Thr Gln Ser Asp
       50                  55                  60
   Gly Thr Gly Arg Pro Asn Gly Ser Gly Leu Pro Lys Ala Phe Ala Cys
   65                  70                  75                  80
   Ser Ser Cys Gly Lys Gly Phe Ala Arg Arg Ser Asp Leu Ala Arg His
                   85                  90                  95
   Glu Arg Ile His Ser Gly Val Arg Pro His Ala Cys Asp Tyr Pro Gly
               100                 105                 110
   Cys Asn Lys Gln Phe Ile Gln Arg Ser Ala Leu Thr Val His Ser Arg
           115                 120                 125
   Val His Thr Gly Glu Lys Pro His Met Cys Glu Arg Cys Gly Lys Pro
       130                 135                 140
   Phe Ser Asp Ser Ser Ser Leu Ala Arg His Arg Arg Ile His Ser Gly
   145                 150                 155                 160
   Lys Arg Pro Tyr Lys Cys Pro Tyr Ala Asp Cys Gln Lys Thr Phe Thr
                   165                 170                 175
   Arg Arg Thr Thr Leu Thr Arg His Gln Asn His His Thr Gly Thr Ile
               180                 185                 190
   Glu Glu Ser Glu Ala Ala Thr Ala Ala Ala Leu Ala Ser Arg Val Asn
           195                 200                 205
   Met Gln Ser Arg Ser Arg Gly Ser Asp Glu Glu Gly Asp Tyr Ser Asp
       210                 215                 220
   Gly Lys Ser Pro Met Pro Gln Gln Pro Asp Arg Val Asn Ser Pro Ala
   225                 230                 235                 240
   Ile Asn Val Asn Gly Val Pro Gln Met Gln Arg Gln Gly Ser Asp Leu
   245 250 255
   Tyr Met Gln Val Met Gly Asn Gly Met Ala Ala Ala Ala Ala Phe Pro
   260 265 270
   Gln His Leu Arg Ser Glu Met Gln Gly Ser Pro Arg Pro Gln Ser Pro
   275 280 285
   Ser Gln Tyr Pro Met Pro Pro Thr Thr Gln Pro Gln Arg Pro Gly Leu
   290 295 300
   Thr Ser Asn Pro Ser Ser Gly Tyr Asn Pro Pro Gln Ile Leu Glu Pro
   305 310 315 320
   Pro Thr Thr Asn Gly Gln Gln Thr Gly Ser Gly Ser Asn Ser Pro His
   325 330 335
   Ile Ser Ala Ala Met Gly Trp Gln Ser Pro His Asn Gly Leu Thr Gln
   340 345 350
   His Gln Gln Ser Asp Tyr Ser Tyr Pro Asp Pro Asn Gly Gln Tyr Ala
   355 360 365
   Thr Ala Asn Ala Ala Ala Met Tyr Tyr Gln Gln Pro Val Gln Arg Pro
   370 375 380
   His Ser Thr Gly Pro Ile Asp Tyr Gln Gln Leu Arg Gly Ala Glu Met
   385 390 395 400
   Trp Ala Gln His Gln Gln
   405
<210> 36
   <211> 406
   <212> PRT
   <213> Artificial Sequence
<220>
   <223> VDAG_01569
<400> 36
   Met Ala Met Gln His Ser His His His Pro Thr Gly Ser Thr Pro Glu
   1               5                   10                  15
   Arg Gln Met His Gln Leu Pro Gln Leu Thr Ala Arg Asn Leu Pro Glu
               20                  25                  30
   Pro Pro Met Asp Arg Ser Gly Ser Pro His Gly Ser Glu His Ser Arg
           35                  40                  45
   Tyr Ser Ala Pro Arg Ser His Met Asp Ser Val Pro Arg Ser Tyr Pro
       50                  55                  60
   Ser Pro Val Pro Met Asn Gly Pro Pro Met Met Gln Ala His Ala Pro
   65                  70                  75                  80
   Pro Gln His Gln His Gln His Gln His Gln His His Ala Gly Val Pro
                   85                  90                  95
   Ile Thr Met Ile Leu Pro Gly Gly Pro Pro Asp Met Asn Ala Gly Met
               100                 105                 110
   His His Tyr Lys Pro Pro Gly Val Pro Val Glu Pro Gln Pro Ala Gln
           115                 120                 125
   Gln Val Lys Ala Tyr Pro Cys Gly Thr Cys Gly Lys Arg Phe Ala Arg
       130                 135                 140
   Arg Ser Asp Leu Ala Arg His Glu Arg Ile His Ser Gly Ile Arg Pro
   145                 150                 155                 160
   His Val Cys Asp Tyr Pro Asn Cys Gly Lys Gln Phe Ile Gln Arg Ser
                   165                 170                 175
   Ala Leu Thr Val His Gln Arg Val His Thr Gly Glu Lys Pro His Met
               180                 185                 190
   Cys Glu Arg Cys Gly Lys Pro Phe Ser Asp Ser Ser Ser Leu Ala Arg
           195                 200                 205
   His Arg Arg Ile His Ser Gly Lys Arg Pro Tyr Lys Cys Pro Tyr Ala
       210                 215                 220
   Asp Cys Gln Lys Thr Phe Thr Arg Arg Thr Thr Leu Thr Arg His Gln
   225                 230                 235                 240
   Asn His His Thr Gly Thr Val Glu Glu Ala Ala Ala Ala Thr Ala Ala
                   245                 250                 255
   Ala Leu Ala Ala Arg Ala Ser Val Lys Pro Gly Ser Thr Arg Ser Asp
               260                 265                 270
   Gly Asp Ser Met Ser Asn Arg Gly Ser Pro Met Thr Thr Pro Ser Pro
           275                 280                 285
   Ala Gln Arg Thr Leu Ser Met Ser Pro Ser Ala Asp Gln Met Gln Arg
   290 295 300
   Ser Asn Ala Glu Phe Gly Tyr Met Gly Asn Thr Ser Leu Pro Val His
   305 310 315 320
   Leu Gln Arg Asp Met His Val Gly Ser Pro Ala Ser Thr Thr Ser Ala
   325 330 335
   Gly Tyr Ser Asn Gly Val Arg Pro Thr Ser His Pro Thr Gly Tyr Gly
   340 345 350
   Met Val Pro Pro Pro Thr Leu Glu Pro Asn Val Glu Gln His Ser Gly
   355 360 365
   Pro Gly Ser Ala Gly Gly Ser Pro His Met Ser Thr Met Gly Trp Gln
   370 375 380
   Ser Pro Ser His Val Ala Ser Pro Ser His Ser Asn Asn Gly Ser Ala
   385 390 395 400
   Tyr Met Tyr Pro Gly Ala
   405
<210> 37
   <211> 283
   <212> PRT
   <213> Artificial Sequence
<220>
   <223> Afu4g11480
<400> 37
   Met Ala Pro Gly Val Gly Arg Asp Phe Asn Cys Ser Trp Glu His Cys
   1               5                   10                  15
   Gly Lys Ser Phe Asn Arg Lys Ser Asp Leu Cys Arg His Tyr Arg Ile
               20                  25                  30
   His Thr Asn Glu Arg Pro Tyr His Cys Thr Val Lys Asp Cys Asn Lys
           35                  40                  45
   Ser Phe Ile Gln Arg Ser Ala Leu Thr Val His Ser Arg Thr His Thr
       50                  55                  60
   Gly Glu Lys Pro His Val Cys Asp His Glu Gly Cys His Lys Ala Phe
   65                  70                  75                  80
   Ser Asp Ser Ser Ser Leu Ala Arg His Arg Arg Ile His Thr Gly Lys
                   85                  90                  95
   Arg Pro Tyr Ile Cys Gln Glu Pro Thr Cys Glu Arg Ser Phe Cys Arg
   100 105 110
   Lys Thr Thr Leu Thr Lys His Gln His Arg Ser His Pro Ser Gly Thr
   115 120 125
   Met Thr Arg Pro Pro Ser Glu Asp Ala Met Ser Glu Gln Ser Tyr Gln
   130 135 140
   Ala Pro Val Ala Ala Pro Ile Pro Asn Asp Gln Tyr Leu Leu Ala Gln
   145 150 155 160
   Gln Gln Tyr Tyr His Gln Pro Ser Thr Pro Ser Gln Glu Phe Tyr Pro
   165 170 175
   Pro Gln Ser Leu Pro Val Thr His Ile Pro Val His Asp Thr Pro Pro
   180 185 190
   Ile Met Thr Gln Ser Val Pro Val Thr Ser Pro Leu Asp Val Gln His
   195 200 205
   Ala Gln Gln Gln Tyr Met Gln Gln Leu Met Gln Gln Arg Tyr Asp Thr
   210 215 220
   Thr Asn Gln Ser Tyr Ile Pro Pro Gly Phe Gln Gln Gln Gln Gln Thr
   225 230 235 240
   Phe Val Pro Gln Pro Met Ala Glu Gly His Pro Leu Met Ile Ser Tyr
   245 250 255
   Asn Gln Asn Phe His Tyr Lys Gln Pro Thr Arg Ile Leu Asn Gln Pro
   260 265 270
   Glu Gly Thr Asp Trp Gly Phe Leu Gly Val Gly
   275 280
<210> 38
   <211> 388
   <212> PRT
   <213> Artificial Sequence
<220>
   <223> crypaestExt_Genewise1.C_80749
<400> 38
   Met Ser Gly His Leu Gln Ala Ala Arg Pro Tyr Gly Ser Pro Thr Ser
   1 5 10 15
   Met His Ala Ser Leu His Met Pro Glu Pro Asn Met Gly Pro Ser Gly
               20                  25                  30
   Met Ala Phe Pro Ser Met Ala Pro Glu Leu Tyr Ala Pro Pro Gln Pro
           35                  40                  45
   Leu Arg Ala Pro Asp Ala Thr Ser Gln Pro Ala Pro Lys Ala Tyr Ala
       50                  55                  60
   Cys Ser Thr Cys Gly Lys Gly Phe Ala Arg Arg Ser Asp Leu Ala Arg
   65                  70                  75                  80
   His Glu Arg Ile His Thr Gly Val Arg Pro His Val Cys Glu Tyr Pro
                   85                  90                  95
   Asn Cys Gly Lys Gln Phe Ile Gln Arg Ser Ala Leu Thr Val His Gln
               100                 105                 110
   Arg Val His Thr Gly Glu Lys Pro His Met Cys Glu Arg Cys Gly Lys
           115                 120                 125
   Pro Phe Ser Asp Ser Ser Ser Leu Ala Arg His Arg Arg Ile His Ser
       130                 135                 140
   Gly Lys Arg Pro Tyr Lys Cys Pro Tyr Ala Asp Cys Gln Lys Thr Phe
   145                 150                 155                 160
   Thr Arg Arg Thr Thr Leu Thr Arg His Gln Asn His His Thr Gly Thr
                   165                 170                 175
   Val Glu Asp Ala Ala Arg Ala Thr Ala Glu Val Leu Ala Arg Asn Ser
               180                 185                 190
   Val Ala Arg Ser Ala Ser Ala Arg Pro Thr Arg Ser Glu Gly Asp Gln
           195                 200                 205
   Ile Ser Asn His Gly Ser Pro Leu Ser Thr Pro Ser Pro Ala Gln Arg
       210                 215                 220
   Thr Met Ser Met Ser Pro Ser Ala Glu Leu Ala Ala Ala Asn Gly Met
   225                 230                 235                 240
   Gln His Gln Tyr Asn Gly Asn Ser Ser Leu Pro Ala His Met Arg Thr
                   245                 250                 255
   Asp Leu His Ser Ser Ser Pro Ala Ala Thr Thr Ser Ser Gly Phe Ser
               260                 265                 270
   Ser Asn Val Arg Pro Thr Ser His Pro Asn Ala Tyr Gly Ala Ala Pro
   275 280 285
   Pro Pro Thr Thr Leu Glu Pro Ser Ile Glu Gly Ser Gln Gly Pro Gly
   290 295 300
   Ser Ala Val Gly Ser Pro His Met Ser Ser Val Gly Trp Ala Ser Pro
   305 310 315 320
   Ser His Val Ala Ser Pro Ala His Ser Asn Ser Gly Gly Asn Ser Ser
   325 330 335
   Tyr Val Tyr Pro Asp Pro Asp Ser Ser Tyr Pro Thr Asn Ala Ala Ala
   340 345 350
   Gln Gly Gln Met Phe Tyr Ser Ala Ala Met Gly Leu Gln Arg Pro Gly
   355 360 365
   Ser Ala Glu Pro Gly Ala Gln Ser Ala Tyr Lys Pro Arg Gln Asn Glu
   370 375 380
   Leu Trp Ala Ala
   385
<210> 39
   <211> 421
   <212> PRT
   <213> Artificial Sequence
<220>
   <223> sc1scSS1G_11373T0
<400> 39
   Met Asp Ile Lys Glu Ile Val Asn Ser Lys Gly Val Lys Gly Ala Ala
   1 5 10 15
   Ala Ala Ala Ala Ala Ser Ala Ser Gly Gln Ala Gln Asp Leu His Leu
   20 25 30
   Leu Gln Ser Ile Ser Gln Ala Asn Gly Leu Arg Met Ser Asp Ala Gly
   35 40 45
   Ser Glu Arg Gly Asn Ser Pro His Gly Ser Glu His Ser Gln Tyr Ser
   50 55 60
   Thr Pro Arg Met Gly Met Asn Gly Met Asn Gly Ala Pro Asn Gly Met
   65 70 75 80
   Arg Tyr Pro Ser Pro Pro Gly Met Gln Asn Ser Met Pro Met Met Gln
                   85                  90                  95
   Gln Pro Tyr Arg Pro Asp Val Ala Phe Asp Asn Gly Met Met Ser Thr
               100                 105                 110
   Gln Gln Gln Asp Asn Thr Arg Gln Arg Gln Pro Gly Asp Ile Thr Val
           115                 120                 125
   Thr Lys Ala Phe Pro Cys Ser Thr Cys Gly Lys Gly Phe Ala Arg Arg
       130                 135                 140
   Ser Asp Leu Ala Arg His Glu Arg Ile His Ser Gly Val Arg Pro His
   145                 150                 155                 160
   Val Cys Asp Tyr Pro Gly Cys Gly Lys Gln Phe Ile Gln Arg Ser Ala
                   165                 170                 175
   Leu Thr Val His Leu Arg Val His Thr Gly Glu Lys Pro His Thr Cys
               180                 185                 190
   Glu Arg Cys Ala Lys Thr Phe Thr Arg Arg Thr Thr Leu Thr Arg His
           195                 200                 205
   Gln Asn His His Thr Gly Thr Val Glu Glu Ala Ala Ala Ala Thr Ala
       210                 215                 220
   Ala Ala Leu Ala Gly Arg Pro Gly Asn Asn Arg Pro Ser Arg Gln Arg
   225                 230                 235                 240
   Ser Asp Gly Glu Ser Tyr Ser Asn Asn Gly Ser Pro Met Ser Thr Pro
                   245                 250                 255
   Ser Pro Gly Arg His Leu Ser Ala Ser Pro Ser Ser Asp Met Ala Pro
               260                 265                 270
   Met Asn Asn Met Gln Arg His Pro Gly Asp Tyr Gly Tyr Met Asn Asn
           275                 280                 285
   Ser Ser Leu Pro Gly His Leu Arg Gly Asp Tyr Gly Val Pro Asn Gln
       290                 295                 300
   Pro Pro Pro Val Ser Thr Ala Phe Thr Asn Gly Met Gln Arg Pro Thr
   305                 310                 315                 320
   Ser His Pro Thr Gly Tyr Gly Pro Pro Ser Ile Leu Glu Pro Pro Ala
                   325                 330                 335
   Asn Met Glu Gln Arg Gln Pro Gly Ser Ala Asn Gly Ser Pro His Met
   340 345 350
   Thr Asn Met Gly Trp Gln Ser Pro Ser Gln Met Ala Ser Pro Ser His
   355 360 365
   Asn Asn Asn Tyr Val Tyr Pro Asp Pro Asp Pro Tyr Gly Ser Gly Ala
   370 375 380
   Ala Met Gly Gln Met Tyr Tyr Pro Asn Ser Asn Met Arg Arg Pro Gln
   385 390 395 400
   Ser Thr Glu Pro Glu Gln Tyr Asp Met Lys Pro Arg Ile Asn Glu Met
   405 410 415
   Trp Ala Thr Ala Gln
   420
<210> 40
   <211> 441
   <212> PRT
   <213> Artificial Sequence
<220>
   <223> cocpoCPAT_02322
<400> 40
   Met Asp Ile Thr Thr Ile Leu Asn Lys Asn Arg Ser Ala Val Ala Ala
   1 5 10 15
   Ala Ala Glu Ala Gln Leu Gln His Gln Leu Ala Gln Thr Ala Gln Ile
   20 25 30
   Lys Ser Arg Ser Pro Ser Glu Leu Gly Ser Glu His Asp Ala Gln Gln
   35 40 45
   Pro Gly Arg His Ser Glu Pro Tyr His Pro Ala His Gln Pro Ile Gln
   50 55 60
   Leu Pro Thr Ile Ser Gln Tyr His Ser Pro Leu His Thr Asn His His
   65 70 75 80
   Ala Ser Met Met Arg Gly Asp Tyr Ala Gln Asn Gly His Glu Asn Met
   85 90 95
   Phe Arg Asn Met Pro Thr Ser Asn Ser Gly Pro Gly Arg Gly Thr Gly
   100 105 110
   Glu Pro Ala Pro Lys Ser Phe His Cys Gln Thr Cys Ser Lys Gly Phe
           115                 120                 125
   Ala Arg Arg Ser Asp Leu Ala Arg His Glu Arg Ile His Ser Gly Ile
       130                 135                 140
   Arg Pro His Val Cys Asp Trp Pro Gly Cys Gly Lys Gln Phe Ile Gln
   145                 150                 155                 160
   Arg Ser Ala Leu Thr Val His Thr Arg Val His Thr Gly Glu Lys Pro
                   165                 170                 175
   His Met Cys Asp Arg Cys Gly Lys Pro Phe Ser Asp Ser Ser Ser Leu
               180                 185                 190
   Ala Arg His Arg Arg Ile His Ser Gly Lys Arg Pro Tyr Lys Cys Pro
           195                 200                 205
   Tyr Ala Asn Cys Gln Lys Thr Phe Thr Arg Arg Thr Thr Leu Thr Arg
       210                 215                 220
   His Gln Asn His His Thr Gly Thr Ile Glu Glu Ala Ala Ala Glu Thr
   225                 230                 235                 240
   Glu Ala Asn Leu Arg Gln Gly Lys Val Val Arg Pro Asp Gly Val Tyr
                   245                 250                 255
   Ser Glu Thr Ala Ser Pro His Gly Thr Ala Ser Pro Ala Gln Arg Ser
               260                 265                 270
   Ser Val Ser Pro Arg His Glu Leu Pro Pro Leu His Ser His Arg Gln
           275                 280                 285
   Met Gly Asp Tyr Phe Met Ala Ser Ser Ser Leu Pro Pro His Leu Arg
       290                 295                 300
   Asp Phe Gln Gln Ala Ser Pro Arg Ala Ser Pro Ser Thr Pro Ser Pro
   305                 310                 315                 320
   Thr Leu Ser Thr Phe Ser Ala Ala His His Pro Arg Pro Ser Leu Thr
                   325                 330                 335
   Ser His Pro Thr Ser Tyr Gly Pro Pro Gln Pro Leu Glu Pro Pro Pro
               340                 345                 350
   Asn Ser Gly Ser Arg Pro Gly Ser Val Thr Gly Ser Pro His Met Thr
           355                 360                 365
   Thr Val Gly Trp Ala Ser Pro Ser Leu Asn Ser Val Pro Ser Pro Gly
       370                 375                 380
   Ser Val Ser Ala Pro Glu Tyr Thr Tyr Pro Glu Pro Ser Gly Pro Pro
   385                 390                 395                 400
   Phe Ala Gly Gly Met Pro Ala His Met Tyr Tyr Pro Asn Ser Thr Ile
                   405                 410                 415
   Arg Arg Pro Gln Ser Thr Glu Pro Glu Asn Tyr Glu Leu Lys Pro Lys
               420                 425                 430
   Val Glu Glu Pro Trp Ala Thr His Ala
           435                 440
<210> 41
   <211> 479
   <212> PRT
   <213> Artificial Sequence
<220>
   <223> pyrtePTTG_20225.1
<400> 41
   Met Glu Leu Asp Thr Ile Leu Asn Ala Arg Leu Thr Ala Ser Leu Gly
   1               5                   10                  15
   Thr Gly Ala Pro Tyr Ala Met Gln His Ala Met Gln Gln Gln His Phe
               20                  25                  30
   Gly Ala Pro Pro Ser Tyr Leu Asn Gly Ser His Ile Lys Ser Glu Asn
           35                  40                  45
   Gly Ser Glu Arg Gly Val Ser Pro His Pro Ser Asp Ser Ser Arg Tyr
       50                  55                  60
   Ser Ser Gln Gln Pro Gln Gln Gln Leu Pro Ser Tyr Pro Ser Ile Pro
   65                  70                  75                  80
   Gln Gln His Met Asn Gly Leu Arg Tyr Pro Ser Pro Ser Gln Met Gln
                   85                  90                  95
   Ala Pro Met Pro Met Leu Asn Asn Ser Asn Tyr Ile Pro Thr Pro Pro
               100                 105                 110
   Asp His Thr Tyr Ala Gln Gln Gln Gln Leu Pro Asp Ala Gln Val Gly
           115                 120                 125
   His Gln His Asn Gly Gly Arg Pro Ala Ser Asp Thr Gly Pro Pro Lys
       130                 135                 140
   Ala Phe Ala Cys Ser Thr Cys Gly Lys Gly Phe Ala Arg Arg Ser Asp
   145                 150                 155                 160
   Leu Ala Arg His Glu Arg Ile His Ser Gly Ile Arg Pro His Val Cys
                   165                 170                 175
   Asp Tyr Pro Asn Cys Gly Lys Gln Phe Ile Gln Arg Ser Ala Leu Thr
               180                 185                 190
   Val His Gln Arg Val His Thr Gly Glu Lys Pro His Met Cys Glu Arg
           195                 200                 205
   Cys Gly Lys Pro Phe Ser Asp Ser Ser Ser Leu Ala Arg His Arg Arg
       210                 215                 220
   Ile His Ser Gly Lys Arg Pro Tyr Lys Cys Pro Tyr Ala Asp Cys Gln
   225                 230                 235                 240
   Lys Thr Phe Thr Arg Arg Thr Thr Leu Thr Arg His Gln Asn His His
                   245                 250                 255
   Thr Gly Thr Val Glu Glu Ala Ala Ala Ala Thr Ala Ala Ala Leu Ala
               260                 265                 270
   Ser Arg Ala Ser Gln Ser Ser Arg Thr Gly Arg Ser Asp Gly Gly Asp
           275                 280                 285
   Tyr Ser Glu Thr Ala Ser Pro Leu Gly Thr Pro Ser Pro Asn Asp Arg
       290                 295                 300
   Thr Leu Ser Leu Ser Pro Ala Asn Gly Met Pro Ala Gly Gly Met Pro
   305                 310                 315                 320
   Gly Leu His Arg Gln Gly Ser Asp Tyr Ala Tyr Met Gly Gly Met Asn
                   325                 330                 335
   Val Pro Pro His Met Arg His Glu Met Pro Gln Pro Ser Pro Arg Ala
               340                 345                 350
   Ser Pro Ala Leu Thr Ala Gln Ser Tyr Ala Ser Asn Val Ser Gly Ser
           355                 360                 365
   Arg Pro Ala Ile Thr Ser His Pro Ser Ala Tyr Gly Pro Pro Pro Ile
       370                 375                 380
   Leu Glu Pro Pro Ala Ser Ala Asn His Gly Gln Ser Gly Gly Thr Ser
   385 390 395 400
   Ser Ala Asn Gly Ser Pro His Met Ser Thr Met Gly Trp Gln Ser Pro
   405 410 415
   Gly Gln Gln Ala Leu Pro Ser Pro Gly Ala Asn Asp Asn Gly Tyr Val
   420 425 430
   Tyr Pro Glu Pro Gln Tyr Gln Ala Gln Asn Gly Met Tyr Tyr Gln His
   435 440 445
   Asn Asn Ile Arg Arg Pro Asn Ser Thr Glu Pro Asp His Tyr Asn Pro
   450 455 460
   Asn Gln Gln Arg Met Gly Asn Glu Met Trp Ala Pro Ala Val Gln
   465 470 475
<210> 42
   <211> 449
   <212> PRT
   <213> Artificial Sequence
<220>
   <223> CHG09160.1
<400> 42
   Met Arg Leu Ser Phe Ser Arg Ile Leu Thr Cys Ala Glu Pro Arg Ala
   1 5 10 15
   Gln Gln Pro Gln Gln Gly Ala Thr Pro Glu Leu Gln Gln Gln Arg Pro
   20 25 30
   Gln Ala Leu Gln Gln Leu Gln Gln Gln Leu Gln Pro Leu Gln Thr Gln
   35 40 45
   Ile Asp Arg Gln Pro Ser Pro His Gly Ser Glu Gln Ser Arg Tyr Ser
   50 55 60
   Gly Pro Met Asn Ala Ser Tyr Pro Ser Pro Thr Ala Met Ala Ala Thr
   65 70 75 80
   Pro Leu Pro Pro Val Pro Asn Ala Asn Met Ala Pro Ala Pro Met Val
   85 90 95
   Pro Asn Glu Met Gln Gln Asn Leu Gly Met Pro Pro Ala Gly Tyr Gln
   100 105 110
   Pro Ala Gln Ala Pro Pro Gln Pro Pro Ala Lys Gln Phe Pro Cys Ser
   115 120 125
   Thr Cys Gly Lys Pro Phe Ala Arg Arg Ser Asp Leu Ala Arg His Glu
   130 135 140
   Arg Ile His Ser Gly Ile Arg Pro His Val Cys Asp Tyr Pro Gly Cys
   145 150 155 160
   Gly Lys Gln Phe Ile Gln Arg Ser Ala Leu Thr Val His Ser Arg Val
   165 170 175
   His Thr Gly Glu Lys Pro His Arg Cys Glu Arg Cys Gly Lys Pro Phe
   180 185 190
   Ser Asp Ser Ser Ser Leu Ala Arg His Arg Arg Ile His Ser Gly Lys
   195 200 205
   Arg Pro Tyr Lys Cys Pro Tyr Ala Asp Cys Gln Lys Thr Phe Thr Arg
   210 215 220
   Arg Thr Thr Leu Thr Arg His Gln Asn His His Val Gly Thr Val Glu
   225 230 235 240
   Glu Ser Ala Arg Ala Arg Ala Glu Ala Leu Ala Gln Gly Ala Asn Ala
   245 250 255
   Ala Ala Ala Ala Val Ala Ala Ala Gln Ser Lys Ser Arg Ser Gly Ser
   260 265 270
   Glu Gln Ala Ser Asn His Glu Ser Pro Leu Thr Asn Thr Pro Ser Pro
   275 280 285
   Gly Gln Arg Pro Met Ser Met Ser Pro Gly Ala Glu Leu Ala Gly Ile
   290 295 300
   Asn Asn Met Gln Tyr Leu Ser Asn Ser Ile Pro Pro His Leu Arg Gly
   305 310 315 320
   Asp Val His Val Gly Ser Pro Ser Pro Thr Ala Ser Ser Gly Tyr Asn
   325 330 335
   Asn Gly Met Arg Pro Thr Ser His Pro Thr Gly Tyr Ala Pro Pro Gln
   340 345 350
   Thr Leu Glu Pro Ser Ile Glu Gln Pro Gln Gln Gly Pro Gly Ser Ala
   355 360 365
   Val Gly Ser Pro His Ile Gly Ser Val Gly Trp Ala Ser Pro Gly His
       370                 375                 380
   Val Gly Ser Pro Thr Gln Ser Pro Ser Gly Asn Gly Tyr Val Tyr Pro
   385                 390                 395                 400
   Asp Pro Glu Ala Tyr Pro Asn Gly Thr Pro Ile Ala Gln Met Phe Phe
                   405                 410                 415
   Asn Ser Ala Val Ala Ser Arg Arg Pro Asp Ser Ala Glu Pro Gly Asn
               420                 425                 430
   Pro Ser Phe Asp Thr Lys Ser Arg Gln Gly Glu Leu Trp Thr Asn Ser
           435                 440                 445
   Gln
<210> 43
   <211> 517
   <212> PRT
   <213> Artificial Sequence
<220>
   <223> geodeGMDG_00992T0
<400> 43
   Met Asp Leu Arg Gly Leu Leu Gln Ser His Pro His His His Thr Ser
   1               5                   10                  15
   His Gln Gln Pro Tyr Asp His Arg Leu Pro Ala Ala Gln Pro Tyr Thr
               20                  25                  30
   Ser Leu Pro Ile Ser Tyr Ala Ser Pro Ser Tyr Ile Ala Ile Gly His
           35                  40                  45
   His His Asn His Gln His His Gln Ala Gln Gly Gln Gln Gln Gly Gln
       50                  55                  60
   Gly Met Tyr Asp Phe Pro Phe Ser His Pro Gln Gln Gln Gln Gln Gln
   65                  70                  75                  80
   Gly Glu Gln Ser Thr Tyr Leu Arg Arg His Gln Gly Tyr Gln Asn Gln
                   85                  90                  95
   Met Pro Thr Ser Ala Gln Gln Gln Gln Gln Ser Ser Tyr Pro Gln Gln
               100                 105                 110
   Gln Gln Gln Leu Pro Arg Ser Tyr Gln Ser Gly Asn Ala Gln Ser Met
   115 120 125
   Tyr Gly Lys Thr Thr Ala Ile Ala Pro Ala Gly Glu Gly Trp Gly Lys
   130 135 140
   His Gln Gln Gln Val Gly Gly Tyr Gln Gln Ser Gln Met Gln Thr Gln
   145 150 155 160
   Val Val Ala Gln Ser Thr Asp Val Asp Ala Leu Met Arg Ala Ile Gln
   165 170 175
   Ala Lys Pro Ala Gly Asp Asn Gly Ser Ala Ser Glu Val Ala Ser Ala
   180 185 190
   Glu Arg Thr Arg Asp Val Ser Ala Glu Arg Glu Lys Asn Ala Ser Arg
   195 200 205
   Gly Gly Gly Ala Thr Gly Met Lys Asn Gly Gly Asn Gly Arg Glu Glu
   210 215 220
   Pro Lys Lys Arg Tyr Glu Cys His Leu Gly Glu Cys Arg Lys Ala Phe
   225 230 235 240
   Phe Gln Lys Thr His Leu Glu Ile His Ile Arg Ala His Thr Gly Ala
   245 250 255
   Lys Pro Tyr Thr Cys Thr His Pro Thr Cys Thr His Ala Phe Ser Gln
   260 265 270
   Leu Gly Asn Leu Lys Thr His Leu Arg Arg His Thr Gly Glu Arg Pro
   275 280 285
   Phe Ala Cys Pro Thr Cys Gly Lys Thr Phe Ala Gln Arg Gly Asn Val
   290 295 300
   Arg Ala His Ala Ala Val His Asp Ala Gly Gly Ala Ala Lys Lys Phe
   305 310 315 320
   Val Cys Arg Leu Asp Gly Cys Gly Lys Cys Phe Thr Gln Leu Gly Asn
   325 330 335
   Leu Lys Ser His Met Asn Lys Phe His Val Glu Thr Leu Arg Gly Leu
   340 345 350
   Thr Val Arg Phe Gly Glu Ser Glu Lys Arg Gly Arg Asp Gly Gly Glu
   355 360 365
   Gly Glu Gly Lys Glu Glu Glu Asp Asp Leu Leu Asp Tyr Phe Arg Ser
   370 375 380
   Leu Tyr Arg Asn Ala Asn Lys Gly Ile Lys Gly Arg Gly Lys Gly Arg
   385 390 395 400
   Lys Val Ala Ser Thr Thr Ser Thr Ser Ser Thr Pro Pro Ser Leu Ser
   405 410 415
   Ser Ser Thr Ser Ser Leu Ser Ser Leu Ala Ser Leu Ser Ser Leu Ala
   420 425 430
   Ser Leu Ala Ser Leu Ala Ser Pro Pro Leu Ser Pro Pro Thr Ser Thr
   435 440 445
   Ser Ser Tyr Ile Gly Gly Ile Pro Ala His Tyr Arg Met Gly Met Gly
   450 455 460
   Met Gly Met Ala Pro Gly Met Asp Gly Ile Glu Leu Phe Asp Gly Ser
   465 470 475 480
   Ser Ser Gly Ile Gly Ser Pro Cys Ala Ser Ser Leu Gly Ser Leu Tyr
   485 490 495
   Glu Val Gly Gly Thr Tyr Glu Glu Glu Glu Gly Gly Glu Leu Ala Phe
   500 505 510
   Gly Asp Arg Met Tyr
   515
<210> 44
   <211> 436
   <212> PRT
   <213> Artificial Sequence
<220>
   <223> fusveFVEG_02052T0
<400> 44
   Met Ser Gly Met Arg Ser Pro Ala Pro Thr Glu Val Met Asp Ile Thr
   1 5 10 15
   Asn Met Leu Asn Asn Lys Gly Gln Met Gln Gln Leu Thr Ser Gly Leu
   20 25 30
   Leu Asp His Gln Gln Tyr Gln His Ala Phe Val Lys His Glu Ser Gly
   35 40 45
   Met Glu Arg Ser Ala Ser Pro His Gly Ser Glu His Ser Gln Tyr Ser
       50                  55                  60
   Asn Pro His Ser Ile Ala Arg Ala Tyr Pro Ser Pro Ser Thr Met Gln
   65                  70                  75                  80
   Ala Pro Met His Met Pro Asn Pro Met Ser Ala Ala Met Thr Met Gln
                   85                  90                  95
   Gly Tyr Pro Glu Met Pro Asn Met Gly Gly Met Pro His Met His Met
               100                 105                 110
   Gln Gln Met Pro Gln Gln Pro Pro Pro Pro Pro Gln Gln Pro Ile Lys
           115                 120                 125
   Ala Tyr Pro Cys Ser Thr Cys Gly Lys Gly Phe Ala Arg Arg Ser Asp
       130                 135                 140
   Leu Ala Arg His Glu Arg Ile His Ser Gly Val Arg Pro His Val Cys
   145                 150                 155                 160
   Asp Trp Pro Gln Cys Gly Lys Gln Phe Ile Gln Arg Ser Ala Leu Thr
                   165                 170                 175
   Val His Gln Arg Val His Thr Gly Glu Lys Pro His His Cys Glu Thr
               180                 185                 190
   Cys Ala Lys Pro Phe Ser Asp Ser Ser Ser Leu Ala Arg His Arg Arg
           195                 200                 205
   Thr His Ser Gly Lys Arg Pro Tyr Lys Cys Pro Tyr Ala Asp Cys Gln
       210                 215                 220
   Lys Thr Phe Thr Arg Arg Thr Thr Leu Thr Arg His Gln Asn His His
   225                 230                 235                 240
   Ser Gly Thr Ile Glu Glu Ala Ala Ala Ala Thr Ala Ala Ala Leu Ala
                   245                 250                 255
   Ala Ser Lys Ser Lys Gly Leu Ser Gln Thr Arg Ser Glu Ser Asp His
               260                 265                 270
   Met Ser Asn His Gly Ser Pro Leu Thr Thr Pro Ser Pro Ser Gln Arg
           275                 280                 285
   Thr Met Ser Met Ser Pro Ser Val Asp Leu Ser Gly Asn Asn Ser Ile
       290                 295                 300
   Pro Arg His Pro Gly Asp Phe Gln Tyr Leu Ser Gln Asn Gly Ser Leu
   305                 310                 315                 320
   Pro Met His Met Arg Val Gly Ser Pro Thr Ser Thr Ser Ser Gly Gly
                   325                 330                 335
   Phe Asn Met Met Arg Pro Thr Ser His Pro Thr Ser Tyr Gly Pro Pro
               340                 345                 350
   Pro Thr Leu Glu Pro Asn Leu Asp Asn Ser Gln Gly Thr Pro Ser Ser
           355                 360                 365
   Asn Gly Gly Ser Pro His Met Thr Asn Val Gly Trp Gln Ser Pro Ser
       370                 375                 380
   His Met Ala Ser Pro Ser Gln Asn Asn Ala Ser Tyr Val Tyr Pro Asp
   385                 390                 395                 400
   Pro Ala Asp Ala Tyr Pro Ala Asn Gln Ala Met Asn Gln Met Tyr Tyr
                   405                 410                 415
   Ser Ala Ala Ala His Met Arg Arg Pro Gln Ser Thr Glu Pro Gly Leu
               420                 425                 430
   Val His Met Ala
           435
<210> 45
   <211> 507
   <212> PRT
   <213> Artificial Sequence
<220>
   <223> AO090003001179
<400> 45
   Met Leu Ala Arg Pro Glu Arg Arg Gly Lys Pro Arg Cys Gly Ala Gly
   1               5                   10                  15
   Ser Ala Leu Lys Pro Glu Gly Val Lys Leu Ser Phe Asp Cys Leu Phe
               20                  25                  30
   Phe Gly Ile Glu Thr Ile Ser Glu Pro Glu Val Thr Pro Ala Phe Val
           35                  40                  45
   Ala His Ile Leu Gly Cys Glu Ser Leu Glu Thr Ser Pro Ala Val Val
       50                  55                  60
   Asn Phe Ser Ala Asp Pro Thr Arg Lys Gly Ser Ala Ala Met Val Ala
   65                  70                  75                  80
   Ala Glu Phe Asp His Gln Gln Phe Ile Gln Asn Pro His Leu Asp Thr
                   85                  90                  95
   Ser Ser Pro Lys Met Lys Pro Glu Pro Gly Val Ser Glu Ala Gly Asp
               100                 105                 110
   Gln Pro Val Leu Ala Tyr Pro Pro His Ala Pro Leu Gly Gln Val Pro
           115                 120                 125
   Asn Met His Pro Asp Met Arg Tyr Gln Pro Gln Thr His Pro Asn Pro
       130                 135                 140
   Ala Leu Pro Leu Leu Gln Asn Pro Tyr Met Pro Gly Gly Tyr Thr Ser
   145                 150                 155                 160
   Ala Pro Pro Met Pro Asn Gly Gly Ala Pro Gln Gly Arg Thr Asp Pro
                   165                 170                 175
   Pro Pro Lys Thr Phe His Cys Gly Thr Cys Ser Lys Gly Phe Ala Arg
               180                 185                 190
   Arg Ser Asp Leu Ala Arg His Glu Arg Ile His Ser Gly Ile Arg Pro
           195                 200                 205
   His Ala Cys Asp Trp Pro Gly Cys Gly Lys Gln Phe Ile Gln Arg Ser
       210                 215                 220
   Ala Leu Thr Val His Ser Arg Val His Thr Gly Glu Lys Pro His Met
   225                 230                 235                 240
   Cys Glu Arg Cys Gly Lys Pro Phe Ser Asp Ser Ser Ser Leu Ala Arg
                   245                 250                 255
   His Arg Arg Ile His Ser Gly Lys Arg Pro Tyr Lys Cys Pro Tyr Ala
               260                 265                 270
   Asn Cys Gln Lys Thr Phe Thr Arg Arg Thr Thr Leu Thr Arg His Gln
           275                 280                 285
   Asn His His Thr Gly Thr Ile Glu Glu Ala Ala Ala Glu Thr Glu Ala
       290                 295                 300
   Asn Leu Arg Gln Asn Lys Glu Arg Val Arg Ala Pro Gly Glu Gly Met
   305                 310                 315                 320
   Tyr Ser Glu His Gly Ser Val His Ser Thr Pro Ser Pro Ala His His
                   325                 330                 335
   Pro Ala Leu Ser Pro Ala Gly Glu Leu Pro Pro Leu Asn Met Pro Arg
               340                 345                 350
   Ser Ser Gly Glu Tyr Tyr Ser Met Gly Asn Gly Ser Ile Pro Pro His
           355                 360                 365
   Val Arg Gly Asp Phe Pro Gln Ala Ser Pro Arg Ala Ser Pro Thr Ala
       370                 375                 380
   Thr Ser Pro Ser Leu Ser Ser Phe Gly Ser Ala Pro His Thr Arg Pro
   385                 390                 395                 400
   Ser Met Thr Ser His Pro Ser Gly Tyr Gly Pro Pro Gln Pro Leu Glu
                   405                 410                 415
   Pro Pro Ala Asn Asn Asp His Arg Pro Asn Ser Val Ser Gly Ser Pro
               420                 425                 430
   His Met Thr Ser Leu Gly Trp Ala Ser Pro Ser His Gly Ser Ile Pro
           435                 440                 445
   Ser Pro Gly Ser Ala Thr Asp Phe Gly Tyr Ser Asp Pro Asn Gly Pro
       450                 455                 460
   Ala Tyr Pro Asn Ser Met Pro Pro His Met Tyr Phe Pro Asn Ser Thr
   465                 470                 475                 480
   Ile Arg Arg Pro Thr Ser Thr Glu Pro Glu Asn Tyr Glu Met Lys Pro
                   485                 490                 495
   Lys Ile Gly Glu Ser Ala Trp Ser Thr Pro Val
               500                 505
<210> 46
   <211> 529
   <212> PRT
   <213> Artificial Sequence
<220>
   <223> BC_P033780.1
<400> 46
   Met Ser His Thr Ser Gly Asn Ala Trp Arg Trp Ser Glu Asp Ser His
   1               5                   10                  15
   Ile Pro Phe Cys Tyr Met Asp Thr Ala Gly Met Val Gln Ser Met Asn
               20                  25                  30
   Pro Gln Thr Thr Thr Ser Ala Gly Leu Gly Arg Ala Thr Val Ala Ala
           35                  40                  45
   Pro Ile Met Pro Ile Ser Ser Tyr Gly Ser Ser Thr Phe Asn Met Thr
       50                  55                  60
   Ser Gly His Gln Gln Ile Pro Gln Asn Ser Tyr Gly Phe Thr Ser Tyr
   65                  70                  75                  80
   Asn Asp Gly Ser Leu Thr Thr Ile Ile Pro Ser Tyr Gly Ala Asn Tyr
                   85                  90                  95
   Ile Gln Pro Arg Pro Leu Pro Gly Met Met Gln Ser Phe Ala Pro Thr
               100                 105                 110
   Pro Lys Gln Ala Pro Phe Asn Gly Ser Ser Arg Gln Glu Phe Val Gly
           115                 120                 125
   Ser Ser His Gln Asn Gln Ser Pro Gln Ile Lys Pro Glu Pro Ser Trp
       130                 135                 140
   Thr Thr Ser Ala Gly His Lys Thr Pro Pro Lys Thr Gln Ser Thr Pro
   145                 150                 155                 160
   Pro Pro Ser Lys Asp Ile Ser Pro Leu Pro Leu Thr Gly Ser Asp Glu
                   165                 170                 175
   Ala His Ile Gly Ser Thr Ala Ile Asp Arg Leu Met Lys Val Ile Gln
               180                 185                 190
   Thr Lys Ala Gln Thr Leu Gln Thr Gln Leu Pro Pro Ile Pro Gln Ile
           195                 200                 205
   Thr Pro Val Val Gly Ala Pro His Thr Leu Ser Arg Gln Asp Ser Met
       210                 215                 220
   Tyr Arg Arg Phe Tyr Gly Tyr Glu Lys Glu Glu Lys Gln Met Leu Thr
   225                 230                 235                 240
   Ser Gly Lys Ala Pro Ser Glu Pro Gly His Ser Lys Arg Lys Glu Lys
                   245                 250                 255
   Lys Tyr Arg Cys Thr Phe Glu Asn Cys Pro Ser Ser Phe Pro Gln Lys
               260                 265                 270
   Thr His Leu Gln Ile His Leu Arg Lys His Thr Gly Ala Lys Pro Tyr
   275 280 285
   Thr Cys Ser Trp Lys Ser Cys Gly Arg Gln Phe Ser Gln His Gly Asn
   290 295 300
   Leu Lys Thr His Met Asn Arg His Ser Gly Glu Arg Arg Phe Pro Cys
   305 310 315 320
   Glu Ile Cys Gly Lys Arg Phe Gly Gln Pro Ser Asn Leu Ser Ala His
   325 330 335
   Arg Ile Val His Thr Gly Glu Lys Pro Phe Thr Cys Lys Leu Asp Gly
   340 345 350
   Cys Glu Lys Arg Phe Thr Gln Leu Gly Asn Leu Lys Ser His His Asn
   355 360 365
   Asn Phe His Gln Gln Thr Ile Glu Asn Leu Met Leu Lys Leu Glu Ser
   370 375 380
   Gly Glu Ile Asp Leu Glu Ala Asn Lys Glu Phe Trp Ser Tyr Phe Phe
   385 390 395 400
   Thr Leu Tyr Lys Asn Ser Asn Lys Gly Ile Lys Gly Arg Gly Lys Asp
   405 410 415
   Arg Asn Ile Ser Pro Arg Ser Arg Leu Ser Lys Ser Gly His Ala Ser
   420 425 430
   Thr Gly Thr Asp Ala Ser Arg Tyr Ile Ser Ser Asn Met Arg Ser Cys
   435 440 445
   His Gly Leu Met Gly Val Asp Ala Thr Ser Pro Phe Ser Ser Asp Val
   450 455 460
   Thr Met Tyr Asp Ser Asp Ala Ser Leu Arg Ser His Thr Ser Gly Ser
   465 470 475 480
   Ile Ser Ala His Ser Ser Phe Gly Ser Val Ser Ser Asp Gly Thr Ser
   485 490 495
   Ser Ser Cys Ser Ser Phe Ser Asp Glu Ile Thr Gly Asp Tyr Lys Asp
   500 505 510
   His Arg Gly Gly Asn Ser Arg Gly Gly Leu Ala Phe Gly Glu Arg Leu
   515 520 525
   Tyr
<210> 47
   <211> 423
   <212> PRT
   <213> Artificial Sequence
<220>
   <223> hiscnHCAG_06087T0
<400> 47
   Met Asp Ile Pro Thr Ile Leu Asn Glu Lys Gly Thr Ala Ala Ala Ala
   1 5 10 15
   Ala Ala Ala Ala Ala Ala Ala Lys Ala Gln Leu Gln Gln Gln Leu Ala
   20 25 30
   Gln Gly Thr His Ile Lys Ser Gln Ser Pro Ser Glu Met Gly Ser Glu
   35 40 45
   Asn Gly Thr Ser Gln Asn Gly Asp Gln Pro Asn Ile Tyr Asn Pro Thr
   50 55 60
   Ser Gln Pro Gln Pro Leu Ser Thr Ile Pro Gln Tyr His Ser Pro Pro
   65 70 75 80
   Gln Ser Ser Val Val Asn Ser Ala Ala Arg Ser Asp Tyr Ile Gln Asn
   85 90 95
   Asp Gln Gly Lys Ile Glu Tyr Leu Gln Asn Asp Ser Ser Gly Arg Thr
   100 105 110
   Arg Ala Asn Gly Glu Pro Ala Pro Lys Thr Phe His Cys Ser Thr Cys
   115 120 125
   Gly Lys Gly Phe Ala Arg Arg Ser Asp Leu Ala Arg His Glu Arg Ile
   130 135 140
   His Ser Gly Ile Arg Pro His Val Cys Asp Trp Pro Gly Cys Gly Lys
   145 150 155 160
   Gln Phe Ile Gln Arg Ser Ala Leu Thr Val His Thr Arg Val His Thr
   165 170 175
   Gly Glu Lys Pro His Met Cys Asp Arg Cys Gly Lys Pro Phe Ser Asp
   180 185 190
   Ser Ser Ser Leu Ala Asp Ile Ala Glu Tyr Ile Pro Glu Asn Asp Leu
   195 200 205
   Thr Asn Ala Gln Ala Ala Ala Gln Thr Glu Ala Asn Leu Arg Gln Asn
   210 215 220
   Lys Glu Arg Ser Ile Arg Pro Leu Glu Gly Ile Tyr Ser Glu Pro Gly
   225 230 235 240
   Ser Ala Gln Ser Thr Pro Ser Cys Gln Arg Leu Ser Ala Ser Pro Gly
   245 250 255
   Asn Glu Leu Pro Pro Met His Leu His Arg Gln Met Gly Glu Tyr Tyr
   260 265 270
   Met Gly Asn Asn Thr Ser Ile Pro Pro His Leu Arg Ser Asp Phe Gln
   275 280 285
   Gln Ala Ser Pro Arg Ala Ser Pro Ser Thr Ser Ser Pro Pro Leu Ser
   290 295 300
   Thr Tyr Asn Ser Val Pro His Ile Arg Pro Ser Leu Thr Ser His Pro
   305 310 315 320
   Ser Gly Tyr Arg Pro Pro Gln Pro Leu Glu Pro Pro Ala Asn Ser Asp
   325 330 335
   Pro Arg Pro Asn Ser Val Ala Gly Ser Pro His Ile Ser Ser Met Gly
   340 345 350
   Trp Ala Ser Pro Thr Leu Asn Ser Ile Ser Ser Pro Gly Ser Ala Ser
   355 360 365
   Ala Thr Glu Tyr Ser Tyr Pro Glu Pro Ser Leu Pro Pro Tyr Ser Ala
   370 375 380
   His Ile Pro Pro His Ile Phe Phe Pro Asn Ser Thr Ile Arg Arg Pro
   385 390 395 400
   Gln Ser Thr Glu Pro Glu Asn Tyr Glu Met Lys Pro Lys Leu Asn Gly
   405 410 415
   Asp Ser Trp Thr Thr Pro Val
   420
<210> 48
   <211> 421
   <212> PRT
   <213> Artificial Sequence
<220>
   <223> picane_gw1.1.994.1
<400> 48
   Met Asn Arg Pro Glu Gln Gln Gln Gln Tyr Asn Gln Tyr Gln Gln Gln
   1               5                   10                  15
   Gln Gln Gln Phe Arg Gln Pro Gln Leu Pro Tyr Asn Asp Tyr Gln Gln
               20                  25                  30
   His Gln Gln His Gln Gln Gln Gln Gln Pro Gln Gln Tyr Gln Gln Tyr
           35                  40                  45
   Gln Gln Tyr Gln Gln Thr Pro Gln Gln Phe His Pro Gln Tyr Asn Ile
       50                  55                  60
   Ser Pro Ser Thr Gln His Ser His Ile Ser Pro Thr Ser Ser Gln Tyr
   65                  70                  75                  80
   Ser Asn Thr Ser Glu Gly Gln Arg Leu Lys Ser Ala Gln Asp Thr Val
                   85                  90                  95
   Thr Thr Asp Asp Gly Arg Pro Leu Leu Gly Ala Thr Lys Val Asp Gln
               100                 105                 110
   Leu Met Leu Val Ile Gln Ala Arg Lys Lys Gly Val His Asn Pro Ile
           115                 120                 125
   Ala Gln Ala Asp Asp Gly Ser Ile Leu Asp Asn Gly Asp Gly Val Leu
       130                 135                 140
   Pro Gln Glu Thr Glu Leu Val Gly Gly Ile Asp Lys Pro Lys Thr Lys
   145                 150                 155                 160
   Gly Thr Lys His His Glu Cys Gln Tyr Cys His Lys Thr Phe Thr Gln
                   165                 170                 175
   Ser Thr His Leu Glu Val His Val Arg Ser His Ile Gly Tyr Lys Pro
               180                 185                 190
   Phe Glu Cys Asn Tyr Cys Gly Lys Arg Phe Thr Gln Gly Gly Asn Leu
           195                 200                 205
   Arg Thr His Leu Arg Leu His Thr Gly Glu Lys Pro Tyr Ile Cys Glu
       210                 215                 220
   Thr Cys Gly Arg Ser Phe Ser Arg Lys Gly Asn Leu Ala Ala His Lys
   225 230 235 240
   Leu Thr His Glu Asn Leu Lys Pro Tyr Glu Cys Lys Leu Asp Gly Cys
   245 250 255
   Asn Lys Ala Phe Thr Gln Leu Gly Asn Leu Lys Ala His Gln Asn Arg
   260 265 270
   Phe His Leu Glu Thr Leu Asn Asn Leu Thr Arg Arg Leu Ala Glu Ile
   275 280 285
   Asp Pro Glu Asp Ala Gln Thr Thr Met Asn Pro Asp Glu Arg Asp Leu
   290 295 300
   Leu Asn Tyr Phe Ala Gly Leu Tyr Lys Asn Ser Asn Arg Gly Ile Lys
   305 310 315 320
   Gly Arg Gly Lys Arg Thr Ser Pro Val Leu Lys Thr Pro Gln Ile Ala
   325 330 335
   Asn Gln Ala Ala Pro Val Asn Asn Gln Pro Gln Pro Gln Gln Gln Gln
   340 345 350
   Gln Gln Gln Gln Arg Pro Gln Gln Gln Gln Ala Lys Gln Glu Gln Gln
   355 360 365
   Gln Gln Tyr Asn Pro Gln Tyr Gln Gln Tyr Gln Tyr Pro Gln Asp Gln
   370 375 380
   Ser Phe Asn Gln Gln Leu Ala Phe Asn Gln Gln Asn Gly Ala Asn Phe
   385 390 395 400
   Ala Pro Pro Met Asn Pro Gly Ala Lys Ala Gly Asn Ile Ser Phe Lys
   405 410 415
   Asn Val Asn Tyr Asn
   420
<210> 49
   <211> 383
   <212> PRT
   <213> Artificial Sequence
<220>
   <223> trivigw1.5.1944.1
<400> 49
   Met Asp Arg Ser Ala Ser Pro His Met Ser Glu His Ser Ser Tyr Ser
   1               5                   10                  15
   Ala His Ser Leu Ser Arg Ala Tyr Pro Ser Pro Thr Ala Met Gln Ala
               20                  25                  30
   Pro Met Gln Ile Gln Asn Pro Met His Ser Ala Met Gln Met Gly Gly
           35                  40                  45
   Phe Ala Asp Met Ser Gly Met Gly Ser Val Pro Ser Met Ala Met His
       50                  55                  60
   His Ile Pro Gln Gln Pro Pro Gln Gln Gln Thr Pro Thr Ala Pro Val
   65                  70                  75                  80
   Lys Ala Tyr Pro Cys Ser Thr Cys Gly Lys Gly Phe Ala Arg Arg Ser
                   85                  90                  95
   Asp Leu Ala Arg His Glu Arg Ile His Thr Gly Val Arg Pro His Val
               100                 105                 110
   Cys Asp Tyr Pro Lys Cys Asn Lys Gln Phe Ile Gln Arg Ser Ala Leu
           115                 120                 125
   Thr Val His Gln Arg Val His Thr Gly Glu Lys Pro His His Cys Glu
       130                 135                 140
   Thr Cys Ala Lys Pro Phe Ser Asp Ser Ser Ser Leu Ala Arg His Arg
   145                 150                 155                 160
   Arg Thr His Ser Gly Lys Arg Pro Tyr Lys Cys Pro Tyr Ala Asp Cys
                   165                 170                 175
   Gln Lys Thr Phe Thr Arg Arg Thr Thr Leu Thr Arg His Gln Asn His
               180                 185                 190
   His Thr Gly Thr Ile Glu Glu Ala Ala Ala Ala Thr Ala Ala Ala Leu
           195                 200                 205
   Ala Ala Ser Arg Ala Lys Asn Gly Ser Gln Ala Arg Ser Glu Gly Asp
       210                 215                 220
   His Met Ser Asn His Gly Ser Pro Leu Thr Thr Pro Ser Pro Ser Gln
   225                 230                 235                 240
   His Thr Met Met Ser Pro Ala Leu Asp Leu Ser Gly Ser Asn Gly Leu
                   245                 250                 255
   Asn Arg His Pro Ala Asp Phe Gln Tyr Met Thr Gln Gly Gly Thr Leu
               260                 265                 270
   Pro Pro His Leu Arg Val Gly Ser Pro Thr Ser Thr Thr Ser Ala Ala
           275                 280                 285
   Ser Tyr Asn Asn Gly Ile Arg Pro Thr Ser His Pro Thr Gly Tyr Gly
       290                 295                 300
   Pro Pro Pro Thr Leu Glu Pro Asn Leu Glu Gln His Pro Ala Gly Ser
   305                 310                 315                 320
   Gly Ser Ala Gly Gly Ser Pro His Met Ser Gln Val Gly Trp Gln Ser
                   325                 330                 335
   Pro Gln Thr His Ser Pro Ala His Asn Ala Gly Ser Tyr Val Tyr Pro
               340                 345                 350
   Asp Pro Asp Gly Tyr Pro Pro Asn Pro Ser Met Ser Gln Met Tyr Tyr
           355                 360                 365
   Gly Ala Pro Gln Gln Met Arg Arg Pro Gln Ser Thr Glu Pro Gly
       370                 375                 380
<210> 50
   <211> 507
   <212> PRT
   <213> Artificial Sequence
<220>
   <223> sormaSMAC_04411
<400> 50
   Met Ala Leu Thr Ala Gln Pro Ser Ala Pro Ala Gly Trp Glu Arg Trp
   1               5                   10                  15
   Pro Gln His His Pro Asn Asn Asp Tyr Val Met Met Asp Ala Asp Val
               20                  25                  30
   Met Pro Tyr Asn Ala Arg Pro Met Thr Thr Ala Pro Met Gln Gln Arg
           35                  40                  45
   Pro Ser Leu Val Pro His Tyr Ile Pro Thr Thr Ser Met Ser Thr Ala
       50                  55                  60
   Pro Ile Thr Ser Ile Ser Ala Pro Ala His Tyr Gln Pro Ser Val Ser
   65                  70                  75                  80
   Tyr Gly Gly Gly Tyr Pro Ala Tyr Pro Leu Pro Ser Pro Thr Thr Met
                   85                  90                  95
   Ser Ser Gln Tyr Arg His Gln His Tyr Gln Glu Arg Pro Ser Leu His
               100                 105                 110
   Met Ile Thr Pro Glu Leu Glu Asp Thr Arg Gly Pro His His Ile Arg
           115                 120                 125
   Asp Ala Arg Arg Tyr Ser Glu Glu Ser Arg Ser Pro Ser Glu Arg Ser
       130                 135                 140
   Asp Ser Gln Ala Ser Thr Ala Glu Thr Thr Ile Ser Asn His Ser Thr
   145                 150                 155                 160
   Cys Ser Arg Thr Ile Thr Pro Asn Ala Pro Val Asn Gly Ala Pro Gln
                   165                 170                 175
   Val Glu Phe Ser Thr Ala Val Asp Lys Leu Met Lys Val Ile Gln Ser
               180                 185                 190
   Lys Met Lys Asp Thr Asp Ala Glu Gln Ser Gly Asp Asp Lys Asp Ile
           195                 200                 205
   Lys Val Glu Gln Gln Ser Ser Pro Asp Cys Gln Ala Arg Val Gln Gln
       210                 215                 220
   Pro Ala Asp Lys Gln Lys Arg Lys Arg Tyr Glu Cys Gln Ile Glu Gly
   225                 230                 235                 240
   Cys Asn Lys Lys Phe Ser Gln Lys Thr His Arg Asp Thr His Val Arg
                   245                 250                 255
   Ser His Thr Gly Asp Arg Pro Tyr Val Cys Pro Ile Pro Gly Cys Gly
               260                 265                 270
   Gly Arg Phe Thr Gln Ala Gly Asn Leu Lys Thr His Lys Arg Arg His
           275                 280                 285
   Thr Gly Glu Arg Pro Tyr Arg Cys Glu Val Cys Asp Lys Gly Phe Val
       290                 295                 300
   Gln Arg Gly Asp Val Lys Ala His Met Lys Thr His Leu Gly Thr Lys
   305                 310                 315                 320
   Ala Phe Leu Cys Arg Leu Asp Asn Cys His Lys Gln Phe Thr Gln Arg
                   325                 330                 335
   Gly Asn Leu Lys Tyr His Gln Asn Lys Tyr His Asn Glu Thr Ile Lys
   340 345 350
   Ala Leu Ala Ala Arg Phe Asp Thr Ile Glu Asp Trp Ser Thr Val Ser
   355 360 365
   Lys Glu Asp Met Glu Ile Phe Lys Asp Phe Ala Val Val His Lys Asn
   370 375 380
   Ser Asn Lys Gly Ile Lys Gly Arg Gly Lys His Arg Lys Val Lys Ser
   385 390 395 400
   Val Pro Gln Ser Ser Pro Thr Ser Pro Thr Gly His Ser Pro Leu Pro
   405 410 415
   Asn Ile Met Ala Ser Gln Tyr Pro Leu Pro Ser Gly Pro Gly Leu Ser
   420 425 430
   His Leu Leu His Ser Pro His Pro Leu His Pro Pro Leu His His Gln
   435 440 445
   Gly Pro Ser His Ser Ala Met Tyr Gly Met Pro Arg Pro Gly Phe His
   450 455 460
   Gly His Tyr Glu Thr Tyr Asp His His Glu Val Asp Thr Val Ala Ser
   465 470 475 480
   Ser Arg Gly Ser Val Ala Glu Pro Ile Tyr His His His Glu Glu His
   485 490 495
   Pro Arg Glu Leu Ala Phe Gly Asp Arg Ile Tyr
   500 505
<210> 51
   <211> 590
   <212> PRT
   <213> Artificial Sequence
<220>
   <223> sclscSS1G_08750T0
<400> 51
   Met Val Met Val Pro His Thr Leu Gly Thr Thr Trp Arg Trp Ser Glu
   1 5 10 15
   Asp Ser His Ile Pro Phe Cys Tyr Met Asp Ala Ser Gly Met Val Gln
   20 25 30
   Ser Leu Asp Pro Gln Thr Thr Ser Ser Ala Gly Leu Gly Arg Thr Ile
           35                  40                  45
   Ile Ala Pro Pro Ile Met Pro Met Asp Ser Phe Ser Gly Ser Ser Phe
       50                  55                  60
   Ile Met Asn Gly His Gln Gln Ile Ser Gln Asn Ala Tyr Gly Phe Thr
   65                  70                  75                  80
   Ser Tyr Thr Asn Gly Ser Ser Thr Asn Leu Ile Thr Thr Tyr Gly Ala
                   85                  90                  95
   Asn Tyr Ile Gln Pro Arg Ser Leu Pro Pro Met Ile Gln Ser Phe Ser
               100                 105                 110
   His Ser Pro Lys Glu Val Ile Phe Asp Glu Ser His Arg Gln Gly Phe
           115                 120                 125
   Ile Glu Ser Thr Ser Pro Ser Gln Ser Pro Gln Ile Lys Pro Glu Pro
       130                 135                 140
   Ser Trp Lys Lys Thr Thr Arg Cys Gln Pro Glu Lys Thr Pro Lys Ala
   145                 150                 155                 160
   Ser Arg Pro Asn Lys Thr Ile Asn Pro Pro Pro Leu Thr Gly Ser Asn
                   165                 170                 175
   Glu Ala His Ile Gly Thr Thr Ala Val Asp Arg Leu Met Lys Ile Ile
               180                 185                 190
   Gln Asp Lys Ala Arg Thr Ser Lys Ser Pro Ser Pro Ser Val Ser Glu
           195                 200                 205
   Ser Thr Ser Val Val Gly Ser Pro His Thr Leu Tyr Asn His Asp Tyr
       210                 215                 220
   Ile Ser Ala Tyr Glu Glu Glu Lys Gln Ile Leu Thr Ser Val Lys Val
   225                 230                 235                 240
   His Asp Glu Ser Gly Arg Ser Lys Val Pro Lys Lys Arg Phe Arg Cys
                   245                 250                 255
   Thr Phe Glu Gly Cys Met Ser Arg Phe Leu Gln Lys Ala Gln Leu Glu
               260                 265                 270
   Ile His Val Arg Lys His Thr Gly Glu Thr Pro Tyr Val Cys Gly Tyr
           275                 280                 285
   Val Gln Cys Lys Arg Arg Phe Thr Gln Gln Gly Asn Leu Asn Ile His
   290 295 300
   Leu Asp Arg His Arg Gly Ile Arg Arg Tyr Pro Cys Glu Ile Cys Gly
   305 310 315 320
   Lys Arg Phe Gly Gln Arg Ser Asn Leu Asp Ala His Arg Ile Val His
   325 330 335
   Thr Gly Ile Lys Pro Phe Ser Cys Lys Leu Asp Gly Cys Gly Lys Arg
   340 345 350
   Phe Thr Gln Arg Gly Asn Leu Lys Ser His His Asn Lys Phe His Arg
   355 360 365
   Gln Thr Leu Glu Arg Leu Gln Ser Gln Leu Ile Asn Gly Glu Ile Asp
   370 375 380
   Ile Glu Ala Asn Lys Glu Phe Trp Ser Tyr Phe Phe Asn Leu Tyr Lys
   385 390 395 400
   Asn Ser Asn Lys Gly Ile Lys Gly Arg Gly Lys Asp Arg Asn Ile Ser
   405 410 415
   Pro Arg Ser Arg Ser Ser Arg Asn Ser His Ala Ser Thr Gly Ser Asn
   420 425 430
   Ala Ser Arg Tyr Val Ser Pro Asn Met Arg Ser Cys His Gly Leu Met
   435 440 445
   Asp Thr Asp Gly Ala Ser Tyr Ser Asp Ala Thr Met Tyr Asp Ser Asp
   450 455 460
   Gly Ser Met Arg Ser His Thr Ser Gly Ser Met Ser Val Asn Ser Ser
   465 470 475 480
   Phe Ser Ser Gly Thr Thr Asp Ser Thr Ala Ser Thr Tyr Ser Asn Phe
   485 490 495
   Ser Asp Asp Ile Ala Thr Asp Tyr Gln Asp Pro His Ala Arg Asn Val
   500 505 510
   Arg Gly Asp Leu Ala Phe Arg Val Thr Arg Asp Val Glu Met Lys Met
   515 520 525
   Ser Thr Gln Asp Glu Asn Thr Glu Met Lys Gly Arg Thr Ala Val Leu
   530 535 540
   His Ser Phe Ile Ile Ile Ile Ile Ile Ile Thr Ile Ile Thr Gln His
   545 550 555 560
   Ser Thr Thr Pro Asn Thr Asn Ile Pro Pro Lys Lys Ala Arg Cys Ile
   565 570 575
   Phe Ile Glu Arg Ile Asn Leu Phe Leu Thr His Gln Ser Ser
   580 585 590
<210> 52
   <211> 320
   <212> PRT
   <213> Artificial Sequence
<220>
   <223> penmagi212542299refXP_002151304.1
<400> 52
   Met Ala Pro Gly Gly Ser Arg Gly Phe Asn Cys Ser Trp Glu Arg Cys
   1               5                   10                  15
   Gly Lys Ser Phe Asn Arg Lys Ser Asp Leu Cys Arg His Tyr Arg Ile
               20                  25                  30
   His Thr Asn Glu Arg Pro Tyr His Cys Thr Val Pro Ser Cys Asn Lys
           35                  40                  45
   Ser Phe Ile Gln Arg Ser Ala Leu Thr Val His Ser Arg Thr His Thr
       50                  55                  60
   Gly Glu Lys Pro His Val Cys Asp His Glu Asp Cys Arg Lys Ala Phe
   65                  70                  75                  80
   Ser Asp Ser Ser Ser Leu Ala Arg His Arg Arg Ile His Thr Gly Arg
                   85                  90                  95
   Arg Pro Tyr Ile Cys Ser Glu Pro Ala Cys Asp Arg Ser Phe Cys Arg
               100                 105                 110
   Lys Thr Thr Leu Thr Lys His Gln Ser Arg Thr His Gln Pro Gly Gly
           115                 120                 125
   Asn Thr Arg Pro Ser Ser Glu Glu Ala Pro Thr Pro Glu Ser Met Thr
       130                 135                 140
   Tyr Gln Thr Pro Val Ile Thr Thr Met Ala Pro Asn Thr Thr Gln Tyr
   145                 150                 155                 160
   Ile Ile Ala Gln Gln Pro Tyr Tyr His Asn Gly Pro Thr Pro Thr Thr
   165 170 175
   Ala Asp Phe Tyr Gln Thr Pro Gln Pro Ala Ile His Phe Thr Gln Pro
   180 185 190
   Gln Ala Met Val Pro Gln Gln Ile Pro Thr Ala Gly Leu Pro Thr Pro
   195 200 205
   Pro Phe Glu Pro Gln His Tyr Ala Gln Ile Met Gln Gln Gln Gln Gln
   210 215 220
   Pro Arg Tyr Asp Pro Thr Pro His Gln Thr Tyr Ile Gln Pro Gln Tyr
   225 230 235 240
   Gln Ser Pro Thr Pro Pro Phe Val Ala Gln His Gln His His Met Ala
   245 250 255
   Ala Asp Val Arg Pro Leu Met Ala Thr Tyr His Pro Asp Gln Phe Lys
   260 265 270
   Pro Pro Ala Arg Ile Leu Ser Ser Gln Glu Gly Thr Asp Trp Ser Phe
   275 280 285
   Leu Gly Cys Leu Asp Arg Cys Ser Pro Gly Pro Gly Thr Gln Glu Tyr
   290 295 300
   Ala Ile Pro Phe Ser Val Asp Glu Lys Gln Pro Leu Leu Gly Leu Ser
   305 310 315 320
<210> 53
   <211> 426
   <212> PRT
   <213> Artificial Sequence
<220>
   <223> ANG45333
<400> 53
   Met Val Ala Ala Asp Gly His Phe Asp His Gln His Leu Val Pro Asn
   1 5 10 15
   Pro Gly Leu Asp His Thr Ser Ser Pro Lys Met Lys Ser Glu Pro Gly
   20 25 30
   Val Ser Asp Ala Gly Asp Gln Ala Val Leu Ala Tyr Pro Pro His Gly
   35 40 45
   Pro Leu Gly Gln Val Pro Asn Met Ala His Asp Met Arg Tyr Ala His
       50              55              60
   Gln Ala His Pro Gly Pro Gly Leu Pro Leu Leu Gln Asn Pro Phe Val
   65              70              75              80
   Pro Gly Ala Tyr Ala Gly Asn Ala Gln Ile Pro Asn Ala Gly Ala Pro
                   85              90              95
   Gln Gly Arg Pro Asp Pro Pro Pro Lys Thr Phe His Cys Gly Thr Cys
               100             105             110
   Gly Lys Gly Phe Ala Arg Arg Ser Asp Leu Ala Arg His Glu Arg Ile
           115             120             125
   His Ser Gly Ile Arg Pro His Ala Cys Asp Trp Pro Gly Cys Gly Lys
       130             135             140
   Gln Phe Ile Gln Arg Ser Ala Leu Thr Val His Ser Arg Val His Thr
   145             150             155             160
   Gly Glu Lys Pro His Met Cys Glu Arg Cys Gly Lys Pro Phe Ser Asp
                   165             170             175
   Ser Ser Ser Leu Ala Arg His Arg Arg Ile His Ser Gly Lys Arg Pro
               180             185             190
   Tyr Lys Cys Pro Tyr Ala Asn Cys Gln Lys Thr Phe Thr Arg Arg Thr
           195             200             205
   Thr Leu Thr Arg His Gln Asn His His Thr Gly Thr Ile Glu Glu Ala
       210             215             220
   Ala Ala Glu Thr Glu Ala Asn Leu Arg Gln Asn Lys Glu Arg Ala Arg
   225             230             235             240
   Met Pro Gly Asp Met Phe Ser Glu His Gly Ser Val His Ser Thr Pro
                   245             250             255
   Ser Pro Ala His His Pro Ile Ser Pro Ala Gly Asp Leu Pro Pro Leu
               260             265             270
   Asn Met Pro Arg Ser Asp Tyr Tyr Met Pro Asn Gly Ser Ile Pro Pro
           275             280             285
   His Val Arg Gly Gly Phe Thr Gln Ala Ser Pro Arg Ala Ser Pro Thr
       290             295             300
   Ala Thr Ser Pro Ser Leu Ser Ser Tyr Gly Ser Ala Pro His Pro Arg
   305 310 315 320
   Pro Ser Met Thr Ser His Pro Ser Gly Tyr Gly Pro Pro Gln Pro Leu
   325 330 335
   Glu Pro Pro Ala Asn Asn Asp His Arg Pro Asn Ser Val Ser Gly Ser
   340 345 350
   Pro His Met Thr Ser Leu Gly Trp Pro Ser Pro Ser His Ala Ser Ile
   355 360 365
   Pro Ser Pro Gly Ser Ala Thr Asp Phe Tyr Pro Glu Thr Ser Gly Pro
   370 375 380
   Ala Tyr Pro Ser Ala Met Pro Pro His Met Tyr Phe Pro Asn Ser Thr
   385 390 395 400
   Ile Arg Arg Pro Thr Ser Thr Glu Pro Asp Ser Tyr Asp Leu Lys His
   405 410 415
   Lys Met Glu Ser Ala Trp Ser Thr Pro Val
   420 425
<210> 54
   <211> 312
   <212> PRT
   <213> Artificial Sequence
<220>
   <223> cersuGenemark.1435_g
<400> 54
   Met Val Arg Arg Arg Phe Pro Arg Thr Lys Val Asp Ala Thr Cys Asp
   1 5 10 15
   Gln Cys His Lys Ser Phe Gly Ser Leu Ala Asp Leu Lys Arg His Ser
   20 25 30
   Met Ile Gln Asp His Leu Asp Leu Arg Pro Tyr Ala Cys Thr Trp Pro
   35 40 45
   Gly Cys Thr Arg Ala Phe Pro Gln Ser Ala Ala Leu Lys Asn His Phe
   50 55 60
   Asn Thr His Thr Gly Lys Lys Pro Tyr Arg Cys Ala Trp Val His Glu
   65 70 75 80
   Cys Asp Arg Ala Phe Gly Asp Lys Ser Thr Cys Ser Arg His Glu Ala
                   85                  90                  95
   Thr Lys His Leu Gly Leu Ile Gly His Lys Cys Pro Val Lys His Cys
               100                 105                 110
   Arg Ser Ser Ile Lys Arg Lys Thr Asp Phe Ala Arg His Leu Arg Asp
           115                 120                 125
   Lys His Gly Ile Arg Thr Lys Gly Leu Asp Val Ser Gln Tyr Ala Val
       130                 135                 140
   Tyr Lys Pro Gly Gly Gly Pro Ile Lys Lys Cys Arg Lys Asn Lys Arg
   145                 150                 155                 160
   Val Glu Ser Ser Pro Glu Asn Ser Pro Leu Ala Cys Pro Leu Asp Asp
                   165                 170                 175
   Phe Val Glu Ser Pro Thr Ser Ser Gly Thr Thr Thr Pro Leu Pro Glu
               180                 185                 190
   Thr Pro Glu Met Gly Gln Gln Ala Glu Leu Pro Val Thr Asp Phe Ser
           195                 200                 205
   His Met Gly Leu Val Val Pro Ala Asp Glu Pro His His Ser Gln Ile
       210                 215                 220
   Ser Asn Ala Pro Phe Val Asp Thr Tyr Glu Lys His Asn Tyr Val His
   225                 230                 235                 240
   Ser Leu Pro Thr Gln Phe Met Ile Pro Pro Ser Ile Ser Pro Ala Val
                   245                 250                 255
   Gln Pro Gln Tyr Tyr Tyr Val Pro Glu Ala Tyr Gln Gln Thr Phe Ala
               260                 265                 270
   Ser Asp Pro Leu Ala Leu Val Ile Asp Pro Val Leu Met Met Glu Ser
           275                 280                 285
   Met Leu Tyr Gly Gly His Gly Thr Gln His Tyr Ala Asn Ala Pro Ile
       290                 295                 300
   Pro Leu Tyr Tyr Pro Ser Ser Cys
   305                 310
<210> 55
   <211> 454
   <212> PRT
   <213> Artificial Sequence
<220>
   <223> hiscnHCAG_06891T0
<400> 55
   Met Glu Thr Ala Val Pro Val Thr Tyr Asn Val Thr Asn His Asp Met
   1               5                   10                  15
   Asn Ala Val Ala His Arg Arg Met Met Ala Pro His Gln His His His
               20                  25                  30
   His His Asp Gln Asn Ile Ala Tyr Phe Ser Asn Pro Pro Ile Pro Tyr
           35                  40                  45
   Ser Thr Pro Leu Gln Pro Pro Ser Phe Ser Gly Phe Gly His Ile Leu
       50                  55                  60
   Asn Asn His Pro His His Thr Ser Tyr Gln Gly Tyr Phe Asn Ser Asn
   65                  70                  75                  80
   Pro Ser His Gln Val Asn Ser His His Pro Arg Leu Pro Thr Glu Thr
                   85                  90                  95
   Asn Pro Val Gln Gln Leu Ser Asp Val Arg His Val Lys Asn Ala Asn
               100                 105                 110
   Pro Arg Leu Ile Arg Pro Ser Pro Pro Lys Glu Asp Thr Leu Pro Pro
           115                 120                 125
   Pro Pro Pro Gln Arg Met Cys Val Pro Pro Ala Arg Asn Thr Thr Gln
       130                 135                 140
   His Glu Asp Ser Lys Pro Met His Lys Pro Glu Val Val Phe Gly Thr
   145                 150                 155                 160
   Glu Val Asp Thr Leu Met Lys Ala Ile Gln Ala Lys Pro Gln Pro Pro
                   165                 170                 175
   Ser Pro Gln Ala Glu His Gln Leu Pro Pro Leu Gln Gln Lys Phe Asn
               180                 185                 190
   Ser Gly Val Thr Asn Trp Ile His Pro Ala Tyr Ala Asn Gln Met Gly
           195                 200                 205
   Gly Asn Gln Ser Ile Phe Pro Asn Pro Pro Gln Asp Arg Ile Pro Pro
       210                 215                 220
   Ala Asn Gln Lys Pro Lys Arg Lys Tyr Asp Cys Thr Leu Pro His Cys
   225 230 235 240
   Arg Lys Ser Phe Phe Gln Lys Thr His Leu Asp Ile His Met Arg Ala
   245 250 255
   His Thr Gly Asp Lys Pro Phe Thr Cys Lys Glu Pro Ser Cys Gly Gln
   260 265 270
   Arg Phe Ser Gln Leu Gly Asn Leu Lys Thr His Glu Arg Arg His Thr
   275 280 285
   Gly Glu Lys Pro Tyr Ser Cys Glu Ile Cys His Lys Lys Phe Ala Gln
   290 295 300
   Arg Gly Asn Val Arg Ala His Lys Ile Thr His Glu Gln Ala Lys Pro
   305 310 315 320
   Phe Thr Cys Arg Leu Asp Asp Cys Gly Lys Gln Phe Thr Gln Leu Gly
   325 330 335
   Asn Leu Lys Ser His Gln Asn Lys Phe His Ala Gln Thr Leu Arg Asn
   340 345 350
   Leu Thr Leu Arg Phe Ala Ser Ile Ala Asp Ile Glu Arg Met Ser Pro
   355 360 365
   Gln Asp Lys Glu Leu Trp Ser Tyr Phe Ser Asn Leu Tyr Arg Asn Ser
   370 375 380
   Asn Lys Gly Ile Lys Gly Arg Gly Lys Asp Arg Arg Val Ser Thr Ala
   385 390 395 400
   Lys Arg Ser Met Ser Ala Tyr Asp Gly Ser Pro Ser Glu Ser Glu Asp
   405 410 415
   Asp Gly Lys Asp Arg Asn Arg Thr Tyr Asp Arg Ala Ser Ala Val Met
   420 425 430
   Thr Ser Asn Asn Asp Glu Pro Asp Tyr Arg Glu Gln Leu Tyr His Asn
   435 440 445
   Arg Asn Gly Gly His His
   450
<210> 56
   <211> 398
   <212> PRT
   <213> Artificial Sequence
<220>
   <223> neuteestExt_Genemark.C_40809
<400> 56
   Met Ala Glu Asp Pro Glu Arg Pro Arg Pro Phe Leu Cys Asp Trp Gln
   1               5                   10                  15
   Gly Cys Gly Lys Arg Phe Asn Arg Lys Ser Asp Leu Gln Arg His His
               20                  25                  30
   Arg Ile His Thr Asn Glu Arg Pro Tyr Gly Cys Asn Trp Gln Asp Cys
           35                  40                  45
   Gly Lys Arg Phe Ile Gln Arg Ser Ala Leu Thr Val His Leu Arg Thr
       50                  55                  60
   His Thr Gly Glu Lys Pro His Gln Cys Glu Val Pro Glu Cys Arg Lys
   65                  70                  75                  80
   Pro Phe Ala Asp Ser Ser Ser Leu Ala Arg His Arg Arg Ile His Arg
                   85                  90                  95
   Gly Glu Lys Pro Tyr Arg Cys Gln Asp Val Gly Cys Asn Lys Ala Phe
               100                 105                 110
   Cys Arg Lys Thr Thr Arg Asp Lys His His Thr Asn Ser His Arg Gly
           115                 120                 125
   Gly Ile Asn Arg Ser Met Thr Pro Arg Gln Gln Leu His Pro Ser Phe
       130                 135                 140
   Gly Ser Gly Phe Asp Gln Phe His Met Ser Ala Pro Pro Glu Leu Thr
   145                 150                 155                 160
   Ala Ser Thr Asp Ser Ser Pro Ala Asp Ser Thr Ala Tyr Ser Pro Ala
                   165                 170                 175
   Ala Leu Glu Gly Asp Trp Ser Asn Gln Asn Phe Asn Asn Tyr Thr His
               180                 185                 190
   Leu Pro Asn Leu Gln Glu Ala Ser Ile Ala Leu Gln Asn Gln Ile Arg
           195                 200                 205
   Ser Gln Gln Ile Pro Gln Leu Met Ser Tyr Met Pro Ser Arg Val Leu
       210                 215                 220
   Pro Ser Tyr Ser Ala Pro Pro Pro Asn Met Ile Thr Pro Gln Met Pro
   225 230 235 240
   Gln Gln Gly Ser Phe Ser Ser Tyr Ser Pro Ser Pro Val Glu Ser Ala
   245 250 255
   Gln Gln Gln Pro Trp Ala Thr Gly Phe Gln Asn Asn Gln Arg Gln Tyr
   260 265 270
   Pro Arg Val Leu Thr Ser Asn Asp Thr Ala Pro Arg Met Phe Ser Pro
   275 280 285
   Tyr Pro Ala Pro Pro Asn Met Leu Pro Gly Gln Asn Asp Phe Asn Ser
   290 295 300
   Gln Tyr Pro Pro Pro Gln Gln Gln Gln Ser Trp Gly Met Pro Gln Asn
   305 310 315 320
   Gln Gly Gln Gln Asp Gln Arg Gln Tyr Ser Leu Pro Ser Pro Ser Gln
   325 330 335
   Pro Leu Thr Pro Val Pro Val Ser Ala Gly Thr Gly Thr Met Gln Pro
   340 345 350
   Leu Gln Pro Met Pro Gly His Ile Tyr Gly Ala Arg Leu Val Gly Asp
   355 360 365
   Asp Leu Asn Asp His Phe Phe Lys Val Glu Thr Thr Ala Asp Asp Asn
   370 375 380
   Asn Pro Gly Met Thr Leu Pro Asp Ala Arg Phe Ala Gly Leu
   385 390 395
<210> 57
   <211> 448
   <212> PRT
   <213> Artificial Sequence
<220>
   <223> spothe_gw1.5.2916.1
<400> 57
   Met Gly Ile Gly Asn Val Leu Asn Ile Lys Ala Glu Ser Gly Gly Gln
   1 5 10 15
   Gln Ala Ser Gln Gln Gly Ala Thr Ser Glu Leu Gln Gln Gln Arg Pro
   20 25 30
   Pro Gln Gln Leu Gln Gln Leu Gln Val Asp Arg Gln Gly Ser Pro Gln
   35 40 45
   Gly Ser Glu Gln Pro Arg Tyr Ser Gly Pro Met Ser Ala Ser Tyr Pro
   50 55 60
   Ser Pro Thr Ala Met Ala Pro Ala Pro Leu Pro Pro Val Pro Asn Ala
   65 70 75 80
   Asn Met Ala Pro Ala Pro Met Val Pro His Glu Met Gln Gln Asn Ile
   85 90 95
   Gly Ile Val His Pro Ala Tyr Gln Gln Thr Gln Gln Pro Pro Gln Ala
   100 105 110
   Pro Ala His Gln Pro Pro Ala Lys Gln Phe Pro Cys Ser Thr Cys Gly
   115 120 125
   Lys Pro Phe Ala Arg Arg Ser Asp Leu Ala Arg His Glu Arg Ile His
   130 135 140
   Ser Gly Ile Arg Pro His Val Cys Asp Tyr Pro Gly Cys Gly Lys Gln
   145 150 155 160
   Phe Ile Gln Arg Ser Ala Leu Thr Val His Ser Arg Val His Thr Gly
   165 170 175
   Glu Lys Pro His Arg Cys Glu Arg Cys Gly Lys Pro Phe Ser Asp Ser
   180 185 190
   Ser Ser Leu Ala Arg His Arg Arg Ile His Ser Gly Lys Arg Pro Tyr
   195 200 205
   Lys Cys Pro Tyr Ala Asp Cys Gln Lys Thr Phe Thr Arg Arg Thr Thr
   210 215 220
   Leu Thr Arg His Gln Asn His His Val Gly Thr Val Glu Asp Ser Ala
   225 230 235 240
   Arg Ala Arg Ala Glu Ala Leu Ala Gln Gly Ala Asn Ala Ala Ala Ala
   245 250 255
   Ala Ala Ala Ala Ala Ala Ala Ala Arg Asn Lys Ser Gln Ser Ala Ser
   260 265 270
   Glu Gln Ala Ser Asn His Glu Ser Pro Ile Thr Thr Pro Ser Pro Gly
   275 280 285
   Gln Arg Pro Ile Ser Met Ser Pro Gly Ala Glu Leu Ala Gly Met Asn
   290 295 300
   Asn Met Gln Tyr Leu Ser Asn Ser Ile Pro Ala His Leu Arg Gly Asp
   305 310 315 320
   Val His Val Gly Ser Pro Ser Pro Thr Ala Ser Thr Gly Tyr Asn Asn
   325 330 335
   Gly Met Arg Pro Thr Ser His Pro Thr Gly Tyr Ala Pro Pro Gln Thr
   340 345 350
   Leu Glu Pro Ser Ile Glu Gln Pro Gln Gln Gly Pro Gly Ser Ala Val
   355 360 365
   Gly Ser Pro His Ile Gly Ser Val Gly Trp Ala Ser Pro Gly Pro Val
   370 375 380
   Gly Ser Pro Thr Gln Ser Pro Asn Gly Asn Gly Tyr Val Tyr Pro Asp
   385 390 395 400
   Pro Glu Ser Tyr Pro Ser Gly Thr Pro Ile Ala Gln Met Phe Tyr Asn
   405 410 415
   Asn Ala Val Ala Thr Arg Arg Pro Glu Ser Ala Glu Pro Ala Asn Pro
   420 425 430
   Ser Phe Asp Ala Lys Gly Arg Gln Gly Glu Leu Trp Thr Asn Ala Gln
   435 440 445
<210> 58
   <211> 416
   <212> PRT
   <213> Artificial Sequence
<220>
   <223> capowCAOG_08885T0
<400> 58
   Asn Gly Leu Val Ala Ala Pro Thr Ala Pro Ser Thr Thr Thr Thr Ala
   1 5 10 15
   Val Gly Ser Ala Ala Ser Gly Ala Gly Met Gly Ile His Asn Asn Pro
   20 25 30
   Ala Val Met Pro Pro Arg Thr Val Pro Met Val Asn Ser Phe Gly Glu
   35 40 45
   Ala Ala Pro Ala Gly Gly Asp His Trp Pro Lys Thr Pro Phe Met Gly
       50                  55                  60
   Ser Ser Asp Ala Ser Val Thr Thr Ser Ser Ile Ser Ser Thr Ser Ser
   65                  70                  75                  80
   Ser Ser His Arg Arg Asp Arg Leu Gly Arg Asp Arg Glu Arg Asp His
                   85                  90                  95
   His Ser Ser Arg Asp His His His His Ala Arg Glu Arg Asp His Ser
               100                 105                 110
   Ser Ser Gly Lys Arg His His His His Ser Ser Arg Arg His Ser His
           115                 120                 125
   His Arg Ser Ser Arg Arg Gln Ser Asp Asp Ser Asp Ser Ala Gly Ser
       130                 135                 140
   Asp Glu Asp Gly Ser Tyr Ala Arg Gly Gly Leu Arg Arg Ala Arg Asn
   145                 150                 155                 160
   His Ser Ser Asp Ser Gly Ser Ser Ser Ala Glu Asp Ser Asp Asp Thr
                   165                 170                 175
   Asp Ala Glu Asp Asp Ser Arg Arg His Thr Thr Ala Val Thr Arg Ala
               180                 185                 190
   Asn Ala Arg His Ala Leu Ala Glu Gly Phe Leu Leu Asp Asp Val Thr
           195                 200                 205
   Cys Gly Gln Cys Gly Lys Ile Phe Arg Arg Pro Asp Arg Leu Ser Ala
       210                 215                 220
   His Val Arg Thr His Ala Thr Met Arg Pro Phe Val Cys Thr Met Ala
   225                 230                 235                 240
   Asp Cys Ser Lys Thr Phe Arg Glu Lys Ala Ala Leu Lys Lys His Met
                   245                 250                 255
   Ile Ser His Gly Asp Ile Arg Pro Tyr Thr Cys Glu Arg Cys Gly Ala
               260                 265                 270
   Gly Phe Lys Phe Asn His His Leu Lys Arg His Asp Thr Arg Cys Leu
           275                 280                 285
   His Arg Leu Asn Arg Ile Cys Gly Ser Lys Ala Pro Pro Thr Val Gln
       290                 295                 300
   Gln Arg Ala Leu Glu Met Leu Ser Gln Ala Gly Phe Arg Ala Val Ala
   305 310 315 320
   Gly Thr Asp Ser Val Asn Leu Val Met Phe Asn Pro Glu Ser Met Ala
   325 330 335
   Thr Thr Lys Pro Glu Glu Ala Met Ser Gly Thr Ala Pro Ser Val Ser
   340 345 350
   Ser Pro Glu His Glu His Asn Met Ser Ala Ser Asp Asp Ala Ser Glu
   355 360 365
   Ala Ser Ser Asn Gln His Ser Ala Ser Ser Leu Ser Ala Thr Phe Asp
   370 375 380
   Leu Pro Thr Ser Trp Lys Pro Glu Asp Ser Gly Ala Ala Asp Ala His
   385 390 395 400
   Ser Arg Tyr Ser Leu Val Pro Val Ala Pro Ser Ala Thr Pro Val Ala
   405 410 415
<210> 59
   <211> 403
   <212> PRT
   <213> Artificial Sequence
<220>
   <223> EfO2.082100.1
<400> 59
   Met Glu Arg Ser Val Ser Pro His Met Ser Glu His Ser Ser Tyr Ser
   1 5 10 15
   Thr Pro His Ser Ile Ala Arg Ser Tyr Thr Ser Pro Ser Ala Met Gln
   20 25 30
   Ala Pro Met His Met Pro Ser Ser Met Thr Gly Gln Met Ala Met Pro
   35 40 45
   Thr Phe Ser Asp Met Pro Gly Ala Met Gly Thr Val His Asn Met Thr
   50 55 60
   Met His His Met Pro Gln Gln His Gln Pro Pro Pro Gln Pro Ser Lys
   65 70 75 80
   Ser Phe Leu Cys Gly Thr Cys Ser Lys Ser Phe Ala Arg Arg Ser Asp
   85 90 95
   Leu Ala Arg His Gly Leu Arg Pro His Val Cys Asp His Leu Gly Cys
   100 105 110
   Gly Lys Gln Phe Ile Gln Arg Ser Ala Leu Thr Val His Lys Arg Val
   115 120 125
   His Thr Gly Glu Lys Pro His His Cys Glu Thr Cys Ala Lys Arg Phe
   130 135 140
   Ser Asp Ser Ser Ser Leu Ala Arg His Arg Arg Thr His Thr Gly Asn
   145 150 155 160
   Arg Pro Tyr Lys Cys Pro Tyr Ala Asp Cys Gln Lys Thr Phe Thr Arg
   165 170 175
   Arg Thr Thr Leu Thr Arg His Gln Asn His His Ser Gly Thr Ile Glu
   180 185 190
   Glu Ala Ala Ala Ala Thr Ala Ala Ala Leu Ala Ala Ser Lys Ala Gln
   195 200 205
   Lys Gly Gly Thr Arg Thr Arg Ala Asp Gly Asp His Leu Ser Ser His
   210 215 220
   Gly Ser Pro Met Thr Thr Pro Ser Pro Ser His Arg Thr Met Ser Met
   225 230 235 240
   Ser Pro Ser Leu Asp Met Gly Ala Ala Asn Asn Met Asn Arg His Pro
   245 250 255
   Gly Asp Phe Gln Tyr Leu Gln Gln Thr Gly Ser Leu Pro Pro His Leu
   260 265 270
   Arg Gly Val Gly Ser Pro Asn Ser Thr Ser Ser Asn Gly Tyr Asn Asn
   275 280 285
   Asn Asn Asn Asn Asn Asn Asn Asn Asn Asn Asn Asn Asn Ser Asn Ser
   290 295 300
   Asn Asn Gly Met Arg Pro Thr Ser His Pro Asn Thr Tyr Gly Pro Pro
   305 310 315 320
   Pro Ile Leu Glu Pro Asn Leu Glu Gln His Gln Ser Gly Pro Gly Ser
   325 330 335
   Met Gly Gly Ser Pro His Met Thr Ser Leu Gly Trp Gln Ser Pro Ser
   340 345 350
   His Val Pro Ser Pro Ser Gln Asn Gly Ala Ser Tyr Val Tyr Pro Glu
   355 360 365
   Pro Asp Thr Tyr Ser Gln Asn Ala Ala Met Asn Gln Met Tyr Tyr Gly
   370 375 380
   Ala Pro Pro Gln Met Arg Arg Gln Gln Ser Thr Glu Pro Gly Leu Val
   385 390 395 400
   His Met Ala
<210> 60
   <211> 453
   <212> PRT
   <213> Artificial Sequence
<220>
   <223> pyrtrPTRT_01511
<400> 60
   Met Gln Gln Gln His Phe Gly Ala Pro Pro Ser Tyr Leu Asn Gly Gly
   1 5 10 15
   His Ile Lys Ser Glu Asn Gly Ser Glu Arg Gly Val Ser Pro His Pro
   20 25 30
   Ser Asp Ser Ser Arg Tyr Ser Ser Gln Gln Pro Gln Gln Gln Leu Pro
   35 40 45
   Ser Tyr Pro Ser Ile Pro Gln Gln His Met Asn Gly Leu Arg Tyr Pro
   50 55 60
   Ser Pro Ser Gln Met Gln Ala Pro Met Pro Met Leu Asn Asn Ser Asn
   65 70 75 80
   Tyr Ile Pro Thr Pro Pro Glu His Thr Tyr Ala Gln Gln Gln Gln Leu
   85 90 95
   Pro Asp Ala Gln Val Gly His Gln His Asn Gly Gly Arg Pro Ala Ser
   100 105 110
   Asp Thr Gly Pro Pro Lys Ala Phe Ala Cys Ser Thr Cys Gly Lys Gly
   115 120 125
   Phe Ala Arg Arg Ser Asp Leu Ala Arg His Glu Arg Ile His Ser Gly
   130 135 140
   Ile Arg Pro His Val Cys Asp Tyr Pro Asn Cys Gly Lys Gln Phe Ile
   145                 150                 155                 160
   Gln Arg Ser Ala Leu Thr Val His Gln Arg Val His Thr Gly Glu Lys
                   165                 170                 175
   Pro His Met Cys Glu Arg Cys Gly Lys Pro Phe Ser Asp Ser Ser Ser
               180                 185                 190
   Leu Ala Arg His Arg Arg Ile His Ser Gly Lys Arg Pro Tyr Lys Cys
           195                 200                 205
   Pro Tyr Ala Asp Cys Gln Lys Thr Phe Thr Arg Arg Thr Thr Leu Thr
       210                 215                 220
   Arg His Gln Asn His His Thr Gly Thr Val Glu Glu Ala Ala Ala Ala
   225                 230                 235                 240
   Thr Ala Ala Ala Leu Ala Ser Arg Ala Ser Gln Ser Ser Arg Thr Gly
                   245                 250                 255
   Arg Ser Asp Gly Gly Asp Tyr Ser Glu Thr Ala Ser Pro Leu Gly Thr
               260                 265                 270
   Pro Ser Pro Asn Asp Arg Thr Leu Ser Leu Ser Pro Ala Asn Gly Met
           275                 280                 285
   Pro Ala Gly Gly Met Pro Gly Leu His Arg Gln Gly Ser Asp Tyr Ala
       290                 295                 300
   Tyr Met Gly Gly Met Asn Val Pro Pro His Met Arg His Glu Met Pro
   305                 310                 315                 320
   Gln Pro Ser Pro Arg Ala Ser Pro Ala Leu Thr Ala Gln Ser Tyr Ala
                   325                 330                 335
   Ser Asn Val Ser Gly Ser Arg Pro Ala Ile Thr Ser His Pro Ser Ala
               340                 345                 350
   Tyr Gly Pro Pro Pro Ile Leu Glu Pro Pro Ala Ser Ala Asn His Gly
           355                 360                 365
   Gln Ser Gly Gly Thr Ser Ser Ala Asn Gly Ser Pro His Met Ser Thr
       370                 375                 380
   Met Gly Trp Gln Ser Pro Gly Gln Gln Ala Leu Pro Ser Pro Gly Ala
   385                 390                 395                 400
   Asn Asp Asn Gly Tyr Val Tyr Pro Glu Pro Gln Tyr Gln Ala Gln Asn
   405 410 415
   Gly Met Tyr Tyr Gln His Asn Asn Ile Arg Arg Pro Asn Ser Thr Glu
   420 425 430
   Pro Asp His Tyr Asn Pro Asn Gln Gln Arg Met Gly Asn Glu Met Trp
   435 440 445
   Ala Pro Ala Val Gln
   450
<210> 61
   <211> 381
   <212> PRT
   <213> Artificial Sequence
<220>
   <223> sormaSMAC_08865
<400> 61
   Met Glu Leu Phe Gln Ile Met Ala Glu Asp Pro Glu Arg Pro Arg Pro
   1 5 10 15
   Phe Leu Cys Asp Trp Gln Gly Cys Gly Lys Arg Phe Asn Arg Lys Ser
   20 25 30
   Asp Leu Gln Arg His His Arg Ile His Thr Asn Glu Arg Pro Tyr Gly
   35 40 45
   Cys Asn Trp Gln Asp Cys Gly Lys Arg Phe Ile Gln Arg Ser Ala Leu
   50 55 60
   Thr Val His Leu Arg Thr His Thr Gly Glu Lys Pro His Gln Cys Glu
   65 70 75 80
   Val Pro Glu Cys Arg Lys Pro Phe Ala Asp Ser Ser Ser Leu Ala Arg
   85 90 95
   His Arg Arg Ile His Arg Gly Glu Lys Pro Tyr Arg Cys Gln Asp Val
   100 105 110
   Gly Cys Asn Lys Ala Lys Thr Thr Arg Asp Lys His His Ser Asn Ser
   115 120 125
   His Arg Gly Gly Ile Asn Arg Ser Met Thr Pro Arg Gln Gln Leu His
   130 135 140
   Pro Thr Phe Ser Ser Glu Phe Asp Gln Phe His Met Ser Ala Pro Pro
   145 150 155 160
   Asp Leu Thr Ala Ser Thr Glu Ser Ser Pro Ala Asp Ser Thr Ala Tyr
   165 170 175
   Ser Pro Ala Ala Leu Glu Gly Asp Trp Ser Asn Gln Ser Phe Asn Asn
   180 185 190
   Tyr Thr His Leu Pro Asn Leu Gln Glu Ala Ser Leu Ala Leu Gln Asn
   195 200 205
   Gln Ile Arg Ser Gln Gln Ile Pro Gln Leu Met Pro Tyr Met Pro Ser
   210 215 220
   Arg Val Met Pro Ser Tyr Ser Ala Pro Pro Ser Asn Met Met Thr Gln
   225 230 235 240
   Gln Met Pro Gln Gln Gly Asn Phe Ser Ser Tyr Ser Pro Ser Pro Val
   245 250 255
   Glu Ser Thr Gln Gln Gln Pro Trp Gly Thr Gly Cys Ser Arg Pro Ile
   260 265 270
   Arg Leu Pro Pro Asn Met Met Pro Gly Gln Asn Asp Phe Asn Ser Gln
   275 280 285
   Tyr Pro Pro Pro Gln Gln Gln Gln Ser Trp Gly Met Pro Gln Asn Gln
   290 295 300
   Gly Gln Gln Glu Gln Arg Gln Tyr Ser Leu Pro Ala Pro Ser Gln Pro
   305 310 315 320
   Met Thr Pro Val Pro Val Ser Ala Gly Thr Gly Asn Ile Gln Pro Met
   325 330 335
   Gln Pro Met Pro Gly His Ile Tyr Gly Ala Arg Leu Val Gly Asp Asp
   340 345 350
   Leu Ser Asp His Phe Phe Lys Val Glu Thr Ser Ala Asp Asp Asn Asn
   355 360 365
   Pro Gly Met Thr Leu Pro Asp Ala Arg Phe Ala Gly Leu
   370 375 380
<210> 62
   <211> 411
   <212> PRT
   <213> Artificial Sequence
<220>
   <223> BC_P041110.1
<400> 62
   Met Asp Val Met Glu Leu Val Glu Asn Glu Gln Asn Ala Arg Pro Phe
   1               5                   10                  15
   Gln Cys Asp Trp Lys Thr Cys Thr Lys Ser Phe Asn Arg Lys Ser Asp
               20                  25                  30
   Leu Gln Arg His Tyr Arg Ile His Thr Asn Glu Arg Pro Tyr Ser Cys
           35                  40                  45
   Val Thr Pro Gly Cys Gly Lys Ser Phe Ile Gln Arg Ser Ala Leu Thr
       50                  55                  60
   Val His Ile Arg Thr His Thr Gly Glu Lys Pro His Gln Cys Gln His
   65                  70                  75                  80
   Ile Gly Cys Gly Lys Arg Phe Ser Asp Ser Ser Ser Leu Ala Arg His
                   85                  90                  95
   Arg Arg Ile His Thr Gly Lys Arg Pro Tyr Lys Cys Ala His Asp Gly
               100                 105                 110
   Cys Leu Lys Ser Phe Cys Arg Lys Thr Thr Met Val Lys His Gln Arg
           115                 120                 125
   Arg Ser His Gln Arg Gly Ile His Ser Ser Glu Leu Asp Asp Gly Glu
       130                 135                 140
   Thr Ser Asp Ser Asp Ser Gly Glu Ser Pro Thr Thr Pro Gln His His
   145                 150                 155                 160
   Ser Ser Gln Ile Gln Trp Pro Gln His Leu Thr Val Pro Gly Val Pro
                   165                 170                 175
   His Gly His Gln Met His Arg Ala His Ser Phe Ala Asp Phe Gly Gln
               180                 185                 190
   His Gln His Ile Asp Gly Tyr Pro Met Pro Gln Ala Tyr Ala His Arg
           195                 200                 205
   His Ser Leu Ser Gly Gly Pro Gln Ala Tyr Gly Pro Ile Pro Asp Gln
       210                 215                 220
   His His His Val Met Gln Arg Gln Pro Ser Leu Gln His Ala Ser Tyr
   225 230 235 240
   Tyr Val Pro Glu Gln Asn Asn Pro Gly Val Ala Thr Leu Asn Thr Asn
   245 250 255
   Pro Ala Pro Ile Gln Thr Tyr His Ile Pro Arg His Ser Met Glu Arg
   260 265 270
   His Pro Gln Glu Ile Leu Gln Ser Ser Pro Gly Ser Tyr Ser Ser His
   275 280 285
   Ser Arg Ala Ser Pro Val Ser Gln Glu Pro Tyr Tyr Thr His Ala Gln
   290 295 300
   His Ser Val His Val Pro Thr Tyr Ala Ile His Gly Gln Ser Pro Thr
   305 310 315 320
   Glu Ser Gln Pro Met Ile His Tyr Gln Gln His Val Pro Thr Pro Gln
   325 330 335
   Ser Ile Pro Ser Pro Val Ala Gln Val Gln His Gln Leu Gln His Gln
   340 345 350
   Tyr Pro Gln Pro Pro Glu His Gly Gln Trp Tyr Glu Ser Thr Pro Tyr
   355 360 365
   Gln Gln Pro Glu Val Ile Ser Asn Val Pro Met Tyr Pro Gln Val Thr
   370 375 380
   Val Phe Ser Asp Pro Trp His Lys Leu Glu Thr Phe Asp Asp Pro Ser
   385 390 395 400
   Leu Gln Met Pro Ser Ala Arg Ile Glu Asn Leu
   405 410
<210> 63
   <211> 367
   <212> PRT
   <213> Artificial Sequence
<220>
   <223> walseestExt_fgeneshl_kg.C_130041
<400> 63
   Met Asp Ile Val Asn Leu Val Asn Asn Ala Asp Thr Arg Lys Ser Ser
   1               5                   10                  15
   Lys Asn Ala Ser Thr Gln Pro Arg Pro Phe Ala Cys Thr Tyr Gly Asn
   20 25 30
   Cys Thr Lys Ala Phe Ala Arg Arg Ser Asp Leu Gln Arg His Leu Arg
   35 40 45
   Ile His Leu Asn Glu Arg Pro Phe Ala Cys Pro Ile Asp Gly Cys Ser
   50 55 60
   Lys Thr Phe Ile Gln Arg Ser Ala Leu Thr Val His Ile Arg Val His
   65 70 75 80
   Thr Gly Glu Arg Pro His Thr Cys Glu Gln Cys Gly Lys Ala Phe Ser
   85 90 95
   Asp Ser Ser Ser Leu Ala Arg His Arg Arg Ile His Thr Gly Lys Arg
   100 105 110
   Pro Tyr Lys Cys Pro Val Glu Gly Cys Asn Lys Thr Phe Cys Arg Lys
   115 120 125
   Thr Thr Leu Thr Lys His Thr Lys Arg Asn His Gln Ser Gln Ser Ser
   130 135 140
   Leu Ser Asn Ser Pro Ser Pro Ser Pro Ser Pro Glu Ala Gln Gln Ser
   145 150 155 160
   Asn Val Pro Ala Leu Thr Ile Pro Gln Pro Leu Thr Gln Gln Leu Ser
   165 170 175
   Gln Ser Pro Leu Gln Cys Leu Pro Ile Thr Gln Pro Ser Leu Tyr Ala
   180 185 190
   Leu Ser Gln Glu Glu Lys Pro Gln Phe Met Asp Asn Ser Gln Phe Arg
   195 200 205
   Leu Gln Ser Pro Ile Cys Met Ser Ser Tyr Gln Ser Gln Glu Ser Pro
   210 215 220
   Ser Asn Ser Thr Val Phe Ser Ser Pro Thr Thr Pro Thr Tyr Ala Thr
   225 230 235 240
   Glu Ala Gln Phe Leu Tyr Gln His Pro Pro Pro Pro Gln Gly Ala Phe
   245 250 255
   Ser Tyr Tyr Glu Ser Thr Ser Pro Phe Leu Tyr Val Asp Thr Ser Asn
   260 265 270
   Gln Lys Ser Gln Met Gly Leu Asn Asn Gln Ser Gln Leu Leu Thr Pro
           275                 280                 285
   Pro Gln Ser Ala Asp Pro Asn Asn Phe Gly Pro Phe Ala Tyr Val Pro
       290                 295                 300
   Pro Pro Leu Ala Val Thr Asp Asp Tyr Asn Thr Tyr Pro Phe Val Pro
   305                 310                 315                 320
   Pro Ser Pro Pro Leu Thr Gly Asn Cys Glu Thr Ser Tyr Ser Asn Gln
                   325                 330                 335
   Ser Leu Ile Asp Glu Ser His Tyr His His Glu Thr Tyr Pro His Pro
               340                 345                 350
   Asn Val Leu Thr Gly Leu Gly Leu Asp Leu Val Gln Asp Gly Glu
           355                 360                 365
<210> 64
   <211> 320
   <212> PRT
   <213> Artificial Sequence
<220>
   <223> talstgi242768578refXP_002341597.1
<400> 64
   Met Ala Pro Gly Gly Ser Arg Gly Phe Asn Cys Ser Trp Glu Asn Cys
   1               5                   10                  15
   Gly Lys Ser Phe Asn Arg Lys Ser Asp Leu Cys Arg His Tyr Arg Ile
               20                  25                  30
   His Thr Asn Glu Arg Pro Tyr His Cys Thr Val Pro Ser Cys Asn Lys
           35                  40                  45
   Ser Phe Ile Gln Arg Ser Ala Leu Thr Val His Ser Arg Thr His Thr
       50                  55                  60
   Gly Glu Lys Pro His Val Cys Asp His Glu Asp Cys Arg Lys Ala Phe
   65                  70                  75                  80
   Ser Asp Ser Ser Ser Leu Ala Arg His Arg Arg Ile His Thr Gly Arg
                   85                  90                  95
   Arg Pro Tyr Ile Cys Ser Glu Pro Ala Cys Asp Arg Ser Phe Cys Arg
               100                 105                 110
   Lys Thr Thr Leu Thr Lys His Gln Ser Arg Thr His Gln Pro Gly Val
               115                 120                 125
   Asn Thr Arg Pro Ser Ser Glu Glu Ala Pro Thr Pro Glu Ser Met Thr
       130                 135                 140
   Tyr Gln Thr Pro Val Met Thr Thr Met Pro Pro Asn Ser Asn Gln Tyr
   145                 150                 155                 160
   Ile Leu Ala Gln Gln Pro Tyr Tyr His Asn Gly Pro Thr Pro Thr Thr
                   165                 170                 175
   Ala Asp Phe Tyr Gln Thr Pro Gln Pro Ala Ile His Phe Pro Gln Pro
               180                 185                 190
   Gln Ala Met Val Pro Gln Pro Ile Pro Thr Ala Gly Leu Pro Thr Pro
           195                 200                 205
   Pro Phe Glu Pro Gln His Tyr Ala Gln Ile Met Gln Gln Gln Gln Pro
       210                 215                 220
   Arg Tyr Asp Pro Thr Tyr Ile Gln Pro Gln Tyr Gln Ser Pro Thr Pro
   225                 230                 235                 240
   Pro Phe Val Ala Gln Pro Gln His His Met Thr Ala Asp Gly Arg Pro
                   245                 250                 255
   Ile Val Ala Thr Tyr His Pro Asp Gln Phe Lys Pro Pro Ala Arg Ile
               260                 265                 270
   Leu Ser Ser Gln Glu Ala Met Leu Ile Arg Phe Ser Pro Ala Leu Ser
           275                 280                 285
   Thr Leu Ala Gly Gly Ala Thr Pro Asp Pro Glu His Arg Thr Pro Asn
       290                 295                 300
   Phe Gly Ser Gly Val Met Arg Thr Ser His Ser Trp Pro Phe Pro Pro
   305                 310                 315                 320
<210> 65
   <211> 295
   <212> PRT
   <213> Artificial Sequence
<220>
   <223> picmegw1.4.641.1
<400> 65
   Gln Gln Gln Gln Gln Gln Gln Arg Gln Gln His Arg Gln Gln Thr Pro
   1               5                   10                  15
   Gln Gln His Glu Arg Tyr Glu Lys Gln Gln His Pro Gln Asp Tyr Asn
   20 25 30
   Ser Asn Ser Asn Asn Ser Asn Asn Asn His Asn Asn Asn Ile Ser Ser
   35 40 45
   Ser Asn Thr Asn Asn Thr Lys Glu Lys Lys Leu Ile Asn Ser Asn Ser
   50 55 60
   Ser Leu Lys Arg Asp Asp Gly Arg Pro Leu Val Gly Ala Thr Lys Val
   65 70 75 80
   Asp Gln Leu Met Leu Ile Ile Gln Ala Arg Gln Lys Gly Val Cys Gly
   85 90 95
   Asn Ile Glu Gln Ser Glu Asp Gly Asn Ile Asp Glu Glu Asn Ile Leu
   100 105 110
   Pro Asn Pro Ile Glu Leu Val Gly Gly Val Glu Lys Thr Ser Leu Lys
   115 120 125
   Gly Asp Lys Ser His Gln Cys Lys Tyr Cys Gln Lys Lys Phe Thr Gln
   130 135 140
   Ser Thr His Leu Asp Val His Leu Arg Ser His Met Gly Val Lys Pro
   145 150 155 160
   Tyr Lys Cys Glu Phe Cys Asp Lys Arg Phe Thr Gln Gly Gly Asn Leu
   165 170 175
   Arg Thr His Met Arg Leu His Thr Gly Glu Lys Pro Phe Lys Cys Asn
   180 185 190
   Ile Cys Asp Lys Gln Phe Ser Arg Lys Gly Asn Leu Gln Ala His His
   195 200 205
   Leu Thr His Asp Asn Ser Lys Pro Phe Ile Cys Lys Phe Asp Gly Cys
   210 215 220
   Asn Lys Gly Phe Thr Gln Leu Gly Asn Leu Lys Ala His Gln Asn Arg
   225 230 235 240
   Phe His Leu Asp Thr Ile Asn Glu Leu Phe Asn Lys Leu Ala Gln Leu
   245 250 255
   Gly Glu Gly Ile Gly Ser Tyr Asp Ser Leu Pro Lys Ser Glu Lys Asp
   260 265 270
   Leu Leu Asp Tyr Phe Ser Ser Leu Tyr Lys Asn Leu Asn Lys Gly Ile
   275 280 285
   Lys Gly Arg Gly Lys Ala Arg
   290 295
<210> 66
   <211> 494
   <212> PRT
   <213> Artificial Sequence
<220>
   <223> podanPa_4_8860chrm4Putative
<400> 66
   Met Ser Gln Ser Pro Ser Ala Ser Gly Ala Met Gly Ile Gly Ser Val
   1               5                   10                  15
   Leu Asn Asn Lys Gly Ala Ala Ala Ala Gln Ala Leu Gln Gln Ala Gly
               20                  25                  30
   Ala Met Pro Val Asp Gln Gln Val Gln Gln Gln Leu Gln Gln Gln Val
           35                  40                  45
   Gln Gln Val Gln Gln Val Gln Gln Gln Val Gln Gln Gln Ala Gln Gln
       50                  55                  60
   Val Gln Gln Ala Gln Gln Leu Gln Gln Ala Gln Gln Pro Leu Gln Gln
   65                  70                  75                  80
   Val Gln Gln Gln Gln Gln His Gln Gln Gly Gln Thr Ile Pro Gln Gln
                   85                  90                  95
   Val Pro Met Asn Arg Pro Asn Ser Pro His Gly Ser Glu Asn Ser Gly
               100                 105                 110
   Tyr Thr Tyr Pro Ser Pro Thr Ala Ile Gly Gly Ala Pro Leu Pro Pro
           115                 120                 125
   Ala Asn Met Ala Pro Ala Pro Met Gly Ile Pro Met Gln Gln Thr Met
       130                 135                 140
   Gln Gln Pro Met Pro Gln Ser Met Ile Pro Val Met His Pro Gly Phe
   145                 150                 155                 160
   Lys Thr Glu Pro Thr Pro Ala Pro Gln Gln Pro Pro Pro Lys Ala Tyr
                   165                 170                 175
   Pro Cys Gln Thr Cys Gly Lys Gly Phe Ala Arg Arg Ser Asp Leu Ala
   180 185 190
   Arg His Glu Arg Ile His Ser Gly Ile Arg Pro His Val Cys Asp Tyr
   195 200 205
   Pro Gly Cys Gly Lys Gln Phe Ile Gln Arg Ser Ala Leu Thr Val His
   210 215 220
   Gln Arg Val His Thr Gly Glu Lys Pro His Gln Cys Glu Arg Cys Gly
   225 230 235 240
   Lys Pro Phe Ser Asp Ser Ser Ser Leu Ala Arg His Arg Arg Ile His
   245 250 255
   Ser Gly Lys Arg Pro Tyr Lys Cys Pro Tyr Val Asp Cys Gln Lys Thr
   260 265 270
   Phe Thr Arg Arg Thr Thr Leu Thr Arg His Gln Asn His His Thr Gly
   275 280 285
   Thr Val Glu Asp Ser Gln Arg Ala Arg Asn Glu Ala Leu Ala Gln Gly
   290 295 300
   Ser Asn Thr Ala Leu Ala Ala Ala Ala Ile Arg Asn Lys Arg Gly Asp
   305 310 315 320
   Ser Glu Gln Ala Ser Asn Gln Glu Ser Pro Ile Thr Thr Pro Ser Pro
   325 330 335
   Ala Gln Arg Pro Met Ser Met Ser Pro Asn Glu Asp Leu Ala Asn Ile
   340 345 350
   Asn Asn Met Gln Gln Tyr Leu Thr Asn Thr Ser Leu Pro Pro His Ile
   355 360 365
   Arg Gly Asp Val His Gly Gly Ser Pro Val Ser Thr Ala Ser Ser Gly
   370 375 380
   Tyr Asn Asn Gly Met Arg Pro Thr Ser His Pro Ser Gly Gly Tyr Ala
   385 390 395 400
   Pro Pro Pro Pro Leu Glu Pro Ser Leu Asp Gln Phe Gln Gln Gly Pro
   405 410 415
   Gly Ser Ala Ser Gly Ser Pro His Ile Gly Ser Val Gly Trp Ala Ser
                   420                 425                 430
   Pro Gly Pro Val Gly Ser Pro Thr Glu Ser His Gly Gln Gly Ala Ser
           435                 440                 445
   Val Tyr Pro Asp Pro Glu Pro Ser Tyr Gln Asn Thr Ala Gln Met Gly
       450                 455                 460
   Gln Ile Tyr Tyr Ala Ser Ala Ala Thr Gln Gly Arg Arg Pro Gly Ser
   465                 470                 475                 480
   Thr Glu Pro Gly Gln Arg Pro Ser Glu Met Trp Thr Gly Gln
                       485                 490
<210> 67
   <211> 498
   <212> PRT
   <213> Artificial Sequence
<220>
   <223> fompiestExt_fgenesh1_kg.C_100065
<400> 67
   Met Pro Arg Ala Leu Ile Pro Lys Pro Asp Gln Pro Arg Gly Ala His
   1               5                   10                  15
   Glu Cys Ala Val Cys Glu Lys Ala Phe Lys Asn Arg Ser Glu Leu Ala
               20                  25                  30
   Arg His Gln Asn Ile His Arg Glu Asp Lys Pro Phe Val Cys Thr Glu
           35                  40                  45
   Cys Gly Lys Lys Phe Ala Gln Asn Gly Gln Leu Thr Asn His Arg Asn
       50                  55                  60
   Thr His Phe Gly Phe Arg Pro Ser Lys Cys Ala Phe Cys Pro Arg Asp
   65                  70                  75                  80
   Phe Ala Glu Gly Gly Ser Thr Gly Arg His Glu Arg Glu Gln His Ser
                   85                  90                  95
   Asn Phe Ala Gly Phe Gln Cys Pro Val Ser Gly Cys Glu Phe Met His
               100                 105                 110
   Lys Arg Lys Gly Asp Tyr Asn Val His Leu Ala Gly His Gly Val Ser
           115                 120                 125
   Pro Ser Ser Val Asn Pro Trp His His Ala Tyr Thr Thr Asp Gly Ser
       130                 135                 140
   Lys Ile Asp Pro Thr Arg Gly Leu Arg Glu Gln Leu Glu Trp Glu Pro
   145 150 155 160
   Phe Leu Lys Asp Gly Arg Asp Gly Ala Gly His Pro Asn Arg Ala Arg
   165 170 175
   Ala Lys Arg Thr Ala Thr Ser Leu Gly Lys Glu Arg Gln Thr Ala Pro
   180 185 190
   Ala Gln Ala Pro Arg Thr Arg Ser Ser Arg Lys Arg Arg Ala Asp Glu
   195 200 205
   Thr Asp Glu Asp Asp Asp Glu Asp Asp Glu Tyr Lys Pro Asn Thr Arg
   210 215 220
   Ala Arg Ala Ala Lys Arg Val Arg Gln Ser Pro Pro Glu Pro Glu Pro
   225 230 235 240
   Glu Val Lys Pro Ala His Ala Val Ser Glu Asp Ala Phe Asp His Leu
   245 250 255
   Phe Thr Ile Ser Pro Thr Ser Asp His Gly Ser Asn Val Gly Leu Pro
   260 265 270
   Ser Gly Ser Ser Ser Ser Leu Thr Ser Leu Ala Ala Thr Pro Asp Pro
   275 280 285
   Gln Gln Gly Glu Leu Gln Tyr Ala Leu Asp Ala Tyr Gly Cys Ala Ser
   290 295 300
   Pro Val Ala Gly Pro Ser Ala Ser Tyr Asn Pro His Asn Val Leu Gly
   305 310 315 320
   Leu Ala Leu Pro Asp Gln Ser Ser Ala Tyr Leu Gly Tyr Gln Ser Pro
   325 330 335
   Leu Ser Gly His Gln Ala Leu Asp Gln Cys Gly Ala Thr Ser Asp Leu
   340 345 350
   Gly Leu Leu Met Ala Gln Gln Ala Pro His Gly His Tyr Glu Phe Asp
   355 360 365
   Met Ala Ala Phe Glu Ala Ser Leu Cys Asp Gly Thr Phe Phe Phe Asp
   370 375 380
   Asn Asn Asp Leu Val His Ala Gln Pro Met Gln Phe Gln Ala Ser Tyr
   385 390 395 400
   Asp Ser Tyr Asn Ile Pro Pro Pro Asn Val Ser Ser Tyr Leu His Gly
   405 410 415
   Gly Leu Tyr Gln Tyr Asp Asn Leu Ala Pro Ala Met Ser Ala Asp Leu
   420 425 430
   Leu Ser Ala Ser Ser Pro Leu Thr Ser Ser Ser Gln Gly Thr Ser Arg
   435 440 445
   Ser Pro Ser Pro Leu Gly Leu Gly Leu Gly Ser Asn Gly Phe Ser Val
   450 455 460
   Pro Ser Ile Ser Ser Leu Gly Arg Ala Arg Ala Asn Ala Gln Leu Ile
   465 470 475 480
   Met Pro Gln Val Tyr Gln Asp Ile Ala Leu Leu His Gly Tyr Pro Thr
   485 490 495
   Ser Met
<210> 68
   <211> 501
   <212> PRT
   <213> Artificial Sequence
<220>
   <223> stano6177
<400> 68
   Met Glu Leu Asp Thr Ile Leu Asn Ala Arg Leu Thr Gln Tyr Ala Gly
   1               5                   10                  15
   His Gly His Pro His Pro Phe Gly Met Gln His Ala Met Gln His Glu
               20                  25                  30
   Met Gln Gln Gln His Phe Gly Ala Pro Pro Tyr Met Ala Asn Gly Arg
           35                  40                  45
   Ile Lys Ser Glu Asn Gly Ser Glu Arg Gly Val Ser Pro His Pro Ser
       50                  55                  60
   Asp Ser Ser Arg Tyr Ser Ser Gln Gln Pro Gln Gln Pro Leu Pro Ser
   65                  70                  75                  80
   Tyr Pro Ala Met Pro Pro Gln His Met Asn Gly Met Arg Tyr Pro Ser
                   85                  90                  95
   Pro Ser Gln Met Gln Ala Pro Met Pro Met Leu Asn Asn Asn Asn Tyr
               100                 105                 110
   Ile Pro Gln Pro Pro Asp His Ala Tyr Gly Gln Gln Gln Gln Gln Gln
           115                 120                 125
   Val Pro Asp Gln Gln Gln His Gln Gly Gly Arg Pro Ala Ser Asp Thr
       130                 135                 140
   Gly Pro Pro Lys Ala Phe Ala Cys Ser Thr Cys Gly Lys Gly Phe Ala
   145                 150                 155                 160
   Arg Arg Ser Asp Leu Ala Arg His Glu Arg Ile His Ser Gly Ile Arg
                   165                 170                 175
   Pro His Val Cys Asp Tyr Pro Asn Cys Gly Lys Gln Phe Ile Gln Arg
               180                 185                 190
   Ser Ala Leu Thr Val His Gln Arg Val His Thr Gly Glu Lys Pro His
           195                 200                 205
   Met Cys Glu Arg Ser Trp Lys Gly Thr Phe Ala His Ser Thr Ala Lys
       210                 215                 220
   Gly Leu Ala Asn Ile Thr Gln Pro Phe Ser Asp Ser Ser Ser Leu Ala
   225                 230                 235                 240
   Arg His Arg Arg Ile His Ser Gly Lys Arg Pro Tyr Lys Cys Pro Tyr
                   245                 250                 255
   Ala Asp Cys Gln Lys Thr Phe Thr Arg Arg Thr Thr Leu Thr Arg His
               260                 265                 270
   Gln Asn His His Thr Gly Thr Val Glu Glu Ala Ala Ala Ala Thr Ala
           275                 280                 285
   Ala Ala Leu Ala Ser Arg Ala Ala Ala Pro Ser Arg Thr Gly Arg Ser
       290                 295                 300
   Asp Gly Gly Asp Tyr Ser Glu Asn Ala Ser Pro Leu Asn Thr Pro Ser
   305                 310                 315                 320
   Pro Gly Asp Arg Thr Leu Ser Met Ser Pro Ala Asn Gly Met Pro Ala
                   325                 330                 335
   Gly Val Val Pro Gly Met His Arg Gln Ala Thr Asp Tyr Ala Tyr Met
                   340                 345                 350
   Gly Gly Met Asn Val Pro Pro His Met Arg Ser Glu Met Pro Gln Pro
           355                 360                 365
   Ser Pro Arg Ala Ser Pro Ala Leu Thr Thr Gln Ser Tyr Ser Ser Val
       370                 375                 380
   Gly Ser Ser Arg Pro Pro His His Leu Pro Pro His Arg Ile Trp Thr
   385                 390                 395                 400
   Ser Pro His Pro Gly Ser Leu Pro Arg Leu Pro Asn His Asn Gln Phe
                   405                 410                 415
   Arg His His Ser Ala Pro Thr Ala Ala Pro His Met Phe Cys His Gly
               420                 425                 430
   Ala Gly Gln Ser Pro Gly Gln Gln Ala Leu Pro Ser Pro Gly Gln Ala
           435                 440                 445
   Asp Asn Gly Tyr Val Tyr Pro Glu Pro Gln Tyr Gly Asn Thr Gln Asn
       450                 455                 460
   Ser Met Tyr Tyr Gln Asn His Asn Ile Arg Arg Pro Asn Ser Thr Glu
   465                 470                 475                 480
   Pro Asp His Tyr Asn Ala Asn Pro Gln Arg Met Gly Asn Asp Leu Trp
                   485                 490                 495
   Ala Pro Ala Val Gln
               500
<210> 69
   <211> 428
   <212> PRT
   <213> Artificial Sequence
<220>
   <223> mycfiestExt_fgeeshl_pg.C_50034
<400> 69
   Met Ala Asp Val Leu Pro Ala Lys Ala Pro Ser Gln Leu Leu Gln Arg
   1               5                   10                  15
   Arg Leu Ser Gln Ala His Phe Asn Ala Met Gln Val Gln Gly Met Met
               20                  25                  30
   Ala Gln Phe Asn Met Ala Phe Arg Pro Asp Ser Ala Ala Thr Ile Pro
           35                  40                  45
   Leu Gln Met Leu Gln Ala Gln Glu Gln Gln Thr Leu Ala Met His Ala
       50                  55                  60
   Ala Ser Val Ala Pro Ser Glu Leu Leu Asn Thr His Pro Ala Gln Thr
   65                  70                  75                  80
   Met Asn Asn Ser Met Tyr Met Asp His Ser Ala Phe Gln Asn Ser Tyr
                   85                  90                  95
   Ser Ile Pro Val Gly Met Asn Asn Ala Thr Phe Gly Ser Leu Ala Trp
               100                 105                 110
   Pro Ala Ser Ile Val Thr Asn Pro Asn Thr Met Ala Val Leu Pro Leu
           115                 120                 125
   Val Arg Asn Glu Ser Tyr Ser Ser Val Glu Gly Ser Glu Pro Ser Ile
       130                 135                 140
   Lys Thr Glu Asp Arg Ser Pro Ile His Pro Ser Gln Val Tyr Phe Asp
   145                 150                 155                 160
   Ala Thr Ala Tyr Ala Ser Ser Pro Glu Ser Gln Thr Ala Thr Asp Ser
                   165                 170                 175
   Ser Glu Glu Thr Lys Pro Val Val Phe Ser Thr Glu Ile Asp Thr Leu
               180                 185                 190
   Met Lys Ala Ile Gln Met Lys Thr Gln Pro Ala Glu Gln Arg Glu Ala
           195                 200                 205
   Pro Lys Pro Lys Pro Ala Ala Ser Asn Asn Arg Ala Lys Lys Arg Tyr
       210                 215                 220
   Thr Cys Asp Val Pro Asp Cys Gly Lys Ala Phe Tyr Gln Lys Thr His
   225                 230                 235                 240
   Leu Glu Ile His Thr Arg Ala His Thr Gly Ile Lys Pro Phe Leu Cys
                   245                 250                 255
   Lys Glu Pro Ser Cys Gly Gln Arg Phe Ser Gln Leu Gly Asn Leu Lys
               260                 265                 270
   Thr His Glu Arg Arg His Thr Gly Glu Arg Pro Tyr His Cys Asp Ile
           275                 280                 285
   Cys Gly Lys Thr Phe Ala Gln His Gly Asn Val Arg Ala His Lys Ile
   290 295 300
   Val His Thr Ala Ala Lys Pro Phe Thr Cys Lys Leu Asp Asn Cys Asn
   305 310 315 320
   Lys Gln Phe Thr Gln Leu Gly Asn Leu Lys Ser His Gln Asn Lys Phe
   325 330 335
   His Val Glu Thr Ile Arg Arg Leu Lys Ser Arg Phe Glu Asp Ile Lys
   340 345 350
   Ala Gly Asp Val Val Glu Lys Trp Glu Lys Asp Met Trp Glu Tyr Phe
   355 360 365
   Ala Gly Leu Tyr Lys Asn Cys Asn Lys Gly Ile Lys Gly Arg Gly Lys
   370 375 380
   Asp Arg Arg Ile Ser Thr Thr Leu Leu Gly Ala Ala Gln Arg Arg Asp
   385 390 395 400
   Ser Met Val Ser Ala Glu Cys Ser Gly Gly Glu Gly Leu Ala Met Gln
   405 410 415
   Ala Arg Arg Asp Ser Leu Thr Gly Thr Met His Gln
   420 425
<210> 70
   <211> 292
   <212> PRT
   <213> Artificial Sequence
<220>
   <223> penmagi212542303refXP_002151306.1
<400> 70
   Met Ala Pro Gly Gly Ser Arg Gly Phe Asn Cys Ser Trp Glu Arg Cys
   1               5                   10                  15
   Gly Lys Ser Phe Asn Arg Lys Ser Asp Leu Cys Arg His Tyr Arg Ile
               20                  25                  30
   His Thr Asn Glu Arg Pro Tyr His Cys Thr Val Pro Ser Cys Asn Lys
           35                  40                  45
   Ser Phe Ile Gln Arg Ser Ala Leu Thr Val His Ser Arg Thr His Thr
       50                  55                  60
   Gly Glu Lys Pro His Val Cys Asp His Glu Asp Cys Arg Lys Ala Phe
   65                  70                  75                  80
   Ser Asp Ser Ser Ser Leu Ala Arg His Arg Arg Ile His Thr Gly Arg
                   85                  90                  95
   Arg Pro Tyr Ile Cys Ser Glu Pro Ala Cys Asp Arg Ser Phe Cys Arg
               100                 105                 110
   Lys Thr Thr Leu Thr Lys His Gln Ser Arg Thr His Gln Pro Gly Gly
           115                 120                 125
   Asn Thr Arg Pro Ser Ser Glu Glu Ala Pro Thr Pro Glu Ser Met Thr
       130                 135                 140
   Tyr Gln Thr Pro Val Ile Thr Thr Met Ala Pro Asn Thr Thr Gln Tyr
   145                 150                 155                 160
   Ile Ile Ala Gln Gln Pro Tyr Tyr His Asn Gly Pro Thr Pro Thr Thr
                   165                 170                 175
   Ala Asp Phe Tyr Gln Thr Pro Gln Pro Ala Ile His Phe Thr Gln Pro
               180                 185                 190
   Gln Ala Met Val Pro Gln Gln Ile Pro Thr Ala Gly Leu Pro Thr Pro
           195                 200                 205
   Pro Phe Glu Pro Gln His Tyr Ala Gln Ile Met Gln Gln Gln Gln Gln
       210                 215                 220
   Pro Arg Tyr Asp Pro Thr Pro His Gln Thr Tyr Ile Gln Pro Gln Tyr
   225                 230                 235                 240
   Gln Ser Pro Thr Pro Pro Phe Val Ala Gln His Gln His His Met Ala
                   245                 250                 255
   Ala Asp Val Arg Pro Leu Met Ala Thr Tyr His Pro Asp Gln Phe Lys
               260                 265                 270
   Pro Pro Ala Arg Ile Leu Ser Ser Gln Glu Gly Thr Asp Trp Ser Phe
           275                 280                 285
   Leu Gly Val Ser
       290
<210> 71
   <211> 416
   <212> PRT
   <213> Artificial Sequence
<220>
   <223> NCH99633
<400> 71
   Met Leu Asn Lys Lys Gly Gln Leu Gln Gln Leu Asn Pro Gly Leu Leu
   1               5                   10                  15
   Asp His Gln His Gln Ala Phe Val Lys His Glu Pro Gly Met Glu Arg
               20                  25                  30
   Ser Ala Ser Pro His Gly Ser Glu His Ser Gln Tyr Ser Asn Pro His
           35                  40                  45
   Ile Ala Arg Ala Tyr Pro Ser Pro Ala Ala Met Gln Ala Pro Met His
       50                  55                  60
   Ile Pro Asn Pro Met Pro Ala Ala Met Gln Leu Pro Gly Phe Pro Glu
   65                  70                  75                  80
   Met Pro Asn Met Gly Gly Met Pro Asn Met His Met Gln Gln Met Gln
                   85                  90                  95
   Gln Gln Pro Pro Pro Pro Pro Gln Gln Pro Ile Lys Ala Tyr Pro Cys
               100                 105                 110
   Ser Thr Cys Gly Lys Gly Phe Ala Arg Arg Ser Asp Leu Ala Arg His
           115                 120                 125
   Glu Arg Ile His Ser Gly Val Arg Pro His Val Cys Asp Trp Pro Asn
       130                 135                 140
   Cys Gly Lys Gln Phe Ile Gln Arg Ser Ala Leu Thr Val His Gln Arg
   145                 150                 155                 160
   Val His Thr Gly Glu Lys Pro His His Cys Glu Thr Cys Ala Lys Pro
                   165                 170                 175
   Phe Ser Asp Ser Ser Ser Leu Ala Arg His Arg Arg Thr His Ser Gly
               180                 185                 190
   Lys Arg Pro Tyr Lys Cys Pro Tyr Ala Asp Cys Gln Lys Thr Phe Thr
           195                 200                 205
   Arg Arg Thr Thr Leu Thr Arg His Gln Asn His His Ser Gly Thr Ile
       210                 215                 220
   Glu Glu Ala Ala Ala Ala Thr Ala Ala Ala Leu Ala Ala Ser Lys Ser
   225                 230                 235                 240
   Lys Gly Met Ser Gln Pro Arg Ser Glu Ser Asp Leu Met Ser Asn Asn
   245 250 255
   Gly Ser Pro His Thr Thr Pro Ser Pro Gly Gln Arg Thr Met Ser Met
   260 265 270
   Ser Pro Ser Val Asp Leu Ser Gly Thr Asn Ser Ile Pro Arg His Pro
   275 280 285
   Gly Asp Phe Gln Tyr Leu Gln Gln Ser Gly Ser Leu Pro Met His Met
   290 295 300
   Arg Val Gly Ser Pro Thr Ser Thr Ser Ser Gly Gly Tyr Asn Met Met
   305 310 315 320
   Arg Pro Thr Ser His Pro Thr Ser Tyr Gly Pro Pro Pro Thr Leu Glu
   325 330 335
   Pro Asn Leu Glu His Ser Gln Gly Ala Pro Ser Ser Asn Gly Gly Ser
   340 345 350
   Pro His Met Ala Asn Val Gly Trp Gln Ser Pro Ser His Val Pro Ser
   355 360 365
   Pro Ser Gln Asn Asn Ala Ser Tyr Val Tyr Pro Asp Pro Ser Glu Ala
   370 375 380
   Tyr Pro Thr Asn Pro Ala Met Asn Gln Met Tyr Tyr Gly Ala Ala Ala
   385 390 395 400
   His Met Arg Arg Pro Gln Ser Ala Glu Pro Gly Leu Val His Met Ala
   405 410 415
<210> 72
   <211> 446
   <212> PRT
   <213> Artificial Sequence
<220>
   <223> parbrPAAG_07074T0
<400> 72
   Met Asp Ile Thr Thr Ile Leu Asn Lys Lys Gly Thr Ala Ala Val Ala
   1 5 10 15
   Ala Ala Glu Ala His Leu Gln Gln His Leu Val His Ala Thr His Met
   20 25 30
   Lys Ser Lys Thr Pro Ser Glu Ile Ser Ser Glu His Gly Thr Pro Gln
   35 40 45
   Asn Gly Asp Gln Ser Asn Ile Tyr His Pro Thr Ser Gln Pro Asn Pro
   50 55 60
   Gln Leu Val Gly Ser Ser Lys Tyr His Ser Gln Ser Gln Gly His Thr
   65 70 75 80
   Gly Ile Ala Pro Val Arg Ser Asp Tyr Val Gln Asp Gly Gln Gly Ile
   85 90 95
   Val Lys Tyr Leu Gln Asn Gly Ser Gly Gly Asn Gly Arg Ser Ser Gly
   100 105 110
   Glu Pro Val Pro Lys Thr Phe His Cys Ser Thr Cys Ser Lys Gly Phe
   115 120 125
   Ala Arg Arg Ser Asp Leu Ala Arg His Glu Arg Ile His Ser Gly Ile
   130 135 140
   Arg Pro His Val Cys Asp Trp Pro Gly Cys Gly Lys Gln Phe Ile Gln
   145 150 155 160
   Arg Ser Ala Leu Thr Val His Thr Arg Val His Thr Gly Glu Lys Pro
   165 170 175
   His Met Cys Asp Arg Cys Gly Lys Pro Phe Ser Asp Ser Ser Ser Leu
   180 185 190
   Ala Arg His Arg Arg Ile His Ser Gly Lys Arg Pro Tyr Lys Cys Pro
   195 200 205
   Tyr Ala Asn Cys Gln Lys Thr Phe Thr Arg Arg Thr Thr Leu Thr Arg
   210 215 220
   His Gln Asn His His Thr Gly Thr Ile Glu Glu Ala Ala Ala Glu Thr
   225 230 235 240
   Glu Ala Asn Leu Arg Gln Asn Lys Glu Arg Ala Val Arg Pro Pro Asp
   245 250 255
   Gly Met Tyr Ser Glu Ala Gly Ser Thr Gln Ser Thr Pro Ser Pro Gly
   260 265 270
   Gln Arg His Ser Leu Ser Pro Gly Asn Glu Leu Pro Pro Met Tyr Leu
   275 280 285
   Pro Arg Gln Met Ser Asp Tyr Tyr Met Gly Asn Thr Pro Ile Pro Ala
   290 295 300
   His Leu Arg Gly Asp Phe Gln Gln Ala Ser Pro Arg Ala Ser Pro Ser
   305 310 315 320
   Thr Ser Ser Pro Pro Leu Ser Thr Tyr Ser Ser Val Pro His Ala Arg
   325 330 335
   Pro Ser Met Thr Ser His Pro Ser Gly Tyr Gly Pro Pro Gln Pro Val
   340 345 350
   Glu Pro Pro Ala Asn Ser Asp Pro Arg Pro Asn Ser Val Ala Gly Ser
   355 360 365
   Pro His Ile Thr Ser Met Gly Trp Ala Ser Pro Thr Leu His Ser Ile
   370 375 380
   Gly Ser Pro Asp Ser Ala Ser Ala Thr Glu Tyr Ser Tyr Pro Glu Pro
   385 390 395 400
   Ser Ala Pro Ser Tyr Ser Ser Gln Ile Pro Pro His Met Tyr Phe Pro
   405 410 415
   Asn Ser Thr Ile Arg Arg Pro Gln Ser Thr Glu Pro Glu Asn Tyr Glu
   420 425 430
   Leu Lys Pro Lys Leu Asn Gly Asp Ser Trp Thr Thr Pro Val
   435 440 445
<210> 73
   <211> 467
   <212> PRT
   <213> Artificial Sequence
<220>
   <223> trieqTEQG_03828T0
<400> 73
   Met Asp Ile Thr Thr Ile Leu Asn Lys Lys Ala Ser Val Ser Ile Val
   1 5 10 15
   Ala Ala Asp Val His Gln Leu Gln Gln His Leu Ala Gln Ala Gln Ser
   20 25 30
   Gln Ser Gln Thr Asp Asp Ala Val Lys Ser Arg Ser Pro Ser Glu Leu
   35 40 45
   Gly Ala Ser Glu His His Arg Ser Ala Ser Ala Pro Pro Ser Glu His
       50                  55                  60
   His His His His His His His His Gln Pro Thr Ser Ser Phe Pro Ser
   65                  70                  75                  80
   Ser Thr Gln Ser Leu Pro Gln Met Ala His Leu Ala Gln Tyr His Val
                   85                  90                  95
   Gln Ala Gln Ala Asn His Ala Gly Thr Ser Pro Gly Tyr Ala His Ser
               100                 105                 110
   Ala His Gly Ser Asp Tyr Gly Arg Ser Ser Thr Ser Met Arg Pro Thr
           115                 120                 125
   Gly Leu Pro Ala Leu Lys Thr Phe His Cys Gln Thr Cys Ser Lys Gly
       130                 135                 140
   Phe Ala Arg Arg Ser Asp Leu Ala Arg His Glu Arg Ile His Ser Gly
   145                 150                 155                 160
   Ile Arg Pro His Ala Cys Asp Trp Pro Gly Cys Gly Lys Gln Phe Ile
                   165                 170                 175
   Gln Arg Ser Ala Leu Thr Val His Thr Arg Val His Thr Gly Glu Lys
               180                 185                 190
   Pro His Met Cys Asp Arg Cys Gly Lys Pro Phe Ser Asp Ser Ser Ser
           195                 200                 205
   Leu Ala Arg His Arg Arg Ile His Ser Gly Lys Arg Pro Tyr Lys Cys
       210                 215                 220
   Pro Tyr Ala Asn Cys Gln Lys Thr Phe Thr Arg Arg Thr Thr Leu Thr
   225                 230                 235                 240
   Arg His Gln Asn His His Thr Gly Thr Ile Glu Glu Ala Ala Ala Glu
                   245                 250                 255
   Thr Glu Ala Asn Leu Arg His Ser Lys Asp Arg Ser Thr Ala Ser Arg
               260                 265                 270
   Pro Ser Glu Tyr Ser Glu Pro Gly Ser Ala His Ser Thr Pro Ser Pro
           275                 280                 285
   Ala Gln Arg Pro Ser Leu Ser Pro Gly Asn Glu Leu Pro Pro Leu Asn
       290                 295                 300
   Leu Ser Ser Arg Pro Ser Ala Ala Gly Gly Pro Thr Ser Ala Gly Gly
   305                 310                 315                 320
   Ser Ala Asp Tyr Tyr Thr Ser Leu Pro Pro His Leu Arg Thr Asp Phe
                   325                 330                 335
   Ala Asn Ser Ser Pro Arg Ala Ser Pro Thr Ser Pro Ser Pro Thr Leu
               340                 345                 350
   Thr Ser Leu Ser Gly Pro Ala Ser Phe His His His Asn His Asn Arg
           355                 360                 365
   Pro Ser Leu Thr Ser His Pro Ser Gly Tyr Ala Pro Pro Gln Pro Leu
       370                 375                 380
   Glu Pro Pro Ala Asn Ser Asp Pro Arg Pro Asn Ser Val Ala Gly Ser
   385                 390                 395                 400
   Pro His Leu Ser Ser Val Gly Trp Ala Ser Pro Thr Leu Ser Ser Met
                   405                 410                 415
   Pro Ser Pro Ser Ser Ala Ser Ala Pro Glu Tyr Pro Tyr Pro Glu Pro
               420                 425                 430
   Ser Ser His Gln Tyr Pro Gly Ser Ile Thr Pro His Met Phe Phe Pro
           435                 440                 445
   Arg Arg Pro Gln Ser Ser Glu Pro Glu Asn Tyr Glu Leu Lys Pro Lys
       450                 455                 460
   Leu Glu Leu
   465
<210> 74
   <211> 490
   <212> PRT
   <213> Artificial Sequence
<220>
   <223> mycfiestExt_fgenesh1_pm.C_30710
<400> 74
   Met Gln Tyr Glu Val Pro Leu Pro Ile Ala Met Asn His Asp Val Ser
   1               5                   10                  15
   Thr Ser Asn Gly His Tyr Ala Gln Thr Leu Tyr Val Pro Asn Gly Asn
               20                  25                  30
   Asn Arg Ile Lys Ser Glu Asn Gly Ser Glu Arg Gly Val Ser Pro His
           35                  40                  45
   Thr Ser Asp His Ser Ser Arg Tyr Ser Ser Gln Thr Pro Gln Asn Ser
       50                  55                  60
   Val Ala Tyr Gln Gln Ile Ala Ala Gln Leu Thr Asn Gly Met Arg Tyr
   65                  70                  75                  80
   Pro Ser Pro Asn Gln Met Gln Gln Asn Asn Asn Met Pro Met Leu Gln
                   85                  90                  95
   His Ser Tyr His Pro Asn Thr Thr Gln Asp Gln Gly Tyr Gln Thr Gln
               100                 105                 110
   Ala Gly Ala Thr Met Gly Ala Val Gln Gln Ala Val Pro Gln Ser Thr
           115                 120                 125
   Asp Gln Thr Ala Met Asp Gly Gly Arg Ala Ser Thr Gly Ser Ser Gly
       130                 135                 140
   Leu Pro Lys Ala Phe Ala Cys Ser Thr Cys Gln Lys Gly Phe Ala Arg
   145                 150                 155                 160
   Arg Ser Asp Leu Ala Arg His Glu Arg Ile His Ser Gly Val Arg Pro
                   165                 170                 175
   His Val Cys Asp His Pro Gly Cys Gly Lys Gln Phe Ile Gln Arg Ser
               180                 185                 190
   Ala Leu Thr Val His Ser Arg Val His Thr Gly Glu Lys Pro His Met
           195                 200                 205
   Cys Glu Arg Cys Gly Lys Pro Phe Ser Asp Ser Ser Ser Leu Ala Arg
       210                 215                 220
   His Arg Arg Ile His Ser Gly Lys Arg Pro Tyr Lys Cys Pro Tyr Ala
   225                 230                 235                 240
   Asp Cys Gln Lys Thr Phe Thr Arg Arg Thr Thr Leu Thr Arg His Gln
                   245                 250                 255
   Asn His His Thr Gly Thr Ile Glu Glu Ser Glu Ala Ala Thr Ala Ala
               260                 265                 270
   Ala Leu Ala Ser Arg Val Gln Met Ser Thr Asn Arg Ser Arg Gly Ser
           275                 280                 285
   Asp Glu Glu Asp Gly Tyr Ser Gly Asp Gly Lys Ser Pro Leu Pro Gln
   290 295 300
   Gln Gln Asp Arg Ala Ala Thr Gly Ser Pro Ala Ala Val Leu Asn Gly
   305 310 315 320
   Met Pro Gln Leu Gln Arg Gln Ala Ser Asp Tyr Tyr Met Asn Ala Met
   325 330 335
   Asn Gly Gly Met Ala Ala Val Pro Pro His Leu Arg Asn Ala Met Gln
   340 345 350
   Pro Ser Pro Arg Ala Gln Ser Pro Gln Gln Tyr Pro Met Pro Val Asn
   355 360 365
   Gly Asn Thr Pro Gln Ser Arg Pro Ser Leu Thr Ser Asn Pro Ser Ser
   370 375 380
   Ser Ala His Leu Pro Gln Ile Leu Glu Pro Pro Thr Thr Asn Gly Gln
   385 390 395 400
   Gln Gln Thr Gly Ser Gly Asn Asn Ser Pro His Leu Asn Asn Asn Asn
   405 410 415
   Asn Asn Pro Met Asn Gly Trp Gln Ser Pro His Pro Gly Met Pro Ala
   420 425 430
   Asn Ala Ser Gln Thr Thr Asp Tyr Thr Tyr Pro Asp Pro Asn Asn His
   435 440 445
   Tyr Gly Val Ser Asn Val Asn Thr Ala Gln Met Tyr Tyr Gln Gln Gln
   450 455 460
   Gly Val Pro Arg Pro His Ser Thr Gln Pro Leu Asp Tyr Gln His Gln
   465 470 475 480
   Gly Gln Pro Met Trp Ala Gln His Gln Gln
   485 490
<210> 75
   <211> 535
   <212> PRT
   <213> Artificial Sequence
<220>
   <223> magpoMAPG_07788T0
<400> 75
   Met Gln Leu Pro Thr Pro Ser Gly Trp Gly Gln Trp Gln Gln Asn Glu
   1               5                   10                  15
   Val Glu Ser Gly Tyr Val His Met Gly Ser Pro Met Met Gly Thr Gln
               20                  25                  30
   Ala Pro Leu Pro Gln Pro Thr Leu Ser Pro Gln Tyr Pro Val Thr Leu
           35                  40                  45
   Ala Pro Ser Pro Ser Leu Gln Gln Pro Met Ser Tyr Gly Pro Tyr Ala
       50                  55                  60
   His Gly Leu Pro Ser Val Ala Pro Leu Gln Ile Pro Thr Ile Gly Ser
   65                  70                  75                  80
   Gln His Ser His His Tyr Gln His Gln His Gln His Gln Gln His Gln
                   85                  90                  95
   Ile Gln Leu Ser Asp Ser Ile Asp Arg Thr Arg Lys Arg Leu Val Asn
               100                 105                 110
   Arg Arg Ser Leu Arg Asp Thr Thr Met Glu Ile Met Glu Ala Arg His
           115                 120                 125
   Arg Gly His Thr Thr Pro Glu Arg Ala Thr His Arg Ile Pro Gln Thr
       130                 135                 140
   Val Val Ala Asn Ala Ser Thr Val Gly Ala Ser His Ala Gly Asn His
   145                 150                 155                 160
   Val Glu Phe Asn Thr Ser Val Asp Lys Leu Met Lys Val Ile Gln Ala
                   165                 170                 175
   Met Pro Asp Thr Gly Pro Leu Val Glu Ser Leu Glu Ala Ala Val Lys
               180                 185                 190
   Gln Glu Leu Pro Gln Val Glu Glu Ser Glu Gln Ala Ala Ser Pro Pro
           195                 200                 205
   His Glu Ser Lys Pro Ala Lys Gln Lys Ala Ala Met Arg Lys Phe Ala
       210                 215                 220
   Cys His Phe Arg Gly Cys Gln Lys Lys Phe Ala Gln Lys Ala Gln Leu
   225                 230                 235                 240
   Asp Thr His Val Arg Ser His Thr Gly Glu Arg Pro Tyr Val Cys Glu
                   245                 250                 255
   Phe Pro Asn Cys Gly Lys Arg Phe Thr Gln Ser Gly Asn Leu His Thr
               260                 265                 270
   His Glu Arg Lys His Thr Gly Glu Arg Pro Tyr Val Cys Asp Val Cys
           275                 280                 285
   Gly Arg Arg Phe Ala Gln Gly Gly Asn Arg Gln Ala His Lys Lys Val
       290                 295                 300
   His Gln Lys Thr Lys Asp Phe Ile Cys Arg Leu Glu Asn Cys Gly Lys
   305                 310                 315                 320
   Glu Phe Thr Gln Arg Gly Asn Leu Lys Asn His Gln Asn Asn Thr His
                   325                 330                 335
   Lys Gln Phe Met Asp Gln Thr Met Ala Arg Leu Glu Arg Ile Ser Pro
               340                 345                 350
   Glu Asn Met Ser Pro Glu Asp Arg Asp Leu Val Val Tyr Leu Ala Pro
           355                 360                 365
   Leu Tyr Lys Asn Ser Asn Lys Gly Ile Lys Gly Arg Gly Ala Arg Pro
       370                 375                 380
   Ala Gly Tyr Arg Leu Ser Arg Ser Arg Arg Ala Pro Ser Val Ser Ser
   385                 390                 395                 400
   Ser Thr Gln Ser Tyr Ser Pro Thr Lys Ser Glu Ala Ser Leu Thr Ser
                   405                 410                 415
   Ser Pro Cys Thr Ala Gly Pro Pro Ser Gln His His Tyr Gly Gly His
               420                 425                 430
   Ala Gln His Gln Tyr His His Gln Gln His His His His Gln Gln Gln
           435                 440                 445
   Gln Gln Arg Pro Gln Gln Gly Leu Ala Tyr Pro Pro Val Ser Phe Gly
       450                 455                 460
   Ser Ala Pro Ser Asp Ser Phe Arg Tyr Pro Tyr Asn Gly Ile Val His
   465                 470                 475                 480
   Arg Tyr Glu Met Pro Glu His Ser His Thr Tyr Ser Gly Ile Pro Val
                   485                 490                 495
   Thr Thr Ser Ser Ser Thr Pro Ser Pro Val Ala Thr Val Tyr Ser Asp
   500 505 510
   Asp Ser Gly Ala Val Tyr His Ser Asp Asp Asn Gly Ala Arg Glu Leu
   515 520 525
   Ala Phe Gly Glu Arg Ile Cys
   530 535
<210> 76
   <211> 341
   <212> PRT
   <213> Artificial Sequence
<220>
   <223> miccaMCYG_06344T0
<400> 76
   Met Val Arg Thr Arg Ser Phe Pro Cys Ser Trp Val Ser Cys Lys Lys
   1               5                   10                  15
   Val Phe Asn Arg Lys Ser Asp Leu Cys Arg His Tyr Arg Ile His Thr
               20                  25                  30
   Asn Glu Arg Pro Tyr Arg Cys Asn Phe Pro Asn Cys Thr Lys Ser Phe
           35                  40                  45
   Ile Gln Arg Ser Ala Leu Thr Val His Ser Arg Thr His Thr Gly Glu
       50                  55                  60
   Lys Pro His Val Cys Asn Phe Ala Asn Cys Ser Lys Ala Phe Ser Asp
   65                  70                  75                  80
   Ser Ser Ser Leu Ala Arg His Arg Arg Ile His Thr Gly Arg Arg Pro
                   85                  90                  95
   Tyr Lys Cys Leu Glu Pro Thr Cys Asp Arg Ser Phe Cys Arg Lys Thr
               100                 105                 110
   Thr Leu Thr Lys His Gln Ser Arg Ser His Gln Pro Glu Ala Val Ser
           115                 120                 125
   Pro Ser Ala Gln Ser Cys Glu Ser Glu Leu Tyr Ser Gln Pro Thr Gln
       130                 135                 140
   His Ser Met Ser Met Met Pro Gln Ser Ser Pro Val Ser Gln His Gln
   145                 150                 155                 160
   Leu Gln Ser Gln Gln Gln Glu Gln Gln Gln His Gln Gln Gln His Leu
                   165                 170                 175
   Gln Pro Gln Pro Arg Pro Gln Gln His Ser Pro Tyr Gln Gln Gln His
   180 185 190
   Ser Pro Ile Ile Ala Val Pro Val Ser Asp Tyr Phe His His Ala Gln
   195 200 205
   Ser Gln Pro Thr Thr Val His Ser Ile Pro Ile Ser Ala Glu Gln Pro
   210 215 220
   Ile Leu Pro Pro Gln Val His Tyr Val Ser Thr Gly Ile Leu Pro Pro
   225 230 235 240
   Gln Ile Pro Arg Tyr Asp Ile Pro Ile Thr Thr Ser Ala Ala Val Ser
   245 250 255
   Ile Pro Thr Thr Ile Asp Gln Leu Thr Tyr Ala His Pro Val Ser Val
   260 265 270
   Ser Met Pro Gln Gln Gln Arg Ile Gln Ala Pro Gln Pro Ile Gln Val
   275 280 285
   Ser His Gln Thr Asp Gly Gly Phe Gln Val Leu Pro Val Val Ala Gly
   290 295 300
   Gly Ala Gly Gly Gln Tyr Met Lys Glu Phe Asp His Met Lys His Gly
   305 310 315 320
   Gln Gln Arg Phe Phe Gly Ser Thr Tyr Pro Glu His Met Asn Trp Asp
   325 330 335
   Phe Leu Gly Leu Ser
   340
<210> 77
   <211> 441
   <212> PRT
   <213> Artificial Sequence
<220>
   <223> penmagi212542027refXP_002151168.1
<400> 77
   Met Glu Ile Pro Thr Ile Leu Asn Arg Lys Ala Ser Ala Ala Val Val
   1 5 10 15
   Ala Asp Pro Arg Phe Gln Ala Gln Leu Ala Gln Ala Val His Leu His
   20 25 30
   Gln Gln Glu Met Ser Glu Gln Ala Asn Ser Gln Pro Gly Thr His Ala
           35                  40                  45
   Val Met Gly Tyr Pro Pro Asn Ala Gln Pro Leu His Gln Gly Pro Met
       50                  55                  60
   Pro Gly Gln His Trp Pro Ile His Pro Asn Gly Ala Pro Met Met Pro
   65                  70                  75                  80
   Asn Asn Trp Asp Pro Asn Pro Phe Ala Gly Ala Pro Pro Gln Pro Pro
                   85                  90                  95
   Gln Pro Ser His Thr Pro Pro Val Pro Gln Gln Gln Pro Arg Ala Glu
               100                 105                 110
   Pro Ala Pro Arg Val Phe His Cys Ser Thr Cys Gln Lys Gly Phe Ala
           115                 120                 125
   Arg Arg Ser Asp Leu Ala Arg His Glu Arg Ile His Ser Gly Ile Arg
       130                 135                 140
   Pro His Ala Cys Asp Trp Pro Gly Cys Gly Lys Gln Phe Ile Gln Arg
   145                 150                 155                 160
   Ser Ala Leu Thr Val His Ala Arg Val His Thr Gly Glu Lys Pro His
                   165                 170                 175
   Met Cys Glu Arg Cys Gly Lys Pro Phe Ser Asp Ser Ser Ser Leu Ala
               180                 185                 190
   Arg His Arg Arg Ile His Ser Gly Lys Arg Pro Tyr Lys Cys Pro Tyr
           195                 200                 205
   Ala Asn Cys Gln Lys Thr Phe Thr Arg Arg Thr Thr Leu Thr Arg His
       210                 215                 220
   Gln Asn His His Thr Gly Thr Ile Glu Glu Ala Ala Ala Gln Thr Glu
   225                 230                 235                 240
   Ala Ser Leu Arg Gln Asn Arg Glu Lys Ser Ala Arg Ala Ser Glu Gly
                   245                 250                 255
   Ile Tyr Ser Glu Thr Gly Ser Gly His Ser Thr Pro Ser Pro Gly Gln
               260                 265                 270
   Gln Ala Ala Met Gly Leu Gly Ser Glu Leu Pro Pro Leu Asn Ile Ile
           275                 280                 285
   Arg Ser Thr Gly Glu Tyr Tyr Ile Pro Asn Gly Gly Ile Pro Pro His
       290                 295                 300
   Val Arg Gly Asp Phe Gln Gln Ala Ser Pro Arg Ala Ser Pro Ala Ala
   305                 310                 315                 320
   Thr Ser Pro Ala Leu Ser Ser Tyr Ser Ser His Pro Arg Pro Ser Met
                   325                 330                 335
   Thr Ser His Pro Ser Ser Tyr Gly Pro Pro Gln Pro Leu Glu Pro Pro
               340                 345                 350
   Ala Asn Thr Asp His Arg Pro Gly Ser Val Thr Gly Ser Pro His Met
           355                 360                 365
   Thr Ser Met Gly Trp Ala Ser Pro Ser His Ala Ser Met Pro Ser Pro
       370                 375                 380
   Gly Ser Pro His Glu Phe Gly Tyr Pro Glu Pro Ser Val Ala Ala Tyr
   385                 390                 395                 400
   Ala Ser Ala Met Pro Pro His Met Tyr Phe Pro Asn Ser Thr Ile Arg
                   405                 410                 415
   Arg Pro Gly Ser Thr Glu Pro Glu Asn Tyr Glu Met Lys Pro Lys Ile
               420                 425                 430
   Gly Pro Asp Gly Trp Ser Thr Pro Ile
           435                 440
<210> 78
   <211> 444
   <212> PRT
   <213> Artificial Sequence
<220>
   <223> neofi9623
<400> 78
   Met Asp Ile Thr Thr Ile Leu Asn Arg Lys Gly Ser Thr Ala Ala Ile
   1               5                   10                  15
   Ala Ala Glu Ala Gln Phe Gln Gln Gln Phe Val His His Asn Leu Asp
               20                  25                  30
   Pro Ser Ser Ser Pro Lys Met Lys Pro Glu Pro Gly Val Ser Glu Ala
           35                  40                  45
   Ser Asp Gln Gln Val Leu Ser Tyr Pro Ser His Ala Pro Leu Asn Pro
       50                  55                  60
   Met Pro Asn Met Ala Gln Asp Met Arg Tyr Pro Pro His Gly Gln Pro
   65                  70                  75                  80
   Asn Ser Gly Met Pro Val Leu Gln Asn Pro Tyr Val Pro Gly Ala Tyr
                   85                  90                  95
   Thr Gly Ser Ala Gln Ile Pro Ser Ser Ala Thr Pro Gln Arg Ala Asp
               100                 105                 110
   Pro Pro Pro Lys Thr Phe His Cys Ser Thr Cys Gly Lys Gly Phe Ala
           115                 120                 125
   Arg Arg Ser Asp Leu Ala Arg His Glu Arg Ile His Thr Gly Ile Arg
       130                 135                 140
   Pro His Ala Cys Asp Trp Pro Gly Cys Gly Lys Gln Phe Ile Gln Arg
   145                 150                 155                 160
   Ser Ala Leu Thr Val His Ser Arg Val His Thr Gly Glu Lys Pro His
                   165                 170                 175
   Met Cys Glu Arg Cys Gly Lys Pro Phe Ser Asp Ser Ser Ser Leu Ala
               180                 185                 190
   Arg His Arg Arg Ile His Ser Gly Lys Arg Pro Tyr Lys Cys Pro Tyr
           195                 200                 205
   Ala Asn Cys Gln Lys Thr Phe Thr Arg Arg Thr Thr Leu Thr Arg His
       210                 215                 220
   Gln Asn His His Thr Gly Thr Ile Glu Glu Ala Ala Ala Glu Thr Glu
   225                 230                 235                 240
   Ala Asn Leu Arg Gln Asn Lys Glu Arg Ala Arg Ala Pro Gly Asp Gly
                   245                 250                 255
   Val Phe Ser Glu His Ala Ser Val His Ser Thr Pro Ser Pro Ala His
               260                 265                 270
   His Pro Val Ser Ile Pro Pro Pro Gly Asp Leu Pro Pro Leu Asn Met
           275                 280                 285
   Pro Arg Ser Ala Gly Asp Tyr Tyr Met Gly Asn Gly Ser Ile Pro Pro
       290                 295                 300
   His Val Arg Gly Asp Phe Ser Gln Ala Ser Pro Arg Ser Ser Pro Thr
   305 310 315 320
   Ala Thr Ser Pro Ser Leu Ser Ser Phe Ser Ser Ala Pro His Gln Arg
   325 330 335
   Pro Ser Met Thr Ser His Pro Ser Gly Tyr Ala Pro Pro Gln Pro Leu
   340 345 350
   Glu Pro Pro Ala Asn Ser Asp His Arg Pro Asn Ser Val Ser Gly Ser
   355 360 365
   Pro His Met Thr Ser Leu Gly Trp Ala Ser Pro Ser His Gly Ser Ile
   370 375 380
   Pro Ser Pro Gly Ser Val Asn Asp Phe Asn Tyr Pro Glu Pro Ser Gly
   385 390 395 400
   Pro Ala Tyr Pro Ser Ser Met Pro Pro His Met Tyr Phe Pro Asn Ser
   405 410 415
   Thr Ile Arg Arg Pro Thr Ser Thr Glu Pro Glu Asn Tyr Glu Leu Lys
   420 425 430
   Pro Arg Leu Gly Asp Asn Gly Trp Ser Thr Pro Val
   435 440
<210> 79
   <211> 456
   <212> PRT
   <213> Artificial Sequence
<220>
   <223> aspflAFL2T_05677
<400> 79
   Met Glu Thr Ala Glu Pro Val Ser Tyr Glu Phe Pro Gly His Thr Ile
   1 5 10 15
   Gly Ala Val Ala Pro Arg Arg Met Met Thr Ser Asn Leu Gly His Asn
   20 25 30
   Phe Pro Phe Tyr Ala Thr Pro Ala Ala Ser Phe Pro Leu Pro Phe His
   35 40 45
   Gln Ser Ser Ser Thr Ala Tyr Gly Phe Gly His Ala Leu Asn His His
   50 55 60
   His His Asn His His Gln Pro Ser Tyr Pro Gln Phe Phe Val Ala Ser
   65                  70                  75                  80
   His Glu Ser Ile Asn Ser Gln Pro Met Arg Leu Ser Ser Glu Pro Pro
                   85                  90                  95
   Pro Val Gln Ser Ile Pro Asp Ile Arg Pro Ala Lys Asn Ala Val Asn
               100                 105                 110
   Arg Val Ser Arg Asp Pro Leu Val Lys Asn Asp Pro Ser Ser Asn Thr
           115                 120                 125
   Gln Gln Thr Pro Met Ala Arg Ser Ser Thr His Gly Ala Ala Ala Gln
       130                 135                 140
   Ser Lys Ser Pro Ser Val Ser Glu Ile Glu Phe Thr Thr Glu Val Asp
   145                 150                 155                 160
   Ile Leu Met Lys Ala Ile Gln Ser Arg Asn Ser Val Gln Pro Pro Asn
                   165                 170                 175
   Thr Gln Ser Leu Pro Pro Leu Gln Gln Leu Thr His Arg Gly Cys His
               180                 185                 190
   Gly Tyr Pro Gln Thr Phe Ser Leu His Pro Ser Gly Asn Thr Arg Cys
           195                 200                 205
   Asn Met Met Ala Glu Glu Val Gln Ser Arg Ser Gly Lys Lys Arg Lys
       210                 215                 220
   Tyr Val Cys Thr Leu Pro His Cys Gly Lys Ser Phe Ala Gln Lys Thr
   225                 230                 235                 240
   His Leu Asp Ile His Thr Arg Ala His Thr Gly Asp Lys Pro Phe Ile
                   245                 250                 255
   Cys Lys Glu Pro Ser Cys Gly Gln Arg Phe Ser Gln Leu Gly Asn Leu
               260                 265                 270
   Lys Arg Glu Lys Thr Asp Ser Asn Pro Gly Ala His Lys Gln Thr His
           275                 280                 285
   Gln Arg Arg His Thr Gly Glu Lys Pro Phe Ser Cys Asp Ile Cys Gln
       290                 295                 300
   Lys Arg Phe Ala Gln Arg Gly Asn Val Arg Ala His Lys Ile Thr His
   305                 310                 315                 320
   Gln His Ala Lys Pro Phe Thr Cys Leu Leu Asp Asp Cys Gly Lys Gln
   325 330 335
   Phe Thr Gln Leu Gly Asn Leu Lys Ser His Gln Asn Lys Phe His Ala
   340 345 350
   Thr Thr Leu Arg Asp Leu Thr Leu Lys Phe Ser Gln Val Thr Ile Gly
   355 360 365
   Asp Pro Met Ser Pro Gln Asp Arg Lys Leu Trp Glu Tyr Phe Ala Thr
   370 375 380
   Leu Tyr Lys Asn Ser Asn Lys Gly Ile Lys Gly Arg Gly Lys Asp Arg
   385 390 395 400
   Arg Ile Ser Pro Thr Ser Arg Ser Gly Pro Gly Lys Arg His Gln Thr
   405 410 415
   Leu Gly Asn Asn Asp Asp Lys Leu Gln Arg Pro Ile Tyr Glu Glu Ser
   420 425 430
   Ser Val Tyr Thr Gly Gly Ser Ser Ser Asp Glu Glu Asp Ala Glu Ala
   435 440 445
   Tyr Tyr Ile Asp Arg Gln Asn His
   450 455
<210> 80
   <211> 423
   <212> PRT
   <213> Artificial Sequence
<220>
   <223> Myg_14264
<400> 80
   Ser Glu Thr Gly Ser Glu Arg Gly Ala Ser Pro His Thr Ser Asp Gln
   1 5 10 15
   Ser Ser Arg Tyr Ser Ser Gln Thr Pro Gln Asn Ser Ala Val Tyr Gln
   20 25 30
   Gln Met Ala Ala Gln Leu Thr Asn Gly Leu Gln Tyr Thr Ala Pro Pro
   35 40 45
   Gln Met Gln Gln Gln Asn Gly Met Ser Leu Leu Gln His Ser Tyr His
   50 55 60
   Pro Ala Thr Gly Gln Glu Pro Ser Tyr Gln Thr Gln Gln Pro Gln Gln
   65                  70                  75                  80
   Gln Gln Ser Gln Gln Gln Ser Thr Gln Leu Gly Leu Pro Lys Ala Phe
                   85                  90                  95
   Ser Cys Asn Thr Cys Gln Lys Gly Phe Ala Arg Arg Ser Asp Leu Ala
               100                 105                 110
   Arg His Glu Arg Ile His Ser Gly Val Arg Pro His Val Cys Glu His
           115                 120                 125
   Pro Asn Cys Gly Lys Gln Phe Ile Gln Arg Ser Ala Leu Thr Val His
       130                 135                 140
   Ser Arg Val His Thr Gly Glu Lys Pro His Met Cys Glu Arg Cys Gly
   145                 150                 155                 160
   Lys Pro Phe Ser Asp Ser Ser Ser Leu Ala Arg His Arg Arg Ile His
                   165                 170                 175
   Ser Gly Lys Arg Pro Tyr Lys Cys Pro Tyr Ala Asp Cys Gln Lys Thr
               180                 185                 190
   Phe Thr Arg Arg Thr Thr Leu Thr Arg His Gln Asn His His Thr Gly
           195                 200                 205
   Thr Ile Glu Glu Ser Glu Ala Ala Thr Ala Ala Ala Leu Ala Ser Arg
       210                 215                 220
   Ala Ser Leu Pro Gln Gln Arg Ser Arg Gly Ser Asp Glu Glu Asn Asp
   225                 230                 235                 240
   Phe Ser Ala Asp Gly Lys Ser Pro Met Pro Pro Gln His Asp Arg Pro
                   245                 250                 255
   Ser Ala Ser Pro Ala Asn Gly Met Asn Gly Ala Pro Gln Leu Gln Arg
               260                 265                 270
   Gln Ala Ser Asp Tyr Tyr Leu Asn Ala Met Asn Gly Gly Met Ala Ser
           275                 280                 285
   Val Pro Ala His Leu Arg Asn Asp Met Gln Pro Thr Pro Arg Ser Gln
       290                 295                 300
   Ser Pro Ala Gln Tyr Thr Met Pro Val Asn Pro Pro Gln Ser Arg Pro
   305                 310                 315                 320
   Thr Leu Ala Ser Asn Pro Ser Ala Ser Tyr Ala Ser Arg Gln Ile Leu
   325 330 335
   Glu Pro Asn Gly Ala Ser Val Gln Gln Gln Gly Thr Ser Gly Thr Asn
   340 345 350
   Ser Pro His Met Asn Gly Asn Ile Ala Trp Gln Pro Ser His His Gly
   355 360 365
   Leu Pro Thr Thr Gln Thr Thr Asp Tyr Thr Tyr Pro Asp His Thr Gly
   370 375 380
   Tyr Ser Val Ala Asn Pro Met Tyr Tyr Gln Gln Gly Ser Val Gln Arg
   385 390 395 400
   Pro His Ser Thr Gly Pro Ile Asp His Tyr Gly Pro Met Arg Gly Gln
   405 410 415
   Glu Met Trp Ala Gln His Gln
   420
<210> 81
   <211> 503
   <212> PRT
   <213> Artificial Sequence
<220>
   <223> NCH48671
<400> 81
   Lys Leu Ala Leu Ala Ala Gln Pro Thr Ala Asn Pro Asn Trp Gly Arg
   1 5 10 15
   Trp Pro Gln Gln Leu Ser Gly Glu Tyr Thr Met Met Glu Ser Pro Ser
   20 25 30
   Met Leu Pro Phe Asp Ser Arg Thr Ala Thr Thr Ala Pro Leu Gln Arg
   35 40 45
   Pro Ile Met Ala Pro Tyr Met Val Pro Asn Ser Tyr Ser Ser Ala Gln
   50 55 60
   Met Asn Ser Leu Thr Ala Thr His Tyr Gln Ala Pro Asn Pro Tyr Gln
   65 70 75 80
   Phe Ser Gly Tyr Gln Gly Pro Pro Thr Pro Pro His Asn Ser Ser Pro
   85 90 95
   Phe Lys Val Glu Tyr His Asp Arg Arg Pro Met Gly Pro Asp Ser Asp
   100 105 110
   Asn Gly Arg Val Pro Ser Tyr His Arg Gly Ala Lys Tyr Ser Tyr Ala
   115 120 125
   Glu Gln Ala Pro Ser Pro Ala Arg Ser Asp Ser Gln Ala Ser Thr Ala
   130 135 140
   Arg Ser Ser Val Asn Asn Pro Thr Leu Cys Ser Lys Thr Ile Thr Ser
   145 150 155 160
   Asn Glu Thr Ile Asn Pro Asp Asp Gln Val Asn Phe Glu Thr Glu Val
   165 170 175
   Asp Glu Leu Met Lys Ala Ile Gln Arg Lys Gly Glu Lys Glu Leu Asp
   180 185 190
   Val Ala Gln Gln Pro Leu Thr Pro Gly Met Ser Pro Val Ser Glu Ala
   195 200 205
   Ser Leu Glu Asn Asn Gly Thr Pro Ala Pro Ala Asp Ser Lys Thr Ser
   210 215 220
   Lys Lys Arg Tyr Val Cys Asn Gly Pro Asn Cys Arg Lys Ser Phe Thr
   225 230 235 240
   Gln Lys Thr His Leu Asp Ile His Arg Arg Thr His Thr Gly Asp Lys
   245 250 255
   Pro Tyr Thr Cys Glu Phe Pro Gly Cys Lys Leu Thr Phe Ser Gln Leu
   260 265 270
   Gly Asn Leu Lys Thr His Met Arg Arg His Thr Gly Glu Arg Pro Tyr
   275 280 285
   Ser Cys Gly Lys Cys Gly Arg Lys Phe Ala Gln Arg Gly Asn Val Arg
   290 295 300
   Ala His Glu Gln Thr His Gln Gly Leu Lys Pro Phe Ile Cys Arg Leu
   305 310 315 320
   Asp Asp Cys Asn Lys Thr Phe Ser Gln Leu Gly Asn Met Lys Thr His
   325 330 335
   Gln Asn Asn Phe His Lys Lys Thr Leu Lys Asn Leu Thr Met Arg Phe
   340 345 350
   Ala Gln Ile Leu Asn Ser Gly Glu Glu Val Pro Glu Ala Glu Arg Glu
   355 360 365
   Leu Phe Glu Tyr Phe Ala Thr His Tyr Lys Asn Ser Asn Lys Gly Ile
   370 375 380
   Lys Gly Arg Gly Lys Ala Arg Thr Val Ala Glu Arg Lys Thr Lys Pro
   385 390 395 400
   Ser Gln Ser Pro Pro Thr Asn Pro Asn Thr Val Thr Pro Gln Tyr Pro
   405 410 415
   Leu Pro Gln Ile Pro Pro Thr Gln Gln Ile Pro Ser Pro His Gln Pro
   420 425 430
   His Gly Leu Ser His Pro Gly Ser Leu Ala Ala Tyr Ser Met Ser Arg
   435 440 445
   Gly His Pro Asn Val Ile Asn Asn Met Pro Arg Glu Ser His Ala Gly
   450 455 460
   Gly Tyr Glu Met Tyr Asp Met Gln Gly Gly His His His Val Gln Pro
   465 470 475 480
   Ser Asn Asn Asn Gly Met Met Tyr Glu Glu Glu His Ala Arg Glu Met
   485 490 495
   Gly Phe Asn Glu Arg Met Tyr
   500
<210> 82
   <211> 446
   <212> PRT
   <213> Artificial Sequence
<220>
   <223> hyspuNODE_451_length_688610_cov_26..g5755.t1
<400> 82
   Met Gln His Glu Gln Leu Gln Gln His Phe Gly Pro Thr Ser Phe Leu
   1 5 10 15
   Asn Gly Arg Ile Lys Ser Glu Asn Gly Ser Asp Arg Gly Val Ser Pro
   20 25 30
   His Pro Ser Asp Ser Ser Arg Tyr Ser Ser Gln Ala Pro Gln Pro Leu
   35 40 45
   Gln Ser Tyr Pro Pro Pro Met Thr Asn Asn Met Asn Gly Met Arg Tyr
       50                  55                  60
   Pro Ser Pro Ser Gln Met Asn Thr Pro Met Pro Met Leu Asn Asn Asn
   65                  70                  75                  80
   Asn Tyr Ile Pro Asn Pro Pro Glu His Ala Tyr Pro Gln Gln Ala Thr
                   85                  90                  95
   Pro Asp Pro Gln Gln Gln His Leu Gly Gly Arg Pro Ala Ser Asp Thr
               100                 105                 110
   Gly Pro Pro Lys Ala Phe Ala Cys Ser Thr Cys Ala Lys Gly Phe Ala
           115                 120                 125
   Arg Arg Ser Asp Leu Ala Arg His Glu Arg Ile His Ser Gly Ile Arg
       130                 135                 140
   Pro His Val Cys Asp His Pro Gly Cys Gly Lys Gln Phe Ile Gln Arg
   145                 150                 155                 160
   Ser Ala Leu Thr Val His Gln Arg Val His Thr Gly Glu Lys Pro His
                   165                 170                 175
   Met Cys Glu Arg Cys Gly Lys Pro Phe Ser Asp Ser Ser Ser Leu Ala
               180                 185                 190
   Arg His Arg Arg Ile His Ser Gly Lys Arg Pro Tyr Lys Cys Pro Tyr
           195                 200                 205
   Ala Asp Cys Gln Lys Thr Phe Thr Arg Arg Thr Thr Leu Thr Arg His
       210                 215                 220
   Gln Asn His His Thr Gly Thr Val Glu Glu Ala Ala Ala Ala Thr Ala
   225                 230                 235                 240
   Ala Ala Leu Ala Ser Arg Ala Ser Ala Pro Ser Arg Ser Ala Arg Ser
                   245                 250                 255
   Asp Ala Asp Glu Tyr Ser Glu Thr Ala Ser Pro Leu Gly Thr Pro Ser
               260                 265                 270
   Pro His Glu Arg Pro Leu Ser Leu Ser Pro Ala Asn Gly Met Pro Val
           275                 280                 285
   Val Pro Ala Met His Arg Gln Ala Ser Asp Tyr Ala Tyr Met Gly Ser
       290                 295                 300
   Met Asn Val Pro Pro His Leu Arg Asn Glu Met Pro Gln Pro Ser Pro
   305 310 315 320
   Arg Ser Ser Pro Ala Leu Thr Ser Gln Ser Tyr Asn Ser Ala Val Ser
   325 330 335
   Asn Thr Arg Pro Thr Ile Thr Ser His Pro Ser Gly Tyr Gly Pro Pro
   340 345 350
   Pro Ile Leu Glu Pro Pro Ala Ser Ala Asn His Asn Gln Pro Gly Ser
   355 360 365
   Val Asn Gly Ser Pro His Met Gly Ala Met Gly Trp Gln Ser Pro Ser
   370 375 380
   Gln Gln Ala Met Pro Ser Pro Gly Pro Gly Asp Asn Tyr Val Tyr Pro
   385 390 395 400
   Glu Pro His Tyr Ala Asn Asn Gly Ser Asn Met Tyr Tyr Ala Asn Ser
   405 410 415
   Asn Ile Arg Arg Pro Asn Ser Thr Glu Pro Asp Gln Tyr Asn Pro Gln
   420 425 430
   Gln Gln Arg Met Ala Gly Asp Met Trp Ala Pro Pro Val Gln
   435 440 445
<210> 83
   <211> 442
   <212> PRT
   <213> Artificial Sequence
<220>
   <223> aspniANID_01997T0
<400> 83
   Met Asp Ile Thr Thr Ile Leu Asn Arg Lys Ala Ser Val Ala Met Val
   1 5 10 15
   Ala Pro Asp Ala Gln Phe Asp Gln Gln Gln Phe Met Gln Ala Ser Asn
   20 25 30
   Leu Asn Thr Ser Pro Ser Pro Gly Met Lys Ser Glu Pro Asn Val Ser
   35 40 45
   Glu Ala Ala Asn Gln Ser Val Leu Ser Tyr Pro Pro His Ala Pro Met
   50 55 60
   Asn Gln Pro Pro Asn Met Asn Gln Asp Ile Arg Tyr Ala Val Gln Gln
   65                  70                  75                  80
   Pro Asn Pro Gln Leu Pro Met Leu Gln Ser Pro Tyr Ala Pro Ser Gly
                   85                  90                  95
   Tyr Ser Ala Ala Ser Val Pro Ile Ser Ala Pro Gln Gly Arg Pro Glu
               100                 105                 110
   Pro Pro Pro Lys Thr Phe His Cys Gly Thr Cys Gly Lys Gly Phe Ala
           115                 120                 125
   Arg Arg Ser Asp Leu Ala Arg His Glu Arg Ile His Ser Gly Ile Arg
       130                 135                 140
   Pro His Ala Cys Asp Trp Pro Gly Cys Gly Lys Gln Phe Ile Gln Arg
   145                 150                 155                 160
   Ser Ala Leu Thr Val His Ser Arg Val His Thr Gly Glu Lys Pro His
                   165                 170                 175
   Met Cys Glu Arg Cys Gly Lys Pro Phe Ser Asp Ser Ser Ser Leu Ala
               180                 185                 190
   Arg His Arg Arg Ile His Ser Gly Lys Arg Pro Tyr Lys Cys Pro Tyr
           195                 200                 205
   Ala Asn Cys Gln Lys Thr Phe Thr Arg Arg Thr Thr Leu Thr Arg His
       210                 215                 220
   Gln Asn His His Thr Gly Thr Ile Glu Glu Ala Ala Ala Glu Thr Glu
   225                 230                 235                 240
   Ala Asn Leu Arg Gln Asn Lys Glu Arg Ala Lys Val Pro Gly Asp Gly
                   245                 250                 255
   Met Phe Ser Asp His Gly Ser Val His Gly Ser Val His Ser Thr Pro
               260                 265                 270
   Ser Pro Ala His His Pro Ile Ser Pro Gly Gly Glu Leu Pro Pro Leu
           275                 280                 285
   Thr Ile Pro Arg Ser Thr Gly Glu Tyr Tyr Leu Gly Asn Gly Ser Ile
       290                 295                 300
   Pro Ala His Val Arg Gly Asp Phe Gln Gln Ala Ser Pro Arg Ala Ser
   305                 310                 315                 320
   Pro Thr Ala Thr Ser Pro Ser Leu Ser Ser Tyr Ser Ser Ala Pro Leu
                   325                 330                 335
   Pro Arg Pro Ser Met Thr Ser His Pro Ser Thr Tyr Ala Pro Pro Gln
               340                 345                 350
   Pro Leu Glu Pro Pro Ala Thr Lys Asp His Arg Pro Asn Ser Val Gly
           355                 360                 365
   Gly Ser Pro His Met Ser Ser Leu Gly Trp Pro Ser Pro Ser His Ser
       370                 375                 380
   Pro Val Ser Ala Pro Asp Phe Gly Tyr Pro Glu Pro Ser Gly Pro Pro
   385                 390                 395                 400
   Tyr Pro Gly Ser Val Pro Pro His Met Tyr Phe Pro Asn Ser Thr Ile
                   405                 410                 415
   Arg Arg Pro Ala Ser Thr Glu Pro Glu Asn Tyr Glu Leu Arg Pro Arg
               420                 425                 430
   Leu Gly Asp Asn Ser Trp Ser Thr Pro Ala
           435                 440
<210> 84
   <211> 460
   <212> PRT
   <213> Artificial Sequence
<220>
   <223> micgyMGYG_06906T0
<400> 84
   Met Asp Ile Thr Thr Ile Leu Asn Arg Lys Ala Ser Val Ser Val Val
   1               5                   10                  15
   Ala Ala Asp Val His Arg Leu Gln Gln His Ile Val His Ala Gln Ala
               20                  25                  30
   Gln Thr Asp Asp Val Leu Lys Ser Arg Ser Pro Ser Glu Met Gly Ser
           35                  40                  45
   Glu His His Arg Ser Ala Ser Ala Pro Pro Ser Glu His His His His
       50                  55                  60
   His His His His Gln Pro Thr Ser Ser Phe Pro Ser Ser Thr Gln Ser
   65                  70                  75                  80
   Leu Pro Gln Met Ala His Leu Ala Gln Tyr His Val Gln Ala Gln Ala
   85 90 95
   Asn His Ser Ser Pro Asn Pro Gly Tyr Ala His Ser Ala His Gly Ser
   100 105 110
   Asp Tyr Gly Arg Ser Ser Gly Gly Asn Met Arg Pro Thr Gly Leu Pro
   115 120 125
   Ala Leu Lys Thr Phe His Cys Gln Thr Cys Ser Lys Gly Phe Ala Arg
   130 135 140
   Arg Ser Asp Leu Ala Arg His Glu Arg Ile His Ser Gly Ile Arg Pro
   145 150 155 160
   His Ala Cys Asp Trp Pro Gly Cys Gly Lys Gln Phe Ile Gln Arg Ser
   165 170 175
   Ala Leu Thr Val His Thr Arg Val His Thr Gly Glu Lys Pro His Met
   180 185 190
   Cys Asp Arg Cys Gly Lys Pro Phe Ser Asp Ser Ser Ser Leu Ala Arg
   195 200 205
   His Arg Arg Ile His Ser Gly Lys Arg Pro Tyr Lys Cys Pro Tyr Ala
   210 215 220
   Asn Cys Gln Lys Thr Phe Thr Arg Arg Thr Thr Leu Thr Arg His Gln
   225 230 235 240
   Asn His His Thr Gly Thr Ile Glu Glu Ala Ala Ala Glu Thr Glu Ala
   245 250 255
   Asn Leu Arg His Ser Lys Asp Arg Thr Thr Thr Thr Arg Pro Ser Asp
   260 265 270
   Tyr Ser Glu Pro Gly Ser Ala His Ser Thr Pro Ser Pro Ala Gln Arg
   275 280 285
   Pro Ser Leu Ser Pro Gly Asn Glu Leu Pro Pro Leu Asn Leu Ser Arg
   290 295 300
   Pro Ser Ala Gly Pro Thr Ser Ser Gly Gly Ala Asp Tyr Tyr Thr Ser
   305 310 315 320
   Leu Pro Pro His Leu Arg Thr Asp Phe Ala Asn Ser Ser Pro Arg Ala
   325 330 335
   Ser Pro Thr Ser Pro Ser Pro Thr Leu Thr Ser Leu Ser Gly Pro Ala
   340 345 350
   Ser Phe His His His Asn His Asn Arg Pro Ser Leu Thr Ser His Pro
   355 360 365
   Ser Gly Tyr Ala Pro Pro Gln Pro Leu Glu Pro Pro Ala Asn Ser Asp
   370 375 380
   Pro Arg Pro Asn Ser Val Ala Gly Ser Pro His Leu Ser Ser Val Gly
   385 390 395 400
   Trp Ala Ser Pro Thr Leu Ser Ser Met Pro Ser Pro Ser Ser Ala Ser
   405 410 415
   Ala Pro Glu Tyr Ser Tyr Pro Glu Pro Ala Ser His Gln Tyr Pro Gly
   420 425 430
   Gly Ile Thr Pro His Met Phe Phe Pro Arg Arg Pro Gln Ser Ser Glu
   435 440 445
   Pro Glu Asn Tyr Glu Met Lys Pro Lys Leu Glu Leu
   450 455 460
<210> 85
   <211> 439
   <212> PRT
   <213> Artificial Sequence
<220>
   <223> veralVDBG_00166T0
<400> 85
   Met Ser Pro Ser Pro Thr Pro Gln Pro Trp Asp Gln Trp Ser Gln Gln
   1 5 10 15
   Gln Gln Gln Gln Gln Gln Pro Gln His His Ser Gly Tyr Pro Leu Met
   20 25 30
   Asp Gln Ser Val Phe Pro Gln Tyr Ala Pro Pro Pro Thr Gln Tyr Asp
   35 40 45
   Pro Arg Thr Ser Gln Tyr Asp Pro Arg Thr Val Val Thr Ser Gly Pro
   50 55 60
   Leu Ile Ala Pro His Met Ala Pro His Tyr Val Thr Ala Pro Ala Tyr
   65 70 75 80
   Gly Val Ser Pro Ile Ser Asn Met Lys Pro His Phe Ser Thr Gln Thr
   85 90 95
   His Tyr Ser Tyr Gly Gln Tyr Asp Gln Pro Thr Gly His Leu Ala Met
   100 105 110
   Pro Tyr Ser Arg Met Ser Pro Gln Gln Glu Arg Leu Gly Leu Pro Met
   115 120 125
   Val Ser Gln Met Pro Gln Gln Gln His Leu His Ser Gln Gln Arg Gln
   130 135 140
   Gln Arg Ser Pro Ser Ala Ala Arg Ser Glu Thr Leu Ser Ala Pro Lys
   145 150 155 160
   Thr Pro Asp Pro Met Asn Thr Lys Glu Ile Thr Tyr Asn Lys Pro Val
   165 170 175
   Met Thr Asp Asp Val Val Phe Thr Thr Pro Val Asp Val Met Met Lys
   180 185 190
   Ala Leu Gln Lys Arg Lys Asp Lys Glu Asp Leu Ala Ser Ser Asn Ser
   195 200 205
   Ser Glu Val Gly Ala Gly Ser Val Lys Ser Glu Pro Ser Ser Pro His
   210 215 220
   Pro Ser Ser Thr Asp Gly Ser Gln Glu Thr Thr Asp Lys Val Lys Lys
   225 230 235 240
   Tyr Arg Cys Pro Tyr Asp Gly Cys Pro Lys Ser Phe Gln Gln Ser Thr
   245 250 255
   His Leu Glu Thr His Lys Arg Ala His Thr Gly Asp Lys Pro Tyr Lys
   260 265 270
   Cys Glu Trp His Gly Cys Gly Arg Arg Phe Ser Gln Pro Gly Asn Leu
   275 280 285
   Lys Thr His Thr Arg Leu His Thr Gly Glu Arg Pro Phe Glu Cys Glu
   290 295 300
   Met Cys Gly Ala Cys Phe Ala Gln Arg Gly Asn Leu Thr Ala His Lys
   305 310 315 320
   Ala Thr His Ser Lys Thr Lys Pro Phe Val Cys Lys Leu Asp Thr Cys
   325 330 335
   Asn Lys Cys Phe Thr Thr Arg Gly Asn Leu Lys Asn His Gln Asn Lys
   340 345 350
   Tyr His Lys Glu Thr Ile Ala Gln Leu Val Asp Trp Ile Ile Ser Leu
   355 360 365
   Thr Asp Val Asp Ala Leu Ser Ala Lys Asp Arg Asp Leu Leu Trp Tyr
   370 375 380
   Phe Ser Asn Ile Tyr Lys Asn Ser Asn Lys Gly Ile Lys Gly Arg Gly
   385 390 395 400
   Arg Asp Arg Arg Val Ser Glu Val Arg Met Gly Lys Thr Lys Leu Ser
   405 410 415
   Ser Pro Gly Met Gly Leu Pro Arg Ser Lys Met Leu Asp Ser Ser Phe
   420 425 430
   Ser Arg Leu Gly Leu Gly Phe
   435
<210> 86
   <211> 405
   <212> PRT
   <213> Artificial Sequence
<220>
   <223> aspcl4643
<400> 86
   Met Lys Pro Glu Pro Gly Val Pro Glu Ala Pro Asp Gln Gln Val Leu
   1 5 10 15
   Ser Tyr Pro Pro His Ala Pro Leu Asn Ser Met Pro Ser Met Val Pro
   20 25 30
   Asp Met Arg Tyr Ser Gln Gly Gln Pro Asn Ser Gly Met Pro Leu Leu
   35 40 45
   Gln Asn Ala Tyr Val Pro Gly Val Tyr Thr Thr Asn Ala Gln Ile Pro
   50 55 60
   Asn Gly Ala Thr Pro Gln Arg Ala Asp Pro Pro Pro Lys Thr Phe His
   65 70 75 80
   Cys Ser Thr Cys Gly Lys Gly Phe Ala Arg Arg Ser Asp Leu Ala Arg
   85 90 95
   His Glu Arg Ile His Thr Gly Ile Arg Pro His Ala Cys Asp Trp Pro
               100                 105                 110
   Gly Cys Gly Lys Gln Phe Ile Gln Arg Ser Ala Leu Thr Val His Ser
           115                 120                 125
   Arg Val His Thr Gly Glu Lys Pro His Met Cys Glu Arg Cys Gly Lys
       130                 135                 140
   Pro Phe Ser Asp Ser Ser Ser Leu Ala Arg His Arg Arg Ile His Ser
   145                 150                 155                 160
   Gly Lys Arg Pro Tyr Lys Cys Pro Tyr Ala Asn Cys Gln Lys Thr Phe
                   165                 170                 175
   Thr Arg Arg Thr Thr Leu Thr Arg His Gln Asn His His Thr Gly Thr
               180                 185                 190
   Ile Glu Glu Ala Ala Ala Glu Thr Glu Ala Asn Leu Arg Gln Asn Lys
           195                 200                 205
   Glu Arg Ala Arg Val Pro Gly Asp Gly Ala Phe Ser Glu His Ala Ser
       210                 215                 220
   Val His Ser Thr Pro Ser Pro Ala His His Pro Ala Ala Asn Pro Pro
   225                 230                 235                 240
   Pro Gly Asp Leu Pro Pro Leu Asn Met Pro Arg Ser Thr Gly Asp Tyr
                   245                 250                 255
   Tyr Met Gly Asn Gly Ser Ile Pro Pro His Val Arg Gly Asp Phe Ser
               260                 265                 270
   Gln Ala Ser Pro Arg Ala Ser Pro Thr Ala Thr Ser Pro Ser Leu Ser
           275                 280                 285
   Ser Phe Ser Asn Ala Pro His Pro Arg Pro Ser Met Thr Ser His Pro
       290                 295                 300
   Ser Gly Tyr Ala Pro Pro Gln Pro Leu Glu Pro Pro Ala Asn Ser Asp
   305                 310                 315                 320
   His Arg Pro Asn Ser Val Ser Gly Ser Pro His Met Thr Ser Leu Gly
                   325                 330                 335
   Trp Ala Ser Pro Ser His Gly Ser Ile Pro Ser Pro Gly Ser Val Asn
               340                 345                 350
   Asp Phe Asn Tyr Pro Glu Pro Ser Gly Pro Ala Tyr Pro Asn Ser Met
   355 360 365
   Pro Pro His Met Tyr Phe Pro Asn Ser Thr Ile Arg Arg Pro Gly Ser
   370 375 380
   Thr Glu Pro Glu Asn Tyr Glu Met Lys Pro Arg Leu Gly Asp Asn Gly
   385 390 395 400
   Trp Ser Thr Pro Val
   405
<210> 87
   <211> 463
   <212> PRT
   <213> Artificial Sequence
<220>
   <223> magpoMAPG_07622T0
<400> 87
   Met Ser Asp Pro Arg Pro Ala Val Ser Pro Thr Val Ser Val Ser Gly
   1 5 10 15
   Ala Gly Ala Ala Ala Leu Gln Ser His Met Ser Leu Glu Ala Val Lys
   20 25 30
   Gln Glu Thr His Gly Leu Gln Pro Leu Gln Gln His Tyr His His Gln
   35 40 45
   Leu Ala Pro Gly Pro Met Asp Arg Ala Asp Ser Pro His Gly Ser Glu
   50 55 60
   His Ser Arg Tyr Ser Val Pro Pro Leu Asn Gly Met Asp Gln Pro Arg
   65 70 75 80
   Pro Tyr Gly Ser Pro Thr Ala Met His Ala Pro Leu His Leu Ser Asp
   85 90 95
   Met Asn Arg Pro Pro Thr Ser Leu Pro Met Asn Ser Leu Pro Leu Pro
   100 105 110
   Ala Ile Gln Pro Thr Leu Ala Pro Ala Pro Gln Ala Ala Gly Ala Val
   115 120 125
   Tyr Gly Ser Gln Glu Thr Pro Gln Pro Pro Pro Lys Ala Tyr Pro Cys
   130 135 140
   Ser Thr Cys Gly Lys Gly Phe Ala Arg Arg Ser Asp Leu Ala Arg His
   145                 150                 155                 160
   Glu Arg Ile His Ser Gly Val Arg Pro His Ile Cys Glu Phe Pro Gly
                   165                 170                 175
   Cys Asn Lys Lys Phe Ile Gln Arg Ser Ala Leu Thr Val His Gln Arg
               180                 185                 190
   Val His Thr Gly Glu Lys Pro His Gln Cys Glu Arg Cys Gly Lys Pro
           195                 200                 205
   Phe Ser Asp Ser Ser Ser Leu Ala Arg His Arg Arg Ile His Ser Gly
       210                 215                 220
   Lys Arg Pro Tyr Lys Cys Pro Tyr Ala Asp Cys Gln Lys Thr Phe Thr
   225                 230                 235                 240
   Arg Arg Thr Thr Leu Thr Arg His Gln Asn His His Thr Gly Thr Ile
                   245                 250                 255
   Glu Glu Ala Ala Ala Ala Thr Arg Glu Ala Leu Ala Arg Gly Pro Thr
               260                 265                 270
   Gly Thr Ala Arg Met Thr Pro Thr His Val Thr Arg Ser Glu Gly Asp
           275                 280                 285
   Ala Gly Ser Gly Ser Asn His Ala Ser Pro Met Ser Thr Pro Ser Pro
       290                 295                 300
   Ala His Arg Thr Asn Ser Met Ser Pro Ser Asn Glu Leu Ala Gly Ile
   305                 310                 315                 320
   Gly Ser Ile Gln Ser Gln Tyr Val Asn Asn Ser Leu Pro Ser His Leu
                   325                 330                 335
   Arg Gly Asp Val His Ala Ala Ser Pro Thr Ser Thr Thr Ser Ser Gly
               340                 345                 350
   Tyr Thr Asn Asn Arg Pro Thr Ser His Pro Thr Gly Tyr Gly Pro Pro
           355                 360                 365
   Gln Val Leu Glu Pro Asn Val Glu Thr Arg Pro Gly Pro Gly Ser Ala
       370                 375                 380
   Ser Gly Ser Pro His Leu Gly Ser Val Gly Trp Gln Ser Pro Ser His
   385                 390                 395                 400
   Val Gly Ser Pro Ser His Ser Thr Ser Gly Phe Val Tyr Pro Asp Pro
   405 410 415
   Glu Ala Tyr Pro Ser Ser Ala Gly Met Gly Pro Leu Phe Tyr Ser Asn
   420 425 430
   Ala Ile Ala Gly Val Arg Ser Gly Ser Ala Glu Pro Gly Gln Asn Asn
   435 440 445
   Asp Tyr Pro Lys Ser Arg Ser Ser Glu Leu Trp Ala Ser Ala Gln
   450 455 460
<210> 88
   <211> 462
   <212> PRT
   <213> Artificial Sequence
<220>
   <223> fusoxFOXG_14211T0
<400> 88
   Met Leu Pro Leu Asp Ser Arg Thr Thr Thr Thr Gly Pro Pro Gln Arg
   1 5 10 15
   Pro Ala Met Ala Pro Tyr Met Met Tyr Gln Ser His Gly Pro Gly Pro
   20 25 30
   Val Asn Ser Phe Ala Ala Ser Tyr Tyr Gln Thr Pro Asn Pro Tyr Gln
   35 40 45
   Phe Gly Glu Tyr Gln Gly Pro Pro Thr Pro Pro His Leu Val Ala Pro
   50 55 60
   Pro Lys Ile Gln Tyr Tyr Asp His Asn Ala Val Gly His Asp Lys Asp
   65 70 75 80
   Asp Gly Arg Thr Pro Ser Tyr Pro Arg Glu Met Glu Tyr Thr Tyr Ala
   85 90 95
   Glu Gln Ala Pro Ser Pro Ala Arg Ser Asp Leu Gln Ala Ser Thr Val
   100 105 110
   Arg Tyr Ser Gly Thr Lys Pro Ser Met Thr Ser Lys Thr Ile Ile Ser
   115 120 125
   Asn Glu Thr Leu Asn Pro Gly Asp Gln Thr Asn Phe Val Thr Glu Val
   130 135 140
   Asp Glu Leu Met Lys Val Ile Gln Arg Lys Ala Asp Leu Gln Val Gly
   145 150 155 160
   Ala Gly Gln Gln Pro Leu Ala Pro Val Met Ser Ser Val Ser Val Ile
   165 170 175
   Ser Gln Gly Thr Pro Gly Ser Ile Asp Asn Lys Thr Ala Arg Lys Arg
   180 185 190
   Tyr Arg Cys Asp Gly Pro Ser Cys Gln Lys Ser Phe Thr Gly Lys Thr
   195 200 205
   His Leu Asp Ile His Arg Arg Thr His Thr Gly Ile Lys Pro Tyr Ile
   210 215 220
   Cys Asp Phe Pro Gly Cys Asp Leu Thr Phe Ser Gln Leu Gly Asn Leu
   225 230 235 240
   Lys Thr His Lys Arg Arg His Thr Gly Glu Arg Pro Phe Ala Cys Glu
   245 250 255
   Gln Cys Asp Arg His Phe Ser Gln Arg Gly Asn Leu Gln Ala His Leu
   260 265 270
   Glu Ser His Lys Gly Leu Lys Pro Phe Ile Cys Ile Leu Asp Asp Cys
   275 280 285
   Asn Lys Thr Phe Thr Leu Leu Gly Asn Met Lys Thr His Gln Asn Ser
   290 295 300
   Leu His Lys Glu Thr Leu Glu Glu Leu Thr Met Lys Phe Thr Lys Ile
   305 310 315 320
   Ile Ala Ser Gly Glu Lys Val Cys Glu Ala Asp Arg Glu Leu Phe Glu
   325 330 335
   Tyr Phe Ala Thr His Tyr Lys Asn Ser Asn Lys Gly Ile Arg Gly Arg
   340 345 350
   Gly Asp Ala Arg Thr Val Ala Asp His Lys Thr Arg Met Glu Met Asn
   355 360 365
   His Pro Val Asn Thr Met Thr Ala Val Pro Gln His Pro Leu Pro Gln
   370 375 380
   Ile Val Ser Thr Pro Ile Pro Leu His Gly Leu Pro Val Ser Val Ser
   385 390 395 400
   Leu Ala Ser Tyr Ser Val Ser Arg Val Gln Pro Gly Pro Ile Asn Pro
   405 410 415
   Met Ser Thr Gln Thr His Ala Gly Gly Tyr Glu Met Tyr Asp Met Asn
   420 425 430
   Gly His His Arg Ile Gln Pro Leu Asn Ser His Gly Leu Ser Tyr Asp
   435 440 445
   Thr Asn Ser Ala Arg Glu Met Gly Tyr His Glu Arg Met Tyr
   450 455 460
<210> 89
   <211> 440
   <212> PRT
   <213> Artificial Sequence
<220>
   <223> PC_255948916
<400> 89
   Met Asp Phe Thr Thr Ile Leu Asn Arg Lys Asn Ser Ala Ala Ala Ala
   1 5 10 15
   Ala Ala Ala Glu Ala Gln Leu Gln Gln Gln Tyr Phe Gln Gln Ser Ala
   20 25 30
   Gln Leu His Thr Gly Ala Ser Pro Thr Met Lys Ser Glu Ser Gly Gly
   35 40 45
   Ser Asp Asn Pro Val Asn Ala Tyr Pro Pro His Gly Pro Pro Pro Met
   50 55 60
   Gln Met Asp Ala Gly Leu Ala Asp Ser Phe Tyr Tyr Ala Gln Pro Thr
   65 70 75 80
   Gly Ser Thr Pro Arg Asn Met Ala Tyr Ala Pro Ala Gly Tyr Ala Gly
   85 90 95
   Asp Pro Gln Met Gln Gln Glu Pro Val Pro Gln Gly Arg Ala Gly Val
   100 105 110
   Glu Pro Pro Pro Lys Thr Phe His Cys Ser Thr Cys Asn Lys Gly Phe
   115 120 125
   Ala Arg Arg Ser Asp Leu Ala Arg His Glu Arg Ile His Thr Gly Val
   130 135 140
   Arg Pro His Ala Cys Glu Trp Pro Gly Cys Gly Lys Gln Phe Ile Gln
   145                 150                 155                 160
   Arg Ser Ala Leu Thr Val His Ser Arg Val His Thr Gly Glu Lys Pro
                   165                 170                 175
   His Met Cys Glu Arg Cys Gly Lys Pro Phe Ser Asp Ser Ser Ser Leu
               180                 185                 190
   Ala Arg His Arg Arg Ile His Ser Gly Lys Arg Pro Tyr Lys Cys Pro
           195                 200                 205
   Tyr Ala Asn Cys Gln Lys Thr Phe Thr Arg Arg Thr Thr Leu Thr Arg
       210                 215                 220
   His Gln Asn His His Thr Gly Thr Ile Glu Glu Ala Ala Ala Glu Thr
   225                 230                 235                 240
   Glu Ala Gln Leu Arg Gln Asn Lys Asp Arg Gly Arg Pro Gly Glu Gly
                   245                 250                 255
   Met Phe Ser Glu His Ala Ser Ile His Ser Thr Pro Ser Pro Ala Gln
               260                 265                 270
   His Pro Ser Met Ser Pro Gly Gly Glu Leu Pro Pro Leu Asn Met His
           275                 280                 285
   Arg Ser Ala Gly Asp Tyr Tyr Met Gly Thr Gly Pro Ile Pro Pro His
       290                 295                 300
   Val Arg Gly Asp Phe Pro Gln Gly Ser Pro Arg Ala Ser Pro Thr Ala
   305                 310                 315                 320
   Thr Ser Pro Ser Leu Ser Ser Tyr Gly Ser Ala Pro His Thr Arg Pro
                   325                 330                 335
   Ser Met Thr Ser His Pro Tyr Ala Pro Pro Gln Pro Leu Glu Pro Pro
               340                 345                 350
   Ala Asn Ser Asp His Arg Pro Asn Ser Val Asn Gly Ser Pro His Met
           355                 360                 365
   Thr Ser Leu Gly Trp Ala Ser Pro Ser His Gly Ser Met Pro Ser Pro
       370                 375                 380
   Gly Ser Ala Asn Asp Phe Thr Tyr Pro Glu Pro Thr Gly Pro Ala Tyr
   385                 390                 395                 400
   Pro Thr Ser Met Pro Pro His Met Tyr Phe Pro Asn Ser Thr Ile Arg
   405 410 415
   Arg Pro Thr Ser Thr Glu Pro Glu Asn Tyr Glu Met Lys Pro Arg Gly
   420 425 430
   Asp His Ser Trp Ser Thr Ala Val
   435 440
<210> 90
   <211> 395
   <212> PRT
   <213> Artificial Sequence
<220>
   <223> neudiestExt_fgenesh3_pm.C_160037
<400> 90
   Met Ala Glu Asp Pro Glu Arg Pro Arg Pro Phe Leu Cys Asp Trp Gln
   1 5 10 15
   Gly Cys Gly Lys Arg Phe Asn Arg Lys Ser Asp Leu Gln Arg His His
   20 25 30
   Arg Ile His Thr Asn Glu Arg Pro Tyr Gly Cys Asn Trp Gln Asp Cys
   35 40 45
   Gly Lys Arg Phe Ile Gln Arg Ser Ala Leu Thr Val His Leu Arg Thr
   50 55 60
   His Thr Gly Glu Lys Pro His Gln Cys Glu Val Pro Glu Cys Arg Lys
   65 70 75 80
   Pro Phe Ala Asp Ser Ser Ser Leu Ala Arg His Arg Arg Ile His Arg
   85 90 95
   Gly Glu Lys Pro Tyr Arg Cys Gln Asp Val Gly Cys Asn Lys Ala Lys
   100 105 110
   Thr Thr Arg Asp Lys His His Thr Asn Ser His Arg Gly Gly Ile Asn
   115 120 125
   Arg Ser Met Thr Pro Arg Gln Gln Leu His Pro Ser Phe Gly Ser Gly
   130 135 140
   Phe Asp Gln Phe His Met Ser Ala Pro Pro Glu Leu Thr Ala Ser Thr
   145 150 155 160
   Asp Ser Ser Pro Ala Asp Ser Thr Ala Tyr Ser Pro Ala Ala Leu Glu
                   165                 170                 175
   Gly Asp Trp Ser Ser Gln Asn Phe Asn Asn Tyr Thr His Met Pro Asn
               180                 185                 190
   Leu Gln Glu Ala Ser Ile Ala Leu Gln Asn Gln Ile Arg Ser Gln Gln
           195                 200                 205
   Ile Pro Gln Leu Met Pro Tyr Met Ser Ser Arg Val Val Pro Ser Tyr
       210                 215                 220
   Ser Ala Pro Pro Pro Asn Met Met Gly Gln Gln Met Pro Gln Gln Gly
   225                 230                 235                 240
   Ser Phe Ser Ser Tyr Ser Pro Ser Pro Val Asp Ser Thr Gln Gln Gln
                   245                 250                 255
   Pro Trp Ala Thr Gly Leu Gln Asn Asn Gln Arg His Tyr Gln Arg Val
               260                 265                 270
   Leu Thr Ser Asn Asp Thr Ala Pro Arg Met Phe Ser Pro Tyr Pro Ala
           275                 280                 285
   Pro Pro Asn Met Met Thr Ser Gln Asn Asp Phe Asn Asn Gln Tyr Pro
       290                 295                 300
   Pro Pro Gln Gln Gln Gln Ser Trp Gly Met Pro Gln Asn Gln Gly Gln
   305                 310                 315                 320
   Gln Asp Gln Arg His Tyr Ser Leu Ser Ser Pro Ser Gln Pro Leu Thr
                   325                 330                 335
   Pro Val Pro Val Ser Ala Gly Pro Gly Thr Met Gln Pro Leu Gln Ser
               340                 345                 350
   Met Pro Gly His Ile Tyr Gly Ala Arg Leu Val Gly Asp Asp Leu Ser
           355                 360                 365
   Asp His Phe Phe Lys Val Glu Thr Thr Ala Asp Glu Asn Asn Pro Gly
       370                 375                 380
   Met Thr Leu Pro Asp Ala Arg Phe Ala Gly Leu
   385                 390                 395
<210> 91
   <211> 471
   <212> PRT
   <213> Artificial Sequence
<220>
   <223> geodeGMDG_02792T0
<400> 91
   Met Ser Asn Ile Asn Gln Asn Gln Asn Pro Gly Ala Asn Gly Gly Ala
   1               5                   10                  15
   Ser Met Asp Ile Thr Asn Ile Leu Asn Thr Lys Gly Gly Ala Ala Ala
               20                  25                  30
   Ala Ala Ala Ala Ala Gly Ala Ser Pro Ser Asp His Thr Phe His Gln
           35                  40                  45
   Gln Leu Met His Ala Ser Gly Gly Gln Ala Asn Ser Glu Thr Ala Ser
       50                  55                  60
   Glu Arg Gly Gly Ser Pro His Gly Ser Glu His Ser Ser Arg Tyr Ser
   65                  70                  75                  80
   Gly Pro Pro Ser Met Gly His Met Asn Gly Met Gly Asn Gly Met Arg
                   85                  90                  95
   Tyr Pro Ser Pro Thr Ala Met Gln Ser Pro Leu Pro Met Gln Ala Gly
               100                 105                 110
   Phe Arg Asn Asp Gly Phe Asp Gly Ser Ala Pro Gln His Gln Glu Ile
           115                 120                 125
   Pro Arg Ser Thr Gly Arg Leu Pro Pro Gly His Gly Val Thr Gln Gln
       130                 135                 140
   Lys Ala Phe Pro Cys Thr Val Cys Gln Lys Gly Phe Ala Arg Arg Ser
   145                 150                 155                 160
   Asp Leu Ala Arg His Glu Arg Ile His Ser Gly Ile Arg Pro His Val
                   165                 170                 175
   Cys Asp Tyr Pro Asn Cys Gly Lys Gln Phe Ile Gln Arg Ser Ala Leu
               180                 185                 190
   Thr Val His Ala Arg Val His Thr Gly Glu Lys Pro His Met Cys Glu
           195                 200                 205
   Arg Cys Gly Lys Arg Phe Ser Asp Ser Ser Ser Leu Ala Arg His Arg
       210                 215                 220
   Arg Ile His Ser Gly Lys Arg Pro Tyr Lys Cys Pro Tyr Ala Asp Cys
   225                 230                 235                 240
   Gln Lys Thr Phe Thr Arg Arg Thr Thr Leu Thr Arg His Gln Asn His
                   245                 250                 255
   His Thr Gly Thr Val Glu Glu Ala Ala Ala Ala Thr Ala Ala Ala Leu
               260                 265                 270
   Ala Ser Arg Ala Gly Ser Asn Arg Ser Gly Arg Gly Pro Arg Ser Asp
           275                 280                 285
   Gly Glu Gln Tyr Ser Ala Thr Gly Ser Pro Met Ser Thr Pro Ser Pro
       290                 295                 300
   Gly Gln Arg Thr His Ser Met Ser Pro Asn Ala Glu Leu Ala Pro Met
   305                 310                 315                 320
   Asn Gly Met Gln Arg His Met Gly Asp Tyr Pro Tyr Met Asn Asn Ser
                   325                 330                 335
   Ser Leu Pro Val His Leu Arg Asn Asp Tyr Ala Gln His Val Pro Met
               340                 345                 350
   Gln Asn Ala Pro Leu Pro Gln Lys Gln Ser Gln Tyr Pro Asn Asn Gln
           355                 360                 365
   Arg Pro Thr Ser His Pro Thr Gly Tyr Gly Pro Pro Gln Ile Leu Glu
       370                 375                 380
   Pro Pro Ala Asn Pro Gln Ser Ala Gly Ser Ala Ser Gly Gly Ser Pro
   385                 390                 395                 400
   His Met Ser Asn Ala Gly Trp His Ser Pro Ser Asn Met Pro Ser Pro
                   405                 410                 415
   Thr Ser Gln Ser Asn Gly Tyr Val Tyr Pro Asp Pro Asp Pro Tyr Gly
               420                 425                 430
   Ser Gly Gly Met Gly His Met Tyr Tyr Pro Asn Ser Asn Ile Arg Arg
           435                 440                 445
   Pro Gln Ser Thr Glu Pro Asp Ser Tyr Glu Thr Lys Pro Arg Leu Asn
       450                 455                 460
   Glu Leu Trp Thr Ala Ala Gln
   465                 470
<210> 92
   <211> 424
   <212> PRT
   <213> Artificial Sequence
<220>
   <223> aspacGenemark1.1898_g
<400> 92
   Met Val Ala Ala Asp Thr Pro Phe Asp Pro Ala His Leu Val Pro Asn
   1               5                   10                  15
   Pro Gly Met Asp His Ser Pro Lys Met Lys Ala Glu Pro Gly Ala Glu
               20                  25                  30
   Ser Thr Asp Gln Gln Val Leu Ser Tyr Pro His His Ala Gln Leu Ser
           35                  40                  45
   Gln Met Pro Ser Met Gln Gln Asp Leu Arg Tyr Ala Pro Gln Ala His
       50                  55                  60
   Pro Asn Gln Gly Leu Pro Leu Leu Gln Asn Thr Phe Ile Pro Gly Ala
   65                  70                  75                  80
   Tyr Ala Gly Ala Gly Gln Leu Pro Ala Asn Val Pro Gln Gly Arg Pro
                   85                  90                  95
   Asp Pro Pro Pro Lys Thr Phe His Cys Gly Thr Cys Ser Lys Gly Phe
               100                 105                 110
   Ala Arg Arg Ser Asp Leu Ala Arg His Glu Arg Ile His Ser Gly Ile
           115                 120                 125
   Arg Pro His Ala Cys Asp Trp Pro Gly Cys Gly Lys Gln Phe Ile Gln
       130                 135                 140
   Arg Ser Ala Leu Thr Val His Ala Arg Val His Thr Gly Glu Lys Pro
   145                 150                 155                 160
   His Met Cys Glu Arg Cys Gly Lys Pro Phe Ser Asp Ser Ser Ser Leu
                   165                 170                 175
   Ala Arg His Arg Arg Ile His Ser Gly Lys Arg Pro Tyr Lys Cys Pro
               180                 185                 190
   Tyr Ala Asn Cys Gln Lys Thr Phe Thr Arg Arg Thr Thr Leu Thr Arg
           195                 200                 205
   His Gln Asn His His Thr Gly Thr Ile Glu Glu Ala Ala Ala Glu Thr
       210                 215                 220
   Glu Ala Asn Leu Arg Gln Asn Lys Glu Arg Ala Arg Val Ser Gly Asp
   225                 230                 235                 240
   Met Phe Ser Asp His Gly Ser Val His Ser Thr Pro Ser Pro Ala His
                   245                 250                 255
   His Pro Val Ser Pro Val Gly Asp Leu Pro Pro Leu Asn Met Pro Arg
               260                 265                 270
   Ser Asp Tyr Tyr Met Thr Asn Gly Ser Ile Pro Pro His Val Arg Gly
           275                 280                 285
   Gly Phe Pro Gln Val Ser Pro Arg Ala Ser Pro Thr Ala Thr Ser Pro
       290                 295                 300
   Ser Leu Ser Ser Tyr Gly Ser Ala Pro His Val Arg Pro Ser Met Thr
   305                 310                 315                 320
   Ser His Pro Ser Gly Tyr Gly Pro Pro Gln Pro Leu Glu Pro Pro Ala
                   325                 330                 335
   Asn Asn Asp His Arg Pro Asn Ser Val Ser Gly Ser Pro His Met Thr
               340                 345                 350
   Ser Leu Gly Trp Ala Ser Pro Ser His Ser Ser Ile Pro Ser Pro Gly
           355                 360                 365
   Ser Ala Thr Asp Phe Gly Tyr Pro Glu Pro Ser Gly Ala Ala Tyr Ala
       370                 375                 380
   Ser Ser Met Pro Pro His Met Tyr Phe Pro Ser Ser Thr Ile Arg Arg
   385                 390                 395                 400
   Pro Ala Ser Thr Glu Pro Asp Asn Tyr Glu Met Lys Pro Arg Leu Gly
                   405                 410                 415
   Asp Ser Ala Trp Ser Thr Pro Val
               420
<210> 93
   <211> 555
   <212> PRT
   <213> Artificial Sequence
<220>
   <223> neuteestExt_Genewise1.C_10702
<400> 93
   Met His Thr Tyr Thr Asp Met Tyr Thr Phe Ala His Arg Leu His Thr
   1               5                   10                  15
   Pro Arg Ser Val Gln Ser Glu Cys Ala Ser Thr Cys Arg Ala Arg Leu
               20                  25                  30
   Leu Ser Arg Val Pro Pro Ile Trp Arg Gly Thr Tyr Pro Thr Asn Arg
           35                  40                  45
   Met Ala Leu Thr Ala Gln Pro Ser Ala Pro Ala Gly Trp Glu Arg Trp
       50                  55                  60
   Pro Gln His His Pro Ser Asn Asp Tyr Val Met Met Asp Ala Asp Val
   65                  70                  75                  80
   Met Pro Tyr Asn Ala Arg Pro Met Thr Thr Ala Pro Met Gln Gln Arg
                   85                  90                  95
   Pro Ser Leu Val Pro His Tyr Met Pro Thr Thr Ser Met Ser Thr Ala
               100                 105                 110
   Ser Ile Asn Ser Ile Ser Ala Pro Ala His Tyr Gln Ser Pro Val Ser
           115                 120                 125
   Tyr Gly Gly Gly Tyr Pro Ala Tyr Pro Leu Pro Thr Pro Thr Thr Met
       130                 135                 140
   Asn Ser Ser Tyr Asn Lys His Gln Gln Tyr Gln Glu Arg Pro Ser Leu
   145                 150                 155                 160
   His Met Ile Thr Pro Glu Phe Glu Glu Thr Arg Gly Pro His His Ile
                   165                 170                 175
   Arg Asn Ala Arg Arg Tyr Ser Glu Glu Ser Arg Ser Pro Ser Glu Arg
               180                 185                 190
   Ser Asp Ser Gln Gly Ser Thr Thr Glu Thr Thr Ile Ser Asn His Ser
           195                 200                 205
   Ser Cys Ser Arg Thr Ile Thr Pro Asn Thr Pro Val Asn Gly Ala Pro
       210                 215                 220
   Gln Val Glu Phe Ser Thr Ala Val Asp Lys Leu Met Lys Val Ile Gln
   225                 230                 235                 240
   Ser Lys Met Lys Asp Ala Asp Pro Glu Gln Ser Gly Asp Asp Lys Asp
   245 250 255
   Ile Lys Ala Glu Gln Gln Ser Ser Pro Val Cys Gln Ala Arg Val Gln
   260 265 270
   Gln Pro Ala Asp Lys His Lys Arg Lys Arg Tyr Glu Cys Gln Ile Glu
   275 280 285
   Gly Cys Asn Lys Lys Phe Ser Gln Lys Thr His Arg Asp Thr His Val
   290 295 300
   Arg Ser His Thr Gly Asp Arg Pro Tyr Val Cys Pro Ile Pro Gly Cys
   305 310 315 320
   Gly Gly Arg Phe Thr Gln Ala Gly Asn Leu Lys Thr His Lys Arg Arg
   325 330 335
   His Thr Gly Glu Arg Pro Tyr Arg Cys Glu Val Cys Asp Lys Gly Phe
   340 345 350
   Val Gln Arg Gly Asp Val Lys Ala His Met Lys Thr His Leu Gly Thr
   355 360 365
   Lys Ala Phe Leu Cys Arg Leu Asp Asn Cys His Lys Gln Phe Thr Gln
   370 375 380
   Arg Gly Asn Leu Lys Tyr His Gln Asn Lys Tyr His Asn Glu Thr Ile
   385 390 395 400
   Lys Ala Leu Ala Ala Arg Phe Asp Ala Ile Glu Asp Trp Ser Thr Val
   405 410 415
   Leu Lys Glu Asp Met Glu Ile Phe Lys Asp Phe Ala Glu Val His Lys
   420 425 430
   Asn Ser Asn Lys Gly Ile Lys Gly Arg Gly Lys His Arg Lys Val Lys
   435 440 445
   Ser Val Pro Leu Ser Ser Pro Thr Ser Pro Thr Gly His Ser Pro Leu
   450 455 460
   Pro Asn Ile Met Ala Ser Gln Tyr Pro Leu Pro Ser Ala Pro Gly Val
   465 470 475 480
   Ser Gln Leu Leu His Thr Pro His Pro Leu His Pro Pro Leu His His
   485 490 495
   Gln Gly Pro Ser His Ser Ala Met Tyr Gly Met Pro Arg Ser Ser Leu
   500 505 510
   His Ser His Tyr Glu Thr Tyr Asp His His Glu Val Asp Thr Val Ala
   515 520 525
   Ser Ser Arg Ala Ser Val Ala Glu Pro Ile Tyr His His Glu Glu His
   530 535 540
   Pro Arg Glu Leu Ala Phe Gly Asp Arg Met Tyr
   545 550 555
<210> 94
   <211> 421
   <212> PRT
   <213> Artificial Sequence
<220>
   <223> picstestExt_gwp_genewisePlus_human.C_chr_3.10541
<400> 94
   Met Arg Asn Gln Leu Pro Ala Gln Gln Gln Ser Asn Tyr Tyr Thr Asn
   1 5 10 15
   Ser Gln Pro Ala Gln Leu Pro Tyr Gln Gln Gln Gln Gln Gln Gln Gln
   20 25 30
   Gln Gln Gln Gln Gln Gln Gln Gln Gln Gln Gln Gln Gln Gln Gln Gln
   35 40 45
   Arg Gln Gln Thr Ser Lys Pro Arg Arg Asn Ser Asn Asn Arg Asp Ala
   50 55 60
   Ile Ser Asp Gln Ser Leu Lys Ser Pro Ile Glu Ser Ser Ser Asn Glu
   65 70 75 80
   Gly Asn Leu Val Pro Ala Gln Gln Tyr Val Lys Ser Glu Asp Gly Arg
   85 90 95
   Pro Leu Leu Gly Ala Thr Lys Ile Asp Gln Leu Met Leu Val Ile Gln
   100 105 110
   Ala Arg Asp Lys Gly Ile Thr Ser Pro Ile Gln Gln Ala Pro Asp Gly
   115 120 125
   Ser Ile Leu Ala Ala Pro Asp Tyr Ser Leu Ser Arg Asp Lys Ser Glu
   130 135 140
   Leu Asp Asn Gly Val Leu Pro Arg Pro Ile Ser Leu Val Gly Gly Val
   145                 150                 155                 160
   Asp Lys Pro Ser Lys Ala Lys Ile Lys Glu Asp Glu Gly Ser Asp Asp
                   165                 170                 175
   Glu Glu Ser Lys Gly Lys Arg Arg Lys His Lys Asn Gln Gln Cys Pro
               180                 185                 190
   Tyr Cys Phe Lys Tyr Phe Thr Gln Ser Thr His Leu Glu Val His Ile
           195                 200                 205
   Arg Ser His Ile Gly Tyr Lys Pro Phe Glu Cys Asn Tyr Cys His Lys
       210                 215                 220
   Lys Phe Thr Gln Gly Gly Asn Leu Arg Thr His Leu Arg Leu His Thr
   225                 230                 235                 240
   Gly Glu Lys Pro Phe Thr Cys Asp Ile Cys Asn Arg Gln Phe Asn Arg
                   245                 250                 255
   Lys Gly Asn Leu Gly Ala His Lys Leu Thr His Glu Asn Leu Lys Pro
               260                 265                 270
   Tyr Glu Cys Lys Leu Asp Gly Cys Asp Lys Ser Phe Thr Gln Leu Gly
           275                 280                 285
   Asn Leu Lys Ser His Gln Asn Arg Phe His Leu Ser Thr Leu Asn His
       290                 295                 300
   Leu Thr Gln Lys Leu Ala Glu Leu Ser Gly Leu Ser Ile Glu Asn Leu
   305                 310                 315                 320
   Pro Pro Asp Glu Lys Asp Leu Leu Met Tyr Phe Lys Asp Leu Tyr Lys
                   325                 330                 335
   Asn Ser Asn Lys Gly Ile Arg Gly Arg Gly Lys Ala Lys Leu Ser Lys
               340                 345                 350
   Asp Asp Thr Gly Gly Ala Thr Ser Ser Ser Pro Asp Asn Ser Gln Phe
           355                 360                 365
   Asn Leu Gln Ser Gln Ser Pro Gln Leu Gln Leu Gln Asn Leu Gln Ala
       370                 375                 380
   Leu Pro Arg Gln Gln Asp Gln Gln Gln Gly Ser Pro Glu Tyr Ser Gln
   385                 390                 395                 400
   Ser Gln His Ser Leu Asp Phe Met Asn Pro His Leu Ala Gly Ser Ile
   405 410 415
   Asn Gly Tyr Gln Gly
   420
<210> 95
   <211> 444
   <212> PRT
   <213> Artificial Sequence
<220>
   <223> talstgi242769920refXP_002341871.1
<400> 95
   Met Glu Ile Pro Thr Ile Leu Asn Arg Lys Ala Ser Ala Ala Val Ala
   1 5 10 15
   Ala Asp Pro Arg Phe Gln Ala Gln Leu Ala Gln Ala Val His Met His
   20 25 30
   Thr Gln Glu Met Ser Glu Gln Ala Thr Ser Gln Pro Gly Asp His Thr
   35 40 45
   Val Met Gly Tyr Pro Pro Asn Ala Gln Pro Leu His Gln Thr Gln Gln
   50 55 60
   Met Ala Pro Gln Leu Trp Pro Thr His Pro Asn Gly Ala Pro Val Met
   65 70 75 80
   Pro Pro Asn Gly Tyr Val His Thr Gly Phe Pro Ala Val Pro Pro Gln
   85 90 95
   Gln Ser His Thr Pro Pro Val Gln Gln Gln Gln Gln Gln Gln Gln Ser
   100 105 110
   Arg Pro Glu Pro Ala Pro Arg Val Phe His Cys Ser Thr Cys Gln Lys
   115 120 125
   Gly Phe Ala Arg Arg Ser Asp Leu Ala Arg His Glu Arg Ile His Ser
   130 135 140
   Gly Ile Arg Pro His Ala Cys Asp Trp Pro Gly Cys Gly Lys Gln Phe
   145 150 155 160
   Ile Gln Arg Ser Ala Leu Thr Val His Ala Arg Val His Thr Gly Glu
   165 170 175
   Lys Pro His Met Cys Glu Arg Cys Gly Lys Pro Phe Ser Asp Ser Ser
   180 185 190
   Ser Leu Ala Arg His Arg Arg Ile His Ser Gly Lys Arg Pro Tyr Lys
   195 200 205
   Cys Pro Tyr Ala Asn Cys Gln Lys Thr Phe Thr Arg Arg Thr Thr Leu
   210 215 220
   Thr Arg His Gln Asn His His Thr Gly Thr Ile Glu Glu Ala Ala Ala
   225 230 235 240
   Gln Thr Glu Ala Ser Leu Arg Gln Asn Arg Glu Arg Ser Ala Arg Ala
   245 250 255
   Ser Glu Gly Ile Tyr Ser Glu Thr Gly Ser Gly His Ser Thr Pro Ser
   260 265 270
   Pro Gly Gln Gln Ala Ser Met Gly Met Gly Ser Glu Leu Pro Pro Leu
   275 280 285
   Asn Ile Ile Arg Ser Thr Gly Glu Tyr Tyr Ile Pro Asn Gly Gly Ile
   290 295 300
   Pro Ala His Val Arg Gly Asp Phe Gln Gln Gly Ser Pro Arg Ala Ser
   305 310 315 320
   Pro Ala Ala Thr Ser Pro Ser Leu Ser Ser Tyr Ser Ser His Pro Arg
   325 330 335
   Pro Ser Met Thr Ser His Pro Ser Gly Tyr Gly Pro Pro Gln Pro Leu
   340 345 350
   Glu Pro Pro Ala Asn Asn Asp His Arg Pro Gly Ser Val Thr Gly Ser
   355 360 365
   Pro His Met Thr Ser Met Gly Trp Ala Ser Pro Ser His Gly Ser Met
   370 375 380
   Pro Ser Pro Gly Ser Pro His Asp Phe Gly Tyr Pro Glu Ser Asn Val
   385 390 395 400
   Ala Ala Tyr Ala Ser Ala Met Pro Pro His Met Tyr Phe Pro Asn Ser
   405 410 415
   Thr Ile Arg Arg Pro Gly Ser Thr Glu Pro Glu Asn Tyr Glu Met Lys
   420 425 430
   Pro Arg Ile Gly Pro Asp Gly Trp Ser Thr Pro Met
   435 440
<210> 96
   <211> 344
   <212> PRT
   <213> Artificial Sequence
<220>
   <223> micgyMGYG_06786T0
<400> 96
   Met Pro Arg Thr Arg Ser Phe Pro Cys Ser Trp Val Ser Cys Lys Lys
   1 5 10 15
   Val Phe Asn Arg Lys Ser Asp Leu Cys Arg His Tyr Arg Ile His Thr
   20 25 30
   Asn Glu Arg Pro Tyr Arg Cys Asn Phe Pro Asn Cys Thr Lys Ser Phe
   35 40 45
   Ile Gln Arg Ser Ala Leu Thr Val His Ser Arg Thr His Thr Gly Glu
   50 55 60
   Lys Pro His Val Cys Asn Phe Ala Asn Cys Ser Lys Ala Phe Ser Asp
   65 70 75 80
   Ser Ser Ser Leu Ala Arg His Arg Arg Ile His Thr Gly Arg Arg Pro
   85 90 95
   Tyr Lys Cys Leu Glu Pro Thr Cys Asp Arg Ser Phe Cys Arg Lys Thr
   100 105 110
   Thr Leu Thr Lys His Gln Ser Arg Ser His Gln Pro Glu Ala Val Ser
   115 120 125
   Pro Ser Ala Gln Ser Cys Glu Ser Glu Leu Tyr Ser Gln Gln Ala Gln
   130 135 140
   His Ser Ile Ser Met Met Ser Gln Ser Pro Gln Val Pro Gln His Gln
   145 150 155 160
   Leu Gln Ser Gln Gln Pro Gln Pro Gln Asp Gln Gln Leu Gln Ser Gln
   165 170 175
   Pro Gln His Leu Gln Ser Gln Pro Arg Pro Gln Gln His Ser Pro Tyr
   180 185 190
   Gln Gln Gln His Ser Pro Ile Ile Ala Val Pro Val Ala Asp Tyr Phe
   195 200 205
   His Ala Gln Ala Gln Pro Thr Thr Val His Ser Ile Pro Ile Ser Ala
   210 215 220
   Glu Gln Pro Ile Leu Pro Pro Gln Val His Tyr Val Thr Thr Gly Met
   225 230 235 240
   Ile Ser Ser Gln Ile Pro Arg Tyr Asp Ile Pro Ile Thr Thr Ser Ala
   245 250 255
   Pro Val Ser Thr Pro Thr Ser Met Asp His Leu Ala Tyr Asn His Pro
   260 265 270
   Ile Ser Val Ser Met Pro Gln Gln Gln Arg Ile Gln Ala Pro Gln Pro
   275 280 285
   Val Pro Ile Pro His Gln Ala Asp Gly Gly Phe Gln Val Leu Pro Val
   290 295 300
   Val Ala Gly Gly Ala Gly Gly Gln Tyr Met Lys Glu Phe Asp Gln Met
   305 310 315 320
   Lys His Gly Gln Gln Arg Phe Phe Gly Ser Ala Tyr Pro Asp Gln Met
   325 330 335
   Asn Trp Glu Phe Leu Gly Leu Ser
   340
<210> 97
   <211> 507
   <212> PRT
   <213> Artificial Sequence
<220>
   <223> NCU04179
<400> 97
   Met Ala Leu Thr Ala Gln Pro Ser Ala Pro Ala Gly Trp Glu Arg Trp
   1 5 10 15
   Pro Gln His His Pro Ser Asn Asp Tyr Val Met Met Asp Ala Asp Val
   20 25 30
   Met Pro Tyr Asn Ala Arg Pro Met Thr Thr Ala Pro Met Gln Gln Arg
   35 40 45
   Pro Ser Leu Val Pro His Tyr Met Pro Thr Thr Ser Met Ser Thr Ala
       50                  55                  60
   Ser Ile Asn Ser Ile Ser Ala Pro Ala His Tyr Gln Ser Pro Val Pro
   65                  70                  75                  80
   Tyr Gly Gly Gly Tyr Pro Ala Tyr Pro Leu Pro Thr Pro Thr Thr Met
                   85                  90                  95
   Asn Ser Ser Tyr His Lys His Gln Gln Tyr Gln Glu Arg Pro Ser Leu
               100                 105                 110
   His Met Ile Thr Pro Glu Phe Glu Asp Thr Arg Gly Pro His His Ile
           115                 120                 125
   Arg Asn Ala Arg Arg Tyr Ser Glu Glu Ser Arg Ser Pro Ser Glu Arg
       130                 135                 140
   Ser Asp Ser Gln Gly Ser Thr Thr Glu Thr Thr Ile Ser Asn His Ser
   145                 150                 155                 160
   Ser Cys Ser Arg Thr Ile Thr Pro Asn Thr Pro Val Asn Gly Ala Pro
                   165                 170                 175
   Gln Val Glu Phe Ser Thr Ala Val Asp Lys Leu Met Lys Val Ile Gln
               180                 185                 190
   Ser Lys Met Lys Asp Ala Asp Pro Glu Gln Ser Gly Asp Asp Lys Asp
           195                 200                 205
   Ile Lys Ala Glu Gln Gln Ser Ser Pro Val Cys Gln Ala Arg Val Gln
       210                 215                 220
   Gln Pro Ala Asp Lys His Lys Arg Lys Arg Tyr Glu Cys Gln Ile Glu
   225                 230                 235                 240
   Gly Cys Asn Lys Lys Phe Ser Gln Lys Thr His Arg Asp Thr His Val
                   245                 250                 255
   Arg Ser His Thr Gly Asp Arg Pro Tyr Val Cys Pro Ile Pro Gly Cys
               260                 265                 270
   Gly Gly Arg Phe Thr Gln Ala Gly Asn Leu Lys Thr His Lys Arg Arg
           275                 280                 285
   His Thr Gly Glu Arg Pro Tyr Arg Cys Glu Val Cys Asp Lys Gly Phe
       290                 295                 300
   Val Gln Arg Gly Asp Val Lys Ala His Met Lys Thr His Leu Gly Thr
   305                 310                 315                 320
   Lys Ala Phe Leu Cys Arg Leu Asp Asn Cys His Lys Gln Phe Thr Gln
                   325                 330                 335
   Arg Gly Asn Leu Lys Tyr His Gln Asn Lys Tyr His Asn Glu Thr Ile
               340                 345                 350
   Lys Ala Leu Ala Ala Arg Phe Asp Ala Ile Glu Asp Trp Ser Thr Val
           355                 360                 365
   Leu Lys Glu Asp Met Glu Ile Phe Lys Asp Phe Ala Glu Val His Lys
       370                 375                 380
   Asn Ser Asn Lys Gly Ile Lys Gly Arg Gly Lys His Arg Lys Val Lys
   385                 390                 395                 400
   Ser Val Pro Leu Ser Ser Pro Thr Ser Pro Thr Gly His Ser Pro Leu
                   405                 410                 415
   Pro Asn Ile Met Ala Ser Gln Tyr Pro Leu Pro Ser Ala Pro Gly Val
               420                 425                 430
   Ser Gln Leu Leu His Thr Pro His Pro Leu His Pro Pro Leu His His
           435                 440                 445
   Gln Gly Pro Ser His Ser Ala Met Tyr Gly Met Pro Arg Ser Ser Leu
       450                 455                 460
   His Ser His Tyr Glu Thr Tyr Asp His His Glu Val Asp Thr Val Ala
   465                 470                 475                 480
   Ser Ser Arg Gly Ser Val Ala Glu Pro Ile Tyr His His Glu Glu His
                   485                 490                 495
   Pro Arg Glu Leu Ala Phe Gly Asp Arg Met Tyr
               500                 505
<210> 98
   <211> 308
   <212> PRT
   <213> Artificial Sequence
<220>
   <223> trive6461
<400> 98
   Met Asp Ile Thr Thr Ile Leu Asn Lys Lys Ala Ser Val Ser Ile Val
   1               5                   10                  15
   Ala Ala Asp Val His Gln Leu Gln Gln His Leu Ala Gln Ala Thr Ala
               20                  25                  30
   Gln Ser Gln Thr Asp Asp Ala Val Lys Ser Arg Ser Pro Ser Glu Leu
           35                  40                  45
   Gly Ala Ser Glu His His Arg Ser Ala Ser Ala Pro Pro Ser Glu His
       50                  55                  60
   His His His His His His His Gln Pro Thr Ser Ser Phe Pro Ser Ser
   65                  70                  75                  80
   Thr Gln Ser Leu Pro Gln Met Ala His Leu Ala Gln Tyr His Val Gln
                   85                  90                  95
   Ala Gln Ala Asn His Ala Ser Thr Ser Pro Gly Tyr Ala His Ser Ala
               100                 105                 110
   His Gly Ser Asp Tyr Gly Arg Ser Thr Thr Ser Met Arg Pro Thr Gly
           115                 120                 125
   Leu Pro Ala Leu Lys Thr Phe His Cys Gln Thr Cys Ser Lys Gly Phe
       130                 135                 140
   Ala Arg Arg Ser Asp Leu Ala Arg His Glu Arg Ile His Ser Gly Ile
   145                 150                 155                 160
   Arg Pro His Ala Cys Asp Trp Pro Gly Cys Gly Lys Gln Phe Ile Gln
                   165                 170                 175
   Arg Ser Ala Leu Thr Val His Thr Arg Val His Thr Gly Glu Lys Pro
               180                 185                 190
   His Met Cys Asp Arg Cys Gly Lys Pro Phe Ser Asp Ser Ser Ser Leu
           195                 200                 205
   Ala Arg His Arg Arg Ile His Ser Gly Lys Arg Pro Tyr Lys Cys Pro
       210                 215                 220
   Tyr Ala Asn Cys Gln Lys Thr Phe Thr Arg Arg Thr Thr Leu Thr Arg
   225                 230                 235                 240
   His Gln Asn His His Thr Gly Thr Ile Glu Glu Ala Ala Ala Glu Thr
                   245                 250                 255
   Glu Ala Asn Leu Arg His Ser Lys Asp Arg Ser Thr Thr Ser Arg Pro
               260                 265                 270
   Ser Glu Tyr Ser Glu Pro Gly Ser Ala His Ser Thr Pro Ser Pro Ala
           275                 280                 285
   Gln Arg Pro Ser Leu Ser Pro Gly Asn Glu Leu Pro Pro Leu Asn Leu
       290                 295                 300
   Ser Ser Arg Pro
   305
<210> 99
   <211> 430
   <212> PRT
   <213> Artificial Sequence
<220>
   <223> aspflAFL2T_01868
<400> 99
   Met Val Ala Ala Glu Phe Asp His Gln Gln Phe Ile Gln Asn Pro His
   1               5                   10                  15
   Leu Asp Thr Ser Ser Pro Lys Met Lys Pro Glu Pro Gly Val Ser Glu
               20                  25                  30
   Ala Gly Asp Gln Pro Val Leu Ala Tyr Pro Pro His Ala Pro Leu Gly
           35                  40                  45
   Gln Val Pro Asn Met His Pro Asp Met Arg Tyr Gln Pro Gln Thr His
       50                  55                  60
   Pro Asn Pro Ala Leu Pro Leu Leu Gln Asn Pro Tyr Met Pro Gly Gly
   65                  70                  75                  80
   Tyr Thr Ser Ala Pro Pro Met Pro Asn Gly Gly Ala Pro Gln Gly Arg
                   85                  90                  95
   Thr Asp Pro Pro Pro Lys Thr Phe His Cys Gly Thr Cys Ser Lys Gly
               100                 105                 110
   Phe Ala Arg Arg Ser Asp Leu Ala Arg His Glu Arg Ile His Ser Gly
           115                 120                 125
   Ile Arg Pro His Ala Cys Asp Trp Pro Gly Cys Gly Lys Gln Phe Ile
       130                 135                 140
   Gln Arg Ser Ala Leu Thr Val His Ser Arg Val His Thr Gly Glu Lys
   145                 150                 155                 160
   Pro His Met Cys Glu Arg Cys Gly Lys Pro Phe Ser Asp Ser Ser Ser
                   165                 170                 175
   Leu Ala Arg His Arg Arg Ile His Ser Gly Lys Arg Pro Tyr Lys Cys
               180                 185                 190
   Pro Tyr Ala Asn Cys Gln Lys Thr Phe Thr Arg Arg Thr Thr Leu Thr
           195                 200                 205
   Arg His Gln Asn His His Thr Gly Thr Ile Glu Glu Ala Ala Ala Glu
       210                 215                 220
   Thr Glu Ala Asn Leu Arg Gln Asn Lys Glu Arg Val Arg Ala Pro Gly
   225                 230                 235                 240
   Glu Gly Met Tyr Ser Glu His Gly Ser Val His Ser Thr Pro Ser Pro
                   245                 250                 255
   Ala His His Pro Ala Leu Ser Pro Ala Gly Glu Leu Pro Pro Leu Asn
               260                 265                 270
   Met Pro Arg Ser Ser Gly Glu Tyr Tyr Ser Met Gly Asn Gly Ser Ile
           275                 280                 285
   Pro Pro His Val Arg Gly Asp Phe Pro Gln Ala Ser Pro Arg Ala Ser
       290                 295                 300
   Pro Thr Ala Thr Ser Pro Ser Leu Ser Ser Phe Gly Ser Ala Pro His
   305                 310                 315                 320
   Thr Arg Pro Ser Met Thr Ser His Pro Ser Gly Tyr Gly Pro Pro Gln
                   325                 330                 335
   Pro Leu Glu Pro Pro Ala Asn Asn Asp His Arg Pro Asn Ser Val Ser
               340                 345                 350
   Gly Ser Pro His Met Thr Ser Leu Gly Trp Ala Ser Pro Ser His Gly
           355                 360                 365
   Ser Ile Pro Ser Pro Gly Ser Ala Thr Asp Phe Gly Tyr Ser Asp Pro
       370                 375                 380
   Asn Gly Pro Ala Tyr Pro Asn Ser Met Pro Pro His Met Tyr Phe Pro
   385                 390                 395                 400
   Asn Ser Thr Ile Arg Arg Pro Thr Ser Thr Glu Pro Glu Asn Tyr Glu
   405 410 415
   Met Lys Pro Lys Ile Gly Glu Ser Ala Trp Ser Thr Pro Val
   420 425 430
<210> 100
   <211> 540
   <212> PRT
   <213> Artificial Sequence
<220>
   <223> neudiestExt_Genewise1Plus.C_12168
<400> 100
   Met Cys Arg Ala Arg Leu Leu Ser Arg Val Pro Pro Gly Trp Arg Glu
   1 5 10 15
   Val Pro Ser Pro Phe Pro Ile Leu Pro Pro Ile Asp Arg Tyr Ser Thr
   20 25 30
   Asp Arg Met Ala Leu Thr Ala Gln Pro Ser Ala Pro Ala Gly Trp Glu
   35 40 45
   Arg Trp Pro Gln His His Pro Asn Asn Asp Tyr Val Met Met Asp Ala
   50 55 60
   Asp Val Met Pro Tyr Asn Ala Arg Pro Met Thr Thr Ala Pro Met Gln
   65 70 75 80
   Gln Arg Pro Ser Leu Val Pro His Tyr Met Pro Thr Thr Ser Met Ser
   85 90 95
   Thr Ala Ser Ile Asn Ser Ile Ser Ala Pro Ala His Tyr Gln Ser Pro
   100 105 110
   Met Ser Tyr Gly Gly Gly Tyr Pro Ala Tyr Pro Leu Pro Thr Pro Thr
   115 120 125
   Thr Met Asn Ser Pro Tyr Lys His Gln Gln Tyr Gln Glu Arg Pro Thr
   130 135 140
   Leu His Met Ile Thr Pro Glu Ile Glu Asp Thr Arg Gly Pro His His
   145 150 155 160
   Ile Arg Asn Ala Arg Arg Tyr Ser Ala Glu Ser Arg Ser Pro Ser Glu
   165 170 175
   Arg Ser Asp Ser Gln Val Ser Thr Thr Glu Ile Thr Ile Ser Asn His
               180                 185                 190
   Ser Ser Cys Ser Arg Thr Ile Thr Pro Asn Thr Pro Val Asn Gly Ala
           195                 200                 205
   Pro Gln Val Glu Phe Ser Thr Ala Val Asp Lys Leu Met Lys Val Ile
       210                 215                 220
   Gln Ser Lys Met Lys Asp Ala Asp Pro Glu Gln Ser Gly Asp Asp Arg
   225                 230                 235                 240
   Asp Ile Lys Ala Glu Gln Gln Ser Ser Pro Val Cys Gln Ala Arg Val
                   245                 250                 255
   Gln Gln Pro Ala Asp Lys Leu Lys Arg Lys Arg Tyr Glu Cys Gln Ile
               260                 265                 270
   Glu Gly Cys Asn Lys Lys Phe Ser Gln Lys Thr His Arg Asp Thr His
           275                 280                 285
   Val Arg Ser His Thr Gly Asp Arg Pro Tyr Val Cys Pro Ile Pro Gly
       290                 295                 300
   Cys Gly Gly Arg Phe Thr Gln Ala Gly Asn Leu Lys Thr His Lys Arg
   305                 310                 315                 320
   Arg His Thr Gly Glu Arg Pro Tyr Arg Cys Glu Val Cys Asp Lys Gly
                   325                 330                 335
   Phe Val Gln Arg Gly Asp Val Lys Ala His Met Lys Thr His Leu Gly
               340                 345                 350
   Thr Lys Ala Phe Leu Cys Lys Leu Asp Asn Cys His Lys Gln Phe Thr
           355                 360                 365
   Gln Arg Gly Asn Leu Lys Tyr His Gln Asn Lys Tyr His Asn Glu Thr
       370                 375                 380
   Ile Lys Ala Leu Ala Ala Arg Phe Asp Ala Ile Glu Asp Trp Ser Thr
   385                 390                 395                 400
   Val Leu Lys Glu Asp Met Glu Ile Phe Lys Asp Phe Ala Glu Val His
                   405                 410                 415
   Lys Asn Ser Asn Lys Gly Ile Lys Gly Arg Gly Lys His Arg Lys Val
               420                 425                 430
   Lys Ser Val Pro Leu Ser Ser Pro Thr Ser Pro Thr Gly His Ser Pro
   435 440 445
   Leu Pro Asn Ile Met Ala Ser Gln Tyr Pro Leu Pro Ser Ala Pro Gly
   450 455 460
   Val Ser Gln Leu Leu His Thr Pro His Pro Leu His Pro Pro Leu His
   465 470 475 480
   His Gln Gly Pro Ser His Ser Ala Met Tyr Gly Met Pro Arg Gln Ser
   485 490 495
   Leu His Ser His Tyr Glu Thr Tyr Asp His His Glu Val Asp Thr Val
   500 505 510
   Pro Ser Ser Arg Gly Pro Val Ala Glu Pro Ile Tyr His His Glu Glu
   515 520 525
   His Pro Arg Glu Leu Ala Ser Gly Asp Arg Met Tyr
   530 535 540
<210> 101
   <211> 432
   <212> PRT
   <213> Artificial Sequence
<220>
   <223> artolgi345566590gbEGX49532.1
<400> 101
   Met Glu Ile His Glu Leu Ile Ile Asp Glu Arg His Ala Pro Lys Gln
   1 5 10 15
   Phe Pro Cys Asp Trp Pro Asp Cys Asn Lys Lys Phe Asn Arg Lys Ser
   20 25 30
   Asp Leu Gln Arg His His Arg Ile His Thr Asn Thr Arg Pro Tyr Ala
   35 40 45
   Cys Thr Phe Ala Gly Cys Lys Lys Ser Phe Ile Gln Arg Ser Ala Leu
   50 55 60
   Thr Val His Ile Arg Thr His Thr Gly Glu Lys Pro His Gln Cys Glu
   65 70 75 80
   Phe Ile Gly Cys Gly Lys Cys Phe Ser Asp Ser Ser Ser Leu Ala Arg
   85 90 95
   His Arg Arg Ile His Thr Gly Arg Arg Pro Tyr Leu Cys Gly Val Asn
   100 105 110
   Arg Cys Gly Lys Ser Phe Cys Arg Lys Thr Thr Leu Ile Lys His Ala
   115 120 125
   Arg Arg Ala His Gly Ile Gly Gln Asn Leu Asp Val Ser Asp Asp Asp
   130 135 140
   Ser Glu Asn Asp Asp Ser Thr Thr Thr Glu Arg Ser His Pro Lys Pro
   145 150 155 160
   Val Ser Gln Ala Arg Ala Glu Val Ile Arg Asn His Gly Pro Glu Ser
   165 170 175
   Val Leu Gln Asn Gln Gly Arg His Leu Ser Leu Phe Arg Pro Glu Leu
   180 185 190
   Ser Leu Pro Ser Ser Pro Arg Ser Asn Ser Asn Thr Ile Gly Phe Gly
   195 200 205
   Asp Thr Tyr Gly Ser Ala Ser Ala Val Tyr His Pro Gln Asn Leu Val
   210 215 220
   Ser Asp His Pro Met Thr Pro Thr Ser Pro Tyr Cys Phe Gln Glu Thr
   225 230 235 240
   Pro Pro His Ala His Val Pro Pro Phe Pro Asn Leu Pro Arg Thr Cys
   245 250 255
   Ala Glu Asp Phe Ile Pro Thr Gly Thr Gly Leu Pro Gln His Pro Gln
   260 265 270
   Asn Pro Gln Tyr Asn Pro Asn Leu Arg Ile Ile Cys Ser Ser Ser Pro
   275 280 285
   Thr Gln Gln Ala Leu Ala Ala Gly His Cys Val Gly Ser Pro Gly Glu
   290 295 300
   Leu Ser Asn Cys Ser Thr Ala Thr Ser Gly Ala Ser Val Asp Tyr Phe
   305 310 315 320
   Tyr Arg Asn Pro Ser Thr Val Asp Gly Val Gly Asn Val Thr Leu Phe
   325 330 335
   Met Gln Gln Ser Ala Ala Ser Asp His Ile Ser His Gln Val Gln Asp
   340 345 350
   Gln Gly Ile Ala Glu Met Pro Ile Ala Val Met Ser Gln Gln Gln His
   355 360 365
   Gln His His Pro Gln Gln Pro Gly Ala Ala Ile Trp Tyr Gly Tyr Ser
   370 375 380
   Gln Phe Asp Gln Pro Gln Phe His Ser Gly Ser Glu Gln Arg Leu Phe
   385 390 395 400
   Tyr Glu Asn Gly Asn Met Glu Asn Ile Thr Pro Val Lys Ala Thr Thr
   405 410 415
   Ser Glu Val Val Leu His Pro Leu Leu Thr Pro Arg Ala Ser Leu Cys
   420 425 430
<210> 102
   <211> 457
   <212> PRT
   <213> Artificial Sequence
<220>
   <223> aspniANID_06503T0
<400> 102
   Met Glu Thr Ala Glu Pro Val Ser Tyr Glu Phe Pro Gly His Ala Val
   1 5 10 15
   Gly Ala Val Ala Ser Arg Arg Met Leu Gly Ser Asn Ile Asn His Asn
   20 25 30
   Phe Ser Phe Tyr Thr Asn Pro Thr Ala Ser Phe Pro Leu Pro Tyr His
   35 40 45
   Gln Pro Pro Ser Ala Thr Tyr Ser Phe Ser His Pro Leu Asn Thr His
   50 55 60
   His His His Gln His His Gln Pro Gln Pro Gln His Pro Gln His His
   65 70 75 80
   Asn His His His Gln His Pro Gly Tyr Gln His Phe Phe Val Pro Asn
   85 90 95
   His Ser Leu Asn Ala Gln Ser Ala Arg Leu Ala Ser Glu Pro Pro Leu
   100 105 110
   Ala Gln Pro Ile Pro Asp Ile Arg Pro Ala Arg Asn Ala Val Asn Arg
   115 120 125
   Val Ser Gly Asp Ser Val Ala Lys Val Glu Gln Arg Ala Ala Pro Gln
       130                 135                 140
   Ser Ser Ser Val Ala Ala Ala Pro Gly Asn Gly Val Ser Ala His Glu
   145                 150                 155                 160
   Lys Asn Pro Asn Ala Ser Glu Val Glu Phe Ser Thr Glu Val Asp Val
                   165                 170                 175
   Leu Met Lys Ala Ile Gln Ala Lys Glu Ala Ser Gln Pro Ala Ala Leu
               180                 185                 190
   Gln Ser Leu Pro Pro Leu Gln Gln Leu Thr His Gly Gly Gly Asn Gly
           195                 200                 205
   Phe Ala Gln Gly Tyr Met Ser Ala Val Val Asn His Arg Gly Ala Val
       210                 215                 220
   Ile Ala Asp Glu Ser Gln Ser Arg Ser Ala Lys Lys Arg Lys Tyr Thr
   225                 230                 235                 240
   Cys Thr Leu Pro His Cys Gly Lys Ser Phe Ala Gln Lys Thr His Leu
                   245                 250                 255
   Asp Ile His Met Arg Ala His Thr Gly Asp Lys Pro Phe Val Cys Lys
               260                 265                 270
   Glu Pro Thr Cys Gly Gln Arg Phe Ser Gln Leu Gly Asn Leu Lys Thr
           275                 280                 285
   His Gln Arg Arg His Thr Gly Glu Lys Pro Phe Thr Cys Asp Val Cys
       290                 295                 300
   Gln Lys Arg Phe Ala Gln Arg Gly Asn Val Arg Ala His Lys Ile Thr
   305                 310                 315                 320
   His Leu Lys Thr Lys Pro Phe Thr Cys Leu Leu Glu Gly Cys Gly Lys
                   325                 330                 335
   Gln Phe Thr Gln Leu Gly Asn Leu Lys Ser His Gln Asn Lys Phe His
               340                 345                 350
   Thr Gln Thr Leu Gln Ala Leu Thr Lys Lys Phe Ser Ala Met Ile Asp
           355                 360                 365
   Gly Glu His Met Ser Pro Gln Asp Arg Lys Leu Trp Glu Tyr Phe Ala
       370                 375                 380
   Gly Leu Tyr Lys Asn Ser Asn Lys Gly Ile Lys Gly Arg Gly Lys Asp
   385 390 395 400
   Arg Arg Ile Ser Pro Ser Ser Lys Thr Asn Val Ser Ala Asp Gly Ser
   405 410 415
   Arg Arg Pro Gly Ala Asp Asp Gln Ala Arg Arg Gly Ser Tyr Glu Asp
   420 425 430
   Ser Val Tyr Thr Gly Ala Ser Ser Ser Asp Glu Asp Asn Glu Asp Pro
   435 440 445
   Tyr Phe Ile Glu Lys Arg Gly Asp Glu
   450 455
<210> 103
   <211> 478
   <212> PRT
   <213> Artificial Sequence
<220>
   <223> settugm1.606_g
<400> 103
   Met Glu Leu Asp Thr Ile Leu Asn Ala Arg Leu Thr Ala Ser Leu Gly
   1 5 10 15
   Thr Gly Ala Leu Phe Thr Met Gln His Ala Met Gln Gln Gln His Phe
   20 25 30
   Gly Ala Pro Pro Ser Tyr Leu Asn Gly Gly His Ile Lys Ser Glu Asn
   35 40 45
   Gly Ser Glu Arg Gly Val Ser Pro His Pro Ser Asp Ser Ser Arg Tyr
   50 55 60
   Ser Ser Gln Gln Pro Gln Gln Ser Leu Pro Ser Tyr Pro Ser Ile Pro
   65 70 75 80
   Gln Gln His Met Asn Gly Leu Arg Tyr Pro Ser Pro Ser Gln Met Gln
   85 90 95
   Ala Pro Met Pro Met Leu Asn Asn Ser Asn Tyr Ile Pro Asn Pro Pro
   100 105 110
   Asp His Ala Tyr Ala Gln Gln Gln Gln Met Pro Asp Thr Gln Val Gln
   115 120 125
   His Gln His Asn Gly Gly Arg Pro Ala Ser Asp Thr Gly Pro Pro Lys
   130 135 140
   Ala Phe Ala Cys Ser Thr Cys Gly Lys Gly Phe Ala Arg Arg Ser Asp
   145 150 155 160
   Leu Ala Arg His Glu Arg Ile His Ser Gly Ile Arg Pro His Val Cys
   165 170 175
   Asp Tyr Pro Asn Cys Gly Lys Gln Phe Ile Gln Arg Ser Ala Leu Thr
   180 185 190
   Val His Gln Arg Val His Thr Gly Glu Lys Pro His Met Cys Glu Arg
   195 200 205
   Cys Gly Lys Pro Phe Ser Asp Ser Ser Ser Leu Ala Arg His Arg Arg
   210 215 220
   Ile His Ser Gly Lys Arg Pro Tyr Lys Cys Pro Tyr Ala Asp Cys Gln
   225 230 235 240
   Lys Thr Phe Thr Arg Arg Thr Thr Leu Thr Arg His Gln Asn His His
   245 250 255
   Thr Gly Thr Val Glu Glu Ala Ala Ala Ala Thr Ala Ala Ala Leu Ala
   260 265 270
   Ser Arg Ala Ser Ala Ser Ser Arg Thr Gly Arg Ser Asp Gly Gly Asp
   275 280 285
   Tyr Ser Glu Thr Ala Ser Pro Leu Gly Thr Pro Ser Pro Asn Asp Arg
   290 295 300
   Thr Leu Ser Leu Ser Pro Ala Asn Gly Met Pro Ala Gly Gly Met Pro
   305 310 315 320
   Gly Leu His Arg Gln Gly Ser Asp Tyr Ala Tyr Met Gly Gly Met Asn
   325 330 335
   Val Pro Pro His Met Arg His Glu Met Pro Gln Pro Ser Pro Arg Ala
   340 345 350
   Ser Pro Ala Leu Thr Ala Gln Ser Tyr Ala Ser Asn Val Ser Gly Thr
   355 360 365
   Arg Pro Ala Ile Thr Ser His Pro Ser Ala Tyr Gly Pro Pro Pro Ile
   370 375 380
   Leu Glu Pro Pro Ala Ser Ala Asn His Asn Gln Ser Gly Thr Asn Ser
   385 390 395 400
   Ala Asn Ala Ser Pro His Met Ser Ala Met Gly Trp Gln Ser Pro Gly
   405 410 415
   Gln Gln Ala Leu Pro Ser Pro Gly Ala Ala Asp Asn Gly Tyr Val Tyr
   420 425 430
   Pro Glu Pro Gln Tyr Gln Ala Gln Asn Gly Met Tyr Tyr Gln His Asn
   435 440 445
   Asn Ile Arg Arg Pro Asn Ser Thr Glu Pro Asp His Tyr Asn Pro Asn
   450 455 460
   Gln Gln Arg Met Gly Asn Glu Met Trp Ala Pro Ala Val Gln
   465 470 475
<210> 104
   <211> 369
   <212> PRT
   <213> Artificial Sequence
<220>
   <223> clagrfgenesh1_kg.91_#_11_#_isotig08397
<400> 104
   Met Asn Arg Ala Asp Ile Ala Met His Thr Tyr Ala His His Ser His
   1 5 10 15
   Val Ala His Gly Ala Ile Pro Ala Ser Asn Phe Glu Leu Thr Ser Ser
   20 25 30
   Phe Phe Ala Asn Pro Ala Phe Thr Tyr Pro Ala Gln Tyr Asn Ser Ile
   35 40 45
   Asp Leu Gly Tyr Asp Leu Gly Leu Gly Tyr Gly Gly Cys Asn Leu Gly
   50 55 60
   His Gly Ala Asn Ala Ser Pro Gln Gln Ile Phe Gly Gln His Ser Ala
   65 70 75 80
   Ser Tyr Ser Glu Gln Gly Leu His Val Pro Ala Ser Gln Gln Pro Ala
   85 90 95
   Pro Gly Glu Pro Ala Gln Arg Ser Pro Ala Ile Lys Val Glu Ala Ala
   100 105 110
   Ser Gln Asp Ala Asp Asn Val Glu Phe Gln Pro Tyr Ser Val Pro Thr
   115 120 125
   Ser Thr Leu Pro Thr Ser Ala Thr Asp Ala Asp Val Gly Thr Asp Val
   130 135 140
   Asp Thr Leu Met Arg Ala Ile Gln Thr Lys Ala Lys Thr Ser Ser Thr
   145 150 155 160
   Gln Ala Pro Ser Ser Ala Ala Leu Ile Asp Gln Arg Arg Ser Lys Ala
   165 170 175
   Arg Arg Lys Tyr Pro Cys Lys Val Gln Ser Cys Ala Lys Val Phe Thr
   180 185 190
   Gln Lys Thr His Leu Glu Ile His Met Arg Ala His Thr Gly Tyr Lys
   195 200 205
   Pro Tyr Leu Cys Arg Glu Thr Ser Cys Gly Gln Arg Phe Ser Gln Leu
   210 215 220
   Gly Asn Leu Lys Thr His Glu Arg Arg His Thr Gly Glu Arg Pro Tyr
   225 230 235 240
   Ser Cys Asp Arg Cys Gly Arg Ser Phe Ala Gln Arg Gly Asn Val Gln
   245 250 255
   Ala His Arg Ile Val His Glu Arg Ile Lys Pro Tyr Cys Cys Gln Leu
   260 265 270
   Glu Gly Cys Ser Lys Arg Phe Thr Gln Leu Gly Asn Leu Lys Ser His
   275 280 285
   Gln Asn Lys Phe His Thr Glu Thr Leu Arg Val Leu Ala Val Lys Phe
   290 295 300
   Ala Ser Leu Arg Glu Glu Asp Ala Met Ser Glu Ala Asp Arg Glu Leu
   305 310 315 320
   Trp Glu Tyr Phe Ser Thr Leu Tyr Lys Asn Ser Asn Lys Gly Ile Lys
   325 330 335
   Gly Arg Gly Lys Asp Arg Arg Ile Ala Ala Val Asp Arg Asn Gly Asp
   340 345 350
   Gly Leu Lys Gln Glu Thr Gln Asp Cys Glu Ser Ser Ser Ser Val Ser
   355 360 365
   Pro
<210> 105
   <211> 437
   <212> PRT
   <213> Artificial Sequence
<220>
   <223> triatestExt_fgenesh1_pg.C_contig_250603
<400> 105
   Met Ser Ser Ala Ser Pro Glu Val Met Asp Ile Thr Asn Met Leu Asn
   1               5                   10                  15
   Asn Lys Gly Gly Ala Met Ala His Gly Leu Gly Gly Ser Met Pro Asp
               20                  25                  30
   His His Gln Gln His His His Leu Gly Leu Val Lys His Glu Ser Ala
           35                  40                  45
   Leu Asp Arg Ser Gly Ser Pro His Met Ser Glu His Ser Ser Tyr Ser
       50                  55                  60
   Ala His Ser Met Pro Arg Ala Tyr Pro Ser Pro Thr Ala Met Gln Thr
   65                  70                  75                  80
   Pro Met Gln Met Gln Asn His Met His Asn Ala Met Gln Met Gly Gly
                   85                  90                  95
   Phe Thr Asp Met Ser Gly Met Gly Ser Val Pro Ser Met Ala Met His
               100                 105                 110
   His Met Pro Gln Gln Pro Gln Gln Gln Gln Ala Pro Thr Ala Pro Val
           115                 120                 125
   Lys Ala Tyr Pro Cys Ser Thr Cys Gly Lys Gly Phe Ala Arg Arg Ser
       130                 135                 140
   Asp Leu Ala Arg His Glu Arg Ile His Thr Gly Val Arg Pro His Val
   145                 150                 155                 160
   Cys Asp Tyr Pro Lys Cys His Lys Gly Phe Ile Gln Arg Ser Ala Leu
                   165                 170                 175
   Thr Val His Gln Arg Val His Thr Gly Glu Lys Pro His Arg Cys Glu
               180                 185                 190
   Thr Cys Ala Lys Leu Phe Ser Asp Ser Ser Ser Leu Ala Arg His Arg
   195 200 205
   Arg Thr His Ser Gly Thr Arg Pro Tyr Lys Cys Pro Tyr Ala Asp Cys
   210 215 220
   Gln Lys Thr Phe Thr Arg Arg Thr Thr Leu Thr Arg His Gln Asn Gln
   225 230 235 240
   His Thr Gly Thr Ile Glu Glu Ala Ala Ala Ala Thr Ala Ala Ala Leu
   245 250 255
   Ala Ala Ser Arg Val Lys Asn Gly Ser Gln Ala Arg Ser Asp Gly Asp
   260 265 270
   His Met Ser Asn Gln Gly Ser Pro Leu Thr Thr Pro Ser Pro Asn His
   275 280 285
   His Ala Met Met Ser Pro Ala Met Asp Leu Ser Gly Ala Asn Gly Leu
   290 295 300
   Gly Arg His Gln Ala Asp Phe Thr Gln Tyr Met Thr Gln Gly Gly Thr
   305 310 315 320
   Leu Pro Pro His Leu Arg Val Gly Ser Pro Thr Ser Thr Asn Ser Ala
   325 330 335
   Ala Ser Tyr Asn Ser Gly Ile Arg Pro Thr Ser His Pro Thr Gly Tyr
   340 345 350
   Gly Pro Pro Pro Thr Leu Glu Pro Ser Leu Glu Gln His Thr Gly Ala
   355 360 365
   Thr Gly Ser Ala Val Gly Ser Pro His Met Ala Ser Gln Leu Gly Trp
   370 375 380
   Gln Ser Pro His Ser Ser Ser Pro Ser Gln His Gly Gly Ser Tyr Val
   385 390 395 400
   Tyr Pro Asp Pro Asp Gly Tyr Pro Pro Asn Pro Ser Met Ser Gln Met
   405 410 415
   Tyr Tyr Gly Ala Pro Gln Gln Met Arg Arg Pro Gln Ser Ala Glu Pro
   420 425 430
   Gly Met Val His Met
   435
<210> 106
   <211> 453
   <212> PRT
   <213> Artificial Sequence
<220>
   <223> cocsae_gw1.2.1546.1
<400> 106
   Met Gln Gln Gln His Phe Gly Ala Pro Pro Ser Tyr Leu Asn Gly Gly
   1               5                   10                  15
   His Ile Lys Ser Glu Asn Gly Ser Glu Arg Gly Val Ser Pro His Pro
               20                  25                  30
   Ser Asp Ser Ser Arg Tyr Ser Ser Gln Gln Pro Gln Gln Gln Leu Pro
           35                  40                  45
   Ser Tyr Pro Ser Ile Pro Gln Gln His Met Asn Gly Leu Arg Tyr Pro
       50                  55                  60
   Ser Pro Ser Gln Met Gln Ala Pro Met Pro Met Leu Asn Asn Ser Asn
   65                  70                  75                  80
   Tyr Ile Pro Asn Pro Pro Asp His Ala Tyr Ala Gln Gln Gln Gln Met
                   85                  90                  95
   Pro Asp Asn Gln Val Gln His Gln His Asn Gly Gly Arg Pro Ala Ser
               100                 105                 110
   Asp Thr Gly Pro Pro Lys Ala Phe Ala Cys Ser Thr Cys Gly Lys Gly
           115                 120                 125
   Phe Ala Arg Arg Ser Asp Leu Ala Arg His Glu Arg Ile His Ser Gly
       130                 135                 140
   Ile Arg Pro His Val Cys Asp Tyr Pro Asn Cys Gly Lys Gln Phe Ile
   145                 150                 155                 160
   Gln Arg Ser Ala Leu Thr Val His Gln Arg Val His Thr Gly Glu Lys
                   165                 170                 175
   Pro His Met Cys Glu Arg Cys Gly Lys Pro Phe Ser Asp Ser Ser Ser
               180                 185                 190
   Leu Ala Arg His Arg Arg Ile His Ser Gly Lys Arg Pro Tyr Lys Cys
           195                 200                 205
   Pro Tyr Ala Asp Cys Gln Lys Thr Phe Thr Arg Arg Thr Thr Leu Thr
       210                 215                 220
   Arg His Gln Asn His His Thr Gly Thr Val Glu Glu Ala Ala Ala Ala
   225                 230                 235                 240
   Thr Ala Ala Ala Leu Ala Ser Arg Ala Ser Ala Ser Ser Arg Thr Gly
                   245                 250                 255
   Arg Ser Asp Gly Gly Asp Tyr Ser Glu Thr Ala Ser Pro Leu Gly Thr
               260                 265                 270
   Pro Ser Pro Asn Asp Arg Thr Leu Ser Leu Ser Pro Ala Asn Gly Met
           275                 280                 285
   Pro Ala Gly Gly Met Pro Gly Leu His Arg Gln Gly Ser Asp Tyr Ala
       290                 295                 300
   Tyr Met Gly Gly Ile Asn Val Pro Pro His Met Arg His Glu Met Pro
   305                 310                 315                 320
   Gln Pro Ser Pro Arg Ala Ser Pro Ala Leu Thr Ala Gln Ser Tyr Ala
                   325                 330                 335
   Ser Asn Val Ser Gly Ser Arg Pro Ala Ile Thr Ser His Pro Ser Ala
               340                 345                 350
   Tyr Gly Pro Pro Pro Ile Leu Glu Pro Pro Ala Ser Ala Asn His Asn
           355                 360                 365
   Gln Ser Gly Gly Thr Asn Ser Ala Asn Gly Ser Pro His Met Ser Ala
       370                 375                 380
   Met Gly Trp Gln Ser Pro Gly Gln Gln Ala Leu Pro Ser Pro Gly Ala
   385                 390                 395                 400
   Gly Asp Asn Gly Tyr Val Tyr Pro Glu Pro Gln Tyr Gln Ala Gln Asn
                   405                 410                 415
   Gly Met Tyr Tyr Gln His Asn Asn Ile Arg Arg Pro Asn Ser Thr Glu
               420                 425                 430
   Pro Asp His Tyr Asn Pro Asn Gln Gln Arg Met Gly Asn Glu Met Trp
           435                 440                 445
   Ala Pro Ala Val Gln
       450
<210> 107
   <211> 518
   <212> PRT
   <213> Artificial Sequence
<220>
   <223> thitefgenesh_kz1_kg.chromosome_1_#_2591_#_18270
<400> 107
   Met Ala Leu Gly Phe Leu Pro Pro Asn Pro Glu Thr Trp Gly Arg Trp
   1               5                   10                  15
   Pro Gln His Gln His Thr Gly Ala Asp Cys Gly Met Met Asp Ala Gly
               20                  25                  30
   Met Val Pro Tyr Asp Cys Arg Pro Gly Ala Thr Ala Ser Ile Pro Arg
           35                  40                  45
   Pro Glu Leu Ala Gln His Phe Leu Pro Gly Pro Phe Asn Pro Ala Ser
       50                  55                  60
   Val Pro Ser Pro Ala Ser Pro Gln Tyr His Ala Pro Val Ser Tyr Gly
   65                  70                  75                  80
   Gly Tyr Ser Pro Tyr Asn Pro Ser Ser Met Leu Glu Ala Pro Phe Lys
                   85                  90                  95
   Pro His Gly Leu Gln Gly Arg Met Asp Gly Leu Pro Leu Val Gly Arg
               100                 105                 110
   Ser Ser Ser Pro Ser Val Lys Ser Glu Ala Lys Ser Thr Lys Ser Glu
           115                 120                 125
   Ser Pro Ser Pro Ser Thr Lys Val Val Ile Pro Asn Val Arg Val Gln
       130                 135                 140
   Gly Ala Pro Val His Glu Phe Asn Thr Ala Ile Asp Arg Leu Ala Lys
   145                 150                 155                 160
   Val Ile Glu Thr Lys Arg Glu Ile Leu Ser Ser Pro Asn Pro Ala Lys
                   165                 170                 175
   Glu Glu Pro Gly Gln Pro Asn Glu Gly Val Glu Gly Gly Glu Met Gln
               180                 185                 190
   Gln Ala Arg Gly Lys Ala Lys Arg Lys Arg Phe Cys Cys Asp Ile Pro
           195                 200                 205
   Gly Cys Ser Lys Met Phe Ala Gln Lys Asn Asn Leu Asp Thr His Arg
       210                 215                 220
   Arg Ala His Thr Gly Glu Ser Pro Tyr Val Cys Pro Ile Cys Leu His
   225                 230                 235                 240
   Arg Phe Thr Gln Ser Val Asn Leu Lys Ser His Ile Arg Arg His Leu
                   245                 250                 255
   Gly Glu Arg Pro Tyr Lys Cys Pro Gln Cys Pro Lys Ala Phe Ser Gln
               260                 265                 270
   Pro Ser Asn Val Lys Ala His Met Lys Thr His Glu Arg Arg Glu Leu
           275                 280                 285
   Arg Ala Arg Trp Val Cys Arg Phe Gly Ser Cys Arg Lys Ser Phe Thr
       290                 295                 300
   Ala Lys Gly Asn Leu Lys Ser His Gln Asn Thr Tyr His Val Glu Ala
   305                 310                 315                 320
   Ile Glu Ala Phe His Ala Lys Leu Ala Ser Ile Glu Asp Lys Ser Met
                   325                 330                 335
   Ile Thr Glu Glu Asp Lys Glu Met Ala Arg Tyr Ile Thr Glu Val His
               340                 345                 350
   Asn Leu Ala Asn Lys Gly Ile Lys Gly Arg Gly Lys Gly Arg Lys Val
           355                 360                 365
   Lys Arg Leu Leu Pro Leu Pro Met Gln Pro Ser Ser Ala Ala Ala Ala
       370                 375                 380
   Ala Thr Thr Thr Thr Thr Thr Thr Ser Leu Ser Ala Ala Thr Ala Ile
   385                 390                 395                 400
   Val His Gly Asn Pro Tyr Ser Met Pro Pro Leu Leu Pro His Ala Pro
                   405                 410                 415
   Pro His His Leu Gln Ala Ala Pro Gln Pro Ser Ala Ala His His Gly
               420                 425                 430
   His Gln His His His Phe Tyr Gly Leu Ser Asn Pro Ala Ala Tyr Ser
           435                 440                 445
   Met Ser Arg Pro Ser Val Ser Ser Ala Met Leu Phe Gly Val Gly Leu
       450                 455                 460
   Gly Ile Asn Thr Arg Asp Val Val His Gly His Gly Ala Tyr Gly Met
   465 470 475 480
   Leu Asp Ser Asp Gln Ile Ser Glu Ala Ser Ser Val His Ser Pro Pro
   485 490 495
   Pro Val Met His His Val Tyr Asp Asp Glu His Ala Arg Glu Leu Ala
   500 505 510
   Phe Gly Glu Arg Leu Tyr
   515
<210> 108
   <211> 414
   <212> PRT
   <213> Artificial Sequence
<220>
   <223> uncreURET_00595
<400> 108
   Met Asp Ile Thr Thr Ile Leu Asn Lys Asn Arg Ser Ala Val Ala Ala
   1 5 10 15
   Ala Ala Glu Ala Gln Leu Gln His Gln Leu Ser Gln Thr Ser His Met
   20 25 30
   Lys Ser Arg Ser Pro Ser Glu Met Gly Ser Glu His Asp Pro Gln His
   35 40 45
   Pro Gly Arg His Thr Glu Ser Tyr Gln Gln Val Pro Gln Pro Ile Gln
   50 55 60
   Leu Pro Asn Ile Ser Gln Tyr His Ser Pro Ile His Ala Asn His His
   65 70 75 80
   Pro Ser Ile Leu Arg Gly Asp Tyr Ser Gln Asn Gly His Glu Thr Met
   85 90 95
   Phe Arg Asn Val Pro Gly Thr Ser Ser Pro Thr Ser Arg Thr Asn Gly
   100 105 110
   Glu Pro Ala Pro Lys Ser Phe His Cys Gln Thr Cys Gly Lys Gly Phe
   115 120 125
   Ala Arg Arg Ser Asp Leu Ala Arg His Glu Arg Ile His Ser Gly Ile
   130 135 140
   Arg Pro His Val Cys Asp Trp Pro Gly Cys Gly Lys Gln Phe Ile Gln
   145                 150                 155                 160
   Arg Ser Ala Leu Thr Val His Thr Arg Val His Thr Gly Glu Lys Pro
                   165                 170                 175
   His Met Cys Asp Arg Cys Gly Lys Lys Thr Phe Thr Arg Arg Thr Thr
               180                 185                 190
   Leu Thr Arg His Gln Asn His His Thr Gly Thr Ile Glu Glu Ala Ala
           195                 200                 205
   Ala Glu Thr Glu Ala Asn Leu Arg Gln Gly Lys Val Val Arg Pro Gly
       210                 215                 220
   Asp Gly Val Tyr Ser Glu Thr Ala Ser Thr His Gly Thr Ala Ser Pro
   225                 230                 235                 240
   Ala Gln Arg Ser Ser Pro Ser Pro Arg His Glu Leu Pro Pro Leu His
                   245                 250                 255
   Ala His Arg Gln Met Gly Asp Tyr Phe Met Gly Asn Ser Ser Leu Pro
               260                 265                 270
   Pro His Leu Arg Gly Asp Phe Gln Gln Ala Ser Pro Arg Ala Ser Pro
           275                 280                 285
   Ser Ala Pro Ser Pro Thr Leu Ser Thr Phe Ser Thr Ser His Pro Ala
       290                 295                 300
   Arg Pro Ser Leu Thr Ser His Pro Thr Gly Tyr Gly Pro Pro Gln Pro
   305                 310                 315                 320
   Leu Glu Pro Pro Pro Asn Gly Gly Ser Arg Pro Gly Ser Val Thr Gly
                   325                 330                 335
   Ser Pro His Met Thr Thr Val Gly Trp Ala Ser Pro Ser Leu Asn Ser
               340                 345                 350
   Ile Pro Ser Pro Gly Ser Ile Ser Ala Pro Glu Tyr Thr Tyr Pro Glu
           355                 360                 365
   Pro Ser Gly Pro Pro Phe Ala Gly Gly Val Pro Ala His Met Tyr Tyr
       370                 375                 380
   Pro Asn Ser Thr Ile Arg Arg Pro Gln Ser Thr Glu Pro Glu Asn Tyr
   385                 390                 395                 400
   Glu Leu Lys Pro Lys Met Glu Glu Asn Trp Thr Ala His Ser
   405 410
<210> 109
   <211> 553
   <212> PRT
   <213> Artificial Sequence
<220>
   <223> gaegrGGTG_07991T0
<400> 109
   Met Gln Leu Pro Thr Pro Pro Gly Trp Gly Gln Trp Gln Gln Asn Glu
   1 5 10 15
   Val Glu Ser Gly Tyr Val His Ile Gly Ser Pro Met Met Gly Thr Gln
   20 25 30
   Thr Pro Leu Gln Gln Pro Thr Leu Ser Ser Gln Tyr Pro Val Thr Leu
   35 40 45
   Ala Pro Ser Pro Ser Leu Gln Gln Pro Ile Ser Tyr Ala Pro Tyr Thr
   50 55 60
   His Gly Leu Pro Ser Val Ala Pro Leu Gln Met Pro Thr Val Gly Ser
   65 70 75 80
   Gln His Asn His Gln His Gln His Gln His His His Ser Gln His His
   85 90 95
   His Gln Gln His Gln Ile Gln Phe Pro Asp His Ser Ile Gln Arg Pro
   100 105 110
   Arg Arg Lys Leu Val Asn Thr Arg Ser Leu Arg Asp Thr Thr Ala Glu
   115 120 125
   Ala Met Glu Ala Gly His Arg Gly Arg Thr Thr Pro Glu Arg Ala Thr
   130 135 140
   Arg Arg Val Pro Lys Thr Ile Val Ala Asn Ala Pro Val Val Gly Ala
   145 150 155 160
   Ser His Val Gly Asn His Val Glu Phe Asn Thr Ser Val Asp Lys Leu
   165 170 175
   Met Lys Ile Ile Gln Ser Met Pro Glu Thr Gly Thr Leu Val Glu Ser
   180 185 190
   Ile Glu Ser Val Val Lys Pro Glu Pro Ala Val Ser Pro Ile Asp Glu
   195 200 205
   Gln Lys Pro Ala Ser Glu Lys Leu Thr Val Arg Arg Phe Val Cys His
   210 215 220
   Phe Arg Gly Cys Leu Lys Lys Phe Ala Gln Lys Ala Gln Leu Asp Thr
   225 230 235 240
   His Val Arg Ser His Thr Gly Glu Arg Pro Tyr Val Cys Glu Phe Pro
   245 250 255
   Asn Cys Gly Lys Arg Phe Ser Gln Ser Gly Asn Leu His Thr His Glu
   260 265 270
   Arg Lys His Thr Gly Glu Arg Pro Tyr Val Cys Asp Ile Cys Gly Arg
   275 280 285
   Arg Phe Ala Gln Gly Gly Asn Arg Gln Ala His Lys Lys Val His Gln
   290 295 300
   Lys Thr Lys Asp Phe Ile Cys Arg Leu Glu Gly Cys Gly Lys Glu Phe
   305 310 315 320
   Thr Gln Arg Gly Asn Leu Lys Ser His Gln Asn Lys Ser His Gln Gln
   325 330 335
   Phe Met Asp Gln Thr Met Ala Arg Leu Glu Lys Ile Ser Leu Glu Asn
   340 345 350
   Met Ser Pro Glu Asp Arg Glu Leu Val Ile Tyr Leu Ala Pro Leu Tyr
   355 360 365
   Lys Asn Ser Asn Lys Gly Ile Lys Gly Arg Gly Val Arg Pro Pro Gly
   370 375 380
   Tyr Arg Leu Ser Arg Ser Asp Arg Ser Ser Ser Met Ser Ser Ser Ala
   385 390 395 400
   His Ser Tyr Ser Pro Thr Lys Ser Glu Ala Ser Leu Thr Ser Ser Pro
   405 410 415
   Cys Thr Ala Gly Pro Pro Pro Gln His His Tyr Gly Gly His Ala Gln
   420 425 430
   Gln His His His Gln Gln Gln Gln Gln Gln His Gln Gln Arg His Pro
   435 440 445
   Asp His Gln Asp Met Ser Ser Tyr His Pro Ser Asn Pro Val Pro Thr
   450 455 460
   His His His Gln Arg Pro Gln Gln Gly Leu Ala Tyr Pro Pro Val Ser
   465 470 475 480
   Phe Gly Ser Ala Pro Ser Gly Asn Ile Arg Tyr Pro Tyr Asn Gly Ile
   485 490 495
   Val His Arg Tyr Glu Met Pro Glu His Ser His Pro Tyr Ser Gly Ile
   500 505 510
   Pro Val Thr Thr Ser Ser Ser Thr Ser Ser Pro Val Ala Thr Val Tyr
   515 520 525
   Ser Asp Asp Ser Gly Ala Val Tyr Gln Ser Asp Asp Asn Gly Ala Arg
   530 535 540
   Glu Leu Ala Phe Gly Glu Arg Met Cys
   545 550
<210> 110
   <211> 463
   <212> PRT
   <213> Artificial Sequence
<220>
   <223> lepmaLema_T018870.1
<400> 110
   Met Ala Ala Val Val Ala Ser His His Val Gly Phe Trp His Glu Arg
   1 5 10 15
   Arg Asp Pro Thr Ile His Val Thr Asn Leu His Leu Ala Asn Met Met
   20 25 30
   Ser Pro Tyr Asp Ile Pro Arg Thr Val Ala Asn Thr Ser Ala Ser Arg
   35 40 45
   Ser Tyr Gln Pro Thr Thr Thr Gln Met Asp Met Gly Met Pro Ile Phe
   50 55 60
   Ser Ala Thr Gly Leu Ser Ser Ser Val Pro Tyr Gln Ser Gly Ala Phe
   65 70 75 80
   Ala Phe Asp Pro Ile Ser Thr Asn Pro Tyr Asn Met Gln Gln Ser Ser
   85 90 95
   Tyr Tyr Ser Ser Asn Ile Ala Gln Ala Val Thr Tyr Gly Thr Ala Pro
   100 105 110
   Asp Val Gln Ser His Pro Thr Val Arg Asp Ala His Ser Ser Phe Leu
   115 120 125
   Met Asn Arg Asn Thr Met Val Lys Ser Glu Ser Ala Ser Pro Ile Ser
   130 135 140
   Ser Ala Pro Met Tyr Asn Asp His Ser Tyr Val Ala Glu Cys Arg Arg
   145 150 155 160
   Ala Ala Ser Glu Gln Thr Asp Arg Thr Gly Val Asn Phe Ala Thr Asp
   165 170 175
   Val Asp Thr Leu Met Arg Ala Ile Gln Ala Lys Glu Pro Thr Thr Pro
   180 185 190
   Gln Ala Lys Glu Ala Lys Gln Glu Thr Glu Val Lys Ile Ser Gln Lys
   195 200 205
   Thr Arg Lys Arg Tyr Gln Cys Asn Ile Pro Asp Cys Ser Lys Ser Phe
   210 215 220
   Phe Gln Lys Thr His Leu Asp Ile His Ile Arg Ala His Thr Gly Ala
   225 230 235 240
   Lys Pro Phe Asn Cys Lys Ala Pro Gly Cys Gly Gln Ser Phe Ser Gln
   245 250 255
   Leu Gly Asn Leu Lys Thr His Glu Arg Arg His Thr Gly Glu Arg Pro
   260 265 270
   Tyr Ser Cys Asp Ile Cys Gly Lys Thr Phe Ala Gln Arg Gly Asn Val
   275 280 285
   Arg Ala His Lys Ile Val His Gln Gln Ile Lys Pro Phe Thr Cys Arg
   290 295 300
   Leu Asp Asp Cys Gly Lys Gln Phe Thr Gln Leu Gly Asn Leu Lys Ser
   305 310 315 320
   His Gln Asn Lys Phe His Ala Ala Thr Leu Arg Tyr Leu Thr Gln Lys
   325 330 335
   Phe Ala Thr Ile Thr Pro Ser Asp Tyr Val Ser Gln Glu Asp Lys Glu
   340 345 350
   Leu Trp Glu Tyr Phe Ala Ser Leu Tyr Lys Asn Ser Asn Lys Gly Ile
           355                 360                 365
   Lys Gly Arg Gly Lys Asp Arg Arg Ile Ser Ala Ile Ser Ser Met Ala
       370                 375                 380
   Asn Ser Asn Pro Ser Ser Tyr Ala Pro Met Pro Met Thr Ser Met Ser
   385                 390                 395                 400
   Gln Ser Tyr Ser Gly Tyr Leu His His Ser Gly Ser Asp Arg Ser Ser
                   405                 410                 415
   Arg Ser Ser Ser Met Ser Thr Asp Ala Ile Arg Arg Ile Asp Asn Ser
               420                 425                 430
   Tyr Asp Phe Asn Ala Pro Met Pro Thr Gly Tyr His His Gln Thr Gln
           435                 440                 445
   Gly Thr Gly Tyr Asp Asp Met Val Phe Pro Glu Arg Lys Leu Tyr
       450                 455                 460
<210> 111
   <211> 449
   <212> PRT
   <213> Artificial Sequence
<220>
   <223> colhiCH063_09026T0
<400> 111
   Met Asp Ile Gly Asn Leu Met Asn Gln Arg Gly Ala Ala Met Gln Gln
   1               5                   10                  15
   His Ala Ser Ile His Thr Gly Ser Thr Pro Glu Arg Gln Met His Gln
               20                  25                  30
   Leu Pro Gln Leu Thr Ala Arg Asn Met Pro Glu Pro Pro Met Asp Arg
           35                  40                  45
   Ala Gly Ser Pro His Gly Ser Glu His Ser Gln Phe Ser Arg His Thr
       50                  55                  60
   Met Asp Gly Val Pro Arg Ser Tyr Pro Ser Pro Ser Ala Met Val Gly
   65                  70                  75                  80
   Ala Pro Met Gln Met Gln Val Pro Val Ser His Met Gly Pro Gly Pro
                   85                  90                  95
   Val Ile Leu Pro Gly Ile Pro Gln His Asp Met Ser Gln Gly Met Pro
               100                 105                 110
   Gln Tyr Lys Ala Pro Glu Gln Gln Val Ser Gln Gln Pro Thr Lys Ala
           115                 120                 125
   Tyr Pro Cys Ser Thr Cys Gly Lys Gly Phe Ala Arg Arg Ser Asp Leu
       130                 135                 140
   Ala Arg His Glu Arg Ile His Ser Gly Ile Arg Pro His Val Cys Asp
   145                 150                 155                 160
   Phe Pro Asn Cys Gly Lys Gln Phe Ile Gln Arg Ser Ala Leu Thr Val
                   165                 170                 175
   His Gln Arg Val His Thr Gly Glu Lys Pro His Met Cys Glu Arg Cys
               180                 185                 190
   Gly Lys Pro Phe Ser Asp Ser Ser Ser Leu Ala Arg His Arg Arg Ile
           195                 200                 205
   His Ser Gly Lys Arg Pro Tyr Lys Cys Pro Tyr Ala Asp Cys Gln Lys
       210                 215                 220
   Thr Phe Thr Arg Arg Thr Thr Leu Thr Arg His Gln Asn His His Thr
   225                 230                 235                 240
   Gly Thr Val Glu Glu Ala Ala Ala Ala Thr Ala Ala Ala Leu Ala Ala
                   245                 250                 255
   Arg Gly Ala Thr Met Lys Pro Asn Ala Thr Arg Ser Asp Gly Asp Ala
               260                 265                 270
   Met Ser Asn Arg Gly Ser Pro Leu Thr Thr Pro Ser Pro Gly Gln Arg
           275                 280                 285
   Thr Leu Ser Met Ser Pro Ser Ala Asp Ser Met His Arg Ala Asn Ser
       290                 295                 300
   Glu Phe Gln Tyr Met Gly Asn Ser Ser Leu Pro Val His Leu Gln Arg
   305                 310                 315                 320
   Asp Met His Val Gly Ser Pro Ala Ser Thr Ser Ser Gly Gly Tyr Asn
                   325                 330                 335
   Thr Gly Met Arg Pro Thr Ser His Pro Thr Ser Tyr Gly Pro Pro Pro
               340                 345                 350
   Thr Leu Glu Pro Ser Val Glu Gln His Ser Gly Pro Gly Ser Ala Gly
   355 360 365
   Gly Ser Pro His Met Ser Asn Val Gly Trp Gln Ser Pro Ser His Val
   370 375 380
   Ala Ser Pro Ser His Ser Ser Asn Gly Gly Gly Tyr Thr Tyr Pro Asp
   385 390 395 400
   Pro Asp Gly Tyr Pro Ala Ala Pro Ser Leu Gly Gly Gln Met Phe Tyr
   405 410 415
   Gly Asn Pro Gly Gln Val Arg Arg Pro Gln Ser Thr Glu Pro Pro Ala
   420 425 430
   Gly Ala Tyr Asp Ile Lys Gly Arg Gln Ser Glu Leu Trp Ala Gly Ala
   435 440 445
   His
<210> 112
   <211> 396
   <212> PRT
   <213> Artificial Sequence
<220>
   <223> CC88663
<400> 112
   Met Asn Gly Leu Arg Tyr Pro Ser Pro Ser Gln Met Gln Ala Pro Met
   1 5 10 15
   Pro Met Leu Asn Asn Ser Asn Tyr Ile Pro Asn Pro Pro Asp His Ala
   20 25 30
   Tyr Ala Gln Gln Gln Gln Met Pro Asp Asn Gln Val Gln His Gln His
   35 40 45
   Asn Gly Gly Arg Pro Ala Ser Asp Thr Gly Pro Pro Lys Ala Phe Ala
   50 55 60
   Cys Ser Thr Cys Gly Lys Gly Phe Ala Arg Arg Ser Asp Leu Ala Arg
   65 70 75 80
   His Glu Arg Ile His Ser Gly Ile Arg Pro His Val Cys Asp Tyr Pro
   85 90 95
   Asn Cys Gly Lys Gln Phe Ile Gln Arg Ser Ala Leu Thr Val His Gln
   100 105 110
   Arg Val His Thr Gly Glu Lys Pro His Met Cys Glu Arg Cys Gly Lys
   115 120 125
   Pro Phe Ser Asp Ser Ser Ser Leu Ala Arg His Arg Arg Ile His Ser
   130 135 140
   Gly Lys Arg Pro Tyr Lys Cys Pro Tyr Ala Asp Cys Gln Lys Thr Phe
   145 150 155 160
   Thr Arg Arg Thr Thr Leu Thr Arg His Gln Asn His His Thr Gly Thr
   165 170 175
   Val Glu Glu Ala Ala Ala Ala Thr Ala Ala Ala Leu Ala Ser Arg Ala
   180 185 190
   Ser Ala Ser Ser Arg Thr Gly Arg Ser Asp Gly Gly Asp Tyr Ser Glu
   195 200 205
   Thr Ala Ser Pro Leu Gly Thr Pro Ser Pro Asn Asp Arg Thr Leu Ser
   210 215 220
   Leu Ser Pro Ala Asn Gly Met Pro Ala Gly Gly Met Pro Gly Leu His
   225 230 235 240
   Arg Gln Gly Ser Asp Tyr Ala Tyr Met Gly Gly Ile Asn Val Pro Pro
   245 250 255
   His Met Arg His Glu Met Pro Gln Pro Ser Pro Arg Ala Ser Pro Ala
   260 265 270
   Leu Thr Ala Gln Ser Tyr Thr Ser Asn Val Ser Gly Ser Arg Pro Ala
   275 280 285
   Ile Thr Ser His Pro Ser Ala Tyr Gly Pro Pro Pro Ile Leu Glu Pro
   290 295 300
   Pro Ala Ser Ala Asn His Asn Gln Ser Gly Gly Thr Asn Ser Ala Asn
   305 310 315 320
   Gly Ser Pro His Met Ser Ala Met Gly Trp Gln Ser Pro Gly Gln Gln
   325 330 335
   Ala Leu Pro Ser Pro Gly Ala Gly Asp Asn Gly Tyr Val Tyr Pro Glu
   340 345 350
   Pro Gln Tyr Gln Ala Gln Asn Gly Met Tyr Tyr Gln His Asn Asn Ile
   355 360 365
   Arg Arg Pro Asn Ser Thr Glu Pro Asp His Tyr Asn Pro Asn Gln Gln
   370 375 380
   Arg Met Gly Asn Glu Met Trp Ala Pro Ala Val Gln
   385 390 395
<210> 113
   <211> 330
   <212> PRT
   <213> Artificial Sequence
<220>
   <223> veralVDBG_08073T0
<400> 113
   Met Asn Ala Gly Ile His His Tyr Lys Pro Pro Gly Val Pro Val Glu
   1 5 10 15
   Pro Gln Pro Ala Gln Gln Val Lys Ala Tyr Pro Cys Gly Thr Cys Gly
   20 25 30
   Lys Arg Phe Ala Arg Arg Ser Asp Leu Ala Arg His Glu Arg Ile His
   35 40 45
   Ser Gly Ile Arg Pro His Val Cys Asp Tyr Pro Asn Cys Gly Lys Gln
   50 55 60
   Phe Ile Gln Arg Ser Ala Leu Thr Val His Gln Arg Val His Thr Gly
   65 70 75 80
   Glu Lys Pro His Met Cys Glu Arg Cys Gly Lys Pro Phe Ser Asp Ser
   85 90 95
   Ser Ser Leu Ala Arg His Arg Arg Ile His Ser Gly Lys Arg Pro Tyr
   100 105 110
   Lys Cys Pro Tyr Ala Asp Cys Gln Lys Thr Phe Thr Arg Arg Thr Thr
   115 120 125
   Leu Thr Arg His Gln Asn His His Thr Gly Thr Val Glu Glu Ala Ala
   130 135 140
   Ala Ala Thr Ala Ala Ala Leu Ala Ala Arg Ala Ser Val Lys Pro Gly
   145 150 155 160
   Ser Thr Arg Ser Asp Gly Asp Ser Met Ser Asn Arg Gly Ser Pro Met
                   165                 170                 175
   Thr Thr Pro Ser Pro Ala Gln Arg Thr Leu Ser Met Ser Pro Ser Ala
               180                 185                 190
   Asp Gln Met Gln Arg Ser Asn Ala Glu Phe Gly Tyr Met Gly Asn Thr
           195                 200                 205
   Ser Leu Pro Val His Leu Gln Arg Asp Met His Val Gly Ser Pro Ala
       210                 215                 220
   Ser Thr Thr Ser Ala Gly Tyr Ser Asn Gly Val Arg Pro Thr Ser His
   225                 230                 235                 240
   Pro Thr Gly Tyr Gly Met Val Pro Pro Pro Thr Leu Glu Pro Asn Val
                   245                 250                 255
   Glu Gln His Ser Gly Pro Gly Ser Ala Gly Gly Ser Pro His Met Ser
               260                 265                 270
   Thr Met Gly Trp Gln Ser Pro Ser His Val Ala Ser Pro Ser His Ser
           275                 280                 285
   Asn Asn Gly Ser Ala Tyr Met Tyr Pro Glu Pro Glu His Tyr Pro Ala
       290                 295                 300
   Gly Pro Thr Leu Gly Asn Gln Met Phe Phe Gly Gln Val Arg Arg Pro
   305                 310                 315                 320
   Gln Ser Thr Glu Pro Pro Ala Ala Ala Tyr
                   325                 330
<210> 114
   <211> 447
   <212> PRT
   <213> Artificial Sequence
<220>
   <223> blaslBDBG_02290T0
<400> 114
   Met Asp Ile Thr Asn Ile Leu Asn Glu Lys Gly Thr Ala Ala Ala Ala
   1               5                   10                  15
   Val Ala Lys Ala Gln Leu His Gln His Leu Thr Gln Gly Thr His Ile
               20                  25                  30
   Lys Ser Gln Thr Pro Ser Glu Met Gly Ser Glu Asn Gly Thr Ser Gln
               35                  40                  45
   Asn Gly Asp Gln Ser Asn Ile Tyr Pro Pro Thr Ser Gln Pro His Ser
       50                  55                  60
   His Leu Ala Gly Ile Pro Gln Tyr His Pro Pro Ser Gln Gly Ala Gly
   65                  70                  75                  80
   Gly Val Pro Ala Ala Arg Ser Asp Phe Val Gln Ala Asp Gln Gly Asp
                   85                  90                  95
   Val Lys Tyr Leu Pro Asn Ala Asn Thr Gly Arg Gly Arg Ala Ser Gly
               100                 105                 110
   Glu Pro Ala Pro Lys Thr Phe His Cys Ser Thr Cys Gly Lys Gly Phe
           115                 120                 125
   Ala Arg Arg Ser Asp Leu Ala Arg His Glu Arg Ile His Ser Gly Ile
       130                 135                 140
   Arg Pro His Val Cys Asp Trp Pro Gly Cys Gly Lys Gln Phe Ile Gln
   145                 150                 155                 160
   Arg Ser Ala Leu Thr Val His Thr Arg Val His Thr Gly Glu Lys Pro
                   165                 170                 175
   His Met Cys Asp Arg Cys Gly Lys Pro Phe Ser Asp Ser Ser Ser Leu
               180                 185                 190
   Ala Arg His Arg Arg Ile His Ser Gly Lys Arg Pro Tyr Lys Cys Pro
           195                 200                 205
   Tyr Ala Asn Cys Gln Lys Thr Phe Thr Arg Arg Thr Thr Leu Thr Arg
       210                 215                 220
   His Gln Asn His His Thr Gly Thr Ile Glu Glu Ala Ala Ala Glu Thr
   225                 230                 235                 240
   Glu Ala Asn Leu Arg Gln Asn Lys Glu Arg Val Ile Arg Pro Pro Asp
                   245                 250                 255
   Gly Ile Tyr Ser Glu Thr Gly Ser Thr His Ser Thr Pro Ser Pro Gly
               260                 265                 270
   Gln Arg His Ser Leu Ser Pro Gly Asn Glu Leu Pro Pro Met Asn Leu
           275                 280                 285
   His Arg Gln Met Gly Asp Tyr Tyr Met Gly Asn Asn Thr Ser Ile Pro
       290                 295                 300
   Pro His Leu Arg Gly Asp Phe Gln Gln Pro Ser Pro Arg Ala Ser Pro
   305                 310                 315                 320
   Ser Thr Ser Ser Pro Pro Val Ser Thr Tyr Asn Asn Val Pro His Ala
                   325                 330                 335
   Arg Pro Ser Leu Thr Ser His Pro Ser Gly Tyr Gly Pro Pro Gln Pro
               340                 345                 350
   Leu Glu Pro Pro Ala Asn Ser Asp Pro Arg Pro Asn Ser Gly Ser Gly
           355                 360                 365
   Ser Pro His Ile Ser Ser Met Gly Trp Ala Ser Pro Thr Leu Asn Ser
       370                 375                 380
   Val Gly Ser Pro Ala Ser Ala Ser Ala Thr Glu Tyr Ser Tyr Pro Glu
   385                 390                 395                 400
   Pro Ser Gly Pro Pro Tyr Ser Ala His Met Pro Pro His Met Tyr Phe
                   405                 410                 415
   Pro Asn Ser Thr Ile Arg Arg Pro Gln Ser Thr Glu Pro Glu Asn Tyr
               420                 425                 430
   Glu Met Lys Pro Lys Leu Asn Gly Asp Ser Trp Thr Thr Pro Val
           435                 440                 445
<210> 115
   <211> 423
   <212> PRT
   <213> Artificial Sequence
<220>
   <223> capowCAOG_06198T0
<400> 115
   Met Ala His Thr Glu Leu Leu Pro Arg Pro Thr Phe Gln Pro Ala Pro
   1               5                   10                  15
   Tyr Gly Ser Ala Gly Gly Val Val Ala Leu Gln Gln Leu Gln Leu Asp
               20                  25                  30
   His Arg Pro Leu Gln Gln His Gln His His Pro Gln Pro Pro Gln Gln
           35                  40                  45
   Gln Gln Gln Gln Gln Gln Gln Gln Gln Gln Gln Gln Gln Gln Arg Leu
   50 55 60
   Gln Gly Phe Val Pro Tyr Gln Gln Thr Ala Leu Ala Gln Ser Ala Pro
   65 70 75 80
   Ser Pro Val Tyr Tyr Ala Ser Ser Gln Met Ala Pro Ile Gly Gln His
   85 90 95
   Val Pro Phe Ala Ser Asp Ala Gln Arg Gln Ser Val Met His Tyr Gln
   100 105 110
   Met Gly Ser Ser Thr His Pro Leu Pro Gln Ser Phe Met Pro His Pro
   115 120 125
   Val Pro Val Thr Leu His Asp Leu Gln Ala Gln Leu Val Gln Asn Pro
   130 135 140
   Ile Ser Gly Leu Ser Gln Pro Gln His Leu His Leu Val Gln Pro Ala
   145 150 155 160
   Thr Thr Gln Gln Gly Gly Met Pro Gln Pro Gln Met Ser Ser Arg Gln
   165 170 175
   Arg Val Arg Arg Lys Ser Ser Thr Glu Gly Ser Thr Thr Ser Ser Asp
   180 185 190
   Asp Lys Thr Ala Gly Leu Ala Gln Ala Ala Ser Ala Gln Val Thr Ala
   195 200 205
   Thr Pro Val Ser Ser Val Gln Ala Ala Thr Ala Pro Ala Val Ala Pro
   210 215 220
   Ala Val Pro Arg Thr Val Phe Val Asp Asn Ser Val Ala Gly Ala Ser
   225 230 235 240
   Ser Thr Phe Gln Cys Glu Trp Gln Gly Cys Ser Asp Ser Phe Leu Ser
   245 250 255
   Phe Ser Asp Phe Val Ser His Val Thr Asp Glu His Ile Gly Ser Gly
   260 265 270
   Leu Arg Phe Tyr Phe Cys Asp Trp Lys Asn Cys Met Gln Ala Gly Asn
   275 280 285
   Pro Phe Arg Lys Arg His Lys Ile Met Thr His Val Arg Ile His Thr
   290 295 300
   Gly Glu Lys Pro His Ser Cys Ser Val Pro Gly Cys Gly Lys Arg Phe
   305                 310                 315                 320
   Pro Arg Ile Asp Ser Leu Arg Ala His Met Gln Val His Ser Gly Thr
                   325                 330                 335
   Arg Lys Phe Thr Cys Pro Leu Ala Thr Cys Met Arg Ser Tyr Tyr His
               340                 345                 350
   Pro Arg Ser Leu Arg Lys His Val Arg Thr Lys Gln His Gly Asp Ala
           355                 360                 365
   Asp Ala Ile Ile Arg Glu Ile Leu Glu Ser Arg Arg Ala Thr Gly Asp
       370                 375                 380
   Ala Gln Asp Asp Ser Asp Asn Leu Glu Glu Glu Glu Glu Glu Glu Glu
   385                 390                 395                 400
   Glu Asp Thr Asp Gly Asp Asp Ser Ala Asp Gly Ser Gly Asp Gly Gly
                   405                 410                 415
   Ser Gln Ser His Ser Pro Arg
               420
<210> 116
   <211> 356
   <212> PRT
   <213> Artificial Sequence
<220>
   <223> CC29363
<400> 116
   Met His Ser Ser Ser Met Ile Pro Gln Tyr Asp Thr Ser Arg Ala Ala
   1               5                   10                  15
   Thr Ser Ala Pro Val Ser Arg Ser Phe Gln Pro Thr Ser Thr Gln Met
               20                  25                  30
   Asp Met Ser Met Pro Leu Tyr Ser Ser Asn Gly Leu Pro Thr Thr Val
           35                  40                  45
   Pro Tyr Gln Pro Gly Gln Phe Ala Tyr Asp Thr Ala Pro Val Asn Pro
       50                  55                  60
   Tyr Thr Met Gln Gln Pro Ser Tyr Tyr Gln Ser Gly Ile Pro His Pro
   65                  70                  75                  80
   Val Ser Tyr Pro Pro Ser Thr Asp Val Gln Gln Leu Pro Thr Glu Asp
                   85                  90                  95
   Ala Thr Lys Pro Ser Gln Lys Pro Arg Lys Arg Tyr Gln Cys Thr Val
               100                 105                 110
   Pro Asn Cys Asn Lys Ser Phe Tyr Gln Lys Thr His Leu Glu Ile His
           115                 120                 125
   Ile Arg Ala His Thr Gly Ala Lys Pro Phe Asn Cys Lys Ala Pro Gly
       130                 135                 140
   Cys Gly Gln Ser Phe Ser Gln Leu Gly Asn Leu Lys Thr His Glu Arg
   145                 150                 155                 160
   Arg His Thr Gly Glu Arg Pro Tyr Ser Cys Asp Ile Cys Gly Lys Thr
                   165                 170                 175
   Phe Ala Gln Arg Gly Asn Val Arg Ala His Lys Ile Val His Gln Gln
               180                 185                 190
   Ile Lys Pro Phe Thr Cys Lys Leu Asp Asp Cys Gly Lys Gln Phe Thr
           195                 200                 205
   Gln Leu Gly Asn Leu Lys Ser His Gln Asn Lys Phe His Ala Thr Thr
       210                 215                 220
   Leu Arg Tyr Leu Thr Thr Lys Phe Ala Thr Ile Ser Met Gly Asp Trp
   225                 230                 235                 240
   Val Ser Lys Glu Asp Lys Glu Leu Trp Glu Tyr Phe Ala Ser Leu Tyr
                   245                 250                 255
   Lys Asn Ser Asn Lys Gly Ile Lys Gly Arg Gly Lys Asp Arg Arg Ile
               260                 265                 270
   Ser Ala Met Ser Ser Ser Ala Ser Ser Tyr Pro Ser Ser Tyr Pro Ala
           275                 280                 285
   Met Pro Met Ala Ser Met Ser Arg Ser Tyr Gly Gly Ser Phe His His
       290                 295                 300
   Ser Ser Glu Arg Ser Ser Arg Ser Ser Ser Met Ser Ser Asp Thr Ile
   305                 310                 315                 320
   Gln Arg Val Asp Ser Gly Tyr Asp Phe Asn Val Pro Met Pro Thr Ser
                   325                 330                 335
   Tyr His Gln Pro Pro Ala Ser Gly Tyr Asp Asp Met Val Phe Pro Glu
   340 345 350
   Arg Lys Leu Tyr
   355
<210> 117
   <211> 475
   <212> PRT
   <213> Artificial Sequence
<220>
   <223> podanPa_6_5230chrm6Putative
<400> 117
   Met Glu Leu Met Glu Leu Val Asp Asn Glu Pro Thr Ala Arg Pro Phe
   1               5                   10                  15
   Gln Cys Asp Trp Asp Gly Cys Glu Thr Lys Lys Gln Ser Phe Asn Arg
               20                  25                  30
   Lys Ser Asp Leu Gln Arg His Tyr Arg Ile His Thr Asn Glu Arg Pro
           35                  40                  45
   Tyr Gln Cys Asn Gln Pro Gly Cys Gly Lys Ser Phe Ile Gln Arg Ser
       50                  55                  60
   Ala Leu Thr Val His Ile Arg Thr His Thr Gly Glu Lys Pro His Gln
   65                  70                  75                  80
   Cys Gln His His Gly Cys Gly Lys Arg Phe Ser Asp Ser Ser Ser Leu
                   85                  90                  95
   Ala Arg His Arg Arg Ile His Thr Gly Lys Arg Pro Tyr Lys Cys Ala
               100                 105                 110
   His Asp Gly Cys Leu Lys Ser Ser Phe Cys Arg Lys Thr Thr Met Val
           115                 120                 125
   Lys His Gln Arg Arg Ser His Gln Arg Gly Ile His Ser Ser Glu Leu
       130                 135                 140
   Asp Asp Ser Met Ser Asp Thr Gly Ser Glu Asp Ser Pro Ser Thr Pro
   145                 150                 155                 160
   Lys Ser His Ser Ala Met Pro Trp Ala Thr Ala Gln Gln Gln His His
                   165                 170                 175
   His Ile Pro Met Met Gly Pro His Gly His His Leu Gln Arg Ala Ala
   180 185 190
   Ser Phe Ala Asp Phe Gly Gln His Met Asn Gly Tyr Ser Leu Gln Gln
   195 200 205
   Gln Tyr Asn Asn Gln His Arg His Ser Leu Ser Ser Gly Gly Ala Ala
   210 215 220
   Glu Tyr His Gly Leu His Gln Asp Pro His Pro Gln Ala Val His His
   225 230 235 240
   His His Gln Gln Gln Pro Gln Pro Pro Gln His His His His Gln Asn
   245 250 255
   Val Pro Val Val Leu Gln Arg Thr Ser Ser Leu Pro His His Ser Tyr
   260 265 270
   Phe Val Ala Asp Ser Asn Asn Pro Gly Val Ala Thr Met Asn Thr Asn
   275 280 285
   His Pro Gly Ala Pro Gln Val His His His Gln His Ala Pro Gln Tyr
   290 295 300
   Gln Thr Ile Pro Arg Gln Ala Pro Val Glu Met Thr Tyr Pro Ser Ala
   305 310 315 320
   Pro Gly Leu Ala Ala Ser Ile Gln Ser Ser Pro Ser Ser Phe Ser Ala
   325 330 335
   Gly Ser Gly Arg Ser Pro Ser Thr Gln Glu Gly Phe Tyr Thr His Ala
   340 345 350
   Pro Pro Ala Gln Ala Ala Thr Tyr Ala Leu His Ala Thr Ser Pro Val
   355 360 365
   Val Glu Thr Gln Gln Gln Gln Gln Gln Gln Gln Gln Gln Gln Gln Gln
   370 375 380
   Gln Gln Gln Asn Met Tyr Pro Pro Pro Gln Gln Ala Gln Ala Gln Pro
   385 390 395 400
   Thr Ser Gln Pro Gln Glu Gln Lys Tyr Tyr Glu Pro Gln Pro Val Arg
   405 410 415
   Glu Glu Glu Pro Gln Gln Asn Trp Tyr Ser Gly Ala His Ala Val Tyr
   420 425 430
   Gln Pro Pro Val Glu Val Ala Thr Ile Gly Gly Tyr Ala Ala Gly Gly
           435                 440                 445
   Val Tyr Asp Pro Trp Gly Thr Gly Pro Lys Leu Glu Phe Asp Asp Gly
       450                 455                 460
   Met Gln Leu Pro Ser Ala Arg Ile Glu Ser Met
   465                 470                 475
<210> 118
   <211> 464
   <212> PRT
   <213> Artificial Sequence
<220>
   <223> settue_gw1.13.578.1
<400> 118
   Met Ala Ala Val Val Ala Pro His Gln Thr Pro Gly Leu Trp Gln Glu
   1               5                   10                  15
   Arg Arg Glu Pro Ser Phe Gln Met Pro Ser Ser Met His Gln Ser Ser
               20                  25                  30
   Met Met Pro Gln Tyr Asp Ala Thr Arg Ser Val Thr Ser Ala Pro Val
           35                  40                  45
   Ser Arg Ser Phe Gln Pro Thr Ser Thr Gln Met Asp Met Ser Met Pro
       50                  55                  60
   Leu Tyr Thr Ser Asn Gly Leu Pro Thr Ser Ala Pro Tyr Gln Ser Gly
   65                  70                  75                  80
   Gly Ala Phe Ala Tyr Asp Pro Ala Ser Val Asn Pro Tyr Thr Met Pro
                   85                  90                  95
   Gln Ser Ser Tyr Tyr Ser Ser Ser Ile Pro Asn Pro Val Ser Tyr Pro
               100                 105                 110
   Pro Ser Ser Asp Val Gln Ser Leu Pro Thr Val Arg Asp Gly Arg Asn
           115                 120                 125
   Leu Phe Asn Pro Met Val Lys Ala Glu Ser Thr Ser Pro Leu Gln Ser
       130                 135                 140
   Asn Pro Ile Tyr Asn Asn Ser Asn Ser Asp Thr Asn Tyr Ser Ala Asp
   145                 150                 155                 160
   Cys Lys Arg Ser Asn Ser Glu Pro Thr Glu Ser Asn Thr Val Asn Phe
   165 170 175
   Ser Thr Asp Val Asp Thr Leu Met Arg Ala Ile Gln Ala Lys Gln Thr
   180 185 190
   Asn Ala Pro Glu Pro Thr Glu Pro Lys Asp Glu Thr Thr Lys Pro Ser
   195 200 205
   Gln Lys Pro Arg Lys Arg Tyr Gln Cys Thr Val Pro Asn Cys Asn Lys
   210 215 220
   Ser Phe Tyr Gln Lys Thr His Leu Glu Ile His Ile Arg Ala His Thr
   225 230 235 240
   Gly Ala Lys Pro Phe Asn Cys Lys Ala Pro Gly Cys Gly Gln Ser Phe
   245 250 255
   Ser Gln Leu Gly Asn Leu Lys Thr His Glu Arg Arg His Thr Gly Glu
   260 265 270
   Arg Pro Tyr Ser Cys Asp Ile Cys Gly Lys Thr Phe Ala Gln Arg Gly
   275 280 285
   Asn Val Arg Ala His Lys Ile Val His Gln Gln Ile Lys Pro Phe Thr
   290 295 300
   Cys Lys Leu Asp Asp Cys Gly Lys Gln Phe Thr Gln Leu Gly Asn Leu
   305 310 315 320
   Lys Ser His Gln Asn Lys Phe His Ala Ala Thr Leu Arg Tyr Leu Thr
   325 330 335
   Thr Lys Phe Ala Thr Ile Ser Met Gly Asp Trp Val Ser Lys Glu Asp
   340 345 350
   Lys Glu Leu Trp Glu Tyr Phe Ala Ser Leu Tyr Lys Asn Ser Asn Lys
   355 360 365
   Gly Ile Lys Gly Arg Gly Lys Asp Arg Arg Ile Ser Ala Met Ser Ser
   370 375 380
   Ser Ala Ser Ser Tyr Pro Ser Ser Tyr Ala Ala Leu Pro Met Ser Ser
   385 390 395 400
   Leu Ser Arg Ser Phe Gly Gly Pro Phe His Gln His Ser Ser Glu Arg
   405 410 415
   Ser Ser Arg Ser Ser Ser Met Ser Ser Asp Ser Ile Gln Arg Val Asp
   420 425 430
   Ser Gly Tyr Asp Phe Asn Val Pro Met Pro Thr Gly Tyr His Gln Pro
   435 440 445
   Pro Ala Ser Gly Tyr Asp Asp Met Val Phe Pro Glu Arg Lys Leu Tyr
   450 455 460
<210> 119
   <211> 312
   <212> PRT
   <213> Artificial Sequence
<220>
   <223> tubmeGSTUMT00010369001
<400> 119
   Met Ala Thr Leu Tyr Leu Gly Leu Glu Glu Asn Leu Leu Ser Ser Ser
   1               5                   10                  15
   Pro Asn Leu Arg Ser Tyr Ser Ile Ser Ser Thr Ser Ser Gly Ala Ser
               20                  25                  30
   Cys Ser Thr Thr Gln Ser His Pro Ser Cys Tyr Arg Thr Leu Ala Pro
           35                  40                  45
   Lys Ala Asp Asn Thr Ser Pro Met Ser Phe Pro Ser Arg Pro Phe Glu
       50                  55                  60
   Asp Gly Phe Leu Gly Leu Arg Glu Arg Lys Thr His Asn Gly Gln Ser
   65                  70                  75                  80
   Ile Ala Pro Asn Leu Leu Ser Ile Lys Val Glu Gly Ala His Gln Thr
                   85                  90                  95
   Ser Asn Pro Pro Leu Asp Pro Ala Phe His Pro Ser Gln Gln Met Glu
               100                 105                 110
   Asp Met Leu Gln Gln Gln Gln Gln Asn Phe Ser Thr Pro Asn Tyr Asp
           115                 120                 125
   Gln Ser Tyr Pro Arg Ile Thr Asn Tyr Pro Glu Tyr Gly Pro Gln Arg
       130                 135                 140
   Tyr Ser Ser Asn Ser Leu Ser Pro Ser Ser Ser Asn Gly Asp Cys Leu
   145                 150                 155                 160
   Tyr Thr Thr His Asn Arg Met Phe Asp Cys Ser Gln Ser Phe Ala Pro
   165 170 175
   Trp Gly His Glu Ser Asn Ser Arg Glu Ala Cys Ala Ala Met Thr Ala
   180 185 190
   Leu Glu Leu Leu Met Pro Lys Val Gly Ile Gly Ser Glu Gly Glu Glu
   195 200 205
   Asp Val Asp Met Ser Asp Arg Asp Ser Ala Gly Ser Ser Pro Gly Pro
   210 215 220
   Thr Val Ala Arg Arg Lys Arg Arg Ala Pro His Pro Leu Val Ile Ser
   225 230 235 240
   Ser Arg Asp Lys Pro His Val Cys Ser Val Gly Asp Cys Glu Ser Arg
   245 250 255
   Phe Lys Arg Pro Glu His Leu Arg Arg His Glu Lys Thr His Thr Gly
   260 265 270
   Glu Arg Pro Phe Ser Cys Thr Ile Cys Gly Arg Arg Phe Gly Arg Asn
   275 280 285
   Asp Asn Leu Gln Thr His Lys Lys Thr His Met Lys Met Thr Gly Arg
   290 295 300
   Asn Glu Tyr Ile Pro Gly Leu Ala
   305 310
<210> 120
   <211> 467
   <212> PRT
   <213> Artificial Sequence
<220>
   <223> artbe3707
<400> 120
   Met Asp Ile Thr Thr Ile Leu Asn Lys Lys Ala Ser Val Ser Ile Val
   1               5                   10                  15
   Ala Ala Asp Val His Gln Leu Gln Gln His Leu Ala Gln Ala Thr Ala
               20                  25                  30
   Gln Ser Gln Thr Asp Asp Ala Val Lys Ser Arg Ser Pro Ser Glu Leu
           35                  40                  45
   Gly Ala Ser Glu His His Arg Ser Ala Ser Ala Pro Pro Ser Glu His
       50                  55                  60
   His His His His His His His Gln Pro Thr Ser Ser Phe Pro Ser Ser
   65 70 75 80
   Thr Gln Ser Leu Pro Gln Met Ala His Leu Ala Gln Tyr His Val Gln
   85 90 95
   Ala Gln Ala Asn His Ala Ser Thr Ser Pro Gly Tyr Ala His Ser Ala
   100 105 110
   His Gly Ser Asp Tyr Gly Arg Ser Thr Thr Ser Met Arg Pro Thr Gly
   115 120 125
   Leu Pro Ala Leu Lys Thr Phe His Cys Gln Thr Cys Ser Lys Gly Phe
   130 135 140
   Ala Arg Arg Ser Asp Leu Ala Arg His Glu Arg Ile His Ser Gly Ile
   145 150 155 160
   Arg Pro His Ala Cys Asp Trp Pro Gly Cys Gly Lys Gln Phe Ile Gln
   165 170 175
   Arg Ser Ala Leu Thr Val His Thr Arg Val His Thr Gly Glu Lys Pro
   180 185 190
   His Met Cys Asp Arg Cys Gly Lys Pro Phe Ser Asp Ser Ser Ser Leu
   195 200 205
   Ala Arg His Arg Arg Ile His Ser Gly Lys Arg Pro Tyr Lys Cys Pro
   210 215 220
   Tyr Ala Asn Cys Gln Lys Thr Phe Thr Arg Arg Thr Thr Leu Thr Arg
   225 230 235 240
   His Gln Asn His His Thr Gly Thr Ile Glu Glu Ala Ala Ala Glu Thr
   245 250 255
   Glu Ala Asn Leu Arg His Ser Lys Asp Arg Ser Thr Thr Ser Arg Pro
   260 265 270
   Ser Glu Tyr Ser Glu Pro Gly Ser Ala His Ser Thr Pro Ser Pro Ala
   275 280 285
   Gln Arg Pro Ser Leu Ser Pro Gly Asn Glu Leu Pro Pro Leu Asn Leu
   290 295 300
   Ser Ser Arg Pro Pro Ala Ala Gly Gly Pro Thr Ser Ala Gly Gly Ala
   305 310 315 320
   Ala Asp Tyr Tyr Thr Ser Leu Pro Pro His Leu Arg Thr Asp Phe Ala
   325 330 335
   Asn Ser Ser Pro Arg Ala Ser Pro Thr Ser Pro Ser Pro Thr Leu Thr
   340 345 350
   Ser Leu Ser Gly Pro Ala Ser Phe His His His His Asn His Asn Arg
   355 360 365
   Pro Ser Leu Thr Ser His Pro Ser Gly Tyr Ala Pro Pro Gln Pro Leu
   370 375 380
   Glu Pro Pro Ala Asn Ser Asp Pro Arg Pro Asn Ser Val Ala Gly Ser
   385 390 395 400
   Pro His Leu Ser Ser Val Gly Trp Ala Ser Pro Thr Leu Ser Ser Met
   405 410 415
   Pro Ser Pro Ser Ser Ala Ser Ala Pro Glu Tyr Pro Tyr Pro Glu Pro
   420 425 430
   Ser Asn His Gln Tyr Pro Gly Ser Ile Thr Pro His Met Phe Phe Pro
   435 440 445
   Arg Arg Pro Gln Ser Ser Glu Pro Glu Asn Tyr Glu Leu Lys Pro Lys
   450 455 460
   Leu Glu Leu
   465
<210> 121
   <211> 402
   <212> PRT
   <213> Artificial Sequence
<220>
   <223> aspteATET_00873
<400> 121
   Met Lys Ser Glu Pro Gly Val Thr Glu Ser Ala Asp Gln Pro Val Leu
   1               5                   10                  15
   Ser Tyr Pro Pro His Ala Pro Leu Pro Ser Met Pro Gln Asp Val Arg
               20                  25                  30
   Tyr Ala Pro Gln Ser His Pro Ala Pro Gly Met Pro Leu Met Gln Thr
           35                  40                  45
   Pro Tyr Val Pro Gly Gly Tyr Ala Ser Gly Ala Pro Met His Asn Gly
       50                  55                  60
   Ala Ala Pro Pro Pro Arg Thr Glu Pro Pro Pro Lys Thr Phe His Cys
   65                  70                  75                  80
   Gly Thr Cys Gly Lys Gly Phe Ala Arg Arg Ser Asp Leu Ala Arg His
                   85                  90                  95
   Glu Arg Ile His Ser Gly Ile Arg Pro His Ala Cys Asp Trp Pro Gly
               100                 105                 110
   Cys Gly Lys Gln Phe Ile Gln Arg Ser Ala Leu Thr Val His Ser Arg
           115                 120                 125
   Val His Thr Gly Glu Lys Pro His Met Cys Glu Arg Cys Gly Lys Pro
       130                 135                 140
   Phe Ser Asp Ser Ser Ser Leu Ala Arg His Arg Arg Ile His Ser Gly
   145                 150                 155                 160
   Lys Arg Pro Tyr Lys Cys Pro Tyr Ala Asn Cys Gln Lys Thr Phe Thr
                   165                 170                 175
   Arg Arg Thr Thr Leu Thr Arg His Gln Asn His His Thr Gly Thr Ile
               180                 185                 190
   Glu Glu Ala Ala Ala Glu Thr Glu Ala Asn Leu Arg Gln Asn Lys Glu
           195                 200                 205
   Arg Ser Arg Gly Pro Gly Glu Pro Met Phe Ser Asp His Ala Ser Val
       210                 215                 220
   His Ser Thr Pro Ser Pro Ala His Ser Gly Ile Ser Ala Gly Gly Glu
   225                 230                 235                 240
   Leu Pro Pro Leu Asn Met Pro Arg Ser Thr Gly Asp Tyr Tyr Met Gly
                   245                 250                 255
   Ser Gly Ser Ile Pro Pro His Val Arg Gly Asp Phe Ser Gln Ala Ser
               260                 265                 270
   Pro Arg Ala Ser Pro Thr Ala Thr Ser Pro Ser Leu Ser Ser Phe Gly
           275                 280                 285
   Ser Ala Pro His Ser Arg Pro Ser Met Thr Ser His Pro Thr Gly Tyr
       290                 295                 300
   Gly Pro Pro Gln Pro Leu Glu Pro Pro Ala Asn Asn Asp His Arg Pro
   305                 310                 315                 320
   Asn Ser Val Ser Gly Ser Pro His Met Pro Thr Leu Gly Trp Ala Ser
                   325                 330                 335
   Pro Ser His Gly Ser Ile Pro Ser Pro Gly Ser Ala Asn Asp Phe Pro
               340                 345                 350
   Tyr Pro Glu Pro Gly Ala Pro Ala Tyr Pro Ser Ser Val Pro Pro His
           355                 360                 365
   Met Tyr Phe Ser His Ser Ala Gly Arg Arg Pro Thr Ser Thr Glu Pro
       370                 375                 380
   Glu Asn Tyr Glu Met Lys Pro Lys Met Gly Asp Asn Ser Trp Ser Thr
   385                 390                 395                 400
   Pro Val
<210> 122
   <211> 404
   <212> PRT
   <213> Artificial Sequence
<220>
   <223> clagrfgenesh1_pm.4_#_42
<400> 122
   Met Leu Asn Asn Lys Val Thr Ser Ala Gln Tyr Gln Gln His Met Ala
   1               5                   10                  15
   Gln Ala Ser Tyr Pro Ser Pro Ser Gln Leu Gln Asp Pro Leu Ser Met
               20                  25                  30
   Leu Gly Thr Asn Tyr Ser Glu Arg Pro Phe Glu Asn Met Tyr Leu Pro
           35                  40                  45
   Gln His Gln Asn Glu His His Met Ser Ser His Asp Ala Ser Asp Ser
       50                  55                  60
   Gln Arg Gln Ser Ser Asn Gly Ala Ile Lys Ala Phe Ala Cys Thr Asn
   65                  70                  75                  80
   Cys Gly Lys Gly Phe Ala Arg Arg Ser Asp Leu Ala Arg His Glu Arg
                   85                  90                  95
   Ile His Thr Gly Asp Arg Pro His Val Cys Pro Glu Lys Gly Cys Gly
               100                 105                 110
   Lys Gln Phe Ile Gln Arg Ser Ala Leu Thr Val His Met Arg Val His
           115                 120                 125
   Ser Gly Glu Lys Pro His Met Cys Glu Arg Leu Ile Arg Val Leu Trp
       130                 135                 140
   Leu Gly Ile Val Ala Tyr Ile Leu Gly Lys Arg Pro Tyr Lys Cys Pro
   145                 150                 155                 160
   Phe Ala Asp Cys Gln Lys Thr Phe Thr Arg Arg Thr Thr Leu Thr Arg
                   165                 170                 175
   His Gln Asn His His Thr Gly Thr Val Glu Glu Ala Ala Val Ala Thr
               180                 185                 190
   Ala Ala Ala Leu Ala Ser Arg Thr Pro Gly Ile His Pro Arg Asn Gly
           195                 200                 205
   Ser Asp Gly Gly Asn Phe Ser Asp Thr Ala Ser Met Ser Ser Ala Met
       210                 215                 220
   Thr Arg Thr Pro Ser Pro Pro Glu Arg Gln Leu Ser Met Ser Pro Asn
   225                 230                 235                 240
   Asp Asp Val Pro His Met Pro His Leu Asn Arg Arg Thr Ser Gly Val
                   245                 250                 255
   Ser Tyr Ser Ser Ser Asp Ser Cys Pro Leu Pro Ala His Phe Arg Pro
               260                 265                 270
   Gly Leu Gln Gln Pro Ser Pro Arg Ser Ser Pro Ser Val Ser Ser Pro
           275                 280                 285
   Thr Leu Ser Ala Phe Gly Gly Asn Asn Ser Tyr His Arg Pro Ser Leu
       290                 295                 300
   Thr Ser His Pro Thr Leu Pro Val Leu Glu Pro Pro Thr Tyr His Asp
   305                 310                 315                 320
   Leu Arg Gln Pro Ala Ser Gly Ser Gly Ser Pro His Phe Gly Ser Thr
                   325                 330                 335
   Gly Trp Gln Ser Pro Val Ala Ala Gly Leu Pro Ser Pro Gly His Asn
   340 345 350
   Asp Gly Gly Phe Trp Asn Pro Asp Pro Gln Tyr Gly Pro Asn Gln His
   355 360 365
   Leu Tyr Tyr Pro Ser Ser Asn Met Arg Arg Pro Gln Ser Thr Glu Pro
   370 375 380
   Asp His Tyr Glu Leu Lys Pro Arg Leu Pro Ser Thr Gly Val Trp Ala
   385 390 395 400
   Gly Thr Met Gly
<210> 123
   <211> 457
   <212> PRT
   <213> Artificial Sequence
<220>
   <223> pyrtePTTG_19009.1
<400> 123
   Met Ala Ala Val Val Ala Ser His His Met Gly Leu Trp Gln Asp Arg
   1               5                   10                  15
   Arg Glu Thr Pro Ala His Leu Ser Asn Met His Leu Ser Asn Met Met
               20                  25                  30
   Pro Ser Tyr Asp Ala Ser Arg Thr Val Ser Asn Ala Pro Val Ser Arg
           35                  40                  45
   Ser Tyr Gln Pro Thr Ser Ser His Met Asp Ile Asn Met Pro Leu Tyr
       50                  55                  60
   Ser Ala Asn Gly Leu Pro Thr Ser Val Pro Tyr Gln Ser Gly Ala Phe
   65                  70                  75                  80
   Ala Tyr Asp Pro Thr Pro Val Asn Ala Tyr Asn Met Gln Gln Pro Ser
                   85                  90                  95
   Tyr Tyr Gln Ser Asn Ile Gln His Pro Val Ser Tyr Ala Pro Thr Ser
               100                 105                 110
   Asp Val Gln Gln Leu Pro Thr Val Arg Asp Ala Arg Asn Val Phe Asn
           115                 120                 125
   Asn Met Val Lys Ser Glu Ser Thr Ser Pro Val Gln Ser Asn Pro Met
       130                 135                 140
   Tyr Asn Asn Thr Ala Tyr Ser Ala Asp Ile Lys Arg Ser Thr Ser Glu
   145                 150                 155                 160
   Pro Thr Glu Gly Ser Ser Ala Ser Phe Ala Thr Asp Val Asp Thr Leu
                   165                 170                 175
   Met Arg Ala Ile Gln Ala Lys Gln Thr Thr Ser Pro Gln Ala Ser Glu
               180                 185                 190
   Pro Lys Glu Glu Ala Val Lys Val Ser Gln Lys Pro Arg Lys Arg Tyr
           195                 200                 205
   Gln Cys Thr Ile Pro Asn Cys Asn Lys Ser Phe Tyr Gln Lys Thr His
       210                 215                 220
   Leu Glu Ile His Ile Arg Ala His Thr Gly Ala Lys Pro Phe Asn Cys
   225                 230                 235                 240
   Lys Ala Pro Gly Cys Gly Gln Ser Phe Ser Gln Leu Gly Asn Leu Lys
                   245                 250                 255
   Thr His Glu Arg Arg His Thr Gly Glu Arg Pro Tyr Ser Cys Asp Ile
               260                 265                 270
   Cys Gly Lys Thr Phe Ala Gln Arg Gly Asn Val Arg Ala His Lys Ile
           275                 280                 285
   Val His Gln Gln Ile Lys Pro Phe Thr Cys Lys Leu Asp Asp Cys Gly
       290                 295                 300
   Lys Gln Phe Thr Gln Leu Gly Asn Leu Lys Ser His Gln Asn Lys Phe
   305                 310                 315                 320
   His Ala Ala Thr Leu Arg Tyr Leu Thr Gln Lys Phe Ala Thr Ile Thr
                   325                 330                 335
   Ser Gly Asp Trp Val Ser Gln Glu Asp Lys Glu Leu Trp Glu Tyr Phe
               340                 345                 350
   Ala Ser Leu Tyr Lys Asn Ser Asn Lys Gly Ile Lys Gly Arg Gly Lys
           355                 360                 365
   Asp Arg Arg Ile Ser Ala Met Ser Ser Ser Ala Ser Ser Tyr Pro Ser
       370                 375                 380
   Ser Tyr Ala Gly Met Pro Met Val Pro Met Ser Arg Ser Tyr Ala Gly
   385 390 395 400
   Ser Phe His Gln Gln Ser Ser Asp Arg Ser Ser Arg Ser Ser Ser Met
   405 410 415
   Ser Ser Asp Ser Leu His Arg Val Asp Ser Ala Tyr Asp Phe Gly Ala
   420 425 430
   Pro Ile Pro Asn Pro Tyr His Gln Gln Pro Gly Thr Gly Tyr Asp Asp
   435 440 445
   Met Val Phe Pro Glu Arg Lys Leu Tyr
   450 455
<210> 124
   <211> 437
   <212> PRT
   <213> Artificial Sequence
<220>
   <223> acrale_gw1.4.916.1
<400> 124
   Met Gln Gln Pro His His Gly Ser Gly Ala Thr Pro Asp Arg Gln Ile
   1               5                   10                  15
   Pro Gln Leu Ser Gln Leu Thr Ala Arg Asn Leu Pro Glu Pro Pro Met
               20                  25                  30
   Asp Arg Ala Ala Ser Pro His Gly Ser Glu His Ser Arg Tyr Ser Ala
           35                  40                  45
   Pro Arg Ser Thr Leu Asp Thr Met Pro Arg Ser Tyr Pro Ser Pro Asn
       50                  55                  60
   Pro Met His Gly Pro Pro Met Thr Met Pro Leu Pro Gly His Gly Gln
   65                  70                  75                  80
   Gln Gln His Ile Ala Ala Pro Ala Ile Thr Met Ile Leu Pro Gly Ala
                   85                  90                  95
   Pro Pro Asp Met Gly Met Pro Lys Pro Ala Pro Ala Pro Gln Ala Pro
               100                 105                 110
   Val Lys Ala Phe Pro Cys Ser Thr Cys Gly Lys Arg Phe Ala Arg Arg
           115                 120                 125
   Ser Asp Leu Ala Arg His Glu Arg Ile His Ser Gly Ile Arg Pro His
       130                 135                 140
   Val Cys Asp Phe Pro Gln Cys Gly Lys Gln Phe Ile Gln Arg Ser Ala
   145                 150                 155                 160
   Leu Thr Val His Gln Arg Val His Thr Gly Glu Lys Pro His Met Cys
                   165                 170                 175
   Glu Arg Cys Gly Lys Pro Phe Ser Asp Ser Ser Ser Leu Ala Arg His
               180                 185                 190
   Arg Arg Ile His Ser Gly Lys Arg Pro Tyr Lys Cys Pro Tyr Ala Asp
           195                 200                 205
   Cys Gln Lys Thr Phe Thr Arg Arg Thr Thr Leu Thr Arg His Gln Asn
       210                 215                 220
   His His Thr Gly Thr Val Glu Glu Ala Ala Ala Ala Thr Ala Ala Val
   225                 230                 235                 240
   Leu Ala Ala Arg Thr Asn Ala Lys Pro Gly Ser Val Arg Ser Glu Gly
                   245                 250                 255
   Glu Gly Ile Ser Asn His Ala Ser Pro Leu Thr Thr Pro Ser Pro Gly
               260                 265                 270
   Gln Arg Thr Leu Ser Met Ser Pro Ser Ala Asp Gln Met Gln Arg Ser
           275                 280                 285
   Ala Thr Glu Phe Gln Tyr Met Gly Asn Ala Ser Leu Pro Val His Met
       290                 295                 300
   Gln Arg Asp Met His Val Gly Ser Pro Val Pro Thr Thr Ser Ala Thr
   305                 310                 315                 320
   Tyr Ser Asn Gly Met Arg Pro Thr Ser His Pro Thr Gly Tyr Gly Met
                   325                 330                 335
   Ile Pro Pro Pro Thr Leu Glu Pro Asn Val Glu Gln His Ser Gly Thr
               340                 345                 350
   Gly Ser Ala Gly Gly Ser Pro Ala Met Ala Asn Met Gly Trp Gln Ser
           355                 360                 365
   Pro Ser His Val Ala Ser Pro Ser His Asn Thr Asn Gly Asn Gly Tyr
       370                 375                 380
   Met Tyr Pro Glu Pro Glu Asn Tyr Pro Ser Gly Pro Thr Leu Gly Gly
   385 390 395 400
   Gln Met Phe Tyr Ser Asn Pro Ala Pro Val Arg Arg Pro Gln Ser Thr
   405 410 415
   Glu Pro Pro Ala Ala Ala Tyr Asp Val Lys Pro Arg Gln Asn Glu Leu
   420 425 430
   Trp Thr Ser Ala His
   435
<210> 125
   <211> 463
   <212> PRT
   <213> Artificial Sequence
<220>
   <223> gaegrGGTG_00870T0
<400> 125
   Met Ser Asp Pro Arg Pro Ala Val Ser Pro Ala Val Ser Val Ser Gly
   1               5                   10                  15
   Val Gly Ser Ala Pro Leu Gln Ser His Met Ser Leu Glu Ala Val Lys
               20                  25                  30
   Gln Glu Thr His Gly Leu Gln Pro Leu Gln Gln His Tyr His His Gln
           35                  40                  45
   Leu Ala Pro Leu Pro Met Asp Arg Ala Asp Ser Pro His Gly Ser Glu
       50                  55                  60
   His Ser Arg Tyr Ser Val Pro Pro Leu Asn Gly Met Asp Gln Pro Arg
   65                  70                  75                  80
   Pro Tyr Gly Ser Pro Ala Ala Met His Ala Pro Leu His Met Ser Asp
                   85                  90                  95
   Met Asn Arg Pro Pro Thr Ser Leu Pro Met Ser Ser Leu Pro Leu Pro
               100                 105                 110
   Ser Ile Gln Pro Val Leu Ala Pro Ala Pro Pro Ala Ala Gly Ala Met
           115                 120                 125
   Tyr Gly Thr Gln Glu Ala Pro Gln Pro Pro Pro Lys Ala Tyr Pro Cys
       130                 135                 140
   Ser Thr Cys Gly Lys Gly Phe Ala Arg Arg Ser Asp Leu Ala Arg His
   145                 150                 155                 160
   Glu Arg Ile His Ser Gly Val Arg Pro His Ile Cys Asp Phe Pro Gly
   165 170 175
   Cys Asn Lys Lys Phe Ile Gln Arg Ser Ala Leu Thr Val His Gln Arg
   180 185 190
   Val His Thr Gly Glu Lys Pro His Gln Cys Glu Arg Cys Gly Lys Pro
   195 200 205
   Phe Ser Asp Ser Ser Ser Leu Ala Arg His Arg Arg Ile His Ser Gly
   210 215 220
   Lys Arg Pro Tyr Lys Cys Pro Tyr Ala Asp Cys Gln Lys Thr Phe Thr
   225 230 235 240
   Arg Arg Thr Thr Leu Thr Arg His Gln Asn His His Thr Gly Thr Ile
   245 250 255
   Glu Glu Ala Ala Ala Ala Thr Arg Glu Ala Leu Ala Arg Gly Pro Thr
   260 265 270
   Gly Thr Ala Arg Met Thr Pro Thr His Val Thr Arg Ser Glu Gly Asp
   275 280 285
   Ala Gly Ser Gly Ser Asn His Ala Ser Pro Met Ser Thr Pro Ser Pro
   290 295 300
   Ala His Arg Thr Asn Ser Met Ser Pro Ser Asn Glu Leu Ala Gly Ile
   305 310 315 320
   Gly Ser Ile Gln Ser Gln Tyr Val Asn Asn Ser Leu Pro Ser His Leu
   325 330 335
   Arg Gly Asp Val His Ala Gly Ser Pro Thr Ser Thr Thr Ser Ser Gly
   340 345 350
   Tyr Thr Asn Asn Arg Pro Thr Ser His Pro Thr Gly Tyr Gly Pro Pro
   355 360 365
   Gln Val Leu Glu Pro Asn Ile Glu Thr Arg Pro Gly Pro Gly Ser Ala
   370 375 380
   Ser Gly Ser Pro His Leu Gly Ser Val Gly Trp Gln Ser Pro Ser His
   385 390 395 400
   Ala Pro Ser Pro Ser His Ser Thr Ser Gly Phe Val Tyr Pro Asp Pro
   405 410 415
   Glu Ala Tyr Pro Ser Ser Ala Gly Met Gly Pro Leu Phe Tyr Ser Ser
   420 425 430
   Ala Ile Ala Gly Val Arg Ser Gly Ser Ala Glu Pro Gly Gln Asn Asn
   435 440 445
   Asp Tyr Pro Lys Ser Arg Ser Ala Glu Leu Trp Ala Ser Ala Gln
   450 455 460
<210> 126
   <211> 440
   <212> PRT
   <213> Artificial Sequence
<220>
   <223> parbrPAAG_03645T0
<400> 126
   Met Glu Thr Ala Val Pro Val Thr Tyr Asn Val Pro Asn His Asp Met
   1               5                   10                  15
   Asn Ala Val Pro Pro Arg Arg Met Met Thr Pro Ser His His Asp Gln
               20                  25                  30
   Asn Ile Ala Tyr Phe Ser Asn Pro Ser Ile Pro Tyr Ser Thr Ala Leu
           35                  40                  45
   Gln Pro Pro Pro Phe Ser Gly Phe Gly His Ile Leu Asn Asn His His
       50                  55                  60
   His Pro Ser Tyr His Ser Tyr Phe Ser Ser Asn Pro Asn His His Val
   65                  70                  75                  80
   Asn Ser His His Pro Arg Leu Pro Ile Glu Ala Leu Pro Val Gln Pro
                   85                  90                  95
   Gln Leu Ser Asp Val Arg His Val Lys Asn Phe Ile Pro Arg Ser Ile
               100                 105                 110
   Arg Pro Ser Pro Pro Lys Glu Asp His Ile Pro His Pro Pro Pro Gln
           115                 120                 125
   Arg Val Asn Val Ala Pro Ala Arg Ala Arg Asn Val Thr Thr Gln His
       130                 135                 140
   Asp Asp Pro Lys Gly Pro Thr Lys Pro Glu Val Asp Phe Gly Thr Glu
   145                 150                 155                 160
   Val Asp Thr Leu Met Lys Thr Ile Gln Ala Lys Pro Gln Ser Ser Ser
   165 170 175
   Pro Gln Val Glu His Gln Leu Pro Pro Leu Gln Gln Lys Phe Asn Asn
   180 185 190
   Gly Val Pro Ser Trp Ile Gln Pro Ala Tyr Ala Asn Gln Met Pro Gly
   195 200 205
   Asn Gln Gly Ile Phe Pro Thr Pro Pro Gln Asp Arg Ile Met Ser Thr
   210 215 220
   Asn Gln Lys Pro Arg Arg Lys Tyr Glu Cys Thr Leu Pro Gln Cys Arg
   225 230 235 240
   Lys Ser Phe Phe Gln Lys Thr His Leu Asp Ile His Met Arg Ala His
   245 250 255
   Thr Gly Asp Lys Pro Phe Thr Cys Lys Glu Pro Ser Cys Gly Gln Arg
   260 265 270
   Phe Ser Gln Leu Gly Asn Leu Lys Thr His Glu Arg Arg His Thr Gly
   275 280 285
   Glu Lys Pro Tyr Ser Cys Glu Ile Cys His Lys Lys Phe Ala Gln Arg
   290 295 300
   Gly Asn Val Arg Ala His Lys Ile Thr His Glu Gln Ala Lys Pro Phe
   305 310 315 320
   Thr Cys Arg Leu Asp Asp Cys Gly Lys Gln Phe Thr Gln Leu Gly Asn
   325 330 335
   Leu Lys Ser His Gln Asn Lys Phe His Thr Gln Thr Leu Arg Asn Leu
   340 345 350
   Thr Leu Arg Phe Glu Ser Ile Gly Asn Ser Asp Arg Met Ser Pro Gln
   355 360 365
   Asp Lys Glu Leu Trp Asp Tyr Phe Ser Thr Leu Tyr Arg Asn Ser Asn
   370 375 380
   Lys Gly Ile Lys Gly Arg Gly Lys Asp Arg Arg Val Ser Thr Ala Lys
   385 390 395 400
   Arg Ser Thr Ser Thr Tyr Glu Gly Ser Val Ser Asp Ser Asp Glu Asp
   405 410 415
   Leu Lys Ile Arg Ser Arg Ser Tyr Asp Arg Val Ser Ala Val Met Thr
   420 425 430
   Leu Gly Gly Glu Asp Leu Gly Arg
   435 440
<210> 127
   <211> 415
   <212> PRT
   <213> Artificial Sequence
<220>
   <223> BC_P074200.1
<400> 127
   Met Ala Phe Tyr Ala Val Ala Ala Phe Lys Gly Ile Lys Asp Ser Lys
   1               5                   10                  15
   Pro Lys Phe Gly Gly Ile Gln Cys Val Asp Val Asn Met Leu Ser Pro
               20                  25                  30
   Gly Thr Leu Asn Pro Gln Ser Gln Leu Ala Lys Leu Ile Gly Leu Met
           35                  40                  45
   Lys Val Ile Pro Glu Asp Asp Leu His Thr Lys Arg Thr Phe Ile Ile
       50                  55                  60
   Ile Thr Lys Phe Gln Val Val Leu Phe Ile Met Tyr Ile Tyr Gln Pro
   65                  70                  75                  80
   Asp Ser Pro Glu Leu Phe Ser Asn Gly Tyr Met Leu Phe Val Glu Arg
                   85                  90                  95
   Trp Thr Gly Lys Ala Val Thr Thr Leu Asn Glu Ala Val Ser Arg Leu
               100                 105                 110
   Gly Gly Met Phe Asp Gly Cys Trp Gln Asp Lys Ser Val Tyr Asn Val
           115                 120                 125
   Asp Asn His Ser Thr Ala Arg Ile Leu Asn His Gln Val Pro Pro Ala
       130                 135                 140
   Cys Ser Thr Arg Phe Thr Glu Ser Met Gln Ala Leu Ala Gly Met Pro
   145                 150                 155                 160
   Met Glu Tyr Gly Gly Phe Pro Ile Thr Thr Thr Thr Ser Thr Glu Cys
                   165                 170                 175
   Thr Thr Lys Asp His Ser Gln Tyr Gln Gln Ser Asp Phe Asn Thr Ile
   180 185 190
   Lys Asn Val Ala Leu Pro Gln Gln Tyr Gln Gln Ser Asp Phe Asn Thr
   195 200 205
   Ile Lys Asn Val Ala Leu Pro Gln Gln Tyr Gln Gln Ser Asp Phe Asn
   210 215 220
   Thr Thr Lys Asn Val Ala Leu Pro Gln Gln Tyr Gln Gln Ser Asp Phe
   225 230 235 240
   Asn Thr Thr Lys Asn Val Ala Leu Pro Gln Gln Tyr Gln Thr Ile Thr
   245 250 255
   Asn Ser Asn Glu Arg Pro His Lys Cys Lys Glu Pro Gly Cys Gly Lys
   260 265 270
   Ala Phe Thr Leu Ser Ser Gly Leu Lys Glu His Lys Asn Phe Thr His
   275 280 285
   Ser Glu Lys Arg Pro His Lys Cys Lys Val Pro Asn Cys Gly Lys Ala
   290 295 300
   Leu Lys Arg Arg Ser Ala Leu Val Ala His Lys Asn Gly Val His Ser
   305 310 315 320
   Asn Val Arg Pro His Lys Cys Glu Val Gln Asn Cys Gly Lys Ala Phe
   325 330 335
   Lys Arg Pro Ser Glu Leu Lys Asn His Gln Asn Phe Thr His Ser Lys
   340 345 350
   Glu Arg Pro Tyr Lys Cys Arg Val Pro Asn Cys Val Lys Ala Phe Arg
   355 360 365
   Arg Pro Ser Asp Leu Tyr Gln His Glu His Gln Val His Ser Asn Glu
   370 375 380
   Lys Pro Tyr Lys Cys Glu Val Pro Asn Cys Gly Lys Ala Tyr Lys Arg
   385 390 395 400
   Phe Thr Ser Leu Gly Lys His Tyr Ile Thr His Thr Asn Asn Ala
   405 410 415
<210> 128
   <211> 475
   <212> PRT
   <213> Artificial Sequence
<220>
   <223> MGG_06507
<400> 128
   Met Asp Ala Pro Val Thr Ser Ser Asp Ile Pro Gly Leu Ser Ser Leu
   1               5                   10                  15
   Val Asp Gln Arg Val Thr Pro Ser Pro Val Pro Ser Met His Leu Pro
               20                  25                  30
   Ala Pro Val Pro Thr Pro Pro His Gly His Gln Glu Pro Leu Gln Leu
           35                  40                  45
   Gln Pro Leu Gln Gln Tyr His Gln Leu Ser Gln Met Pro Val Asp Arg
       50                  55                  60
   Ala Asp Ser Pro His Gly Ser Glu His Ser Arg Tyr Ser Ala Pro Pro
   65                  70                  75                  80
   Met Asn Gly Ser Gly Met Asp Asn Ser Arg Pro Tyr Gly Ser Pro Ser
                   85                  90                  95
   Ala Met His Ala Pro Leu His Ile Ser Pro Pro Thr Ser Leu Pro Leu
               100                 105                 110
   Gly Ser Asn Ile Ser Leu Pro Ala Ile Gln Gln Pro Ile Leu Ala Pro
           115                 120                 125
   Ala Pro Pro Ala Met Met Ser Val Tyr Pro Pro Val Asn Gln Pro Pro
       130                 135                 140
   Pro Pro Val Lys Ala Tyr Pro Cys Ser Thr Cys Gly Lys Gly Phe Ala
   145                 150                 155                 160
   Arg Arg Ser Asp Leu Ala Arg His Glu Arg Ile His Ser Gly Ile Arg
                   165                 170                 175
   Pro His Val Cys Asp His Pro Gly Cys Gly Lys Gln Phe Ile Gln Arg
               180                 185                 190
   Ser Ala Leu Thr Val His Met Arg Val His Thr Gly Glu Lys Pro His
           195                 200                 205
   Met Cys Glu Arg Cys Gly Lys Pro Phe Ser Asp Ser Ser Ser Leu Ala
       210                 215                 220
   Arg His Arg Arg Ile His Ser Gly Lys Arg Pro Tyr Lys Cys Pro Tyr
   225 230 235 240
   Ala Asn Cys Gln Lys Thr Phe Thr Arg Arg Thr Thr Leu Thr Arg His
   245 250 255
   Gln Asn His His Thr Gly Thr Ile Glu Glu Ala Ala Leu Ala Thr Arg
   260 265 270
   Glu Ala Leu Ser Arg Asp Pro Asn Thr Thr Ala Arg Leu Thr Pro Gly
   275 280 285
   Ala His His Arg Ser Asp Gly Glu Ala Val Val Thr Ser Asn His Ala
   290 295 300
   Ser Pro Leu Thr Thr Pro Ser Pro Ala His Arg Thr His Ser Met Ser
   305 310 315 320
   Pro Ser Asn Glu Leu Ala Gly Ile His Thr Leu Gln Thr Gln Tyr Pro
   325 330 335
   Asn Ser Ser Leu Pro Ser His Leu Arg Ser Asp Val His Ile Ala Ser
   340 345 350
   Pro Thr Ser Thr Thr Ser Ser Gly Tyr Asn Gly Asn Arg Pro Thr Ser
   355 360 365
   His Pro Ser Gly Tyr Gly Pro Pro Ser Val Leu Glu Pro Ser Ile Glu
   370 375 380
   Thr Arg Pro Gly Pro Gly Ser Ala Ser Gly Ser Pro His Met Gly Ser
   385 390 395 400
   Val Gly Trp Gln Ser Pro Ser His Ala Gly Ser Pro Ser His Ser Ala
   405 410 415
   Ser Gly Phe Val Tyr Pro Asp Pro Glu Ala Tyr Pro Gly His Ala Gly
   420 425 430
   Leu Ser Gly Asn Leu Gly Ser Leu Phe Tyr Gly Ser Ala Val Asn Ser
   435 440 445
   Thr Arg Ala Gly Ser Ser Glu Pro Gly Gln Gly Gly Asp Tyr Pro Lys
   450 455 460
   Pro Arg Thr Ser Glu Leu Trp Ala Ser Ala Gln
   465 470 475
<210> 129
   <211> 447
   <212> PRT
   <213> Artificial Sequence
<220>
   <223> ANG40300
<400> 129
   Met Glu Thr Ala Glu Pro Val Ser Tyr Glu Phe Pro Gly His Ala Val
   1               5                   10                  15
   Gly Ala Val Ala Pro Arg Arg Met Met Gly Ser Asn Val Gly His Asn
               20                  25                  30
   Phe Pro Phe Tyr Thr Asn Pro Thr Gly Gly Tyr Thr Leu Pro Phe His
           35                  40                  45
   Gln Ser Ser Ser Pro Ala Tyr Ser Phe Gly His Thr Leu Asn His His
       50                  55                  60
   His His His His His His His Pro Gly Tyr Gln His Tyr Phe Val Ala
   65                  70                  75                  80
   Gly Gln Gln Pro Ile Asn Pro Gln Pro Val Arg Leu Ser Ser Glu Pro
                   85                  90                  95
   Pro Ser Ile Gln Gln Ile Pro Asp Ile Arg Pro Ala Lys Asn Ala Val
               100                 105                 110
   Asn Arg Val Ala Arg Asp Pro Leu Met Lys Ile Glu His Asn Gly Ala
           115                 120                 125
   Ser Gln Gln Pro Pro Gly Ala Gln Ser Ser Ser Asn Glu Glu Gly Ala
       130                 135                 140
   Gln Gly Lys Ser Ser Ser Ser Asn Glu Val Glu Phe Ser Thr Glu Val
   145                 150                 155                 160
   Asp Ile Leu Met Lys Ala Ile Gln Ala Lys Ala Ser Ala Gln Ser Pro
                   165                 170                 175
   Gly Val Gln Ser Leu Pro Pro Leu Gln Gln Leu Thr His Gly Gly Ser
               180                 185                 190
   Asn Gly Tyr Pro Gln Ser Tyr Ser Met Pro Val Thr Thr Pro Arg Cys
           195                 200                 205
   Thr Val Met Val Glu Glu Ala Pro Ser Arg Ser Gly Lys Lys Arg Lys
       210                 215                 220
   Tyr Ala Cys Thr Leu Pro Gln Cys Gly Lys Ser Phe Ala Gln Lys Thr
   225                 230                 235                 240
   His Leu Asp Ile His Met Arg Ala His Thr Gly Asp Lys Pro Phe Val
                   245                 250                 255
   Cys Lys Glu Pro Ser Cys Gly Gln Arg Phe Ser Gln Leu Gly Asn Leu
               260                 265                 270
   Lys Thr His Gln Arg Arg His Thr Gly Glu Lys Pro Phe Ser Cys Asp
           275                 280                 285
   Ile Cys Gln Lys Arg Phe Ala Gln Arg Gly Asn Val Arg Ala His Lys
       290                 295                 300
   Ile Thr His Gln His Ala Lys Pro Phe Thr Cys Leu Leu Asp Asp Cys
   305                 310                 315                 320
   Gly Lys Gln Phe Thr Gln Leu Gly Asn Leu Lys Ser His Gln Asn Lys
                   325                 330                 335
   Phe His Ala Thr Thr Leu Arg Asn Leu Thr Met Lys Phe Ser Gln Val
               340                 345                 350
   Thr Glu Gly Asp His Met Ser Pro Gln Asp Arg Lys Leu Trp Glu Tyr
           355                 360                 365
   Phe Ala Thr Leu Tyr Lys Asn Ser Asn Lys Gly Ile Lys Gly Arg Gly
       370                 375                 380
   Lys Asp Arg Arg Ile Ser Pro Thr Ser Arg Ser Asp Pro Gly Ser Glu
   385                 390                 395                 400
   Ser Arg Arg Arg Ile Glu Pro Leu Ser Ser Thr Asp Asp Lys Met Arg
                   405                 410                 415
   Arg Ala Ser Tyr Gly Asp Thr Ser Met Tyr Asn Gly Gly Ser Ser Ser
               420                 425                 430
   Asp Asp Asp Asp Ala Glu Pro Tyr Phe Ile Glu Arg Gln Ala His
           435                 440                 445
<210> 130
   <211> 439
   <212> PRT
   <213> Artificial Sequence
<220>
   <223> fusgrFGSG_08617T0
<400> 130
   Met Ser Gly Met Arg Ser Pro Ala Pro Thr Glu Val Met Asp Ile Thr
   1               5                   10                  15
   Asn Met Leu Asn Lys Lys Gly Gln Met Gln Gln Leu Thr Ser Gly Leu
               20                  25                  30
   Leu Asp His His Gln Tyr Gln His Ser Phe Val Lys His Glu Pro Gly
           35                  40                  45
   Met Glu Arg Ser Ala Ser Pro His Gly Ser Glu His Ser Gln Tyr Ser
       50                  55                  60
   Asn Pro His Asn Ile Ala Arg Ala Tyr Pro Ser Pro Ser Thr Met Gln
   65                  70                  75                  80
   Ala Pro Met His Ile Pro Asn Pro Met Pro Ser Ala Met Gly Leu Ser
                   85                  90                  95
   Gly Tyr Pro Glu Met Pro Asn Met Gly Gly Met Pro His Met His Met
               100                 105                 110
   Gln His Met Pro Gln Gln Gln Gln His His Gln Pro Pro Pro Gln Gln
           115                 120                 125
   Pro Ile Lys Ala Tyr Pro Cys Ser Thr Cys Gly Lys Gly Phe Ala Arg
       130                 135                 140
   Arg Ser Asp Leu Ala Arg His Glu Arg Ile His Ser Gly Val Arg Pro
   145                 150                 155                 160
   His Val Cys Asp Trp Pro Gly Cys Gly Lys Gln Phe Ile Gln Arg Ser
                   165                 170                 175
   Ala Leu Thr Val His Gln Arg Val His Thr Gly Glu Lys Pro His His
               180                 185                 190
   Cys Glu Thr Cys Ala Lys Pro Phe Ser Asp Ser Ser Ser Leu Ala Arg
           195                 200                 205
   His Arg Arg Thr His Ser Gly Lys Arg Pro Tyr Lys Cys Pro Tyr Ala
       210                 215                 220
   Asp Cys Gln Lys Thr Phe Thr Arg Arg Thr Thr Leu Thr Arg His Gln
   225                 230                 235                 240
   Asn His His Ser Gly Thr Ile Glu Glu Ala Ala Ala Ala Thr Ala Ala
                   245                 250                 255
   Ala Leu Ala Ala Ser Lys Thr Lys Gly Met Ser Gln Ala Arg Ser Glu
               260                 265                 270
   Ser Asp His Met Ser Asn His Gly Ser Pro Met Thr Thr Pro Ser Pro
           275                 280                 285
   Ser Gln Arg Thr Met Ser Met Ser Pro Ser Val Asp Leu Ser Gly Thr
       290                 295                 300
   Asn Ser Ile Pro Arg His Pro Gly Asp Phe Gln Tyr Leu Gln Gln Thr
   305                 310                 315                 320
   Gly Ser Leu Pro Met His Met Arg Val Gly Ser Pro Thr Ser Thr Ser
                   325                 330                 335
   Ser Gly Gly Tyr Asn Met Met Arg Pro Thr Ser His Pro Thr Ser Tyr
               340                 345                 350
   Gly Pro Pro Pro Thr Leu Glu Pro Asn Leu Asp Asn Ser Gln Gly Thr
           355                 360                 365
   Pro Ser Ser Asn Gly Gly Ser Pro His Met Ala Asn Val Gly Trp Gln
       370                 375                 380
   Ser Pro Ser His Met Ala Ser Pro Ser Gln Asn Asn Ala Ser Tyr Val
   385                 390                 395                 400
   Tyr Pro Asp Pro Ala Asp Ala Tyr Pro Thr Asn Asn Ala Met Asn Gln
                   405                 410                 415
   Met Tyr Tyr Gly Ala Ala Thr His Met Arg Arg Pro Gln Ser Ala Glu
               420                 425                 430
   Pro Gly Leu Val His Met Ala
           435
<210> 131
   <211> 468
   <212> PRT
   <213> Artificial Sequence
<220>
   <223> tubmeGSTUMT00003625001
<400> 131
   Met Asp Ile Leu Glu Leu Ile Asp Asp Arg Glu Gln Thr Val Ala Glu
   1               5                   10                  15
   Ser Ser Pro Arg Pro Phe Cys Cys Pro Glu Gln Ser Cys Asn Lys Ser
               20                  25                  30
   Phe Asn Arg Lys Ser Asp Leu Gln Arg His His Arg Ile His Thr Asn
           35                  40                  45
   Glu Arg Pro Tyr Ser Cys Thr Phe Pro Asn Cys Gly Lys Ser Phe Ile
       50                  55                  60
   Gln Arg Ser Ala Leu Thr Val His Thr Arg Thr His Thr Gly Glu Lys
   65                  70                  75                  80
   Pro His Ser Cys Glu Tyr Ile Gly Cys Gly Lys Cys Phe Ser Asp Ser
                   85                  90                  95
   Ser Ser Leu Ala Arg His Arg Arg Ile His Thr Gly Lys Arg Pro Tyr
               100                 105                 110
   Ser Cys Pro Ile Asp Lys Cys Gly Lys Ser Phe Cys Arg Lys Thr Thr
           115                 120                 125
   Leu Thr Lys His Ala Arg Lys Asn His Gln Leu Arg Ala Glu Asn Asp
       130                 135                 140
   Val Ser Asp Asp Asp Val Glu Glu Ser Asp Asp Asp Asp Leu Ser Pro
   145                 150                 155                 160
   Lys Ala Ser Gly Lys Ala Gly Ser Arg Ala Gln Ser Lys Arg Leu Thr
                   165                 170                 175
   Lys Ala Ser Ala Arg Arg Ala Ala Pro Pro Ser Ala Pro Ser Arg Pro
               180                 185                 190
   Ser Val Leu Arg Thr Ser Ser Phe Ala Glu Pro Leu Thr Pro His Ser
           195                 200                 205
   Pro Gln Ser Thr Val Arg Ser Gly His Ser Ser Arg Asn Thr Ser Phe
       210                 215                 220
   Ser Ala Ala Ser Asp Ser Tyr Arg His Ser Met Pro Met Ala Met Thr
   225                 230                 235                 240
   His Ser Val His Gln His Gln Pro Pro Thr Pro Gln Ser Pro Tyr Tyr
   245 250 255
   Thr Glu Glu Asp Val Gly Asp Gly Arg Glu Ile Ser Pro Asn Ala Ile
   260 265 270
   Ile His Arg Asp Asp Glu Tyr Gly Thr Thr Pro Thr Gly Pro Pro Pro
   275 280 285
   Pro Leu Gln Gly Asn Arg Ser Phe Asp Thr Leu Ser Ile Val Cys Ser
   290 295 300
   Thr Pro Thr Thr His Gln Leu Leu Ala Ala Gln Gln Thr Leu Gln Ser
   305 310 315 320
   Ser Pro Gly Ser Leu Ser Ser Cys Ser Ser Ala Thr Thr Ala Ser Cys
   325 330 335
   Gly Ser Asp Tyr Phe Tyr Arg Ala Pro Gln Pro Thr Ser Ser Gly His
   340 345 350
   Tyr Gln Asn Met Gly Gln Ala Ile Ser Pro Gly Gly Ile Pro Pro Tyr
   355 360 365
   Pro Ala Gln Met Pro Val His Thr Gly Pro Ser Gln His Pro Ile Val
   370 375 380
   Met Tyr Ser Gln Asn Gly Gln His Gln Ser Asn Gly Ile Leu Ser Pro
   385 390 395 400
   His Ser Gln Ala Gln Ala Gln Gln Gln Gln Gln Gln Gln Pro Val Trp
   405 410 415
   Tyr Asp Tyr Pro Ala Tyr Gln Gln Gln Ile Leu Ala Ala Gln Thr Gln
   420 425 430
   Pro Pro Gln His Arg Ile Tyr Tyr Thr Gly Val Pro Val Gln Asp Pro
   435 440 445
   Asn Phe Ile Lys Thr Glu Gln Glu Gln Asn Leu Leu Pro Thr Pro Arg
   450 455 460
   Gly Ser Phe Cys
   465
<210> 132
   <211> 470
   <212> PRT
   <213> Artificial Sequence
<220>
   <223> rhyruNODE_1332_length_15500_cov_80..g6069.t1
<400> 132
   Met Glu Leu Gly Asn Ile Leu Asn Thr Lys Phe Pro Ala Ala Ala Val
   1               5                   10                  15
   Leu Ala Ser Pro Phe Asn Met Gln His Ala Met Gln His Glu Leu Gln
               20                  25                  30
   Gln Gln His Phe Ala Pro Thr Tyr Leu Asn Gly Arg Ile Lys Ser Glu
           35                  40                  45
   Thr Gly Ser Asp Arg Gly Val Ser Pro His Pro Ser Asp Ser Ser Arg
       50                  55                  60
   Tyr Ser Ser Gln Ala Pro Gln Pro Leu Gln Ser Tyr Pro Pro Pro Met
   65                  70                  75                  80
   Ser Asn Asn Met Asn Gly Met Arg Tyr Pro Ser Pro Ser Gln Met Gln
                   85                  90                  95
   Thr Pro Met Pro Leu Leu Asn Asn Asn Asn Tyr Ile Pro Asn Pro Pro
               100                 105                 110
   Glu His Thr Tyr Pro Gln Gln Pro Thr Pro Asp Pro Gln Gln Gln Met
           115                 120                 125
   Gly Gly Arg Pro Ala Ser Asp Thr Gly Pro Pro Lys Ala Phe Ala Cys
       130                 135                 140
   Ser Thr Cys Ser Lys Gly Phe Ala Arg Arg Ser Asp Leu Ala Arg His
   145                 150                 155                 160
   Glu Arg Ile His Ser Gly Ile Arg Pro His Val Cys Asp His Pro Gly
                   165                 170                 175
   Cys Gly Lys Gln Phe Ile Gln Arg Ser Ala Leu Thr Val His Gln Arg
               180                 185                 190
   Val His Thr Gly Glu Lys Pro His Met Cys Glu Arg Cys Gly Lys Pro
           195                 200                 205
   Phe Ser Asp Ser Ser Ser Leu Ala Arg His Arg Arg Ile His Ser Gly
       210                 215                 220
   Lys Arg Pro Tyr Lys Cys Pro Tyr Ala Asp Cys Gln Lys Thr Phe Thr
   225                 230                 235                 240
   Arg Arg Thr Thr Leu Thr Arg His Gln Asn His His Thr Gly Thr Val
                   245                 250                 255
   Glu Glu Ala Ala Ala Ala Thr Ala Ala Ala Leu Ala Ser Arg Ala Ala
               260                 265                 270
   Asn Pro Asn Arg Ser Ala Arg Ser Glu Ala Asp Glu Phe Ser Glu Thr
           275                 280                 285
   Ala Ser Pro Leu Gly Thr Pro Ser Pro Asn Glu Arg Pro Leu Ser Leu
       290                 295                 300
   Ser Pro Ala Ala Gly Met Pro Val Val Pro Asn Met His Arg Gln Ala
   305                 310                 315                 320
   Ser Asp Tyr Ala Tyr Leu Gly Asn Met Asn Val Pro Pro His Leu Arg
                   325                 330                 335
   Asn Glu Ile Pro Gln Pro Ser Pro Arg Ser Ser Pro Ser Leu Thr Ala
               340                 345                 350
   Gln Ser Tyr Asn Ser Ala Val Ser Asn Gly Arg Pro Ala Ile Thr Ser
           355                 360                 365
   His Pro Ser Gly Tyr Gly Pro Pro Ser Ile Leu Glu Pro Pro Ala Ser
       370                 375                 380
   Ala Thr His Asn Gln Pro Gly Ser Ala Asn Gly Ser Pro His Met Gly
   385                 390                 395                 400
   Thr Met Gly Trp Gln Ser Pro Ser Gln Gln Ala Met Pro Ser Pro Gly
                   405                 410                 415
   Ala Gly Asp Ser Tyr Val Tyr Pro Glu Pro His Tyr Gln Ser Asn Gly
               420                 425                 430
   Gly Asn Met Tyr Tyr Gln Asn Ala Gly Ile Gln Arg Pro Asn Ser Thr
           435                 440                 445
   Glu Pro Asp Arg Tyr Asn Pro Gln Gln Gln Arg Met Thr Asn Glu Met
       450                 455                 460
   Trp Ala Pro Ala Val Gln
   465 470
<210> 133
   <211> 491
   <212> PRT
   <213> Artificial Sequence
<220>
   <223> colgrGLRG_03066T0
<400> 133
   Met Ala Leu Pro Glu Gln Thr Ala Ala Pro Gln Ala Trp Gly Arg Trp
   1               5                   10                  15
   Gln Gln Gln Pro Ala Gly His Asp Tyr Gly Met Met Asp Asn Ser Thr
               20                  25                  30
   Phe Val Gln Tyr Asp Ser Arg Ala Ala Thr Ser Ala Pro Met Asn Gly
           35                  40                  45
   Ala Ile Met Ala Pro Gln Tyr Met Val Ala Pro Gln Tyr Asn Met Thr
       50                  55                  60
   Gln Met Pro Ala Met Gly His Gln Cys Pro Thr Gln Asn His Phe Ala
   65                  70                  75                  80
   Tyr Thr Gln Tyr Asp Ser Ser Pro Thr Asn Met Asn Met Pro Phe Arg
                   85                  90                  95
   Leu Pro Pro Gln Pro Glu Arg Pro Val Met Pro Val Ala Ala Ser Glu
               100                 105                 110
   His Asp Ile Pro Arg Ala Ser Ser Tyr Pro Gln Glu Ser Pro Ser Val
           115                 120                 125
   Lys Asn Asp Arg His Arg Ser Pro Ser Ser Arg Ser Glu Thr Lys Met
       130                 135                 140
   Ser Asn Pro Lys Thr Pro His Pro Leu Asn Thr Lys Glu Ile Lys Tyr
   145                 150                 155                 160
   Asn Lys Pro Thr Ser Ala Asp Pro Val Asn Phe Thr Thr Ala Ile Asp
                   165                 170                 175
   Thr Leu Met Lys Ala Ile Gln Lys Arg Asn Asp Ser Glu Asp Ile Val
               180                 185                 190
   Lys Gly Val Pro Glu Ile Glu Gln Val Val Lys Pro Glu Arg Met Leu
           195                 200                 205
   Ser Leu Glu Pro Glu Ala Gly Ser Ile Ala Pro Val Gln Ala Pro Ala
   210 215 220
   Pro Ile Ala Thr Glu Ser Thr Asp Ala Pro Lys Pro Lys Arg Tyr Ile
   225 230 235 240
   Cys Asp Val Glu Gly Cys Gly Lys Ser Phe Tyr Gln Ser Thr His Leu
   245 250 255
   Asp Thr His Arg Arg Ala His Thr Gly Glu Lys Pro Tyr Gln Cys Asn
   260 265 270
   Trp Pro Arg Cys Gly Arg Thr Phe Ser Gln Pro Gly Asn Leu Lys Thr
   275 280 285
   His Met Arg Arg His Thr Gly Glu Lys Pro Phe Arg Cys Glu Gln Cys
   290 295 300
   Ser Lys Val Phe Ala Gln Arg Gly Asn Leu Gln Thr His Met Ala Thr
   305 310 315 320
   His Thr Asn Ala Lys Pro Phe Val Cys Lys Leu Asp Asp Cys Asn Lys
   325 330 335
   Met Phe Thr Gln Arg Gly Asn Leu Lys Asn His Gln Asn Lys Tyr His
   340 345 350
   Glu Lys Thr Leu Met Glu Met Thr Asp Trp Ile Val Ser Ile Ser Asp
   355 360 365
   Ile Asp Ala Leu Ser Asp Asp Gln Arg Glu Met Tyr Trp Tyr Phe Ala
   370 375 380
   Asn Leu Tyr Lys Asn Ser Asn Lys Gly Ile Lys Gly Arg Gly Lys Asp
   385 390 395 400
   Arg Arg Val Ser Asn Arg Gly Ala Lys Ser Arg Thr Ala Pro Ser Ser
   405 410 415
   Tyr Pro Val Lys Arg Val Pro Gly Pro Ala Ser Leu Ala Ala Tyr Ser
   420 425 430
   Ser Lys Arg Leu Pro Pro Pro Glu Gln Tyr Met Gln Gln Gln Gln His
   435 440 445
   His Gln His Gly Tyr Glu Val Tyr Asp Thr Asp Met Asp Gln Ser Ser
   450 455 460
   Ser Ala Ser Ser Pro Arg Tyr Asp Gly Leu Pro Ala Thr Tyr Arg Asp
   465 470 475 480
   Arg Met Tyr Gln Gln Ser Thr His Ala Met Tyr
   485 490
<210> 134
   <211> 397
   <212> PRT
   <213> Artificial Sequence
<220>
   <223> ML_117645
<400> 134
   Met Arg Leu Ser Glu Ala Asp Ala Pro Leu Glu Val Lys Ala Ala Arg
   1               5                   10                  15
   Pro Phe Ala Cys Thr Tyr Asp Ala Cys Gln Lys Ser Phe Ser Arg Arg
               20                  25                  30
   Ser Asp Leu Val Arg His Val Arg Ile His Thr Asn Glu Arg Pro Phe
           35                  40                  45
   Lys Cys Asp Phe Leu Ser Cys Gly Lys Cys Phe Ile Gln Arg Ser Ala
       50                  55                  60
   Leu Thr Val His Met Arg Thr His Thr Gly Glu Arg Pro His Val Cys
   65                  70                  75                  80
   Glu Leu Cys Gly Arg Ala Phe Ser Asp Ser Ser Ser Leu Ala Arg His
                   85                  90                  95
   Arg Arg Val His Thr Gly Lys Arg Pro Tyr Arg Cys Pro Phe Asn Gly
               100                 105                 110
   Cys Asp Lys Thr Phe Cys Arg Lys Thr Thr Leu Ile Lys His His Lys
           115                 120                 125
   Gly His Thr Glu Asp Gly Leu Leu Leu Asn Ser Pro Asp Tyr Ser Ser
       130                 135                 140
   Lys Ser Ser Pro Thr Gly Asp Ser Thr Ser Ala Ser Ser Ser Ser Thr
   145                 150                 155                 160
   Met Thr Pro Leu Ser Thr Asn Phe Ser Phe Ala Thr Tyr Ser Ser His
                   165                 170                 175
   Pro Glu Gln Ile Tyr Ser Ala Pro Ala Ser Ser Gln Tyr Phe Asn Ile
   180 185 190
   Pro Ala Leu His His Ser Gln Asp Ser Asn Phe Ser Trp Gly Asn Pro
   195 200 205
   Ser Asn Gln Phe Ser Pro Ser Pro Ser Phe Pro Arg Pro Asn Asn Thr
   210 215 220
   Thr Ser Gly Trp Ser Ser Arg Ala Pro Leu Ser Glu Phe Pro Met Thr
   225 230 235 240
   Ser Ile Ser Pro Glu Tyr Asn Arg Phe Pro Arg Glu Thr Ala Phe Asn
   245 250 255
   Asn Pro Pro His Ser Thr Asp Ala Thr Ser Ser Ser Arg Ser Phe Ser
   260 265 270
   Val Ser Gln Gly Tyr Gly Ser Asn Pro Gln Asp Asn Ile Leu Pro Asp
   275 280 285
   Ile Lys Pro Ser Phe Pro Phe Pro Pro Thr Thr Leu His Gln Ser Ala
   290 295 300
   Pro Thr Val Tyr His Asp Thr Asn Pro Tyr Thr Ser Ser Phe Ala Asn
   305 310 315 320
   Thr Val Val Lys Glu Val Pro Asn Glu Pro Ser Tyr Ser Ser Gly Thr
   325 330 335
   His Met Leu Asp Val Gly Arg His Thr Asn Val Ala Asp Phe Asn Asn
   340 345 350
   Arg Glu Met Leu Pro Pro Leu Gly Asn Ile Pro Ile Gln Pro Leu Ser
   355 360 365
   Leu Pro Arg Met Ser Asn Ile Asp Leu Ser Ser Ser Lys Leu Asn Gly
   370 375 380
   Ile Gly Ser Tyr Trp Lys Ser Glu Ser Thr Trp Glu Gly
   385 390 395
<210> 135
   <211> 468
   <212> PRT
   <213> Artificial Sequence
<220>
   <223> stano9345
<400> 135
   Met Ala Ala Val Val Ala His Gln Gln Ala Ala Leu Trp His Arg Arg
   1               5                   10                  15
   Leu Asp His Phe His Ile Pro Asp Val His Phe Thr Gly Met Met Pro
               20                  25                  30
   Gln Tyr Glu Asn Gln Arg Thr Gly Gly Ser Ala Pro Thr Ser Arg Ser
           35                  40                  45
   Phe Leu Pro Ala Thr Thr Gln Met Asp Leu Ser Leu Pro Leu Phe Ser
       50                  55                  60
   Ala Asn Gly Leu Pro Thr Ser Val Pro Tyr Gln Ser Ser Gly Thr Phe
   65                  70                  75                  80
   Ala Tyr Gly Ser Ser Val Asp Pro Tyr Asn Met Gln Gln Ser Asn Met
                   85                  90                  95
   Gln His Ser Tyr Ser Met Asn Tyr Thr Ser Asn Leu Ser Pro Ala Val
               100                 105                 110
   Ser Tyr Ala Asp Arg Thr Glu His Gln Ser Leu Ala Asn Val His Glu
           115                 120                 125
   Ala Arg His Ala Phe Ala Leu Asp Asn Ala His Leu Val Lys Ala Glu
       130                 135                 140
   Ser Ala Ser Pro Ala Gln Ser Ser Pro Val Tyr Asn Asn Thr Ser Tyr
   145                 150                 155                 160
   Thr Ser Glu Cys Lys Arg Ser Ser Ser Glu Pro Met Asp Pro Ser Asn
                   165                 170                 175
   Ile Asn Phe Ala Thr Asp Val Asp Thr Leu Met Lys Ala Ile Gln Ala
               180                 185                 190
   Lys Gln Thr Thr Ser Thr Gln Gln Gln Glu Ala Pro Pro Lys Arg Glu
           195                 200                 205
   Glu Pro Lys Val Ser Gln Lys Pro Arg Lys Arg Tyr Gln Cys His Met
       210                 215                 220
   Pro Asp Cys Asn Lys Ser Phe Phe Gln Lys Thr His Leu Glu Ile His
   225                 230                 235                 240
   Ile Arg Ala His Thr Gly Ala Lys Pro Phe Glu Cys Lys Ala Pro Gly
                   245                 250                 255
   Cys Gly Gln Arg Phe Ser Gln Leu Gly Asn Leu Lys Thr His Glu Arg
               260                 265                 270
   Arg His Thr Gly Glu Arg Pro Tyr Ser Cys Asp Ile Cys Gly Lys Thr
           275                 280                 285
   Phe Ala Gln Arg Gly Asn Val Arg Ala His Lys Ile Val His Gln Gln
       290                 295                 300
   Ile Lys Pro Phe Thr Cys Arg Leu Asp Asp Cys Gly Lys Gln Phe Thr
   305                 310                 315                 320
   Gln Leu Gly Asn Leu Lys Ser His Gln Asn Lys Phe His Ala Ser Thr
                   325                 330                 335
   Leu Lys Tyr Leu Thr Gln Lys Phe Ala Thr Ile Thr Pro Gly Asp Tyr
               340                 345                 350
   Val Ser His Ala Asp Lys Glu Leu Trp Glu Tyr Phe Ala Ser Leu Tyr
           355                 360                 365
   Lys Asn Ser Asn Lys Gly Ile Lys Gly Arg Gly Lys Asp Arg Arg Ile
       370                 375                 380
   Ser Ala Met Ser Ser Ser Ala Ser Val His Pro Leu Ser Tyr Ser Thr
   385                 390                 395                 400
   Met Pro Thr Ser Asn Asn Arg Ser Tyr Pro Gly Leu Tyr Asn His Asn
                   405                 410                 415
   Gly Ser Asp Arg Ser Ser Arg Ser Ser Ser Ala Cys Thr Asp Leu Ser
               420                 425                 430
   Asn Gln Arg Pro Glu Pro Asn Tyr Asp Phe Asn Ala Pro Met Gln Ala
           435                 440                 445
   Gly Tyr Pro Thr Gln Gly Thr Gly Tyr Asp Asp Met Val Phe Pro Glu
       450                 455                 460
   Arg Lys Met Tyr
   465
<210> 136
   <211> 452
   <212> PRT
   <213> Artificial Sequence
<220>
   <223> aspacfgenesh1_pm.14_#_237
<400> 136
   Met Glu Ala Ala Glu Pro Val Ser Tyr Glu Phe Pro Gly His Ala Ile
   1               5                   10                  15
   Gly Ala Val Ala Pro Arg Arg Met Met Asn Thr Gly Leu Gly His Thr
               20                  25                  30
   Leu Pro Phe Tyr Pro Gly Pro Thr Ala Ser Phe Pro Leu Ser Phe His
           35                  40                  45
   Gln Gln Ser Ser Ala Ser Tyr Gly Phe Gly His Pro Met Asn Gln His
       50                  55                  60
   His His His Pro Gln Gln His His His His Gln Thr Ser Tyr Gln His
   65                  70                  75                  80
   Tyr Phe Val Pro Gly Gln Gln Pro Leu Asn Ser Gln Pro Val Arg Leu
                   85                  90                  95
   Leu Ser Glu Pro Pro Ser Ile Gln Pro Ile Pro Glu Ile Arg Pro Ala
               100                 105                 110
   Lys Asn Ala Val Ser Arg Met Met Lys Asp Ala Ser Ala Ser Ile Gly
           115                 120                 125
   His Lys Ser Ser Val Gln Gln Pro Leu Gly Gln His Pro Thr Ser Gly
       130                 135                 140
   Gly Pro Thr Gln Gly Arg Ser Pro Cys Pro Asn Glu Ile Glu Phe Ser
   145                 150                 155                 160
   Thr Glu Val Asp Ile Leu Met Lys Ala Ile Gln Ser Lys Ala Ser Thr
                   165                 170                 175
   Gln Ser Ala Ile Val Gln Ser Leu Pro Pro Leu Gln Gln Met Thr His
               180                 185                 190
   Gly Val Asn Pro Ser Thr Phe Ala Pro Gln Tyr Ser Ile Gln Ala Ala
           195                 200                 205
   Thr Asn Pro Arg Cys Thr Val Ile Val Glu Asp Gln Pro Leu Leu Ser
       210                 215                 220
   Ala Lys Lys Arg Lys Tyr Ala Cys Thr Leu Pro His Cys Gly Lys Ser
   225 230 235 240
   Phe Ala Gln Lys Thr His Leu Asp Ile His Met Arg Ala His Thr Gly
   245 250 255
   Asp Lys Pro Phe Val Cys Lys Glu Pro Leu Cys Gly Gln Arg Phe Ser
   260 265 270
   Gln Leu Gly Asn Leu Lys Thr His Gln Arg Arg His Thr Gly Glu Lys
   275 280 285
   Pro Phe Ser Cys Glu Ile Cys His Lys Arg Phe Ala Gln Arg Gly Asn
   290 295 300
   Val Arg Ala His Lys Ile Thr His Gln His Ala Lys Pro Phe Thr Cys
   305 310 315 320
   Leu Leu Asp Asp Cys Gly Lys Gln Phe Thr Gln Leu Gly Asn Leu Lys
   325 330 335
   Ser His Gln Asn Lys Phe His Ala Ala Thr Leu Lys Ile Leu Thr Leu
   340 345 350
   Lys Phe Ser Gln Val Thr Asp Ala Asp Asn Met Ser Ser Gln Asp Gln
   355 360 365
   Lys Leu Trp Glu Tyr Phe Ala Thr Leu Tyr Lys Asn Ser Asn Lys Gly
   370 375 380
   Ile Lys Gly Arg Gly Lys Asp Arg Arg Ile Ser Pro Thr Ala Arg Ser
   385 390 395 400
   Glu Thr Arg Pro Asp Thr Arg Arg Arg Leu Gln Ser Leu Thr Asn Ser
   405 410 415
   Asp Gly Lys Gly Arg Arg Ala Ser Tyr Gly Glu Met Ser Thr Cys Asn
   420 425 430
   Gly Ala Ser Ser Ser Asp Glu Glu Asp Ile Gly Pro Tyr Phe Leu Asp
   435 440 445
   Arg Gln Gly His
   450
<210> 137
   <211> 479
   <212> PRT
   <213> Artificial Sequence
<220>
   <223> dotsefgenesh1_kg.4_#_271_#_isotig02754
<400> 137
   Met Gln Tyr Glu Val Pro Met Pro Ile Thr Met Ser His Asp Met Ser
   1               5                   10                  15
   Thr Ala Thr Gly His Tyr Ala His Gln Asn Leu Tyr Val Pro Asn Asn
               20                  25                  30
   Asn Ala Asn Arg Ile Lys Ser Glu Thr Gly Ser Glu Arg Gly Val Ser
           35                  40                  45
   Pro His Ser Asn Ser Asp His Ser Arg Tyr Ser Ser Gln Thr Pro His
       50                  55                  60
   Asn Asn Ser Val Ala Tyr Gln Gln Ile Ala Ala Gln Leu Gln Asn Gly
   65                  70                  75                  80
   Met Arg Tyr Pro Ser Pro Asn Gly Leu Gln Gln Gln Asn Gly Met Ser
                   85                  90                  95
   Met Leu Gln His Asn Tyr Gln Pro Ser Ala Thr Pro Glu Gln Pro Phe
               100                 105                 110
   Gln Gln Gln Gln Ser Ala Ser Met Gly Ala Val Gln Gln Ala Val Ser
           115                 120                 125
   Gln Pro Gln Gln Glu Gln Ser Pro Met Glu Gly Gly Arg Ala Ser Thr
       130                 135                 140
   Gly Ser Thr Gly Leu Pro Lys Ala Phe Ala Cys Ser Thr Cys Gln Lys
   145                 150                 155                 160
   Gly Phe Ala Arg Arg Ser Asp Leu Ala Arg His Glu Arg Ile His Ser
                   165                 170                 175
   Gly Val Arg Pro His Val Cys Asp His Pro Gly Cys Gly Lys Gln Phe
               180                 185                 190
   Ile Gln Arg Ser Ala Leu Thr Val His Ser Arg Val His Thr Gly Glu
           195                 200                 205
   Lys Pro His Met Cys Glu Arg Cys Gly Lys Pro Phe Ser Asp Ser Ser
       210                 215                 220
   Ser Leu Ala Arg His Arg Arg Ile His Ser Gly Lys Arg Pro Tyr Lys
   225                 230                 235                 240
   Cys Pro Tyr Ala Asp Cys Gln Lys Thr Phe Thr Arg Arg Thr Thr Leu
                   245                 250                 255
   Thr Arg His Gln Asn His His Thr Gly Thr Ile Glu Glu Ser Glu Ala
               260                 265                 270
   Ala Thr Ala Ala Ala Leu Ala Ser Arg Val Ser Leu Gln Asn Ser Arg
           275                 280                 285
   Ser Arg Gly Ser Asp Glu Glu Asn Asp Tyr Ser Asp Gly Lys Ser Pro
       290                 295                 300
   Met Pro Asn Ala Ser Glu Arg Thr Ala Thr Pro Val Asn Gly Asn Gln
   305                 310                 315                 320
   Asn Leu Gln Arg Gln Ala Ser Asp Tyr Tyr Met Asn Ala Met Gln Gly
                   325                 330                 335
   Gly Met Ala Ala Val Pro Thr His Ile Arg Ala Glu Met Pro Asn Gly
               340                 345                 350
   Val Pro Ser Arg Ala Ala Ser Pro Ala Gln Tyr Pro Met Pro Ala Val
           355                 360                 365
   Asn Gly Gln Gln Gln Arg Pro Pro Leu Thr Ser Asn Pro Ser Ser Gly
       370                 375                 380
   Tyr Asn Pro Pro Gln Ile Met Glu Pro Ser Val Gly Asn Gly Gln Gln
   385                 390                 395                 400
   Thr Gly Ser Gly Thr Asn Ser Pro His Met Gln Gly Trp Gln Ser Pro
                   405                 410                 415
   His Thr Gly Val Gln Ala Gln Pro Asn Asp Tyr Ser Tyr Thr Asp Ala
               420                 425                 430
   Asn Asn Asn Tyr Asn Gly Thr Asn Val Asn Val Leu Gln Gln Tyr Tyr
           435                 440                 445
   Thr Thr Pro Asp Val Gln Arg Pro His Ser Thr Gly Pro Val Gly Tyr
       450                 455                 460
   His Asn Gln Met Arg Gly Gln Glu Met Trp Ala Gln His Gln Gln
   465 470 475
<210> 138
   <211> 468
   <212> PRT
   <213> Artificial Sequence
<220>
   <223> tubmeGSTUMT00001918001
<400> 138
   Met Asp Leu Thr Asn Ile Leu Asn Thr Lys Asp Ala Ala Ala Ala Ala
   1               5                   10                  15
   Ala Gly Ala Ala Pro Pro Leu Thr Leu Ser Leu Glu Met Gln Lys Asn
               20                  25                  30
   Pro Tyr Leu Ala Asn Pro Gly Ala His Pro His Ser Pro Ala Ala Ser
           35                  40                  45
   Asp Phe Ile Ser Asp Arg Glu Gly Ser Pro His Ser Ser Thr Ser Pro
       50                  55                  60
   Ser Ala Ser Glu His Ser Pro Ser Ala Tyr Pro Leu Pro Ser Asn Ala
   65                  70                  75                  80
   Leu Val Ser Ala Asn Gly Ser Tyr Ser Gly Val Pro Ser Gly Ala Val
                   85                  90                  95
   Asn Thr Val Pro Arg Gly Pro Gly Arg Pro Ser Ser Gly Asp Pro Tyr
               100                 105                 110
   Ser Lys Ala Phe Pro Cys Ser Val Cys Ser Lys Gly Phe Ala Arg Arg
           115                 120                 125
   Ser Asp Leu Ala Arg His Glu Arg Ile His Thr Gly Val Arg Pro His
       130                 135                 140
   Val Cys Asp Tyr Pro Gly Cys Asp Lys Gln Phe Ile Gln Arg Ser Ala
   145                 150                 155                 160
   Leu Thr Val His Ala Arg Val His Thr Gly Glu Lys Pro His Met Cys
                   165                 170                 175
   Asp Thr Cys Gly Lys Pro Phe Ser Asp Ser Ser Ser Leu Ala Arg His
               180                 185                 190
   Arg Arg Ile His Ser Gly Lys Arg Pro Tyr Lys Cys Pro Tyr Ala Asp
           195                 200                 205
   Cys Gln Lys Thr Phe Thr Arg Arg Thr Thr Leu Thr Arg His Gln Asn
   210 215 220
   His His Thr Gly Thr Ile Ser Glu Ala Ala Ala Ala Thr Ala Ala Ala
   225 230 235 240
   Leu Ala Ala Arg Pro Thr Leu Pro Lys Pro Gly Arg Pro Pro Arg Ser
   245 250 255
   Pro Asn Ala Arg Pro Leu Gly Arg Pro Pro Asn Asp Ser Asn Pro Ser
   260 265 270
   Ser Ala His Ser Thr Pro Ser Pro Gly Gln Arg His Ala Ser Leu Ser
   275 280 285
   Pro Ser Thr Glu Leu Pro Pro Pro Phe Ser Ala His Arg Gln Gly Gly
   290 295 300
   Asp Tyr His Tyr Arg Gln Ser Asn Ile Thr Ser His Pro Ser Thr Ser
   305 310 315 320
   Leu Met Asn Glu Leu Gln Pro Leu Thr Pro Arg Pro Thr Pro Thr Thr
   325 330 335
   Thr Pro Thr Ile Ser Ser Gly Met Val Pro Ala Gly Pro Arg Gln Pro
   340 345 350
   Pro Thr Ser Asn Pro Thr Phe Met Ser Ser Leu Pro Pro Ile Leu Glu
   355 360 365
   Pro Pro Pro Lys Pro Asp Ala Gln Arg Gln Gly Ser Pro His Leu Ser
   370 375 380
   Gly Ser Pro His Met Ser Ala Ala Gly Ser Pro His Met Ser His Pro
   385 390 395 400
   Ser Pro Leu Phe Ser Pro Gly Tyr Arg Ser Pro Ser Cys His Ser Thr
   405 410 415
   Val Ser Glu Asp Thr Val Phe Phe Pro Pro His Asn Ser Phe Gln Ser
   420 425 430
   Pro Val Leu Gly Ser His Tyr Gly Ser Asp His Leu Arg Arg Pro His
   435 440 445
   Ser Ser Asp Ser Leu His Ser Tyr Glu Gln Arg Glu Val Arg Leu Pro
   450 455 460
   Gly Val Tyr Pro
   465
<210> 139
   <211> 492
   <212> PRT
   <213> Artificial Sequence
<220>
   <223> colhiCH063_11465T0
<400> 139
   Met Ala Leu Thr Glu Gln Thr Ala Thr Pro Gln Ala Trp Gly Arg Trp
   1               5                   10                  15
   Gln Gln Gln Pro Thr Gly His Gly Tyr Ala Met Met Asp Asn Ser Thr
               20                  25                  30
   Phe Val Pro Tyr Glu Ser Arg Ala Ala Thr Ser Ala Pro Met Asn Gly
           35                  40                  45
   Ala Ile Met Ala Pro Gln Tyr Met Val Ala Pro Gln Tyr Asn Met Thr
       50                  55                  60
   Pro Met Pro Ala Met Gly His Cys Thr Ser Gln Asn His Phe Ala Tyr
   65                  70                  75                  80
   Ala Gln Tyr Asp Ser Pro Pro Thr Asn Met Asn Met Pro Phe Arg Leu
                   85                  90                  95
   Pro Ala His Gln Glu Arg Pro Val Met Pro Val Ala Ala Pro Glu His
               100                 105                 110
   Asp Ile Pro Arg Ala Ser Ser Tyr Pro Gln Glu Ser Pro Ser Ser Gln
           115                 120                 125
   Asn Asp Arg His Ser Ser Pro Ser Thr Arg Ser Glu Thr Lys Met Ser
       130                 135                 140
   Asn Pro Lys Thr Pro His Pro Leu Asn Thr Lys Asp Ile Lys Phe Asn
   145                 150                 155                 160
   Lys Pro Thr Ser Ala Glu Pro Val Asn Phe Thr Thr Ala Ile Asp Thr
                   165                 170                 175
   Leu Met Lys Ala Ile Gln Lys Arg Arg Asp Ser Glu Asp Ile Val Lys
               180                 185                 190
   Gly Val Pro Glu Ile Glu Gln Leu Val Lys Pro Glu Pro Arg Ser Pro
           195                 200                 205
   Lys Pro Glu Ala Gly Pro Val Thr Ser Val Pro Thr Pro Ala Pro Ala
       210                 215                 220
   Ala Thr Glu Ser Thr Asp Thr Pro Lys Pro Lys Arg Tyr Val Cys Thr
   225                 230                 235                 240
   Ile Asp Gly Cys Gly Lys Ser Phe Tyr Gln Ser Thr His Leu Asp Thr
                   245                 250                 255
   His Arg Arg Ala His Thr Gly Glu Lys Pro Tyr Gln Cys Asn Trp Pro
               260                 265                 270
   Arg Cys Gly Arg Thr Phe Ser Gln Pro Gly Asn Leu Lys Thr His Met
           275                 280                 285
   Arg Arg His Thr Gly Glu Lys Pro Phe Arg Cys Glu Gln Cys Ser Lys
       290                 295                 300
   Val Phe Ala Gln Arg Gly Asn Leu Gln Thr His Met Ala Thr His Thr
   305                 310                 315                 320
   Asn Ala Lys Pro Phe Val Cys Lys Leu Asp Asp Cys Asn Lys Met Phe
                   325                 330                 335
   Thr Gln Arg Gly Asn Leu Lys Asn His Gln Asn Lys Tyr His Glu Lys
               340                 345                 350
   Thr Leu Met Glu Met Thr Asp Trp Ile Val Ser Ile Ser Asp Ile Asp
           355                 360                 365
   Ala Leu Ser Asp Asp Gln Arg Glu Met Tyr Trp Tyr Phe Ala Asn Leu
       370                 375                 380
   Tyr Lys Asn Ser Asn Lys Gly Ile Lys Gly Arg Gly Lys Asp Arg Arg
   385                 390                 395                 400
   Val Ser Asn Arg Gly Ser Lys Ser Arg Thr Ala Pro Ser Ser Tyr Pro
                   405                 410                 415
   Val Lys Arg Val Pro Gly Pro Ser Ser Leu Ala Ala Tyr Ser Ser Lys
               420                 425                 430
   Arg Leu Pro Pro Pro Glu Gln Tyr Met Gln Gln Gln Gln Gln Gln Gln
           435                 440                 445
   His His Gln Gln Ser Tyr Glu Val Tyr Asp Thr Asp Met Asp Gln Ser
       450                 455                 460
   Ser Ser Ala Ser Ser Pro Arg Tyr Asp Gly Ile Pro Val Ala Tyr Arg
   465                 470                 475                 480
   Asp Arg Met Tyr Gln Gln Ser Thr His Ala Met Tyr
                   485                 490
<210> 140
   <211> 453
   <212> PRT
   <213> Artificial Sequence
<220>
   <223> TRI38080
<400> 140
   Arg Ser Asn Ala Val Met Ser Ser Ala Ser Pro Glu Val Met Asp Ile
   1               5                   10                  15
   Thr Asn Met Leu Asn Asn Lys Gly Gly Ala Met Thr His Gly Leu Gly
               20                  25                  30
   Gly Gly Met His Asp His His Gln His Gln His His His Leu Gly Leu
           35                  40                  45
   Val Lys His Glu Pro Thr Met Asp Arg Ser Gly Ser Pro His Met Ser
       50                  55                  60
   Glu His Ser Ser Tyr Ser Ala His Ser Met Ser Arg Ala Tyr Pro Ser
   65                  70                  75                  80
   Pro Thr Ala Met Gln Ala Pro Met Gln Ile Pro Asn Ser Met His Asn
                   85                  90                  95
   Ala Met Gln Met Gly Gly Phe Ala Asp Met Ser Gly Met Gly Gly Val
               100                 105                 110
   Pro Ser Met Ala Met His His Ile Pro Gln His Pro Pro Gln Gln Gln
           115                 120                 125
   Gln Gln Gln Gln Gln Gln Gln Pro Gln Gln Pro Gln Ala Pro Val Lys
       130                 135                 140
   Ala Tyr Pro Cys Ser Thr Cys Gly Lys Gly Phe Ala Arg Arg Ser Asp
   145                 150                 155                 160
   Leu Ala Arg His Glu Arg Ile His Thr Gly Val Arg Pro His Val Cys
                   165                 170                 175
   Asp Tyr Pro Lys Cys Asn Lys Gln Phe Ile Gln Arg Ser Ala Leu Thr
               180                 185                 190
   Val His Gln Arg Val His Thr Gly Glu Lys Pro His His Cys Glu Thr
           195                 200                 205
   Cys Ala Lys Pro Phe Ser Asp Ser Ser Ser Leu Ala Arg His Arg Arg
       210                 215                 220
   Thr His Ser Gly Lys Arg Pro Tyr Lys Cys Pro Tyr Ala Asp Cys Gln
   225                 230                 235                 240
   Lys Thr Phe Thr Arg Arg Thr Thr Leu Thr Arg His Gln Asn His His
                   245                 250                 255
   Thr Gly Thr Ile Glu Glu Ala Ala Ala Ala Thr Ala Ala Ala Leu Ala
               260                 265                 270
   Ala Ser Arg Ala Lys Asn Gly Ser Gln Ala Arg Ser Asp Gly Asp His
           275                 280                 285
   Met Ser Ser His Gly Ser Pro Leu Ser Thr Pro Ser Pro Ser Gln His
       290                 295                 300
   Gly Met Met Ser Pro Ala Leu Asp Leu Ser Gly Ser Asn Gly Ile Pro
   305                 310                 315                 320
   Arg His Ala Ala Asp Phe Gln Gln Tyr Ile Thr Gln Ser Gly Gly Leu
                   325                 330                 335
   Pro Pro His Leu Arg Val Gly Ser Pro Thr Ser Thr Thr Ser Ala Ala
               340                 345                 350
   Ser Tyr Asn Thr Gly Ile Arg Pro Thr Ser His Pro Thr Gly Tyr Gly
           355                 360                 365
   Pro Pro Pro Thr Leu Glu Pro Asn Leu Glu Gln His Pro Ala Gly Ser
       370                 375                 380
   Gly Ser Ala Gly Gly Ser Pro His Met Ser Gln Val Ser Trp Gln Ser
   385                 390                 395                 400
   Pro Gln Thr His Ser Pro Ser His Asn Gly Gly Ser Tyr Ile Tyr Pro
   405 410 415
   Asp Pro Asp Gly Gly Tyr Pro Pro Asn Pro Ala Met Ser Gln Met Tyr
   420 425 430
   Tyr Gly Ala Pro Gln Gln Met Arg Arg Pro Gln Ser Ala Glu Pro Gly
   435 440 445
   Leu Val His Met Ala
   450
<210> 141
   <211> 1437
   <212> PRT
   <213> Artificial Sequence
<220>
   <223> région promotrice du gène glyceraldehyde-3-phosphate déshydrogénase (GPDp) de T. reesei
<400> 141
   Gly Ala Cys Gly Cys Ala Gly Ala Ala Gly Ala Ala Gly Gly Ala Ala
   1               5                   10                  15
   Ala Thr Cys Gly Cys Cys Cys Cys Gly Cys Cys Gly Gly Thr Thr Cys
               20                  25                  30
   Cys Gly Thr Ala Ala Ala Ala Ala Ala Ala Ala Thr Ala Thr Gly Ala
           35                  40                  45
   Gly Cys Gly Cys Ala Gly Gly Gly Ala Cys Ala Ala Gly Cys Ala Cys
       50                  55                  60
   Ala Gly Cys Cys Thr Thr Gly Gly Cys Cys Cys Thr Gly Gly Gly Cys
   65                  70                  75                  80
   Cys Thr Ala Gly Cys Cys Thr Thr Gly Cys Gly Cys Cys Thr Thr Gly
                   85                  90                  95
   Thr Thr Thr Gly Ala Thr Gly Cys Ala Ala Thr Cys Gly Gly Cys Gly
               100                 105                 110
   Ala Cys Ala Thr Gly Thr Cys Gly Ala Ala Thr Gly Cys Thr Gly Thr
           115                 120                 125
   Ala Ala Thr Thr Thr Thr Thr Thr Thr Thr Gly Thr Thr Thr Ala Gly
       130                 135                 140
   Gly Thr Thr Cys Cys Cys Cys Thr Thr Thr Thr Cys Cys Thr Thr Thr
   145 150 155 160
   Thr Gly Thr Gly Thr Thr Ala Ala Thr Ala Ala Thr Ala Ala Thr Thr
   165 170 175
   Cys Thr Cys Gly Ala Ala Gly Gly Gly Cys Gly Cys Thr Gly Ala Thr
   180 185 190
   Thr Thr Thr Gly Ala Ala Ala Thr Thr Thr Gly Thr Cys Gly Gly Thr
   195 200 205
   Gly Ala Gly Ala Gly Cys Cys Ala Ala Ala Cys Gly Gly Ala Thr Ala
   210 215 220
   Thr Ala Cys Ala Gly Gly Cys Gly Cys Gly Gly Cys Thr Gly Ala Thr
   225 230 235 240
   Gly Ala Ala Thr Ala Ala Thr Gly Ala Thr Gly Ala Ala Thr Cys Gly
   245 250 255
   Ala Gly Cys Thr Gly Ala Cys Thr Thr Gly Ala Thr Gly Cys Thr Gly
   260 265 270
   Thr Ala Thr Gly Thr Ala Cys Ala Ala Thr Ala Thr Thr Gly Ala Cys
   275 280 285
   Thr Gly Cys Gly Ala Gly Gly Ala Cys Cys Ala Thr Cys Ala Gly Gly
   290 295 300
   Thr Gly Thr Thr Gly Thr Ala Thr Gly Gly Ala Thr Gly Gly Ala Ala
   305 310 315 320
   Thr Cys Ala Thr Thr Cys Thr Gly Thr Ala Ala Cys Cys Ala Cys Cys
   325 330 335
   Ala Ala Gly Gly Thr Gly Cys Ala Thr Gly Cys Ala Thr Cys Ala Thr
   340 345 350
   Ala Ala Gly Gly Thr Ala Thr Thr Cys Thr Cys Cys Thr Cys Ala Gly
   355 360 365
   Cys Thr Cys Ala Cys Cys Ala Ala Cys Ala Ala Cys Gly Ala Ala Cys
   370 375 380
   Gly Ala Thr Gly Gly Cys Cys Ala Thr Gly Thr Thr Ala Gly Thr Ala
   385 390 395 400
   Ala Ala Gly Gly Cys Ala Cys Cys Gly Thr Gly Ala Thr Gly Gly Cys
   405 410 415
   Ala Ala Gly Ala Thr Ala Gly Ala Ala Cys Cys Ala Cys Thr Ala Thr
   420 425 430
   Thr Gly Cys Ala Thr Cys Thr Gly Cys Gly Cys Thr Thr Cys Cys Cys
   435 440 445
   Ala Cys Gly Cys Ala Cys Ala Gly Thr Ala Cys Gly Thr Cys Ala Ala
   450 455 460
   Thr Gly Thr Ala Ala Cys Gly Thr Cys Ala Ala Ala Gly Cys Cys Gly
   465 470 475 480
   Cys Cys Cys Thr Cys Cys Cys Gly Thr Ala Ala Cys Cys Thr Cys Gly
   485 490 495
   Cys Cys Cys Gly Thr Thr Gly Thr Thr Gly Cys Thr Cys Cys Cys Cys
   500 505 510
   Cys Cys Gly Ala Thr Thr Gly Cys Cys Thr Cys Ala Ala Thr Cys Ala
   515 520 525
   Cys Ala Thr Ala Gly Thr Ala Cys Cys Thr Ala Cys Cys Thr Ala Thr
   530 535 540
   Gly Cys Ala Thr Thr Ala Thr Gly Gly Cys Gly Cys Cys Thr Cys Ala
   545 550 555 560
   Ala Cys Cys Cys Ala Cys Cys Cys Cys Cys Cys Cys Ala Gly Ala Thr
   565 570 575
   Thr Gly Ala Gly Ala Gly Cys Thr Ala Cys Cys Thr Thr Ala Cys Ala
   580 585 590
   Thr Cys Ala Ala Thr Ala Thr Gly Gly Cys Cys Ala Gly Cys Ala Cys
   595 600 605
   Cys Thr Cys Thr Thr Cys Gly Gly Cys Gly Ala Thr Ala Cys Ala Thr
   610 615 620
   Ala Cys Thr Cys Gly Cys Cys Ala Cys Cys Cys Cys Ala Gly Cys Cys
   625 630 635 640
   Gly Gly Gly Gly Cys Gly Ala Thr Thr Gly Thr Gly Thr Gly Thr Ala
   645 650 655
   Cys Thr Ala Gly Gly Thr Ala Gly Gly Cys Thr Cys Gly Thr Ala Cys
   660 665 670
   Thr Ala Thr Ala Cys Cys Ala Gly Cys Ala Gly Gly Ala Gly Ala Gly
   675 680 685
   Gly Thr Gly Cys Thr Gly Cys Thr Thr Gly Gly Cys Ala Ala Thr Cys
   690 695 700
   Gly Thr Gly Cys Thr Cys Ala Gly Cys Thr Gly Thr Thr Ala Gly Gly
   705 710 715 720
   Thr Thr Gly Thr Ala Cys Thr Thr Gly Thr Ala Thr Gly Gly Thr Ala
   725 730 735
   Cys Thr Thr Gly Thr Ala Ala Gly Gly Thr Gly Gly Thr Cys Ala Thr
   740 745 750
   Gly Cys Ala Gly Thr Thr Gly Cys Thr Ala Ala Gly Gly Thr Ala Cys
   755 760 765
   Cys Thr Ala Gly Gly Gly Ala Gly Gly Gly Ala Thr Thr Cys Ala Ala
   770 775 780
   Cys Gly Ala Gly Cys Cys Cys Thr Gly Cys Thr Thr Cys Cys Ala Ala
   785 790 795 800
   Thr Gly Thr Cys Cys Ala Thr Cys Thr Gly Gly Ala Thr Ala Gly Gly
   805 810 815
   Ala Thr Gly Gly Cys Gly Gly Cys Thr Gly Gly Cys Gly Gly Gly Gly
   820 825 830
   Cys Cys Gly Ala Ala Gly Cys Thr Gly Gly Gly Ala Ala Cys Thr Cys
   835 840 845
   Gly Cys Cys Ala Ala Cys Ala Gly Thr Cys Ala Thr Ala Thr Gly Thr
   850 855 860
   Ala Ala Thr Ala Gly Cys Thr Cys Ala Ala Gly Thr Thr Gly Ala Thr
   865 870 875 880
   Gly Ala Thr Ala Cys Cys Gly Thr Thr Thr Thr Gly Cys Cys Ala Gly
   885 890 895
   Gly Ala Thr Thr Ala Gly Gly Ala Thr Gly Cys Gly Ala Gly Ala Ala
   900 905 910
   Gly Cys Ala Gly Cys Ala Thr Gly Ala Ala Thr Gly Thr Cys Gly Cys
   915 920 925
   Thr Cys Ala Thr Cys Cys Gly Ala Thr Gly Cys Cys Gly Cys Ala Thr
   930 935 940
   Cys Ala Cys Cys Gly Thr Thr Gly Thr Gly Thr Cys Ala Gly Ala Ala
   945 950 955 960
   Ala Cys Gly Ala Cys Cys Ala Ala Gly Cys Thr Ala Ala Gly Cys Ala
   965 970 975
   Ala Cys Thr Ala Ala Gly Gly Thr Ala Cys Cys Thr Thr Ala Cys Cys
   980 985 990
   Gly Thr Cys Cys Ala Cys Thr Ala Thr Cys Thr Cys Ala Gly Gly Thr
   995 1000 1005
   Ala Ala Cys Cys Ala Gly Gly Thr Ala Cys Thr Ala Cys Cys Ala
   1010 1015 1020
   Gly Cys Thr Ala Cys Cys Cys Thr Ala Cys Cys Thr Gly Cys Cys
   1025 1030 1035
   Gly Thr Gly Cys Cys Thr Ala Cys Cys Thr Gly Cys Thr Thr Thr
   1040 1045 1050
   Ala Gly Thr Ala Thr Thr Ala Ala Thr Cys Thr Thr Thr Cys Cys
   1055 1060 1065
   Ala Cys Cys Thr Cys Cys Cys Thr Cys Cys Thr Cys Ala Ala Thr
   1070 1075 1080
   Cys Thr Thr Cys Thr Thr Thr Thr Cys Cys Cys Thr Cys Cys Thr
   1085 1090 1095
   Cys Thr Cys Cys Thr Cys Thr Thr Thr Thr Thr Thr Thr Thr Thr
   1100 1105 1110
   Thr Cys Thr Thr Cys Cys Thr Cys Cys Thr Cys Thr Thr Cys Thr
   1115 1120 1125
   Thr Cys Thr Cys Cys Ala Thr Ala Ala Cys Cys Ala Thr Thr Cys
   1130 1135 1140
   Cys Thr Ala Ala Cys Ala Ala Cys Ala Thr Cys Gly Ala Cys Ala
   1145 1150 1155
   Thr Thr Cys Thr Cys Thr Cys Cys Thr Ala Ala Thr Cys Ala Cys
   1160 1165 1170
   Cys Ala Gly Cys Cys Thr Cys Gly Cys Ala Ala Ala Thr Cys Cys
   1175 1180 1185
   Thr Cys Ala Gly Gly Thr Thr Ala Gly Thr Ala Thr Thr Ala Cys
   1190 1195 1200
   Thr Ala Cys Thr Ala Cys Thr Ala Cys Ala Ala Thr Cys Ala Thr
   1205 1210 1215
   Cys Ala Cys Cys Ala Cys Gly Ala Thr Gly Cys Thr Cys Cys Gly
   1220 1225 1230
   Cys Cys Cys Gly Ala Cys Gly Ala Thr Gly Cys Gly Gly Cys Thr
   1235 1240 1245
   Thr Cys Thr Gly Thr Thr Cys Gly Cys Cys Thr Gly Cys Cys Cys
   1250 1255 1260
   Cys Thr Cys Cys Thr Cys Thr Cys Ala Cys Thr Cys Gly Thr Gly
   1265 1270 1275
   Cys Cys Cys Thr Thr Gly Ala Cys Gly Ala Gly Cys Thr Ala Cys
   1280 1285 1290
   Cys Cys Cys Gly Cys Cys Ala Gly Ala Cys Thr Cys Thr Cys Cys
   1295 1300 1305
   Thr Gly Cys Gly Thr Cys Ala Cys Cys Ala Ala Thr Thr Thr Thr
   1310 1315 1320
   Thr Thr Thr Cys Cys Cys Thr Ala Thr Thr Thr Ala Cys Cys Cys
   1325 1330 1335
   Cys Thr Cys Cys Thr Cys Cys Cys Thr Cys Thr Cys Thr Cys Cys
   1340 1345 1350
   Cys Thr Cys Thr Cys Gly Thr Thr Thr Cys Thr Thr Cys Cys Thr
   1355 1360 1365
   Ala Ala Cys Ala Ala Ala Cys Ala Ala Cys Cys Ala Cys Cys Ala
   1370 1375 1380
   Cys Cys Ala Ala Ala Ala Thr Cys Thr Cys Thr Thr Thr Gly Gly
   1385 1390 1395
   Ala Ala Gly Cys Thr Cys Ala Cys Gly Ala Cys Thr Cys Ala Cys
   1400 1405 1410
   Gly Cys Ala Ala Gly Cys Thr Cys Ala Ala Thr Thr Cys Gly Cys
   1415 1420 1425
   Ala Gly Ala Thr Ala Cys Ala Ala Ala
   1430 1435
<210> 142
   <211> 909
   <212> PRT
   <213> Artificial Sequence
<220>
   <223> région terminateur du gène glyceraldehyde-3-phosphate déshydrogénase (GPDt) de T. reesei
<400> 142
   Gly Thr Gly Cys Thr Gly Thr Gly Thr Thr Cys Cys Thr Cys Ala Gly
   1 5 10 15
   Ala Ala Thr Gly Gly Gly Cys Cys Cys Cys Ala Gly Ala Ala Gly Gly
   20 25 30
   Gly Cys Gly Thr Cys Gly Ala Gly Cys Ala Thr Thr Gly Thr Cys Thr
   35 40 45
   Ala Thr Gly Ala Ala Thr Gly Cys Ala Ala Ala Cys Ala Ala Ala Ala
   50 55 60
   Ala Thr Ala Gly Thr Ala Ala Ala Thr Ala Ala Ala Thr Ala Gly Thr
   65 70 75 80
   Ala Ala Thr Thr Cys Thr Gly Gly Cys Cys Ala Thr Gly Ala Cys Gly
   85 90 95
   Ala Ala Thr Ala Gly Ala Gly Cys Cys Ala Ala Thr Cys Thr Gly Cys
   100 105 110
   Thr Cys Cys Ala Cys Thr Thr Gly Ala Cys Thr Ala Thr Cys Cys Thr
   115 120 125
   Thr Gly Thr Gly Ala Cys Thr Gly Thr Ala Thr Cys Gly Thr Ala Thr
   130 135 140
   Gly Thr Cys Gly Ala Ala Cys Cys Cys Thr Thr Gly Ala Cys Thr Gly
   145 150 155 160
   Cys Cys Cys Ala Thr Thr Cys Ala Ala Ala Cys Ala Ala Thr Thr Gly
   165 170 175
   Thr Ala Ala Ala Gly Gly Ala Ala Thr Ala Thr Gly Ala Gly Cys Thr
   180 185 190
   Ala Cys Ala Ala Gly Thr Thr Ala Thr Gly Thr Cys Thr Cys Ala Cys
   195 200 205
   Gly Thr Thr Thr Gly Cys Gly Thr Gly Cys Gly Ala Gly Cys Cys Cys
   210 215 220
   Gly Thr Thr Thr Gly Thr Ala Cys Gly Thr Thr Ala Thr Thr Thr Thr
   225 230 235 240
   Gly Ala Gly Ala Ala Ala Gly Cys Gly Thr Thr Gly Cys Cys Ala Thr
   245 250 255
   Cys Ala Cys Ala Thr Gly Cys Thr Cys Ala Cys Ala Gly Thr Cys Ala
   260 265 270
   Cys Thr Thr Gly Gly Cys Thr Thr Ala Cys Gly Ala Thr Cys Ala Thr
   275 280 285
   Gly Thr Thr Thr Gly Cys Gly Ala Thr Cys Thr Thr Thr Cys Gly Gly
   290 295 300
   Thr Ala Ala Gly Ala Ala Thr Ala Cys Ala Cys Ala Gly Ala Gly Thr
   305 310 315 320
   Ala Ala Cys Gly Ala Thr Thr Ala Thr Ala Cys Ala Thr Cys Cys Ala
   325 330 335
   Thr Cys Gly Cys Thr Thr Thr Cys Thr Ala Thr Gly Ala Thr Thr Ala
   340 345 350
   Gly Gly Thr Ala Cys Thr Cys Ala Gly Ala Cys Ala Ala Cys Ala Cys
   355 360 365
   Ala Thr Gly Gly Gly Ala Ala Ala Cys Ala Ala Gly Ala Thr Ala Ala
   370 375 380
   Cys Cys Ala Thr Cys Gly Cys Ala Thr Gly Cys Ala Ala Gly Gly Thr
   385 390 395 400
   Cys Gly Ala Thr Thr Cys Cys Ala Ala Thr Cys Ala Thr Gly Ala Thr
                   405                 410                 415
   Cys Thr Gly Gly Ala Cys Thr Gly Gly Gly Gly Thr Ala Thr Thr Cys
               420                 425                 430
   Cys Ala Thr Cys Thr Ala Ala Gly Cys Cys Ala Thr Ala Gly Thr Ala
           435                 440                 445
   Cys Cys Cys Thr Cys Gly Ala Gly Ala Gly Ala Ala Gly Gly Ala Ala
       450                 455                 460
   Thr Gly Gly Thr Ala Gly Gly Ala Cys Cys Thr Cys Thr Cys Ala Gly
   465                 470                 475                 480
   Gly Cys Gly Thr Cys Cys Ala Cys Cys Ala Thr Cys Thr Gly Thr Gly
                   485                 490                 495
   Cys Thr Gly Cys Ala Ala Ala Thr Cys Cys Ala Ala Gly Ala Ala Ala
               500                 505                 510
   Cys Cys Cys Cys Cys Cys Ala Ala Ala Ala Gly Cys Ala Cys Cys Thr
           515                 520                 525
   Ala Cys Cys Thr Ala Thr Cys Thr Ala Cys Cys Thr Ala Gly Ala Gly
       530                 535                 540
   Thr Ala Ala Thr Cys Thr Gly Cys Ala Cys Gly Ala Gly Ala Ala Ala
   545                 550                 555                 560
   Ala Gly Ala Ala Ala Ala Gly Gly Ala Gly Cys Ala Gly Ala Ala Gly
                   565                 570                 575
   Ala Ala Gly Ala Ala Thr Gly Ala Thr Cys Thr Cys Ala Ala Gly Ala
               580                 585                 590
   Gly Gly Cys Cys Gly Thr Gly Ala Ala Cys Gly Cys Ala Gly Ala Ala
           595                 600                 605
   Ala Cys Ala Cys Ala Cys Thr Cys Cys Thr Cys Cys Cys Ala Ala Cys
       610                 615                 620
   Thr Thr Thr Thr Cys Ala Ala Gly Thr Thr Thr Thr Gly Ala Ala Cys
   625                 630                 635                 640
   Ala Ala Ala Ala Ala Ala Ala Gly Ala Ala Ala Gly Ala Thr Gly Ala
                   645                 650                 655
   Gly Gly Ala Cys Thr Ala Gly Ala Ala Gly Ala Thr Gly Gly Ala Gly
   660 665 670
   Thr Ala Thr Thr Thr Cys Cys Thr Thr Cys Thr Thr Ala Gly Ala Gly
   675 680 685
   Ala Gly Cys Thr Cys Thr Cys Gly Gly Thr Gly Ala Gly Gly Thr Gly
   690 695 700
   Ala Cys Cys Thr Gly Thr Cys Ala Gly Gly Gly Thr Thr Thr Ala Cys
   705 710 715 720
   Cys Thr Cys Ala Ala Ala Cys Cys Gly Thr Cys Thr Gly Thr Gly Gly
   725 730 735
   Thr Thr Cys Thr Ala Thr Cys Cys Ala Ala Thr Thr Ala Ala Thr Cys
   740 745 750
   Ala Ala Gly Thr Cys Cys Cys Thr Cys Thr Cys Cys Thr Cys Thr Cys
   755 760 765
   Cys Thr Cys Thr Thr Cys Thr Cys Thr Cys Cys Thr Gly Thr Cys Cys
   770 775 780
   Thr Thr Thr Cys Ala Thr Ala Gly Ala Ala Thr Cys Cys Cys Thr Thr
   785 790 795 800
   Thr Thr Cys Cys Thr Thr Gly Thr Thr Gly Cys Thr Thr Gly Ala Thr
   805 810 815
   Cys Gly Ala Ala Gly Cys Gly Gly Gly Thr Thr Ala Thr Cys Gly Ala
   820 825 830
   Cys Gly Cys Cys Ala Cys Cys Ala Ala Ala Gly Thr Ala Thr Thr Gly
   835 840 845
   Thr Cys Thr Thr Gly Gly Thr Gly Ala Cys Thr Thr Ala Thr Cys Ala
   850 855 860
   Ala Ala Thr Cys Cys Cys Thr Thr Thr Gly Gly Thr Gly Ala Thr Cys
   865 870 875 880
   Ala Ala Ala Cys Ala Gly Cys Cys Cys Cys Cys Gly Ala Gly Thr Gly
   885 890 895
   Ala Thr Cys Ala Gly Ala Thr Cys Cys Gly Thr Ala Ala
   900 905
<210> 143
   <211> 2603
   <212> PRT
   <213> Artificial Sequence
<220>
   <223> cassette HygroR
<400> 143
   Cys Ala Ala Gly Thr Gly Thr Ala Cys Cys Thr Gly Thr Gly Cys Ala
   1               5                   10                  15
   Thr Thr Cys Thr Gly Gly Gly Thr Ala Ala Ala Cys Gly Ala Cys Thr
               20                  25                  30
   Cys Ala Thr Ala Gly Gly Ala Gly Ala Gly Thr Thr Gly Thr Ala Ala
           35                  40                  45
   Ala Ala Ala Ala Gly Thr Thr Thr Cys Gly Gly Cys Cys Gly Gly Cys
       50                  55                  60
   Gly Thr Ala Thr Thr Gly Gly Gly Thr Gly Thr Thr Ala Cys Gly Gly
   65                  70                  75                  80
   Ala Gly Cys Ala Thr Thr Cys Ala Cys Thr Ala Gly Gly Cys Ala Ala
                   85                  90                  95
   Cys Cys Ala Thr Gly Cys Ala Thr Cys Cys Thr Thr Ala Cys Thr Ala
               100                 105                 110
   Thr Thr Gly Thr Ala Thr Ala Cys Cys Ala Thr Cys Thr Thr Ala Gly
           115                 120                 125
   Thr Ala Gly Gly Ala Ala Thr Gly Ala Thr Thr Thr Cys Gly Ala Gly
       130                 135                 140
   Gly Thr Thr Thr Ala Thr Ala Cys Cys Thr Ala Cys Gly Ala Thr Gly
   145                 150                 155                 160
   Ala Ala Thr Gly Thr Gly Thr Gly Thr Cys Cys Thr Gly Thr Ala Gly
                   165                 170                 175
   Gly Cys Thr Thr Gly Ala Gly Ala Gly Thr Thr Cys Ala Ala Gly Gly
               180                 185                 190
   Ala Ala Gly Ala Ala Ala Cys Ala Thr Gly Cys Ala Ala Thr Thr Ala
           195                 200                 205
   Thr Cys Thr Thr Thr Gly Cys Gly Ala Ala Cys Cys Cys Ala Gly Gly
   210 215 220
   Gly Cys Thr Gly Gly Thr Gly Ala Cys Gly Gly Ala Ala Thr Thr Thr
   225 230 235 240
   Thr Cys Ala Thr Ala Gly Thr Cys Ala Ala Gly Cys Thr Ala Thr Cys
   245 250 255
   Ala Gly Ala Gly Thr Ala Ala Ala Gly Ala Ala Gly Ala Gly Gly Ala
   260 265 270
   Gly Cys Ala Thr Gly Thr Cys Ala Ala Ala Gly Thr Ala Cys Ala Ala
   275 280 285
   Thr Thr Ala Gly Ala Gly Ala Cys Ala Ala Ala Thr Ala Thr Ala Thr
   290 295 300
   Ala Gly Thr Cys Gly Cys Gly Thr Gly Gly Ala Gly Cys Cys Ala Ala
   305 310 315 320
   Gly Ala Gly Cys Gly Gly Ala Thr Thr Cys Cys Thr Cys Ala Gly Thr
   325 330 335
   Cys Thr Cys Gly Thr Ala Gly Gly Thr Cys Thr Cys Thr Thr Gly Ala
   340 345 350
   Cys Gly Ala Cys Cys Gly Thr Thr Gly Ala Thr Cys Thr Gly Cys Thr
   355 360 365
   Thr Gly Ala Thr Cys Thr Cys Gly Thr Cys Thr Cys Cys Cys Gly Ala
   370 375 380
   Ala Ala Ala Thr Gly Ala Ala Ala Ala Thr Ala Gly Cys Thr Cys Thr
   385 390 395 400
   Gly Cys Thr Ala Ala Gly Cys Thr Ala Thr Thr Cys Thr Thr Cys Thr
   405 410 415
   Cys Thr Thr Cys Gly Cys Cys Gly Gly Ala Gly Cys Cys Thr Gly Ala
   420 425 430
   Ala Gly Gly Cys Gly Thr Thr Ala Cys Thr Ala Gly Gly Thr Thr Gly
   435 440 445
   Cys Ala Gly Thr Cys Ala Ala Thr Gly Cys Ala Thr Thr Ala Ala Thr
   450 455 460
   Gly Cys Ala Thr Thr Gly Cys Ala Gly Ala Thr Gly Ala Gly Cys Thr
   465 470 475 480
   Gly Thr Ala Thr Cys Thr Gly Gly Ala Ala Gly Ala Gly Gly Thr Ala
   485 490 495
   Ala Ala Cys Cys Cys Gly Ala Ala Ala Ala Cys Gly Cys Gly Thr Thr
   500 505 510
   Thr Thr Ala Thr Thr Cys Thr Thr Gly Thr Thr Gly Ala Cys Ala Thr
   515 520 525
   Gly Gly Ala Gly Cys Thr Ala Thr Thr Ala Ala Ala Thr Cys Ala Cys
   530 535 540
   Thr Ala Gly Ala Ala Gly Gly Cys Ala Cys Thr Cys Thr Thr Thr Gly
   545 550 555 560
   Cys Thr Gly Cys Thr Thr Gly Gly Ala Cys Ala Ala Ala Thr Gly Ala
   565 570 575
   Ala Cys Gly Thr Ala Thr Cys Thr Thr Ala Thr Cys Gly Ala Gly Ala
   580 585 590
   Thr Cys Cys Thr Gly Ala Ala Cys Ala Cys Cys Ala Thr Thr Thr Gly
   595 600 605
   Thr Cys Thr Cys Ala Ala Cys Thr Cys Cys Gly Gly Cys Thr Ala Gly
   610 615 620
   Cys Gly Ala Ala Thr Thr Cys Thr Cys Gly Ala Cys Thr Cys Ala Thr
   625 630 635 640
   Thr Cys Cys Thr Thr Thr Gly Cys Cys Cys Thr Cys Gly Gly Ala Cys
   645 650 655
   Gly Ala Gly Thr Gly Cys Thr Gly Gly Gly Gly Cys Gly Thr Cys Gly
   660 665 670
   Gly Thr Thr Thr Cys Cys Ala Cys Thr Ala Thr Cys Gly Gly Cys Gly
   675 680 685
   Ala Gly Thr Ala Cys Thr Thr Cys Thr Ala Cys Ala Cys Ala Gly Cys
   690 695 700
   Cys Ala Thr Cys Gly Gly Thr Cys Cys Ala Gly Ala Cys Gly Gly Cys
   705 710 715 720
   Cys Gly Cys Gly Cys Thr Thr Cys Thr Gly Cys Gly Gly Gly Cys Gly
   725 730 735
   Ala Thr Thr Thr Gly Thr Gly Thr Ala Cys Gly Cys Cys Cys Gly Ala
   740 745 750
   Cys Ala Gly Thr Cys Cys Cys Gly Gly Cys Thr Cys Cys Gly Gly Ala
   755 760 765
   Thr Cys Gly Gly Ala Cys Gly Ala Thr Thr Gly Cys Gly Thr Cys Gly
   770 775 780
   Cys Ala Thr Cys Gly Ala Cys Cys Cys Thr Gly Cys Gly Cys Cys Cys
   785 790 795 800
   Ala Ala Gly Cys Thr Gly Cys Ala Thr Cys Ala Thr Cys Gly Ala Ala
   805 810 815
   Ala Thr Thr Gly Cys Cys Gly Thr Cys Ala Ala Cys Cys Ala Ala Gly
   820 825 830
   Cys Thr Cys Thr Gly Ala Thr Ala Gly Ala Gly Thr Thr Gly Gly Thr
   835 840 845
   Cys Ala Ala Gly Ala Cys Cys Ala Ala Thr Gly Cys Gly Gly Ala Gly
   850 855 860
   Cys Ala Thr Ala Thr Ala Cys Gly Cys Cys Cys Gly Gly Ala Gly Thr
   865 870 875 880
   Cys Gly Thr Gly Gly Cys Gly Ala Thr Cys Cys Thr Gly Cys Ala Ala
   885 890 895
   Gly Cys Thr Cys Cys Gly Gly Ala Thr Gly Cys Cys Thr Cys Cys Gly
   900 905 910
   Cys Thr Cys Gly Ala Ala Gly Thr Ala Gly Cys Gly Cys Gly Thr Cys
   915 920 925
   Thr Gly Cys Thr Gly Cys Thr Cys Cys Ala Thr Ala Cys Ala Ala Gly
   930 935 940
   Cys Cys Ala Ala Cys Cys Ala Cys Gly Gly Cys Cys Thr Cys Cys Ala
   945 950 955 960
   Gly Ala Ala Gly Ala Ala Gly Ala Thr Gly Thr Thr Gly Gly Cys Gly
   965 970 975
   Ala Cys Cys Thr Cys Gly Thr Ala Thr Thr Gly Gly Gly Ala Ala Thr
   980 985 990
   Cys Cys Cys Cys Gly Ala Ala Cys Ala Thr Cys Gly Cys Cys Thr Cys
   995 1000 1005
   Gly Cys Thr Cys Cys Ala Gly Thr Cys Ala Ala Thr Gly Ala Cys
   1010 1015 1020
   Cys Gly Cys Thr Gly Thr Thr Ala Thr Gly Cys Gly Gly Cys Cys
   1025 1030 1035
   Ala Thr Thr Gly Thr Cys Cys Gly Thr Cys Ala Gly Gly Ala Cys
   1040 1045 1050
   Ala Thr Thr Gly Thr Thr Gly Gly Ala Gly Cys Cys Gly Ala Ala
   1055 1060 1065
   Ala Thr Cys Cys Gly Cys Gly Thr Gly Cys Ala Cys Gly Ala Gly
   1070 1075 1080
   Gly Thr Gly Cys Cys Gly Gly Ala Cys Thr Thr Cys Gly Gly Gly
   1085 1090 1095
   Gly Cys Ala Gly Thr Cys Cys Thr Cys Gly Gly Cys Cys Cys Ala
   1100 1105 1110
   Ala Ala Gly Cys Ala Thr Cys Ala Gly Cys Thr Cys Ala Thr Cys
   1115 1120 1125
   Gly Ala Gly Ala Gly Cys Cys Thr Gly Cys Gly Cys Gly Ala Cys
   1130 1135 1140
   Gly Gly Ala Cys Gly Cys Ala Cys Thr Gly Ala Cys Gly Gly Thr
   1145 1150 1155
   Gly Thr Cys Gly Thr Cys Cys Ala Thr Cys Ala Cys Ala Gly Thr
   1160 1165 1170
   Thr Thr Gly Cys Cys Ala Gly Thr Gly Ala Thr Ala Cys Ala Cys
   1175 1180 1185
   Ala Thr Gly Gly Gly Gly Ala Thr Cys Ala Gly Cys Ala Ala Thr
   1190 1195 1200
   Cys Gly Cys Gly Cys Ala Thr Ala Thr Gly Ala Ala Ala Thr Cys
   1205 1210 1215
   Ala Cys Gly Cys Cys Ala Thr Gly Thr Ala Gly Thr Gly Thr Ala
   1220 1225 1230
   Thr Thr Gly Ala Cys Cys Gly Ala Thr Thr Cys Cys Thr Thr Gly
   1235 1240 1245
   Cys Gly Gly Thr Cys Cys Gly Ala Ala Thr Gly Gly Gly Cys Cys
   1250 1255 1260
   Gly Ala Ala Cys Cys Cys Gly Cys Thr Cys Gly Thr Cys Thr Gly
   1265 1270 1275
   Gly Cys Thr Ala Ala Gly Ala Thr Cys Gly Gly Cys Cys Gly Cys
   1280 1285 1290
   Ala Gly Cys Gly Ala Thr Cys Gly Cys Ala Thr Cys Cys Ala Thr
   1295 1300 1305
   Ala Gly Cys Cys Thr Cys Cys Gly Cys Gly Ala Cys Cys Gly Gly
   1310 1315 1320
   Thr Thr Gly Thr Ala Gly Ala Ala Cys Ala Gly Cys Gly Gly Gly
   1325 1330 1335
   Cys Ala Gly Thr Thr Cys Gly Gly Thr Thr Thr Cys Ala Gly Gly
   1340 1345 1350
   Cys Ala Gly Gly Thr Cys Thr Thr Gly Cys Ala Ala Cys Gly Thr
   1355 1360 1365
   Gly Ala Cys Ala Cys Cys Cys Thr Gly Thr Gly Cys Ala Cys Gly
   1370 1375 1380
   Gly Cys Gly Gly Gly Ala Gly Ala Thr Gly Cys Ala Ala Thr Ala
   1385 1390 1395
   Gly Gly Thr Cys Ala Gly Gly Cys Thr Cys Thr Cys Gly Cys Thr
   1400 1405 1410
   Ala Ala Ala Cys Thr Cys Cys Cys Cys Ala Ala Thr Gly Thr Cys
   1415 1420 1425
   Ala Ala Gly Cys Ala Cys Thr Thr Cys Cys Gly Gly Ala Ala Thr
   1430 1435 1440
   Cys Gly Gly Gly Ala Gly Cys Gly Cys Gly Gly Cys Cys Gly Ala
   1445 1450 1455
   Thr Gly Cys Ala Ala Ala Gly Thr Gly Cys Cys Gly Ala Thr Ala
   1460 1465 1470
   Ala Ala Cys Ala Thr Ala Ala Cys Gly Ala Thr Cys Thr Thr Thr
   1475 1480 1485
   Gly Thr Ala Gly Ala Ala Ala Cys Cys Ala Thr Cys Gly Gly Cys
   1490 1495 1500
   Gly Cys Ala Gly Cys Thr Ala Thr Thr Thr Ala Cys Cys Cys Gly
   1505 1510 1515
   Cys Ala Gly Gly Ala Cys Ala Thr Ala Thr Cys Cys Ala Cys Gly
   1520 1525 1530
   Cys Cys Cys Thr Cys Cys Thr Ala Cys Ala Thr Cys Gly Ala Ala
   1535 1540 1545
   Gly Cys Thr Gly Ala Ala Ala Gly Cys Ala Cys Gly Ala Gly Ala
   1550 1555 1560
   Thr Thr Cys Thr Thr Cys Gly Cys Cys Cys Thr Cys Cys Gly Ala
   1565 1570 1575
   Gly Ala Gly Cys Thr Gly Cys Ala Thr Cys Ala Gly Gly Thr Cys
   1580 1585 1590
   Gly Gly Ala Gly Ala Cys Gly Cys Thr Gly Thr Cys Gly Ala Ala
   1595 1600 1605
   Cys Thr Thr Thr Thr Cys Gly Ala Thr Cys Ala Gly Ala Ala Ala
   1610 1615 1620
   Cys Thr Thr Cys Thr Cys Gly Ala Cys Ala Gly Ala Cys Gly Thr
   1625 1630 1635
   Cys Gly Cys Gly Gly Thr Gly Ala Gly Thr Thr Cys Ala Gly Gly
   1640 1645 1650
   Cys Thr Thr Thr Thr Thr Cys Ala Thr Gly Ala Thr Gly Gly Cys
   1655 1660 1665
   Cys Cys Thr Cys Cys Thr Ala Cys Cys Gly Gly Thr Gly Ala Thr
   1670 1675 1680
   Cys Thr Cys Ala Gly Cys Thr Gly Thr Ala Gly Gly Ala Ala Ala
   1685 1690 1695
   Gly Ala Gly Ala Ala Gly Ala Ala Gly Gly Thr Thr Ala Gly Thr
   1700 1705 1710
   Ala Gly Thr Cys Gly Ala Cys Ala Thr Gly Gly Thr Gly Gly Cys
   1715 1720 1725
   Cys Cys Thr Cys Cys Thr Ala Thr Ala Gly Thr Gly Ala Gly Thr
   1730 1735 1740
   Cys Gly Thr Ala Thr Thr Ala Thr Ala Cys Thr Ala Thr Gly Cys
   1745 1750 1755
   Cys Gly Ala Thr Ala Thr Ala Cys Thr Ala Thr Gly Cys Cys Gly
   1760 1765 1770
   Ala Thr Gly Ala Thr Thr Ala Ala Thr Thr Gly Thr Cys Ala Ala
   1775 1780 1785
   Cys Ala Cys Thr Ala Gly Gly Cys Gly Cys Cys Gly Gly Thr Cys
   1790 1795 1800
   Ala Cys Ala Ala Cys Thr Ala Gly Thr Ala Gly Ala Thr Ala Thr
   1805 1810 1815
   Cys Ala Cys Thr Thr Ala Cys Gly Thr Gly Thr Thr Gly Ala Gly
   1820 1825 1830
   Ala Gly Gly Cys Gly Gly Cys Ala Thr Gly Cys Gly Ala Thr Ala
   1835 1840 1845
   Ala Gly Ala Gly Gly Thr Gly Thr Ala Ala Thr Thr Ala Cys Cys
   1850 1855 1860
   Thr Gly Ala Gly Ala Ala Cys Ala Thr Cys Thr Thr Gly Thr Thr
   1865 1870 1875
   Gly Cys Cys Cys Thr Gly Cys Thr Thr Thr Cys Cys Gly Thr Gly
   1880 1885 1890
   Cys Gly Ala Ala Ala Thr Ala Cys Thr Ala Cys Cys Gly Gly Thr
   1895 1900 1905
   Ala Cys Thr Thr Thr Thr Gly Gly Gly Ala Ala Ala Cys Ala Ala
   1910 1915 1920
   Gly Gly Gly Ala Ala Cys Ala Gly Gly Ala Gly Gly Gly Cys Gly
   1925 1930 1935
   Cys Thr Gly Cys Thr Gly Thr Gly Cys Gly Cys Gly Gly Thr Thr
   1940 1945 1950
   Cys Thr Gly Ala Gly Thr Gly Thr Thr Cys Ala Gly Gly Ala Thr
   1955 1960 1965
   Thr Gly Ala Ala Gly Cys Thr Gly Ala Ala Gly Ala Ala Gly Gly
   1970 1975 1980
   Thr Gly Cys Thr Gly Ala Gly Gly Ala Ala Gly Cys Gly Thr Ala
   1985 1990 1995
   Gly Ala Ala Cys Thr Gly Thr Thr Gly Cys Gly Gly Ala Cys Gly
   2000 2005 2010
   Cys Gly Ala Gly Thr Thr Cys Thr Gly Ala Gly Ala Ala Gly Ala
   2015 2020 2025
   Gly Cys Thr Gly Thr Ala Cys Cys Gly Ala Thr Thr Gly Gly Thr
   2030 2035 2040
   Gly Ala Ala Ala Gly Cys Cys Gly Ala Ala Gly Ala Ala Gly Thr
   2045 2050 2055
   Gly Ala Gly Thr Thr Gly Gly Thr Gly Cys Cys Cys Thr Gly Thr
   2060 2065 2070
   Thr Gly Cys Cys Thr Gly Gly Ala Thr Ala Ala Thr Gly Thr Thr
   2075 2080 2085
   Thr Gly Cys Ala Ala Cys Thr Cys Gly Cys Thr Gly Gly Thr Thr
   2090 2095 2100
   Cys Thr Gly Cys Ala Gly Ala Gly Ala Cys Gly Gly Ala Gly Ala
   2105 2110 2115
   Cys Ala Ala Ala Thr Gly Cys Thr Gly Gly Cys Thr Ala Cys Gly
   2120 2125 2130
   Ala Thr Gly Thr Thr Gly Cys Thr Gly Ala Thr Thr Cys Ala Gly
   2135 2140 2145
   Gly Thr Thr Gly Ala Thr Ala Cys Cys Thr Cys Gly Gly Thr Cys
   2150 2155 2160
   Gly Ala Gly Ala Cys Ala Cys Thr Gly Thr Thr Thr Thr Gly Gly
   2165 2170 2175
   Thr Thr Thr Gly Ala Thr Ala Gly Gly Gly Thr Gly Gly Ala Thr
   2180 2185 2190
   Thr Thr Gly Gly Thr Thr Gly Cys Ala Gly Ala Gly Ala Ala Gly
   2195 2200 2205
   Ala Gly Ala Ala Ala Gly Gly Ala Ala Gly Gly Thr Cys Ala Ala
   2210 2215 2220
   Ala Gly Ala Gly Gly Gly Ala Ala Ala Ala Cys Thr Gly Gly Gly
   2225 2230 2235
   Cys Gly Gly Ala Gly Gly Gly Ala Ala Gly Gly Ala Thr Thr Thr
   2240 2245 2250
   Thr Gly Thr Ala Thr Cys Ala Gly Gly Cys Ala Gly Cys Ala Ala
   2255 2260 2265
   Ala Cys Thr Gly Cys Cys Ala Cys Thr Gly Cys Ala Gly Thr Gly
   2270 2275 2280
   Gly Cys Cys Cys Thr Gly Gly Cys Ala Gly Thr Gly Cys Cys Gly
   2285 2290 2295
   Gly Gly Cys Gly Ala Gly Gly Cys Ala Cys Cys Cys Ala Cys Gly
   2300 2305 2310
   Cys Ala Cys Gly Gly Cys Cys Gly Cys Gly Cys Ala Ala Cys Cys
   2315 2320 2325
   Gly Gly Thr Thr Gly Gly Thr Cys Cys Thr Thr Gly Cys Cys Cys
   2330 2335 2340
   Ala Cys Cys Ala Cys Gly Ala Ala Ala Cys Cys Cys Thr Thr Cys
   2345 2350 2355
   Thr Gly Ala Ala Ala Gly Gly Thr Cys Ala Gly Ala Thr Gly Gly
   2360 2365 2370
   Ala Ala Gly Thr Gly Thr Gly Cys Gly Ala Cys Ala Gly Thr Gly
       2375                2380                2385
   Cys Gly Cys Gly Thr Cys Cys Cys Cys Ala Ala Gly Cys Cys Ala
       2390                2395                2400
   Ala Thr Gly Cys Ala Gly Gly Cys Gly Cys Cys Ala Thr Gly Cys
       2405                2410                2415
   Ala Cys Thr Cys Cys Cys Cys Ala Cys Cys Cys Gly Cys Ala Ala
       2420                2425                2430
   Gly Ala Thr Thr Cys Ala Cys Thr Gly Thr Gly Cys Gly Thr Thr
       2435                2440                2445
   Cys Thr Thr Ala Thr Thr Gly Gly Thr Thr Gly Cys Cys Gly Cys
       2450                2455                2460
   Ala Ala Gly Gly Cys Cys Ala Gly Cys Cys Ala Ala Ala Gly Gly
       2465                2470                2475
   Gly Gly Gly Ala Ala Gly Thr Ala Thr Gly Ala Gly Thr Cys Ala
       2480                2485                2490
   Cys Ala Gly Cys Ala Cys Cys Gly Ala Thr Ala Cys Ala Ala Gly
       2495                2500                2505
   Ala Ala Ala Ala Thr Thr Gly Cys Ala Gly Ala Ala Cys Thr Ala
       2510                2515                2520
   Ala Cys Ala Thr Ala Thr Gly Gly Ala Thr Gly Cys Gly Cys Gly
       2525                2530                2535
   Cys Gly Cys Thr Ala Thr Thr Cys Thr Gly Thr Ala Gly Ala Gly
       2540                2545                2550
   Cys Thr Cys Thr Gly Gly Gly Cys Ala Ala Ala Gly Cys Ala Cys
       2555                2560                2565
   Cys Ala Ala Thr Cys Cys Thr Gly Cys Gly Gly Gly Thr Cys Gly
       2570                2575                2580
   Gly Thr Ala Cys Ala Cys Ala Cys Ala Cys Thr Ala Gly Cys Ala
       2585                2590                2595
   Cys Thr Gly Cys Cys
       2600
<210> 144
   <211> 40
   <212> DNA
   <213> Artificial Sequence
<220>
   <223> amorce sens
<400> 144
   attgggtacc gggccccccc gacgcagaag aaggaaatcg 40
<210> 145
   <211> 30
   <212> DNA
   <213> Artificial Sequence
<220>
   <223> amorce antisens
<400> 145
   cgagggccat tttgtatctg cgaattgagc 30
<210> 146
   <211> 30
   <212> DNA
   <213> Artificial Sequence
<220>
   <223> amorce sens
<400> 146
   cagatacaaa atggccctcg cagctcaaca 30
<210> 147
   <211> 32
   <212> DNA
   <213> Artificial Sequence
<220>
   <223> amorce antisens
<400> 147
   aacacagcac tcaggatagg tggctcgcaa tg 32
<210> 148
   <211> 30
   <212> DNA
   <213> Artificial Sequence
<220>
   <223> amorce sens
<400> 148
   cctatcctga gtgctgtgtt cctcagaatg 30
<210> 149
   <211> 30
   <212> PRT
   <213> Artificial Sequence
<220>
   <223> amorce antisens
<400> 149
   Gly Gly Thr Ala Cys Ala Cys Thr Thr Gly Thr Thr Ala Cys Gly Gly
   1               5                   10                  15
   Ala Thr Cys Thr Gly Ala Thr Cys Ala Cys Thr Cys Gly Gly
               20                  25                  30
<210> 150
   <211> 31
   <212> DNA
   <213> Artificial Sequence
<220>
   <223> amorce sens
<400> 150
   agatccgtaa caagtgtacc tgtgcattct g 31
<210> 151
   <211> 40
   <212> DNA
   <213> Artificial Sequence
<220>
   <223> amorce antisens
<400> 151
   tggatccccc gggctgcagg ggcagtgcta gtgtgtgtac 40
<210> 152
   <211> 20
   <212> DNA
   <213> Artificial Sequence
<220>
   <223> amorce sens
<400> 152
   gacgcagaag aaggaaatcg 20
<210> 153
   <211> 21
   <212> DNA
   <213> Artificial Sequence
<220>
   <223> amorce antisens
<400> 153
   ggtacacaca ctagcactgc c 21
<210> 154
   <211> 6384
   <212> DNA
   <213> Artificial Sequence
<220>
   <223> séquence de l'exemple 2
<400> 154
   gacgcagaag aaggaaatcg ccccgccggt tccgtaaaaa aaatatgagc gcagggacaa 60
   gcacagcctt ggccctgggc ctagccttgc gccttgtttg atgcaatcgg cgacatgtcg 120
   aatgctgtaa ttttttttgt ttaggttccc cttttccttt tgtgttaata ataattctcg 180
   aagggcgctg attttgaaat ttgtcggtga gagccaaacg gatatacagg cgcggctgat 240
   gaataatgat gaatcgagct gacttgatgc tgtatgtaca atattgactg cgaggaccat 300
   caggtgttgt atggatggaa tcattctgta accaccaagg tgcatgcatc ataaggtatt 360
   ctcctcagct caccaacaac gaacgatggc catgttagta aaggcaccgt gatggcaaga 420
   tagaaccact attgcatctg cgcttcccac gcacagtacg tcaatgtaac gtcaaagccg 480
   ccctcccgta acctcgcccg ttgttgctcc ccccgattgc ctcaatcaca tagtacctac 540
   ctatgcatta tggcgcctca acccaccccc ccagattgag agctacctta catcaatatg 600
   gccagcacct cttcggcgat acatactcgc caccccagcc ggggcgattg tgtgtactag 660
   gtaggctcgt actataccag caggagaggt gctgcttggc aatcgtgctc agctgttagg 720
   ttgtacttgt atggtacttg taaggtggtc atgcagttgc taaggtacct agggagggat 780
   tcaacgagcc ctgcttccaa tgtccatctg gataggatgg cggctggcgg ggccgaagct 840
   gggaactcgc caacagtcat atgtaatagc tcaagttgat gataccgttt tgccaggatt 900
   aggatgcgag aagcagcatg aatgtcgctc atccgatgcc gcatcaccgt tgtgtcagaa 960
   acgaccaagc taagcaacta aggtacctta ccgtccacta tctcaggtaa ccaggtacta 1020
   ccagctaccc tacctgccgt gcctacctgc tttagtatta atctttccac ctccctcctc 1080
   aatcttcttt tccctcctct cctctttttt ttttcttcct cctcttcttc tccataacca 1140
   ttcctaacaa catcgacatt ctctcctaat caccagcctc gcaaatcctc aggttagtat 1200
   tactactact acaatcatca ccacgatgct ccgcccgacg atgcggcttc tgttcgcctg 1260
   cccctcctct cactcgtgcc cttgacgagc taccccgcca gactctcctg cgtcaccaat 1320
   ttttttccct atttacccct cctccctctc tccctctcgt ttcttcctaa caaacaacca 1380
   ccaccaaaat ctctttggaa gctcacgact cacgcaagct caattcgcag atacaaaatg 1440
   gccctcgcag ctcaacagca tactcaggcc gattggggcc gctggtctca ccagattcca 1500
   cagagttttc ccatgatggg ctctccagga ttcatgtcat acgatcccag agctcaggac 1560
   ggcagtcaga tgcagcgtca ggtgtctgct cagtacctgg tgaactcgaa ctacaaccag 1620
   cccccgatgc ccactgcttc gtctccccag tatcaacacg cagggccatt ttcctatgtg 1680
   ccttaccaca gcccgccgcc gtccactcct cttggttccc cattcaagag cgaatttccc 1740
   gagcaccctc ttacgcgcat gacacactct acggtcgatc gacaccattc tcaggccatg 1800
   agggactacc aaccttattc tcctgtatcg aggaggggat cgatttcttc agtcgccacc 1860
   aagccctcag cagctcccgt cacaccaggt ccaactactc ctggctcttt cacttcaagt 1920
   tccgacgccc agagccccag cactccaaac ccccagactg cgtctcagcc tgtcagctcc 1980
   aagactctca cttacaatga gaccgttcat ccgggcgata ggatcagctt cagaaccgat 2040
   gttgatgaac tcatgaaggc catccagaag acacagacga ccgacgagtg tcagcaaaca 2100
   ctcacacctg cgcgaacacc aaagaactgt accacaagta ctcccgtact tcgtacacaa 2160
   agcgggaagc cgagaaaaca gtgggtttgc gatggcccca actgcggcaa ggcctttgtc 2220
   cagaagacgc atcgcgacat tcaccgacgc actcacaccg gccatcgacc atacgtacgc 2280
   gcccagctcc tcttcactgc aacgccggct aattaaatgt tgataggtct gcaccatgga 2340
   aaattgcggt cttacgttct cgcagcgagg aaacctcaag gtaagcttca gctgctaaga 2400
   atctcctttg agaatgcgta tactgaccag atggtgtgtg gacagactca catacgacgc 2460
   cacacaggtg aaaagccgtt ctcttgcgct gcttgtggca agtgcttcgc tcagcgtggg 2520
   aatcttcgat cccacgagga gacacacaaa ggcctgaagc ccttcgtctg ccggctcgat 2580
   gattgcaaca agtcgttttc tcagctgggc aatatgaagg tatgcaacat ctagcacatg 2640
   aaagcagtat gagaacgctc taacgctgag ggaactgcag actcatcaga acaactttca 2700
   caaagaaacg ctccagaaac tcacacacat gtttgtgcaa ttctcggaga acggcgaggt 2760
   gcccagagac tatcaggatc ttttcgaata cttccagaag cactacaaga atagcaacaa 2820
   gggagtcaag ggccgaggaa agactcgcgc tgtggcagct cgtgggcctc aagattccgc 2880
   gtttcggcag gctgcctccc cagtgcccgc gttactgaag acgccggcta cgactcattt 2940
   gccccagatg acaatgccag cccatgatcc ccatggcaga atctcaccat acgccatgac 3000
   ccagggagct gcgaacactc tgagcaatgt cctgcgcaac cccaacccct cttacggcct 3060
   ttatggaccc acgtttgccc cgggccctgt acgagatggc gtctttcaca tgggcattgc 3120
   gagccaccta tcctgatcta gaaagaagga ttacctctaa acaagtgtac ctgtgcattc 3180
   tgggtaaacg actcatagga gagttgtaaa aaagtttcgg ccggcgtatt gggtgttacg 3240
   gagcattcac taggcaacca tgcatcctta ctattgtata ccatcttagt aggaatgatt 3300
   tcgaggttta tacctacgat gaatgtgtgt cctgtaggct tgagagttca aggaagaaac 3360
   atgcaattat ctttgcgaac ccagggctgg tgacggaatt ttcatagtca agctatcaga 3420
   gtaaagaaga ggagcatgtc aaagtacaat tagagacaaa tatatagtcg cgtggagcca 3480
   agagcggatt cctcagtctc gtaggtctct tgacgaccgt tgatctgctt gatctcgtct 3540
   cccgaaaatg aaaatagctc tgctaagcta ttcttctctt cgccggagcc tgaaggcgtt 3600
   actaggttgc agtcaatgca ttaatgcatt gcagatgagc tgtatctgga agaggtaaac 3660
   ccgaaaacgc gttttattct tgttgacatg gagctattaa atcactagaa ggcactcttt 3720
   gctgcttgga caaatgaacg tatcttatcg agatcctgaa caccatttgt ctcaactccg 3780
   gcaagtgtac ctgtgcattc tgggtaaacg actcatagga gagttgtaaa aaagtttcgg 3840
   ccggcgtatt gggtgttacg gagcattcac taggcaacca tgcatcctta ctattgtata 3900
   ccatcttagt aggaatgatt tcgaggttta tacctacgat gaatgtgtgt cctgtaggct 3960
   tgagagttca aggaagaaac atgcaattat ctttgcgaac ccagggctgg tgacggaatt 4020
   ttcatagtca agctatcaga gtaaagaaga ggagcatgtc aaagtacaat tagagacaaa 4080
   tatatagtcg cgtggagcca agagcggatt cctcagtctc gtaggtctct tgacgaccgt 4140
   tgatctgctt gatctcgtct cccgaaaatg aaaatagctc tgctaagcta ttcttctctt 4200
   cgccggagcc tgaaggcgtt actaggttgc agtcaatgca ttaatgcatt gcagatgagc 4260
   tgtatctgga agaggtaaac ccgaaaacgc gttttattct tgttgacatg gagctattaa 4320
   atcactagaa ggcactcttt gctgcttgga caaatgaacg tatcttatcg agatcctgaa 4380
   caccatttgt ctcaactccg gctagcgaat tctcgactca ttcctttgcc ctcggacgag 4440
   tgctggggcg tcggtttcca ctatcggcga gtacttctac acagccatcg gtccagacgg 4500
   ccgcgcttct gcgggcgatt tgtgtacgcc cgacagtccc ggctccggat cggacgattg 4560
   cgtcgcatcg accctgcgcc caagctgcat catcgaaatt gccgtcaacc aagctctgat 4620
   agagttggtc aagaccaatg cggagcatat acgcccggag tcgtggcgat cctgcaagct 4680
   ccggatgcct ccgctcgaag tagcgcgtct gctgctccat acaagccaac cacggcctcc 4740
   agaagaagat gttggcgacc tcgtattggg aatccccgaa catcgcctcg ctccagtcaa 4800
   tgaccgctgt tatgcggcca ttgtccgtca ggacattgtt ggagccgaaa tccgcgtgca 4860
   cgaggtgccg gacttcgggg cagtcctcgg cccaaagcat cagctcatcg agagcctgcg 4920
   cgacggacgc actgacggtg tcgtccatca cagtttgcca gtgatacaca tggggatcag 4980
   caatcgcgca tatgaaatca cgccatgtag tgtattgacc gattccttgc ggtccgaatg 5040
   ggccgaaccc gctcgtctgg ctaagatcgg ccgcagcgat cgcatccata gcctccgcga 5100
   ccggttgtag aacagcgggc agttcggttt caggcaggtc ttgcaacgtg acaccctgtg 5160
   cacggcggga gatgcaatag gtcaggctct cgctaaactc cccaatgtca agcacttccg 5220
   gaatcgggag cgcggccgat gcaaagtgcc gataaacata acgatctttg tagaaaccat 5280
   cggcgcagct atttacccgc aggacatatc cacgccctcc tacatcgaag ctgaaagcac 5340
   gagattcttc gccctccgag agctgcatca ggtcggagac gctgtcgaac ttttcgatca 5400
   gaaacttctc gacagacgtc gcggtgagtt caggcttttt catgatggcc ctcctaccgg 5460
   tgatctcagc tgtaggaaag agaagaaggt tagtagtcga catggtggcc ctcctatagt 5520
   gagtcgtatt atactatgcc gatatactat gccgatgatt aattgtcaac actaggcgcc 5580
   ggtcacaact agtagatatc acttacgtgt tgagaggcgg catgcgataa gaggtgtaat 5640
   tacctgagaa catcttgttg ccctgctttc cgtgcgaaat actaccggta cttttgggaa 5700
   acaagggaac aggagggcgc tgctgtgcgc ggttctgagt gttcaggatt gaagctgaag 5760
   aaggtgctga ggaagcgtag aactgttgcg gacgcgagtt ctgagaagag ctgtaccgat 5820
   tggtgaaagc cgaagaagtg agttggtgcc ctgttgcctg gataatgttt gcaactcgct 5880
   ggttctgcag agacggagac aaatgctggc tacgatgttg ctgattcagg ttgatacctc 5940
   ggtcgagaca ctgttttggt ttgatagggt ggatttggtt gcagagaaga gaaaggaagg 6000
   tcaaagaggg aaaactgggc ggagggaagg attttgtatc aggcagcaaa ctgccactgc 6060
   agtggccctg gcagtgccgg gcgaggcacc cacgcacggc cgcgcaaccg gttggtcctt 6120
   gcccaccacg aaacccttct gaaaggtcag atggaagtgt gcgacagtgc gcgtccccaa 6180
   gccaatgcag gcgccatgca ctccccaccc gcaagattca ctgtgcgttc ttattggttg 6240
   ccgcaaggcc agccaaaggg ggaagtatga gtcacagcac cgatacaaga aaattgcaga 6300
   actaacatat ggatgcgcgc gctattctgt agagctctgg gcaaagcacc aatcctgcgg 6360
   gtcggtacac acactagcac tgcc 6384
<210> 155
   <211> 21
   <212> DNA
   <213> Artificial Sequence
<220>
   <223> amorce sens
<400> 155
   gtcagaaacg accaagctaa g 21
<210> 156
   <211> 20
   <212> DNA
   <213> Artificial Sequence
<220>
   <223> amorce antisens
<400> 156
   gcctgagaat ggtgtcgatc 20
<210> 157
   <211> 18
   <212> DNA
   <213> Artificial Sequence
<220>
   <223> amorce sens
<400> 157
   tcgtgggcct caagattc 18
<210> 158
   <211> 18
   <212> DNA
   <213> Artificial Sequence
<220>
   <223> amorce antisens
<400> 158
   gacgcctgag aggtccta 18
<210> 159
   <211> 20
   <212> DNA
   <213> Artificial Sequence
<220>
   <223> amorce sens
<400> 159
   ccttcttaga gagctctcgg
<210> 160
   <211> 20
   <212> DNA
   <213> Artificial Sequence
<220>
   <223> amorce antisens
<400> 160
   cgggtttacc tcttccagat 20

## Description

La présente invention concerne un procédé de production d'une protéine dans une cellule de champignon filamenteux, comprenant la surexpression du gène *TrAZF1,* dans ladite cellule.

La possibilité de produire de l'éthanol à partir de cellulose a reçu beaucoup d'attention en raison de la disponibilité de grandes quantités de matière première ainsi que de l'intérêt de l'éthanol à titre de carburant. Les matières premières naturelles cellulosiques pour un tel processus sont désignées par le terme "biomasse". De nombreux types de biomasse, par exemple le bois, les résidus agricoles, les cultures herbacées et les déchets solides municipaux, ont été considérés comme des matières premières potentielles pour la production de biocarburant. Ces matières sont constituées principalement de cellulose, d'hémicellulose et de lignine.

La demande WO1992003541 décrit par exemple un procédé d'hydrolyse d'hémicellulose.

La cellulose est un polymère constitué de molécules de glucose reliées par des liaisons beta 1-4, qui sont très résistantes à la dégradation ou à la dépolymérisation. Une fois la cellulose convertie en glucose, celui-ci est facilement fermenté en biocarburant, par exemple l'éthanol, en utilisant une levure.

Les plus anciennes méthodes étudiées pour convertir la cellulose en glucose sont basées sur l'hydrolyse acide. Ce processus peut se faire en présence d'acides concentrés ou dilués. Cependant, plusieurs inconvénients, tels que la mauvaise récupération de l'acide lors de l'utilisation d'acides concentrés et la faible production de glucose dans le cadre de l'utilisation d'acides dilués, nuisent à l'économie du processus d'hydrolyse acide.

Pour surmonter les inconvénients du processus d'hydrolyse acide, les processus de conversion de la cellulose ont porté plus récemment sur l'hydrolyse enzymatique, à l'aide d'enzymes de type cellulase. Les microorganismes comportant des enzymes qui hydrolysent la cellulose sont par exemple les champignons *Trichoderma, Aspergillus, Humicola* et *Fusarium.* Cette hydrolyse enzymatique de la biomasse lignocellulosique (par exemple, la cellulose), à l'échelle industrielle, présente cependant l'inconvénient d'être coûteuse.

La demande de brevet FR 2 979 111 décrit par exemple un procédé de production de cellulases par un champignon filamenteux adapté à un fermenteur ayant un faible coefficient de transfert volumétrique d'oxygene Kla.
La demande internationale WO 2008/156605 décrit la sélection de souches et leur utilisation pour la production de cellulases
Les demandes WO 2011/151515, FR 3 018 522, ou encore les articles Shaowen et al. (Journal of Industrial Microbiology & Biotechnology, Vol. 40, n°6, 7 mars 2013, pages 633-641), Junxin et al. (Microbial Cell Factories, Biomed Central, Vol. 11, n°1, 18 juin 2012, page 84) décrivent également des souches de champignon filamenteux améliorées pour la production d'enzymes.
Anderson *et al.* ont également étudié la production des enzymes cellulolytiques dans *Trichoderma reesei* (Molecular Biosystems, Vol. 9, n°12, 1er décembre 2013, pages 2992-3000).
Des variants d'exoglucanases à activité améliorée ont également été décrits dans la demande de brevet FR 3 022 558.

Le rôle du facteur de transcription Azf1 chez *Saccharomyces cerevisiae* a été étudié par Slattery et al. (Eukaryotic Cell, Vol. 5, n°2, 1er février 2006, pages 313-320), mais également par Newcomb et al. (Molecular and Cellular Biology, Vol. 22, n°5, 1er mars 2002, pages 1607-1614).
La demande CN104862558 décrit également une souche de *Trichoderma reesei* comprenant un domaine de transcription de type « zinc finger »).

Pour diminuer le coût associé à l'hydrolyse enzymatique de la lignocellulose, l'industrie est à la recherche constante de procédés plus productifs ou présentant un meilleur rendement.

Il existe donc un besoin d'optimisation de la production d'enzymes dans le processus industriel. En particulier, il existe un besoin inassouvi et longtemps attendu de développer un procédé de production d'enzymes amélioré, économiquement avantageux.

Les inventeurs ont ainsi développé un procédé amélioré de production d'une protéine d'intérêt dans un champignon filamenteux, dans lequel la protéine est en particulier une enzyme cellulolytique.

La présente illustration concerne un procédé de production d'une protéine dans une cellule de champignon filamenteux, comprenant la surexpression du gène *TrAZF1* ou d'un de ses variants, dans ladite cellule.

La présente illustration concerne également une souche de champignon filamenteux, de préférence une souche de *Trichoderma reesei,* surexprimant le gène *TrAZF1* ou un de ses variants.

La présente invention est telle que définie dans les revendications 1-6.

Par « surexprimant le gène *TrAZF1* ou un de ses variants », on entend que ladite souche possède au moins le gène *TrAZF1* ou un de ses variants, ledit gène ou ses variants étant exprimé de manière constitutive.
Selon un mode de réalisation, la souche de champignon filamenteux selon l'invention, de préférence *Trichoderma reesei,* comprend le gène *TrAZF1* endogène, ledit gène étant sous la dépendance d'un promoteur constitutif. Dans ce cas, le gène *TrAZF1* endogène est présent dans le génome natif de la souche, et le promoteur a été modifié, muté ou remplacé, pour être constitutif.
Selon un autre mode de réalisation, la souche de champignon filamenteux selon l'invention, de préférence *Trichoderma reesei,* comprend, outre le gène *TrAZF1* présent dans son génome, une copie supplémentaire du gène *TrAZF1,* ladite copie étant exprimée de façon constitutive. Elle comprend donc dans ce cas au moins deux copies du gène *TrAZF1,* l'une de ces copies étant exprimée de façon constitutive.

Cette souche mutée selon l'invention est caractérisée par une amélioration de la production d'enzymes cellulolytiques par rapport à la même souche de *T. reesei* non modifiée, ou par rapport à une souche de référence de *T. reesei.*

*Trichoderma reesei* est un champignon filamenteux cellulolytique. Compte tenu de la capacité de *T. reesei* à sécréter de grandes quantités de cellulases et d'hémicellulases, cette souche est hautement intéressante pour la production d'enzymes pour la transformation des matières de la biomasse végétale en bioproduits utiles à l'industrie, tels que le bioéthanol.

Par **"souche de référence de *T. reesei",*** on entend une souche de *Trichoderma reesei* choisie parmi les souches QM6a, NG14, RutC30 et QM9414. Ces souches sont accessibles au public et ont notamment fait l'objet de dépôts, respectivement sous les numéros:
- ATCC 13631 (souche QM6a);
- ATCC 56767 (souche NG14);
- ATCC 56765 (souche RutC30); et
- ATCC 26921 (souche QM9414).

Dans un mode de réalisation particulier, la souche est la souche CL847. Cette souche est une souche hyperproductive.

Parmi les champignons filamenteux utilisables selon l'invention, on peut citer certains champignons du phylum des Ascomycètes (*Ascomycota*), des Basidiomycètes (*Basidiomycota*) et des Zygomycètes (*Zygomycota*)*.* Typiquement, les champignons sont choisis dans les classes des orbiliomycètes, des pézizomycètes, des dothideomycètes, des eurotiomycètes, des lecanoromycètes, des léotiomycètes, des sordariomycètes et des saccharomycètes. Notamment, on peut citer *Trichoderma reesei, Arthrobotrys oligospora, Tuber melanosporum, Alternaria brassicicola, Baudoinia compniacensis, Cochliobolus heterostrophus, Cochliobolus sativus, Hysterium pulicare, Leptosphaeria maculans, Mycosphaerella pini, Mycosphaerella populorum, Phaeosphaeria nodorum, Pseudocercospora fijiensis, Pyrenophora teres, Pyrenophora triticirepentis, Rhytidhysteron rufulum, Setosphaeria turcica, Zymoseptoria tritici, Ajellomyces capsulatus, Ajellomyces dermatitidis, Arthroderma benhamiae, Arthroderma gypseum, Arthroderma otae, Aspergillus aculeatus, Aspergillus carbonarius, Aspergillus clavatus, Aspergillus flavus, Aspergillus fumigatus, Aspergillus niger, Aspergillus oryzae, Aspergillus terreus, Coccidioides immitis, Coccidioides posadasii, Emericella nidulans, Neosartorya fischeri, Paracoccidioides brasiliensis, Paracoccidioides sp. lutzii, Penicillium chrysogenum, Penicillium marneffei, Talaromyces stipitatus, Trichophyton equinum, Trichophyton rubrum, Trichophyton tonsurans, Trichophyton verrucosum, Uncinocarpus reesii, Cladonia grayi, Botryotinia fuckeliana, Geomyces destructans, Sclerotinia sclerotiorum, Acremonium alcalophilum, Chaetomium globosum, Colletotrichum higginsianum, Cryphonectria parasitica, Epichloe festucae, Fusarium oxysporum, Gaeumannomyces graminis, Gibberella moniliformis, Gibberella zeae, Glomerella graminicola, Magnaporthe grisea, Magnaporthe poae, Myceliophthora thermophila, Nectria haematococca, Neurospora crassa, Neurospora discreta, Neurospora tetrasperma, Podospora anserina, Sordaria macrospora, Thielavia terrestris, Trichoderma atroviride, Trichoderma virens, Verticillium albo-atrum, Verticillium dahliae, Clavispora lusitaniae, Pichia membranifaciens, Scheffersomyces stipitis, Wickerhamomyces anomalus, Ceriporiopsis subvermispora, Fomitopsis pinicola, Phlebiopsis gigantea, Wallemia sebi, Melampsora larici-populina* ou *Rhizopus oryzae.* De préférence, le champignon filamenteux utilisable selon l'invention est *Trichoderma reesei.*

Dans le cadre de la présente invention, par « gène ***TrAZF1*** », on entend le gène de séquence SEQ ID NO:1 ou SEQ ID NO :3. Ce gène code pour un facteur de transcription dont la séquence protéique native est représentée en SEQ ID NO :2. Cette séquence est disponible en ligne sous les numéros d'accession GenBank: XM_006961831.1 (souche *T. reesei* QM6a) et sur le site dédié au génome de *T. reesei,* sous le numéro ID103275 à l'adresse suivante (*http:*//*genome.jgi.doe.gov*/*cgi-bin*/*dispGeneModel?db*=*Trire2&id*=*103275*)*.* La séquence SEQ ID NO:1 est la séquence nucléotidique du gène *TrAZF1* sans intron. C'est donc la séquence d'ADNc.
La séquence SEQ ID NO :3 est la séquence nucléotidique génomique du gène *TrAZF1.* Elle est constituée de quatre exons et de trois introns. Elle est reproduite ci-dessous, et les trois introns sont surlignés: Ainsi, la séquence SEQ ID NO:1 correspond à la séquence SEQ ID NO :3 sans les introns.
Une souche selon l'invention comprend donc de préférence soit au moins deux copies de la séquence SEQ ID NO :3, soit au moins une copie de la séquence SEQ ID NO:1.

Dans la présente illustration, par « **variant** » du gène *TrAZF1,* on entend un gène codant pour une protéine ayant la même fonction que celle de séquence SEQ ID NO :2, à savoir un facteur de transcription. De préférence, le variant du gène *TrAZF1* est un orthologue.
De préférence, le variant du gène *TrAZF1* code pour une protéine ayant la même fonction que celle de séquence SEQ ID NO :2. De préférence, le variant du gène *TrAZF1* code pour une protéine choisie parmi les séquences SEQ ID NO :6 à SEQ ID NO:140.

Les inventeurs ont maintenant montré, pour la première fois, que la surexpression du gène *TrAZF1* conduit à une augmentation significative de la production de protéines cellulolytiques.
En particulier, les inventeurs ont mis en évidence que des souches de *T.reesei* RutC30 et CL847 mutées selon l'invention, i.e. comprenant deux copies du gène *TrAZF1* dont une exprimée de façon constitutive, présentent une amélioration de la production de protéines extracellulaires, en particulier d'enzymes cellulolytiques.

Les protéines produites selon l'invention sont de préférence des enzymes cellulolytiques, plus préférentiellement des cellulases ou des hémicellulases, encore plus préférentiellement il s'agit de cellulases.

Le procédé selon l'invention concerne la production d'une protéine dans une cellule de champignon filamenteux, comprenant la surexpression du gène *TrAZF1,* dans ladite cellule.

Cette surexpression du gène *TrAZF1* est effectuée par introduction d'une cassette comprenant ledit gène dans le génome de la cellule. La connaissance de telles méthodes appartient aux connaissances de l'homme du métier.

Cette cassette comprend :
a) au moins un promoteur constitutif ;
b) le gène de séquence SEQ ID NO:1 ou SEQ ID NO :3 ; et
c) optionnellement, un terminateur.

Le promoteur constitutif a) est un promoteur fort. Par promoteur constitutif, on entend un promoteur qui n'est pas inductible. Ce promoteur constitutif exprime tout le temps le gène ; ainsi, une production constante et forte de protéines a lieu. Ce promoteur peut notamment provenir de *Trichoderma reesei,* mais aussi *d'Aspergillus nidulans.*
De préférence, le promoteur constitutif a) est choisi parmi :
- le promoteur gpd de *Trichoderma reesei* (Li J. et al (2012), Achieving efficient protein expression in Trichoderma reesei by using strong constitutive promoters, Microbial cell factories, 11(1), 84. doi:10.1186/1475-2859-11-84). Ce promoteur a pour séquence SEQ ID NO :4,
- le promoteur gpd *d'Aspergillus nidulans* (Penttilä M. et al (1987), A versatile transformation system for the cellulolytic filamentous fungus Trichoderma reesei, Gene, 61(2), 155-64 ; http://www.ncbi.nlm.nih.gov/pubmed/3127274), et
- le promoteur tef1 de *Trichoderma reesei* (Nakari-Setälä T, Penttilä M., Production of Trichoderma reesei cellulases on glucose-containing media, Applied and Environmental Microbiology. 1995;61(10):3650-3655)

De préférence, le promoteur constitutif a) est le promoteur gpd de séquence SEQ ID NO:4.

La cassette utilisée dans le procédé de l'invention comprend également l'élément b), i.e. le gène de séquence SEQ ID NO : 1 ou SEQ ID NO :3.

Enfin, la cassette utilisée dans le procédé de l'invention peut comprendre, de manière optionnelle, un terminateur c).
Le terminateur est de préférence le terminateur gpd de *Trichoderma reesei,* de séquence SEQ ID NO :5.

La présente invention se rapporte également à l'utilisation d'une cassette telle que décrite ci-dessus pour la production de protéines, notamment d'enzymes cellulolytiques.

La surexpression du gène *TrAZF1* est effectuée de préférence par introduction d'un fragment d'ADN comprenant le gène *TrAZF1* avec un promoteur constitutif, un terminateur et un marqueur de sélection. Cette cassette peut aussi être introduite dans la cellule par un vecteur, tel qu'un plasmide, comprenant la cassette. Selon l'invention, on entend par « **vecteur** » toute séquence d'ADN dans laquelle il est possible d'insérer des fragments d'acide nucléique étranger, les vecteurs permettant d'introduire de l'ADN étranger dans une cellule hôte. Des exemples de vecteurs sont les plasmides, les cosmides, les chromosomes artificiels de levures (YAC), les chromosomes artificiels de bactéries (BAC) et les chromosomes artificiels dérivés du bactériophage P1 (PAC), les vecteurs dérivés de virus.
Le vecteur selon l'invention pourra également porter un marqueur de sélection. On entend par « marqueur de sélection » un gène dont l'expression confère aux cellules qui le contiennent une caractéristique permettant de les sélectionner. Il s'agit par exemple d'un gène de résistance aux antibiotiques.

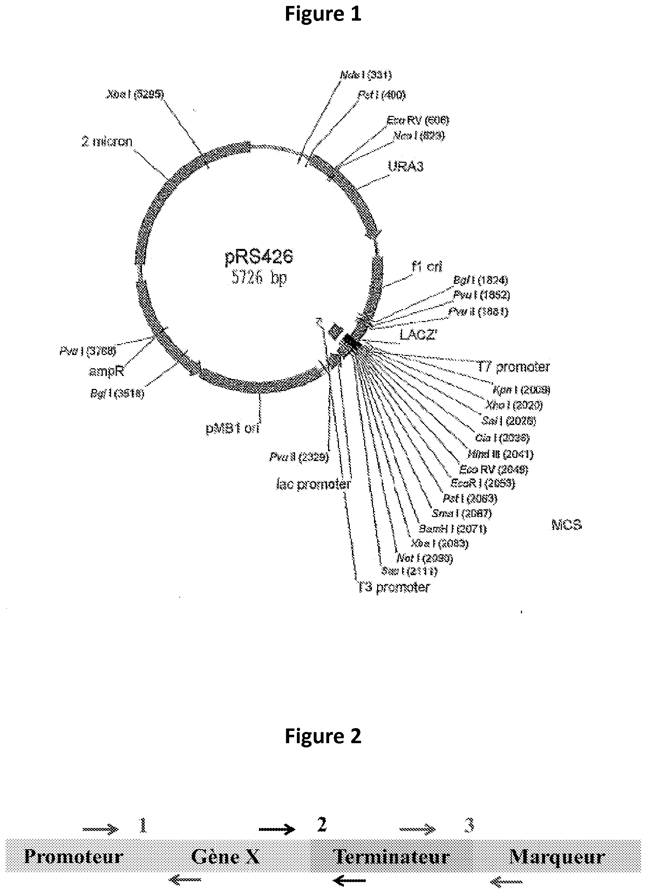

Préférentiellement, ledit vecteur est un plasmide. Plus préférentiellement, le plasmide est un plasmide de type pRS426, tel que décrit dans les exemples et en Figure 1.

La cassette selon l'invention, une fois insérée dans le génome, permet la traduction du gène d'intérêt *TrAZF1,* ce qui donne la protéine correspondante.

L'invention porte également sur l'utilisation de la souche selon l'invention pour la production d'enzymes cellulolytiques. Aussi, dans un mode de réalisation particulier, l'invention concerne l'utilisation d'une souche de *Trichoderma reesei* pour la production d'enzymes cellulolytiques, ladite souche surexprimant le gène *TrAZF1.*

L'invention porte également sur l'utilisation de la souche selon l'invention pour l'hydrolyse de la cellulose et ses produits de dégradation, dont le cellobiose, en glucose.

L'invention a également pour objet l'utilisation de la souche selon l'invention pour la production de biocarburant. Selon l'invention, le terme **"biocarburant"** peut être défini comme tout produit issu de la transformation de la biomasse et pouvant être utilisé à des fins énergétiques. D'une part et sans vouloir se limiter, on peut citer à titre d'exemple des biogaz, des produits pouvant être incorporés (éventuellement après transformation ultérieure) à un carburant ou être un carburant à part entière, tels que des alcools (l'éthanol, le butanol et/ou l'isopropanol selon le type d'organisme fermentaire utilisé), des solvants (acétone), des acides (butyrique), des lipides et leurs dérivés (acides gras à courtes ou longues chaînes, esters d'acides gras), ainsi que l'hydrogène.

De manière préférée, le biocarburant selon l'invention est un alcool, par exemple l'éthanol, le butanol et/ou l'isopropanol. Plus préférentiellement, le biocarburant selon l'invention est l'éthanol. Dans un autre mode de réalisation, le biocarburant est du biogaz.

L'invention concerne également l'utilisation de la souche selon l'invention pour l'hydrolyse des beta-oligosaccharides.

Les exemples suivants illustrent l'invention sans en limiter la portée.

### FIGURES

**Figure 1****:** Représentation du plasmide pRS426.
**Figure 2** : Position des amorces utilisées pour vérifier la présence de la cassette de surexpression des souches transformées.
**Figure 3** **:** Production de protéines extracellulaires par les souches RutC30 transformées selon l'invention et une souche RutC30 de *T. reesei* non modifiée après 7 jours de culture.
**Figure 4** **:** Production de protéines extracellulaires par les souches C1847 transformées selon l'invention et une souche C1847 de *T. reesei* non modifiée après 7 jours de culture.
**Figure 5** : Vitesse spécifique de production de la souche C1847 et deux transformants en conditions de cultures de type Fioles alimentées.

### EXEMPLES

### Exemple 1 : Etudes transcriptomiques en induction lactose des souches de Trichoderma reesei ATCC 56767 et ATCC 56765

Dans cette étude, les inventeurs ont utilisé les souches de *Trichoderma reesei* suivantes:
- ATCC 56767 (NG14), et
- ATCC 56765 (RUT C30).
La souche RutC30 est issue par mutagénèse de la souche NG14 et a une production de cellulase accrue.
Le gène *TrAZF1* (ID 103275) est un facteur de transcription (protéine impliquée dans la régulation d'autres gènes) identifié comme différentiellement exprimé lors d'une cinétique d'induction sur lactose (Poggi-Parodi et al. 2014). Plus précisément, l'étude transcriptomique montre que l'expression de *TrAZF1* diminue lors de l'induction dans la souche NG14 alors que dans la RutC30 son expression ne varie pas.

### Exemple 2 : Construction d'une cassette de surexpression pour le gène TrAZF1

La cassette de surexpression est composée de trois fragments d'ADN :
- la région promotrice du gène glyceraldehyde-3-phosphate déshydrogénase (GPDp) de *T. reesei.* La taille de la région promotrice a été définie dans l'article de Li et al. 2012 (SEQ ID NO : 141)
- la région codante du gène d'intérêt (*TrAZF1*) (SEQ ID NO : 3)
- la région terminateur du gène glyceraldehyde-3-phosphate déshydrogénase (GPDt) de *T. reesei.* La taille de la région promotrice a été définie dans l'article de Li et al. 2012 (SEQ ID NO : 142).

Pour pouvoir sélectionner les transformants, une cassette appelée HygroR (SEQ ID NO: 143) contenant le gène Hph est fusionnée avec la cassette de surexpression. Le gène Hph code pour l'hygromycine B phosphotransférase responsable de la résistance à l'hygromycine B. Il a été isolé d'*Escherichia coli.* Pour l'expression du gène dans *Trichoderma reesei,* le gène Hph est placé sous le contrôle du promoteur du gène cpc-1 de *Neurospora crassa* (NCU04050) et le terminateur du gène trpC *d'Aspergillus nidulans* ANID_00648)

Le plasmide pRS426 (ATCC 77107) a été utilisé comme vecteur pour la construction de la cassette de délétion par recombinaison *in vivo* dans S. *cerevisiae* selon une méthode inspirée de la littérature (Schuster et al. 2012). Pour ce faire, le plasmide a été digéré avec EcoRI et XhoI (New England Biolabs) et purifiée par électrophorèse sur gel en utilisant QIAquick Gel Extraction Kit (Qiagen). Le plasmide pRS426 est représenté en figure 1.

Les parties d'amorces spécifiques du gène d'intérêt *TrAZF1* ont été conçues sur la base de la prédiction ORF dans la version v2.0 du génome (http://genome.jgi-psf.org/Trire2/Trire2.home.html).

### Amplification de la région promotrice du gène glyceraldehyde-3-phosphate déshydrogénase de T. reesei GPDp

Les amorces utilisées pour l'amplification de la région promotrice GPDp à partir de l'ADN de la souche Rut C30 sont décrites ci-après :
- L'amorce sens est constituée de deux parties : une partie de 20
   nucléotides (nt) homologue de la séquence du vecteur pRS426 (figure 1) à proximité du site de restriction XhoI et une partie de 20 nt homologue à l'extrémité 5' de GPDp (SEQ ID NO : 141), le tout formant la séquence SEQ ID NO : 144
   (ATTGGGTACCGGGCCCCCCCGACGCAGAAGAAGGAAATCG).
- L'amorce antisens est constituée de deux parties : une partie de 10 nt homologue à l'extrémité 5' de la région codante du gène *TrAZF1* (SEQ ID NO : 3) et une partie de 20 nt homologue à l'extrémité 3' de GPDp (SEQ ID NO : 141), le tout formant la séquence SEQ ID NO : 145
   (CGAGGGCCATTTTGTATCTGCGAATTGAGC)

### Amplification de la région codante du gène d'intérêt TrAZF1

Les amorces utilisées pour l'amplification du gène d'intérêt *TrAZF1* à partir de l'ADN de la souche Rut C30 sont décrites ci-après :
- L'amorce sens est constituée de deux parties : une partie de 10 nt homologue de l'extrémité 3' de GPDp (SEQ ID NO : 141) et une partie de 20 nt homologue à la région codante 5' du gène *TrAZF1* (SEQ ID NO : 3), le tout formant la séquence SEQ ID NO : 146
   (CAGATACAAAATGGCCCTCGCAGCTCAACA).
- L'amorce antisens est constituée de deux parties : une partie de 10 nt homologue à l'extrémité 5' de la région GPDt (SEQ ID NO : 142) et une partie de 20 nt homologue à la région codante 3' du gène *TrAZF1* (SEQ ID NO : 3), le tout formant la séquence SEQ ID NO : 147
   (AACACAGCACTCAGGATAGGTGGCTCGCAATG)

### Amplification de la région terminateur du gène olyceraldehyde-3-phosphate déshydrogénase de T. reesei GPDt

Les amorces utilisées pour l'amplification de la région terminateur GPDp à partir de l'ADN de la souche Rut C30 sont décrites ci-après :
- L'amorce sens est constituée de deux parties : une partie de 10 nt homologue à l'extrémité 3' de la région codante du gène *TrAZF1* (SEQ ID NO : 3) et une partie de 20 nt homologue à l'extrémité 5' de GPDt (SEQ ID NO : 142), le tout formant la séquence SEQ ID NO : 148
   (CCTATCCTGAGTGCTGTGTTCCTCAGAATG)
- L'amorce antisens est constituée de deux parties : une partie de 10 nt homologue à l'extrémité 5' de la cassette HygroR (SEQ ID NO : 143) et une partie de 20 nt homologue à l'extrémité 3' de GPDt (SEQ ID NO : 142), le tout formant la séquence SEQ ID NO : 149
   (GGTACACTTGTTACGGATCTGATCACTCGG)

### Amplification de la cassette HygroR

Les amorces utilisées pour l'amplification de la cassette HygroR sont décrites ci-après :
- L'amorce sens est constituée de deux parties : une partie de 10 nt homologue à l'extrémité 3' de GPDt (SEQ ID NO : 142), et une partie de 20 nt homologue à l'extrémité 5' de la cassette HygroR (SEQ ID NO : 143), le tout formant la séquence SEQ ID NO : 150
   (AGATCCGTAACAAGTGTACCTGTGCATTCTG).
- L'amorce antisens est constituée de deux parties : une partie de 20 nt homologue à la séquence de vecteur pRS426 à proximité du site de restriction EcoRI et une partie de 20 nt homologue à l'extrémité 3' de la cassette HygroR (SEQ ID NO : 143), le tout formant la séquence SEQ ID NO : 151
   (TGGATCCCCCGGGCTGCAGGGGCAGTGCTAGTGTGTGTAC).

Les PCR mises en œuvre à l'aide des amorces ci-dessus ont donné lieu à des fragments d'ADN à extrémités homologues. Ces fragments d'ADN ainsi que le plasmide pRS426 digéré ont été transformés dans une souche *Saccharomyces cerevisiae* W303 compétente.
Les plasmides pRS426 contenant la cassette de surexpression du gène *TrAZF1* et la cassette HygroR ont été extraits de *S*. *cerevisiae* et utilisés comme matrice pour amplifier par PCR la cassette de surexpression fusionnée à la cassette HygroR (SEQ ID NO : 154) en utilisant l'amorce sens (SEQ ID NO : 152) et l'amorce antisens (SEQ ID NO : 153).

### Exemple 3 : Transformation des souches de T. reesei RutC30 et C1847 avec la cassette de surexpression TrAZF1

Le produit de la PCR (SEQ ID NO 154) a ensuite été transformé dans les souches de *T. reesei* RutC30 (ATCC 56765) et C1847 (Durand et al., 1988). Les transformants ont été sélectionnés sur la base de la fonction de gène marqueur de sélection Hph. Les transformants ont été purifiés à partir des colonies issues de spores individuelles.

L'intégration ectopique de la cassette a été confirmée par trois amplifications par PCR. La position des amorces utilisées ainsi que les fragments amplifiés sont indiqués dans la figure 2. Les amorces utilisées pour les PCR sont décrites ci-dessous :
- Amorces pour amplification de vérification 1 :
   - Une amorce sens dans le promoteur GPDp (SEQ ID NO: 155): GTCAGAAACGACCAAGCTAAG
   - Une amorce anti-sens dans le gène *TrAZF1* (SEQ ID NO: 156): GCCTGAGAATGGTGTCGATC
- Amorces pour amplification de vérification 2 :
   - Une amorce sens dans le gène *TrAZF1* (SEQ ID NO: 157) : TCGTGGGCCTCAAGATTC
   - Une amorce anti-sens dans le terminateur GPDt (SEQ ID NO : 158): GACGCCTGAGAGGTCCTA
- Amorces pour amplification de vérification 3 :
   - Une amorce sens dans le terminateur GPDt (SEQ ID NO: 159): CCTTCTTAGAGAGCTCTCGG
   - Une amorce anti-sens dans la cassette HygroR (SEQ ID NO : 160) : CGGGTTTACCTCTTCCAGAT

Les amplifications ont été réalisées à partir de l'ADN génomique des transformants purifiés : 4 transformants ont été retenus pour chaque souche.

### Exemple 4 : Culture de la souche de T. reesei RutC30 avec la cassette de surexpression TrAZF1 et analyse des cultures pour la production de protéines

Les spores provenant de quatre transformants de la souche RutC30 qui présentent une intégration ectopique de la cassette de surexpression *TrAZF1* ont été utilisés pour ensemencer une plaque de culture à 24 puits contenant 2 ml de milieu de culture par puits.

Le milieu était composé de K2HPO4 8,7 g.L-1 ; (NH4)2SO4 4,2 g.L-1 ; MgSO4.7H2O 0,3 g.L-1 ; cornsteep 1,5 g.L-1 ; Lactose 10 g.L-1 ; Cellulose 10 g.L-1; acide maléique 11,6 g.L-1; CaCl2 0,3 g.L-1; FeSO4.7H2O 5,0 mg.L-1 ; MnSO4.H2O 1,6 mg.L-1; ZnSO4.7H2O 1,4 mg.L-1; CoCl2.6H2LO 2,0 mg.L-1 ; pH 6.

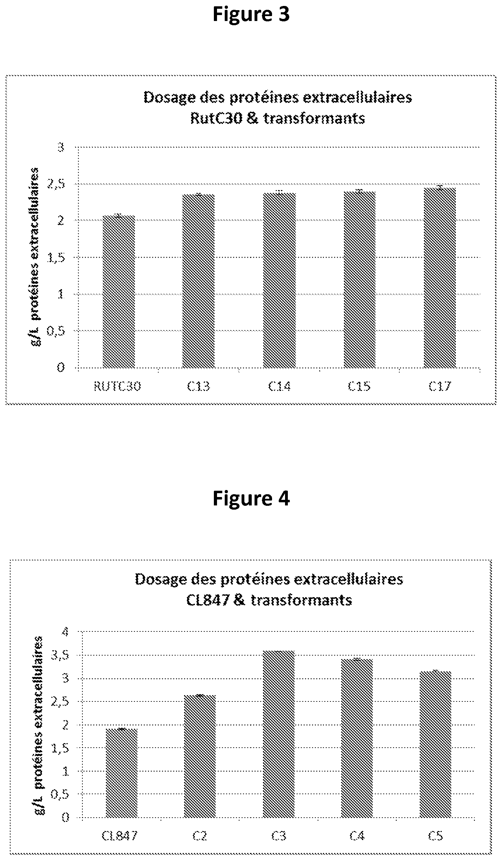

La culture a été effectuée à 30°C sous agitation à 150 rpm, en double exemplaire. Après 7 jours de culture, le surnageant a été recueilli pour mesurer la concentration en protéines dans le milieu (Méthode Folin). La production de protéines extracellulaires des cultures est présentée dans la Figure 3. Cette figure montre une augmentation de la production de protéines pour les transformants par rapport à la souche de *T. reesei* non modifiée.

### Exemple 5 : Culture de la souche de T. reesei C1847 avec la cassette de surexpression TrAZF1 et analyse des cultures pour la production de protéines

Les spores provenant de quatre transformants de la souche C1847 qui présentent une intégration ectopique de la cassette de surexpression *TrAZF1* ont été utilisés pour ensemencer une plaque de culture à 24 puits contenant 2 ml de milieu de culture par puits.

Le milieu était composé de K2HPO4 8,7 g.L-1 ; (NH4)2SO4 4,2 g.L-1 ; MgSO4.7H2O 0,3 g.L-1 ; cornsteep 1,5 g.L-1 ; Lactose 10 g.L-1 ; Cellulose 10 g.L-1; acide maléique 11,6 g.L-1; CaC12 0,3 g.L-1; FeSO4.7H2O 5,0 mg.L-1 ; MnSO4.H2O 1,6 mg.L-1; ZnSO4.7H2O 1,4 mg.L-1; CoCl2.6H2O 2,0 mg.L-1 ; pH 6.

La culture a été effectuée à 30°C sous agitation à 150 rpm, en double exemplaire. Après 7 jours de culture, le surnageant a été recueilli pour mesurer la concentration en protéines dans le milieu (Méthode Folin). La production de protéines extracellulaires des cultures est présentée dans la Figure 4. Cette figure montre une augmentation de la production de protéines pour les transformants par rapport à la souche de *T. reesei* non modifiée.

### Exemple 6 : Culture de la souche de T. reesei C1847 avec AZF1 surexprimé en fioles alimentés et analyse des cultures pour la production de protéines et la croissance

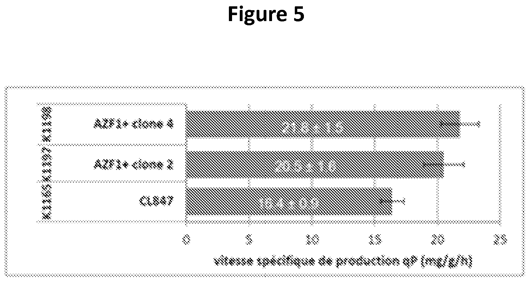

Une culture de deux transformants de la souche T. reesei C1847 ainsi que de la souche non transformée en utilisant la méthode de production de cellulases décrite dans le brevet WO 2013/026964 A1. La production de protéines extracellulaires ainsi que la biomasse ont été mesurée au cours du temps pour obtenir une mesure de la vitesse spécifique de production de la protéine (production de protéine par mg de biomasse fongique par unité de temps, ou qP) à la fin de la culture. Ces valeurs sont présentées en Figure 5.

Les données montrent des valeurs qP améliorées chez les mutants par rapport à la souche de *T. reesei* non modifiée.

### Bibliographie

Durand, H., Clanet, M., & Tiraby, G. (1988). Genetic Improvement of Trichoderma reesei for large scale cellulase production. Enzyme and Microbial Technology, 10, 341-346
Li, Junxin; Wang, Juan; Wang, Shaowen; Xing, Miao; Yu, Shaowen; Liu, Gang (2012) Achieving efficient protein expression in Trichoderma reesei by using strong constitutive promoters. In : Microbial cell factories, vol. 11, p. 84. DOI: 10.1186/1475-2859-11-84.
Poggi-Parodi, Dante; Bidard, Frédérique; Pirayre, Aurélie; Portnoy, Thomas; Blugeon, Corinne; Seiboth, Bernhard et al. (2014) Kinetic transcriptome analysis reveals an essentially intact induction system in a cellulase hyper-producer Trichoderma reesei strain. In : Biotechnology for biofuels, vol. 7, n° 1, p. 173. DOI: 10.1186/sl3068-014-0173-z.
Schuster, André; Bruno, Kenneth S.; Collett, James R.; Baker, Scott E.; Seiboth, Bernhard; Kubicek, Christian P.; Schmoll, Monika (2012) A versatile toolkit for high throughput functional genomics with Trichoderma reesei. In : Biotechnology for biofuels, vol. 5, n° 1, p. 1. DOI: 10.1186/1754-6834-5-1.
WO09026716 (A1) - METHOD FOR CELLULASE PRODUCTION

## Revendications

1. Procédé de production d'une protéine dans une cellule de champignon filamenteux, comprenant la surexpression du gène *TrAZF1,* dans ladite cellule, **caractérisé en ce que** la surexpression du gène *TrAZF1* est effectuée par introduction dans le génome de la cellule d'une cassette comprenant :
a) au moins un promoteur constitutif;
b) le gène de séquence SEQ ID NO : 1 ou SEQ ID NO : 3; et
c) optionnellement, un terminateur.

2. Procédé selon la revendication **1**, **caractérisé en ce que** la protéine est choisie parmi les enzymes cellulolytiques, préférentiellement les cellulases et les hémicellulases, encore plus préférentiellement les cellulases.

3. Procédé selon l'une des revendications **1 à 2**, **caractérisé en ce que** le champignon filamenteux est choisi les classes des orbiliomycètes, des pézizomycètes, des dothideomycètes, des eurotiomycètes, des lecanoromycètes, des léotiomycètes, des sordariomycètes et des saccharomycètes ; de préférence le champignon filamenteux est *Trichoderma reesei.*

4. Utilisation d'une cassette pour la production de protéines, notamment d'enzymes cellulolytiques, ladite cassette comprenant :
a) au moins un promoteur constitutif;
b) le gène de séquence SEQ ID NO : 1 ou SEQ ID NO : 3; et
c) optionnellement, un terminateur.

5. Souche de champignon filamenteux, de préférence souche de *Trichoderma reesei,* surexprimant le gène de séquence SEQ ID NO : 1 ou SEQ ID NO : 3, **caractérisé en ce que** la surexpression du gène *TrAZF1* est effectuée par introduction dans le génome de la cellule d'une cassette comprenant :
a) au moins un promoteur constitutif;
b) le gène de séquence SEQ ID NO : 1 ou SEQ ID NO : 3; et
c) optionnellement, un terminateur.

6. Souche selon la revendication **5**, **caractérisée en ce qu'**elle est une souche de *Trichoderma reesei* comprenant soit au moins deux copies de la séquence SEQ ID NO : 3, soit au moins une copie de la séquence SEQ ID NO : 1.

## Patentansprüche

1. Verfahren zur Herstellung eines Proteins in einer Zelle eines filamentösen Pilzes, umfassend die Überexpression des *TrAZF1*-Gens in dieser Zelle, **dadurch gekennzeichnet, dass** die Überexpression des *TrAZF1*-Gens durch Einführen einer Kassette in das Genom der Zelle bewirkt wird, umfassend:
a) wenigstens einen konstituierenden Promotor;
b) das Gen der Sequenz SEQ ID NO: 1 oder SEQ ID NO: 3; und
c) gegebenenfalls einen Terminator.

2. Verfahren nach Anspruch **1, dadurch gekennzeichnet, dass** das Protein gewählt ist aus zellulolytischen Enzymen, bevorzugt Cellulasen und Hemicellulasen, noch stärker bevorzugt Cellulasen.

3. Verfahren nach einem der Ansprüche **1 bis 2, dadurch gekennzeichnet, dass** der filamentöse Pilz gewählt ist aus den Klassen der Obiliomyceten, Pezizomyceten, Dothideomyceten, Eurotiomyceten, Lecanoromyceten, Leotiomyceten, Sordariomyceten und Saccharomyceten, und der filamentöse Pilz bevorzugt *Trichoderma reesei* ist.

4. Verwendung einer Kassette für die Herstellung von Proteinen, insbesondere von zellulolytischen Enzymen, wobei die Kassette umfasst:
a) wenigstens einen konstituierenden Promotor;
b) das Gen der Sequenz SEQ ID NO: 1 oder SEQ ID NO: 3; und
c) gegebenenfalls einen Terminator.

5. Filamentöser Pilzstamm, bevorzugt ein Stamm von *Trichoderma reesei,* der das Gen der Sequenz SEQ ID NO: 1 oder SEQ ID NO: 3 überexprimiert, **dadurch gekennzeichnet, dass** die Überexpression des *TrAZF1*-Gens durch Einführen einer Kassette in das Genom der Zelle durchgeführt wird, umfassend:
**a)** wenigstens einen konstituierenden Promotor;
**b)** das Gen der Sequenz SEQ ID NO: 1 oder SEQ ID NO: 3; und
**c)** gegebenenfalls einen Terminator.

6. Stamm nach Anspruch **5**, **dadurch gekennzeichnet, dass** es sich um einen Stamm von *Trichoderma reesei* handelt, welcher entweder wenigstens zwei Kopien der Sequenz SEQ ID NO: 3 oder wenigstens eine Kopie der Sequenz SEQ ID NO: 1 umfasst.

## Claims

1. A method for producing a protein in a filamentous fungus cell, comprising the overexpression of the TrAZF1 gene, in said cell, **characterised in that** the overexpression of the TrAZF1 gene is performed by introducing into the genome of the cell a cassette comprising:
a) at least one constitutive promoter;
b) the gene of sequence SED ID NO: 1 or SEQ ID NO: 3; and
c) optionally, a terminator.

2. The method according to claim 1, **characterised in that** the protein is selected from cellulolytic enzymes, preferentially cellulases and hemicellulases, more preferentially cellulases.

3. The method according to one of claims 1 to 2, **characterised in that** the filamentous fungus is selected from the orbiliomycetes, pezizomycetes, dothideomycetes, eurotiomycetes, lecanoromycetes, leotiomycetes, sordariomycetes and saccharomycetes classes; the filamentous fungus is preferably *Trichoderma reesei.*

4. A use of a cassette for producing proteins, in particular cellulolytic enzymes, said cassette comprising:
a) at least one constitutive promoter;
b) the gene of sequence SED ID NO: 1 or SEQ ID NO: 3; and
c) optionally, a terminator.

5. A filamentous fungus strain, preferably a *Trichoderma reesei* strain, overexpressing the gene of sequence SEQ ID NO: 1 or SEQ ID NO: 3, **characterised in that** the overexpression of the TrAZF1 gene is performed by introducing into the genome of the cell a cassette comprising:
a) at least one constitutive promoter;
b) the gene of sequence SED ID NO: 1 or SEQ ID NO: 3; and
c) optionally, a terminator.

6. The strain according to claim 5, **characterised in that** it is a *Trichoderma reesei* strain comprising either at least two copies of the sequence SEQ ID NO: 3, or at least one copy of the sequence SEQ ID NO: 1.
